# EUROPEAN PATENT SPECIFICATION

(11) **EP 4 003 367 B1**
(45) Date of publication and mention of the grant of the patent: **24.12.2025**
(21) Application number: 20757462.5
(22) Date of filing: 31.07.2020
(51) Int. Cl.: A61K 31/7028, A61K 31/7052, A61K 31/7056, A61K 35/28, A61K 38/19, A61P 7/06, A61P 35/00, A61P 35/02, A61P 37/00, A61P 37/06, A61P 43/00

(54) **E-SELECTIN ANTAGONISTS FOR USE IN ENHANCING THE SURVIVAL OF RECONSTITUTED, BONE MARROW-DEPLETED HOSTS**
E-SELEKTIN-ANTAGONISTEN ZUR VERWENDUNG BEI DER ERHÖHUNG DES ÜBERLEBENS VON REKONSTITUIERTEN, KNOCHENMARKABGEREICHERTEN WIRTEN
ANTAGONISTES D'E-SÉLECTINE POUR UTILISATION DANS L'AMÉLIORATION DE LA SURVIE D'HÔTES DE MOELLE OSSEUSE APPAUVRIE RECONSTITUÉE

(30) Priority: 31.07.2019 US 201962881307 P; 04.10.2019 US 201962910738 P; 31.05.2020 US 202063032680 P
(43) Date of publication of application: 01.06.2022
(73) Proprietor: Crescent Biopharma, Inc., Waltham, MA 02453 (US)
(72) Inventor: MAGNANI, John L., Gaithersburg, Maryland 20878 (US); FOGLER, William E., Baltimore, Maryland 21212 (US)
(74) Representative: Mathys & Squire
(86) International application number: PCT/US2020/044449
(87) International publication number: WO 2021/022132

(56) References cited:
- WO-A1-2009/073911
- WO-A1-2018/068010
- WO-A1-2019/133878
- WO-A1-2020/219417
- US-A1- 2017 305 951
- IULIA OANCEA ET AL: "A novel mouse model of veno-occlusive disease provides strategies to prevent thioguanine-induced hepatic toxicity", GUT MICROBIOTA, vol. 62, no. 4, 7 July 2012 (2012-07-07), UK, pages 594 - 605, XP055741733, ISSN: 0017-5749, DOI: 10.1136/gutjnl-2012-302274
- INGRID WINKLER ET AL: "Mobilisation of Reconstituting HSC Is Boosted By Synergy Between G-CSF and E-Selectin Antagonist GMI-1271.", BLOOD, 6 December 2014 (2014-12-06), pages 1 - 6, XP055741137, Retrieved from the Internet <URL:https://ashpublications.org/blood/article/124/21/317/96789/Mobilisation-of-Reconstituting-HSC-Is-Boosted-By> [retrieved on 20201019]
- I. WINKLER ET AL: "Vascular E-Selectin Acts As a Gatekeeper Inducing Commitment and Loss of Self-Renewal in HSC Transmigrating through the Marrow Vasculature", BLOOD, 29 November 2018 (2018-11-29), pages 1 - 6, XP055741099, Retrieved from the Internet <URL:https://ashpublications.org/blood/article/132/Supplement%201/4552/275618/Vascular-E-Selectin-Acts-As-a-Gatekeeper-Inducing> [retrieved on 20201018]
- INGRID G WINKLER ET AL: "Vascular niche E-selectin regulates hematopoietic stem cell dormancy, self renewal and chemoresistance", NATURE MEDICINE, vol. 18, no. 11, 21 October 2012 (2012-10-21), New York, pages 1651 - 1657, XP055560999, ISSN: 1078-8956, DOI: 10.1038/nm.2969
- SAHIN UGUR ET AL: "An overview of hematopoietic stem cell transplantation related thrombotic complications", CRITICAL REVIEWS IN ONCOLOGY/HEMATOLOGY, vol. 107, no. 6, 2016, pages 149 - 155, XP029795636, ISSN: 1040-8428, DOI: 10.1016/J.CRITREVONC.2016.09.004

## Description

### FIELD OF THE INVENTION

The invention relates to E-selectin inhibitors for use in a method of increasing survival of a subject that receives a hematopoietic stem cell (HSC) transplantation, or for use in a method of increasing engraftment and reconstitution of a hematopoietic stem cell (HSC) transplant in a subject receiving HSC transplantation.

### BACKGROUND

Hematopoietic stem cell (HSC) transplantation represents a curative modality for the treatment of patients with hematological malignant and non-malignant diseases, immunodeficiency, autoimmune disorders, and other genetic disorders. Considerable work continues to strive toward the identification of critical factors involved in the successful engraftment and reconstitution of HSC recipients. The identification of these critical components and the understanding of how they may be therapeutically targeted would result in improved patient survival.

Selectins are a group of structurally similar cell surface receptors important for mediating leukocyte binding to endothelial cells. These proteins are type 1 membrane proteins and are composed of an amino terminal lectin domain, an epidermal growth factor (EGF)-like domain, a variable number of complement receptor related repeats, a hydrophobic domain spanning region and a cytoplasmic domain. The binding interactions appear to be mediated by contact of the lectin domain of the selectins and various carbohydrate ligands.

There are three known selectins: E-selectin, P-selectin, and L-selectin. E-selectin is found on the surface of activated endothelial cells and binds to the carbohydrate sialyl-Lewis^{x} (SLe^{x}) which is presented as a glycoprotein or glycolipid on the surface of certain leukocytes (monocytes and neutrophils) and helps these cells adhere to capillary walls in areas where surrounding tissue is infected or damaged. E-selectin also binds to sialyl-Lewis^{a} (SLe^{a}) which is expressed on many tumor cells. P-selectin is expressed on inflamed endothelium and platelets and also recognizes SLe^{x} and SLe^{a} but also contains a second site that interacts with sulfated tyrosine. The expression of E-selectin and P-selectin is generally increased when the tissue adjacent to a capillary is infected or damaged. L-selectin is expressed on leukocytes.

Previous studies have investigated the involvement of E-selectin and its interaction with E-selectin ligands in transplantation of HSC (Winkler et al. 2012; Winkler et al. 2014). These studies first demonstrated a novel function for E-selectin that involved the activation of otherwise dormant HSC with the induction of lineage commitment. Winkler et al. 2018 describes administration of G-CSF together with E-selectin antagonist to mice.

However, previous work has also suggested that an E-selectin antagonist could have either a negative or a positive effect on early and/or late complications in patients with transplantation of HSC. For example, antagonism of E-selectin in an HSC recipient could lead to an inhibition of homing and subsequent lack of engraftment and reconstitution with donor cells. Lethally irradiated recipient mice deficient in both P-and E-selectins (P/E-/-), reconstituted with minimal numbers of wild-type bone marrow cells, poorly survived the procedure compared with wild-type recipients (P.S. Frenette et al., 1998). Excess mortality in P/E-/- mice, after a lethal dose of irradiation, was likely caused by a defect of hematopoietic progenitor cell (HPC) homing, since it was observed that the recruitment of HPC to the BM was reduced in P/E-/- animals. Moreover, homing into the bone marrow (BM) of P/E-/- recipient mice was further compromised when a function-blocking VCAM-1 antibody was administered. However, since these studies used mice deficient in both P- and E-selectin, it is not possible to ascertain the direct impact of E-selectin deficiency on HSC recruitment.

Therefore, a need exists in the field to resolve and clarify the role of selectin inhibition, particularly that of E-selectin, as a beneficial factor in increased overall survival of HSC-reconstituted subjects.

In the following description, certain specific details are set forth in order to provide a thorough understanding of varius embodiments. However, one skilled in the art will understand that the disclosed embodiments may be practiced without these details. In other instances, well-known structures have not been shown or described in detail to avoid unnecessarily obscuring descriptions of the embodiments. These and other embodiments will become apparent upon reference to the following detailed description and attached drawings.

### SUMMARY OF THE INVENTION

The invention provides at least one E-selectin inhibitor for use in a method of increasing survival of a subject that receives a hematopoietic stem cell (HSC) transplantation, wherein the subject has a hematological disease chosen from malignant and non-malignant diseases, the method comprising administering to the subject receiving HSC transplantation an effective amount of the at least one E-selectin inhibitor in combination with HSC transplantation, wherein the at least one E-selectin inhibitor is chosen from: 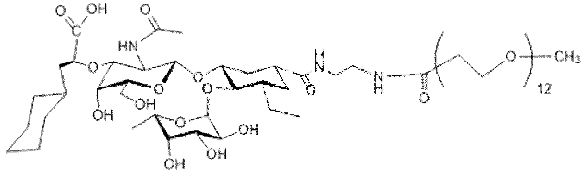 and pharmaceutically acceptable salts thereof.

The invention also provides at least one E-selectin inhibitor for use in a method of increasing engraftment and reconstitution of a hematopoietic stem cell (HSC) transplant in a subject receiving HSC transplantation, wherein the subject has a hematological disease chosen from malignant and non-malignant diseases, the method comprising administering to the subject receiving HSC transplantation an effective amount of the at least one E-selectin inhibitor in combination with HSC transplantation, wherein the at least one E-selectin inhibitor is chosen from: 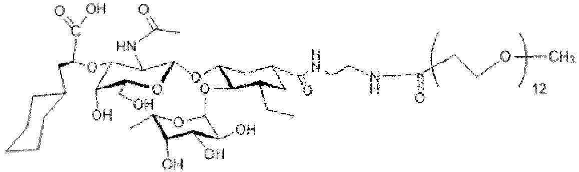 and pharmaceutically acceptable salts thereof.

The invention is as set out in the appended claims.

### BRIEF DESCRIPTION OF THE DRAWINGS

FIGURE 1 is a diagram illustrating the prophetic synthesis of compound **11.**
FIGURE 2 is a diagram illustrating the prophetic synthesis of compound **14.**
FIGURE 3 is a diagram illustrating the prophetic synthesis of multimeric compounds **21** and **22.**
FIGURE 4 is a diagram illustrating the prophetic synthesis of multimeric compounds **36** and **37.**
FIGURE 5 is a diagram illustrating the prophetic synthesis of multimeric compounds **44, 45,** and **46.**
FIGURE 6 is a diagram illustrating the prophetic synthesis of multimeric compounds **55** and **56.**

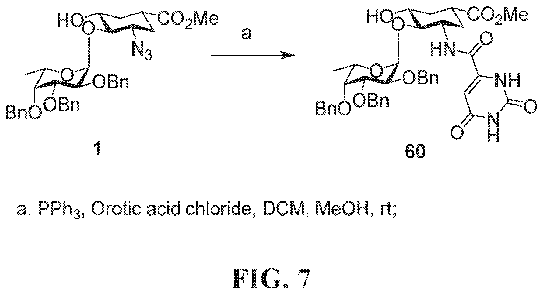

FIGURE 7 is a diagram illustrating the prophetic synthesis of compound **60.**

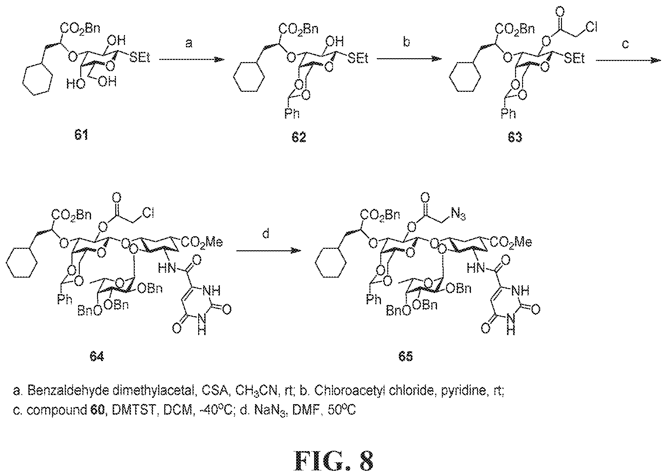

FIGURE 8 is a diagram illustrating the prophetic synthesis of compound **65.**
FIGURE 9 is a diagram illustrating the prophetic synthesis of multimeric compounds **66, 67,** and **68.**

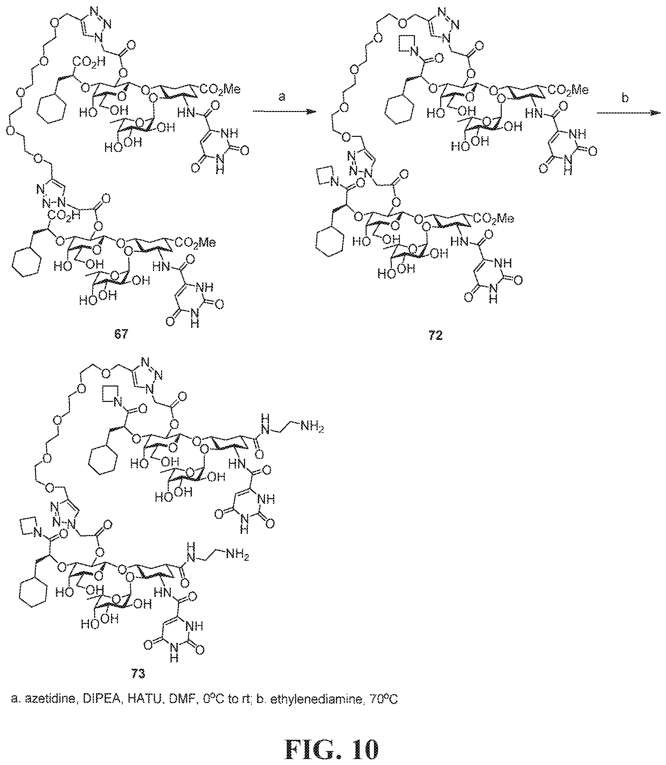

FIGURE 10 is a diagram illustrating the prophetic synthesis of multimeric compounds **72** and **73.**
FIGURE 11 is a diagram illustrating the prophetic synthesis of multimeric compounds **76, 77,** and **78.**
FIGURE 12 is a diagram illustrating the prophetic synthesis of multimeric compounds **86** and **87.**
FIGURE 13 is a diagram illustrating the prophetic synthesis of multimeric compound **95.**
FIGURE 14 is a diagram illustrating the prophetic synthesis of multimeric compound **146.**
FIGURE 15 is a diagram illustrating a prophetic synthesis of multimeric compound **197.**
FIGURE 16 is a diagram illustrating a synthesis of compound **205.**
FIGURE 17 is a diagram illustrating the synthesis of multimeric compound **206.**
FIGURE 18 is a diagram illustrating the synthesis of compound **214.**
FIGURE 19 is a diagram illustrating the synthesis of multimeric compounds **218, 219,** and **220.**
FIGURE 20 is a diagram illustrating the synthesis of multimeric compound **224.**
FIGURE 21 is a diagram illustrating the prophetic synthesis of compound **237.**
FIGURE 22 is a diagram illustrating the prophetic synthesis of compound **241.**
FIGURE 23 is a diagram illustrating the prophetic synthesis of compound **245.**
FIGURE 24 is a diagram illustrating the prophetic synthesis of multimeric compound **257.**
FIGURE 25 is a diagram illustrating the prophetic synthesis of multimeric compounds **261, 262,** and **263.**
FIGURE 26 is a diagram illustrating the prophetic synthesis of multimeric compounds **274, 275,** and **276.**
FIGURE 27 is a diagram illustrating the prophetic synthesis of compound **291.**
FIGURE 28 is a diagram illustrating the prophetic synthesis of multimeric compounds **294** and **295.**
FIGURE 29 is a diagram illustrating the prophetic synthesis of multimeric compounds **305, 306,** and **307.**

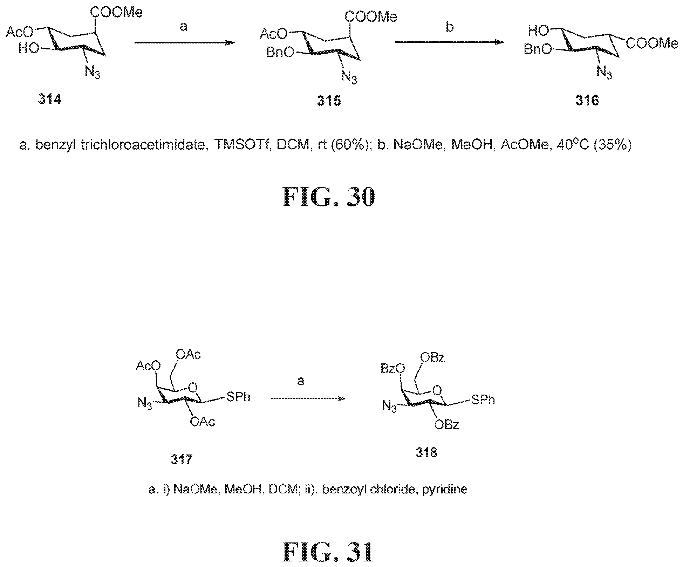

FIGURE 30 is a diagram illustrating the synthesis of compound **316.**
FIGURE 31 is a diagram illustrating the synthesis of compound **318.**

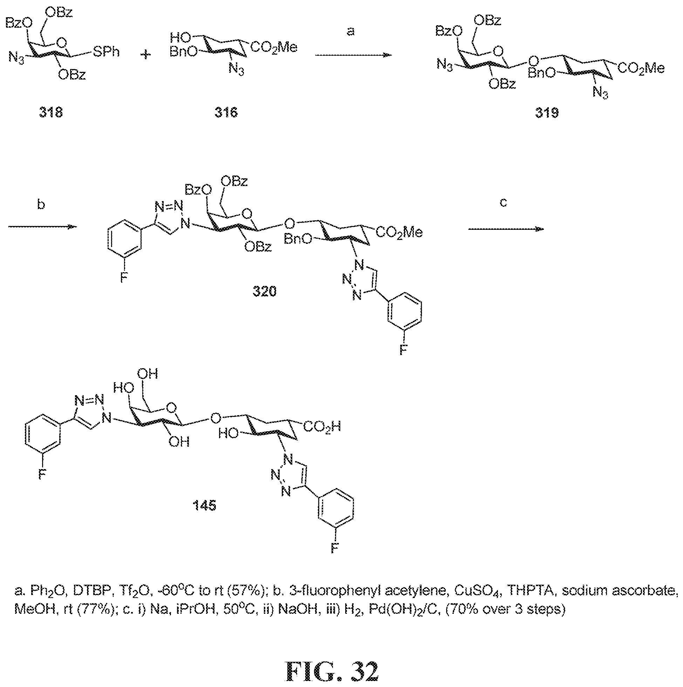

FIGURE 32 is a diagram illustrating the synthesis of compound **145.**
FIGURE 33 is a diagram illustrating the synthesis of compound **332.**
FIGURE 34 is a schematic illustrating an experimental model to determine hematopoietic reconstitution of lethally irradiated C57/BL6 (CD45.2+) mice with CD45.1+ congenic B6.SJL cells.
FIGURE 35 is a graph illustrating the effect of compound A on the survival of bone marrow-depleted, reconstituted mice.
FIGURE 36 is a chart illustrating flow cytometry data evaluating percentage of donor versus recipient cells in blood and bone marrow among reconstituted mice at day 30 post-ablation.

### DETAILED DESCRIPTION

The technical information set out below may in some respects go beyond the scope of the invention, which is defined by the appended claims. The additional technical information is provided to place the actual invention in a broader technical context and to illustrate possible related technical developments.

Any references to methods for treatment of the human or animal body by surgery or therapy and diagnostic methods practised on the human or animal body are not to be construed as claiming protection for such methods as such, but are instead to be interpreted as references to products, in particular compounds, pharmaceutical compositions and medicaments of the present invention for use in any of these methods.

In order to better understand the disclosure, certain exemplary embodiments are discussed herein. In addition, certain terms are discussed to aid in the understanding.

Disclosed herein are products for use in a method of increasing survival of subjects that receive HSC transplantation by treating them with an effective amount of at least one E-selectin inhibitor. Also disclosed herein are products for use in a method of increasing engraftment and reconstitution in subjects receiving HSC transplantation with the use of at least one E-selectin inhibitor.

In these methods, when subjects suffering from a condition resulting in depletion or compromise of bone marrow receive a transplantation of HSCs to reconstitute the absent marrow, inhibition and/or antagonism of selectins may result in increased survival of the subjects.

According to one instance, the HSC quiescence and/or HSC mobilization in the subject may be increased. In some instances, the subject is a transplant donor. In some instances, , the subject is a transplant recipient.

In the invention, the subject is suffering from a hematological disease, which may be malignant or non-malignant. Examples of diseases include multiple myeloma, Hodgkin and non-Hodgkin lymphoma, acute myeloid leukemia (AML), acute lymphoblastic leukemia (ALL), myelodysplastic syndrome, chronic myeloid leukemia (CML), chronic lymphocytic leukemia, myelofibrosis, essential thrombocytosis, polycythemia vera, immunodeficiency, autoimmune disorders, and genetic disorders, aplastic amemia, severe combined immune deficiency syndrome (SCID), thalassemia, sickle cell anemia, chronic granulomatous disease, leukocyte adhesion deficiency, Chediak-Higashi syndrome, Kostman syndrome, Fanconi anemia, Blackfan-Diamond anemia, enzymatic disorders, systemic sclerosis, systemic lupus erythematosus, mucopolysaccharidosis, pyruvate kinase deficiency, and multiple sclerosis.

### Definitions

Unless otherwise defined, all technical and scientific terms used herein have the same meaning as commonly understood by one of ordinary skill in the art to which this disclosure belongs.

To the extent terms or discussion in references conflict with this disclosure, the latter shall control.

As used herein, the singular forms of a word also include the plural form of the word, unless the context clearly dictates otherwise; as examples, the terms "a," "an," and "the" are understood to be singular or plural. By way of example, "an element" means one or more element. The term "or" shall mean "and/or" unless the specific context indicates otherwise.

The term "E-selectin ligand" as used herein, refers to a carbohydrate structure that contains the epitope shared by sialyl Le^{a} and sialyl Le^{x}. Carbohydrates are secondary gene products synthesized by enzymes known as glycosyltransferases which are the primary gene products coded for by DNA. Each glycosyltransferase adds a specific monosaccharide in a specific stereochemical linkage to a specific donor carbohydrate chain.

The terms "E-selectin antagonist" and "E-selectin inhibitor" are used interchangeably herein. E-selectin inhibitors are known in the art. Some E-selectin inhibitors are specific for E-selectin only. Other E-selectin inhibitors have the ability to inhibit not only E-selectin but additionally P-selectin or L-selectin or both P-selectiti and L-selectin. In some embodiments, an E-selectin inhibitor inhibits E-selectin, P-selectin, and L-selectin.

In some instances, an E-selectin inhibitor is a specific glycomimetic antagonist of E-selectin. Examples of E-selectin inhibitors (specific for E-selectin or otherwise) are disclosed in U.S. Patent No. 9,109,002.

In some instances, the E-selectin antagonists suitable for the disclosed compounds and methods include pan-selectin antagonists.

Non-limiting examples of suitable E-selectin antagonists include small molecules, such as nucleic acids, peptides, polypeptides, peptidomimetics, carbohydrates, glycomimetics, lipids and other organic (carbon containing) or inorganic molecules. Suitably, the selectin antagonist is selected from antigen-binding molecules that are immuno-interactive with a selectin, peptides that bind to the selectin and that block cell-cell adhesion, and carbohydrate or peptide mimetics of selectin ligands. In some instances, the E-selectin antagonist reduces the expression of a selectin gene or the level or functional activity of an expression product of that gene. For example, the E-selectin antagonist may antagonize the function of the selectin, including reducing or abrogating the activity of at least one of its ligand-binding sites.

In some instances, the E-selectin antagonist inhibits an activity of E-selectin or inhibits the binding of E-selectin to one or more E-selectin ligands (which in turn may inhibit a biological activity of E-selectin).

E-selectin antagonists include the glycomimetic compounds described herein. E-selectin antagonists also include antibodies, polypeptides, peptides, peptidomimetics, and aptamers which bind at or near the binding site on E-selectin to inhibit E-selectin interaction with sialyl Le^{a} (sLe^{a}) or sialyl Le^{x} {sLe^{x}).

Further disclosure regarding E-selectin antagonists suitable for the disclosed methods and compounds may be found in U.S. Patent No. 9,254,322, issued Feb. 9, 2016, and U.S. Patent No. 9,486,497, issued Nov. 8, 2016 .

As disclosed herein, a selectin antagonist may be chosen from E-selectin antagonists disclosed in U.S. Patent No. 9,109,002, issued Aug. 18, 2015 .

As disclosed herein, an E-selectin antagonist may be chosen from heterobifunctional antagonists disclosed in U.S. Patent No. 8,410,066, issued Apr. 2, 2013, and US Publication No. US2017/0305951, published Oct. 26, 2017 .

Further disclosure regarding E-selectin antagonists suitable for the disclosed methods and compounds may be found in PCT Publication Nos. WO2018/068010, published Apr. 12, 2018, WO2019/133878, published July 4, 2019, and WO02020/139962, published July 2, 2020 . The E-selectin inhibitor for use according to the invention is Compound A, as defined in claims 1 and 2.

The term "at least one" refers to one or more, such as one, two, etc. For example, the term "at least one C₁₋₄ alkyl group" refers to one or more C₁₋₄ alkyl groups, such as one C₁₋₄ alkyl group, two C₁₋₄ alkyl groups, etc.

The term "pharmaceutically acceptable salts" includes both acid and base addition salts. Non-limiting examples of pharmaceutically acceptable acid addition salts include chlorides, bromides, sulfates, nitrates, phosphates, sulfonates, methane sulfonates, formates, tartrates, maleates, citrates, benzoates, salicylates, and ascorbates. Non-limiting examples of pharmaceutically acceptable base addition salts include sodium, potassium, lithium, ammonium (substituted and unsubstituted), calcium, magnesium, iron, zinc, copper, manganese, and aluminum salts. Pharmaceutically acceptable salts may, for example, be obtained using standard procedures well known in the field of pharmaceuticals.

The term "prodrug" includes compounds that may be converted, for example, under physiological conditions or by solvolysis, to a biologically active compound described herein. Thus, the term "prodrug" includes metabolic precursors of compounds described herein that are pharmaceutically acceptable. A discussion of prodrugs can be found, for example, in Higuchi, T., et al., "Pro-drugs as Novel Delivery Systems," A.C.S. Symposium Series, Vol. 14, and in Bioreversible Carriers in Drug Design, ed. Edward B. Roche, American Pharmaceutical Association and Pergamon Press, 1987. The term "prodrug" also includes covalently bonded carriers that release the active compound(s) as described herein *in vivo* when such prodrug is administered to a subject. Non-limiting examples of prodrugs include ester and amide derivatives of hydroxy, carboxy, mercapto and amino functional groups in the compounds described herein.

This application contemplates all the isomers of the compounds disclosed selected from the group consisting of optical isomers (such as stereoisomers, *e.g.,* enantiomers and diastereoisomers), geometric isomers (such as Z (zusammen) or E (entgegen) isomers), and tautomers. The present disclosure includes within its scope all the possible geometric isomers, *e.g.,* Z and E isomers (*cis* and *trans* isomers), of the compounds as well as all the possible optical isomers, e.g. diastereomers and enantiomers, of the compounds. Furthermore, the present disclosure includes in its scope both the individual isomers and any mixtures thereof, e.g. racemic mixtures. The individual isomers may be obtained using the corresponding isomeric forms of the starting material or they may be separated after the preparation of the end compound according to conventional separation methods. For the separation of optical isomers, *e.g.,* enantiomers, from the mixture thereof conventional resolution methods, e.g. fractional crystallization, may be used.

The present disclosure includes within its scope all possible tautomers. Furthermore, the present disclosure includes in its scope both the individual tautomers and any mixtures thereof. Each compound disclosed herein includes within its scope all possible tautomeric forms. Furthermore, each compound disclosed herein includes within its scope both the individual tautomeric forms and any mixtures thereof. With respect to the methods, uses and compositions of the present application, reference to a compound or compounds is intended to encompass that compound in each of its possible isomeric forms and mixtures thereof. Where a compound of the present application is depicted in one tautomeric form, that depicted structure is intended to encompass all other tautomeric forms.

E-selectin inhibitors, such as compound A, can be useful for increasing survival of individuals that receive HSC transplantation for reconstitution of depleted and compromised bone marrow.

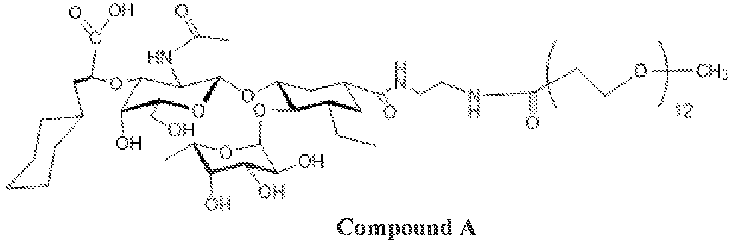

Accordingly, the invention provides at least one E-selectin inhibitor for use in a method of increasing survival of a subject that receives a HSC transplantation, wherein the subject has a hematological disease chosen from malignant and non-malignant diseases, the method comprising administering to the subject receiving HSC transplantation an effective amount of the at least one E-selectin inhibitor in combination with HSC transplantation, wherein the at least one E-selectin inhibitor is chosen from Compound A and pharmaceutically acceptable salts thereof.

Also contemplated is increasing engraftment and reconstitution in a subject receiving HSC transplantation , wherein the subject in need thereof is administered an effective amount of at least one E-selectin inhibitor, such as compound A. Accordingly, the invention provides at least one E-selectin inhibitor for use in a method of increasing engraftment and reconstitution of a HSC transplant in a subject receiving HSC transplantation, wherein the subject has a hematological disease chosen from malignant and non-malignant diseases, the method comprising administering to the subject receiving HSC transplantation an effective amount of the at least one E-selectin inhibitor in combination with HSC transplantation, wherein the at least one E-selectin inhibitor is chosen from Compound A and pharmaceutically acceptable salts thereof.

In some embodiments, the HSC quiescence in the subject is increased. In some embodiments, the HSC mobilization in the subject is increased. In some embodiments, the HSC quiescence and the HSC mobilization in the subject is increased.

In some embodiments, the method further includes inhibiting sinusoidal obstruction syndrome (SOS) in the subject. In some embodiments, the SOS is a hepatic veno-occlusive disease.

In some embodiments, the subject has depleted and/or compromised bone marrow.

In some embodiments, the HSC transplantation is from the subject's peripheral blood. In some embodiments, the HSC transplantation is from the subject's bone marrow.

In some embodiments, the subject is a transplant donor. In some embodiments, the subject is a transplant recipient. In the invention, the subject receives or is receiving HSC transplantation.

In some embodiments, the subject has received an effective amount of a granulocyte colony-stimulating factor (GCSF).

In the invention, the subject has a hematological disease.

In some embodiments the hematological disease is a malignant disease. In some embodiments, the malignant disease is multiple myeloma. In some embodiments, the malignant disease is Hodgkin lymphoma. In some embodiments, the malignant disease is non-flodgkin lymphoma. In some embodiments, the malignant disease is acute myeloid leukemia (AML). In some embodiments, the malignant disease is acute lymphoblastic leukemia (ALL). In some embodiments, the malignant disease is myelodysplastic syndrome. In some embodiments, the malignant disease is chronic myeloid leukemia (CML). In some embodiments, the malignant disease is chronic lymphocytic leukemia. In some embodiments, the malignant disease is myelofibrosis. In some embodiments, the malignant disease is essential thrombocytosis. In some embodiments, the malignant disease is polycythemia vera.

In some embodiments, the hematological disease is a non-malignant disease. In some embodiments, the non-malignant disease is immunodeficiency. In some embodiments, the non-malignant disease is an autoimmune disorder. In some embodiments, the non-malignant disease is a genetic disorder. In some embodiments, the non-malignant disease is aplastic amemia. In some embodiments, the non-malignant disease is severe combined immune deficiency syndrome (SCID). In some embodiments, the non-malignant disease is thalassemia. In some embodiments, the non-malignant disease is sickle cell anemia. In some embodiments, the non-malignant disease is chronic granulomatous disease. In some embodiments, the non-malignant disease is leukocyte adhesion deficiency. In some embodiments, the non-malignant disease is Chediak-Higashi syndrome. In some embodiments, the non-malignant disease is Kostman syndrome. In some embodiments, the non-malignant disease is Fanconi anemia. In some embodiments, the non-malignant disease is Blackfan-Diamond anemia. In some embodiments, the non-malignant disease is systemic sclerosis. In some embodiments, the non-malignant disease is systemic lupus erythematosus. In some embodiments, the non-malignant disease is mucopolysaccharidosis. In some embodiments, the non-malignant disease is pyruvate kinase deficiency. In some embodiments, the non-malignant disease is multiple sclerosis.

In the invention, the at least one E-selectin inhibitor is chosen from Compound A: 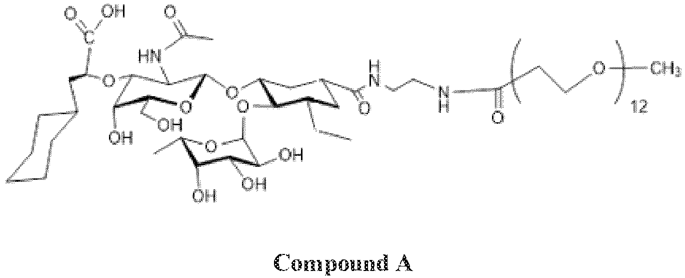 and pharmaceutically acceptable salt thereof.

In some embodiments, the one or more E-selectin inhibitor is admmistered as a pharmaceutical composition comprising the one or more E-selectin inhibitor, in combination with one or more pharmaceutically acceptable excipients. In the invention, the at least one E-selectin inhibitor is selected from Compound A and pharmaceutically acceptable salts thereof.

In some embodiments, the pharmaceutical composition is delivered by subcutaneous delivery. In some embodiments, the pharmaceutical composition is delivered by subcutaneous delivery to the upper arm. In some embodiments, the pharmaceutical composition is delivered by subcutaneous delivery to the abdomen. In some embodiments, the pharmaceutical composition is delivered by subcutaneous delivery to the thigh. In some embodiments, the pharmaceutical composition is delivered by subcutaneous delivery to the upper back. In some pharmaceutical embodiments the composition is delivered by subcutaneous delivery to the buttock. In some embodiments, the pharmaceutical composition is delivered by intravenous infusion.

In various embodiments, the pharmaceutical composition is administered over one or more doses, with one or more intervals between doses. In some embodiments, the pharmaceutical composition is administered over 1, 2, 3, 4, 5, 6, 7, 8, 9, 10, 11, 12, 13, 14, 15, 16, 17, 18, 19, or 20 doses. In some embodiments, the pharmaceutical composition is administered at 6-hour, 12-hour, 18-hour, 24-hour, 48-hour, 72-hour, or 96-hour intervals. In some embodiments, the pharmaceutical composition is administered at one interval, and then administered at a different interval, e.g., 1 dose 24 hours before transplantation, then twice-daily doses throughout transplantation. In some embodiments, the pharmaceutical composition is administered at 1 dose 24 hours before transplantation, then twice-daily doses throughout transplantation up till 48 hours post-transplantation.

The selectin antagonists suitable for the disclosed methods include pan selectin antagonists.

As disclosed herein, any method of inhibiting E-selectin may be used to enhance the survival of reconstituted, bone marrow depleted hosts. Inhibition can be by any means, for example, antibody, small molecule, biologic, inhibitors of gene expression, etc.

Non-limiting examples of suitable selectin antagonists include small molecules, such as nucleic acids, peptides, polypeptides, peptidomimetics, carbohydrates, lipids and other organic (carbon containing) or inorganic molecules. Suitably, the selectin antagonist is selected from antigen-binding molecules that are immuno-interactive with a selectin, peptides that bind to the selectin and that block cell-cell adhesion, and carbohydrate or peptide mimetics of selectin ligands. In some instances, the selectin antagonist reduces the expression of a selectin gene or the level or functional activity of an expression product of that gene. For example, the selectin antagonist may antagonize the function of the selectin, including reducing or abrogating the activity of at least one of its ligand-binding sites.

As disclosed herein, an E-selectin antagonist may be chosen from compounds of Formula Ix: 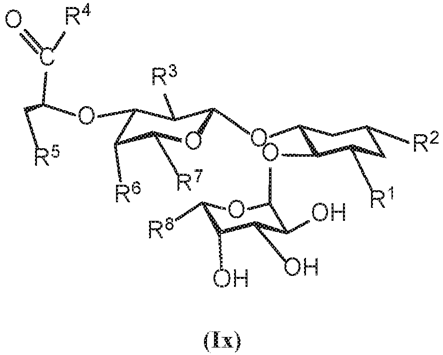 prodrugs of Formula Ix, and pharmaceutically acceptable salts of any of the foregoing, wherein:
R¹ is chosen from C₁-C₈ alkyl, C₂-C₈ alkenyl, C₂-C₈ alkynyl, C₁-C₈ haloalkyl, C₂-Cs haloalkenyl, and C₂-C₈ haloalkynyl groups;
R² is chosen from H, -M, and -L-M;
R³ is chosen from -OH, -NH₂, -OC(=O)Y¹, -NHC(=O)Y¹, and -NHC(=O)NHY¹ groups, wherein Y¹ is chosen from C₁₋₈ alkyl, C₂₋₈ alkenyl, C₂₋₈ alkynyl, C₁₋₈ haloalkyl, C₂₋₈ haloalkenyl, C₂₋₈ haloalkynyl, C₆₋₁₈ aryl, and C₁₋₁₃ heteroaryl groups;
R⁴ is chosen from -OH and -NZ¹Z² groups, wherein Z¹ and Z², which may be identical or different, are each independently chosen from H, C₁-C₈ alkyl, C₂-C₈ alkenyl, C₂-C₈ alkynyl, C₁-C₈ haloalkyl, C₂-C₈ haloalkenyl, and C₂-C₈ haloalkynyl groups, wherein Z¹ and Z² may join together to form a ring;
R⁵ is chosen from C₃-C₈ cycloalkyl groups;
R⁶ is chosen from -OH, C₁-C₈ alkyl, C₂-C₈ alkenyl, C₂-C₈ alkynyl, C₁-C₈ haloalkyl, C₂-C₈ haloalkenyl, and C₂-C₈ haloalkynyl groups;
R⁷ is chosen from -CH₂OH, C₁-C₈ alkyl, C₂-C₈ alkenyl, C₂-C₈ alkynyl, C₁-C₈ haloalkyl, C₂-C₈ haloalkenyl, and C₂-C₈ haloalkynyl groups;
R⁸ is chosen from C₁-C₈ alkyl, C₂-C₈ alkenyl, C₂-C₈ alkynyl, C₁-C₈ haloalkyl, C₂-Cs haloalkenyl, and C₂-C₈ haloalkynyl groups;
L is chosen from linker groups; and
M is a non-glycomimetic moiety chosen from polyethylene glycol, thiazolyl, chromenyl, -C(=O)NH(CH₂)₁₋₄NH₂, C₁₋₈ alkyl, and -C(=O)OY groups, wherein Y is chosen from C₁₋₄ alkyl, C₂₋₄ alkenyl, and C₂₋₄ alkynyl groups.

As disclosed herein, an E-selectin antagonist may be chosen from compounds of Formula Ix, wherein the non-glycomimetic moiety comprises polyethylene glycol.

As disclosed herein, an E-selectin antagonist may be chosen from compounds of Formula Ix, wherein the linker is -C(=O)NH(CH₂)₁₋₄NHC(=O)- and the non-glycomimetic moiety comprises polyethylene glycol.

As disclosed herein, an E-selectin antagonist may be chosen from the compound of Formula Ix, prodrugs of compounds of Formula Ix and pharmaceutically acceptable salts of any of the foregoing. In some instances, an E-selectin inhibitor is the compound of Formula Ix. As disclosed herein, an E-selectin antagonist may be chosen from pharmaceutically acceptable salts of the compound of Formula Ix.

As disclosed herein, an E-selectin antagonist may be chosen from compounds of Formula Ia: 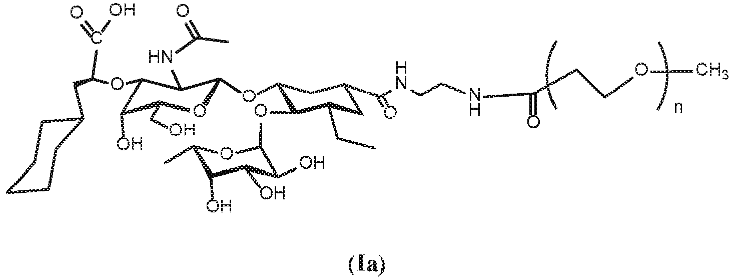 and pharmaceutically acceptable salts thereof, wherein n is chosen from integers ranging from 1 to 100. In some instances, n is chosen from 4, 8, 12, 16, 20, 24, and 28. In some instances, n is 12. In the invention, the at least one E-selectin inhibitor is chosen from compounds of Formula Ia wherein n is 12, and pharmaceutically acceptable salts thereof.

As disclosed herein, an E-selectin antagonist may be a heterobifunctional antagonist chosen from compounds of Formula II: 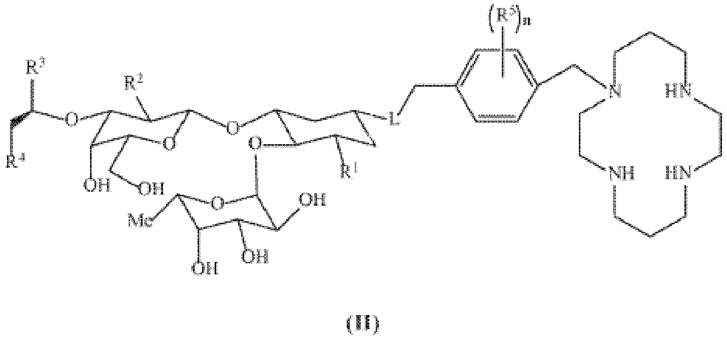 prodrugs of compounds of Formula II, and pharmaceutically acceptable salts of any of the foregoing, wherein:
R¹ is chosen from H, C₁₋₈ alkyl, C₂₋₈ alkenyl, C₂₋₈ alkynyl, C₁₋₈ haloalkyl, C₂₋₈ haloalkenyl, and C₂₋₈ haloalkynyl groups;
R² is chosen from -OH, -NH₂, -OC(=O)Y¹, -NHC(=O)Y¹, and -NHC(=O)NHY¹ groups, wherein Y¹ is chosen from C₁₋₈ alkyl, C₂₋₈ alkenyl, C₂₋₈ alkynyl, C₁₋₈ haloalkyl, C₂₋₈ haloalkenyl, C₂₋₈ haloalkynyl, C₆₋₁₈ aryl, and C₁₋₁₃ heteroaryl groups;
R³ is chosen from -CN, -CH₂CN, and -C(=O)Y² groups, wherein Y² is chosen from C₁₋₈ alkyl, C₂₋₈ alkenyl, C₂₋₈ alkynyl, -OZ¹, -NHOH, -NHOCH₃, -NHCN, and -NZ¹Z² groups, wherein Z¹ and Z², which may be identical or different, are independently chosen from H, C₁₋₈ alkyl, C₂₋₈ alkenyl, C₂₋₈ alkynyl, C₁₋₈ haloalkyl, C₂₋₈ haloalkenyl, and C₂₋₈ haloalkynyl groups, wherein Z¹ and Z² may join together to form a ring;
R⁴ is chosen from C₃₋₈ cycloalkyl groups;
R⁵ is independently chosen from H, halo, C₁₋₈ alkyl, C₂₋₈ alkenyl, C₂₋₈ alkynyl, C₁₋₃ haloalkyl, C₂₋₈ haloalkenyl, and C₂₋₈ haloalkynyl groups;
n is chosen from integers ranging from 1 to 4; and
L is chosen from linker groups.

As disclosed herein, an E-selectin antagonist may be a heterobifunctional antagonist chosen from compounds of Formula IIa: 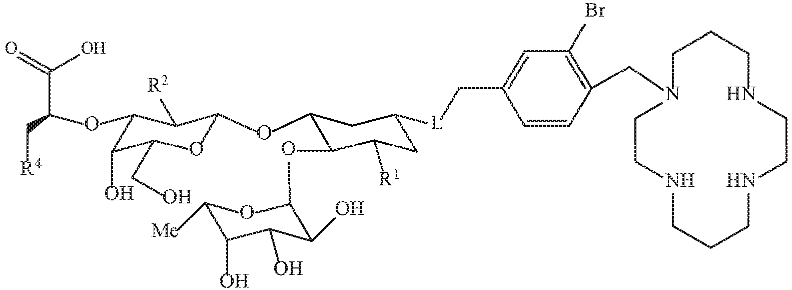 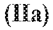 and pharmaceutically acceptable salts thereof.

In some instances, the linker groups of Formula Ix and/or Formula II are independently chosen from groups comprising spacer groups, such spacer groups as, for example, -(CH₂)ₚ- and -O(CH₂)ₚ-, wherein p is chosen from integers ranging from 1 to 30. In some instances, p is chosen from integers ranging from 1 to 20.

Other non-limiting examples of spacer groups include carbonyl groups and carbonyl-containing groups such as, for example, amide groups. A non-limiting example of a spacer group is

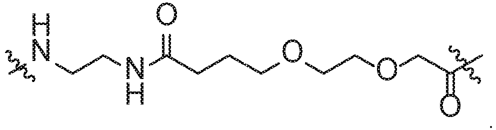

In some instances, the linker groups of Formula Ix and/or Formula II are independently chosen from

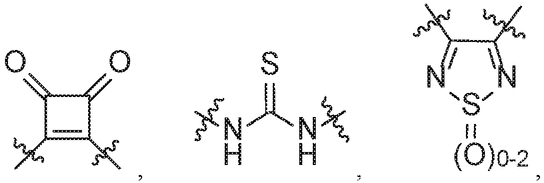

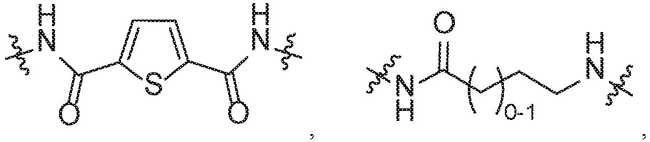

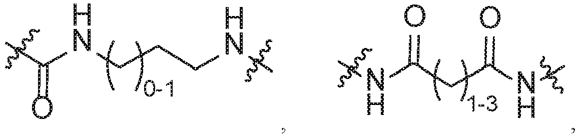

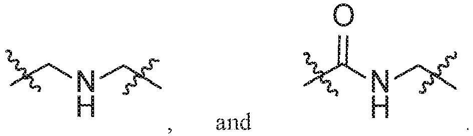

Other linker groups, such as, for example, polyethylene glycols (PEGs) and -C(=O)-NR-(CH₂)ₚ-C(=O)-NH-, wherein p is chosen from integers ranging from 1 to 30, or wherein p is chosen from integers ranging from 1 to 20, will be familiar to those of ordinary skill in the art and/or those in possession of the present disclosure.

In some instances, at least one linker group of Formula Ix and/or Formula II is

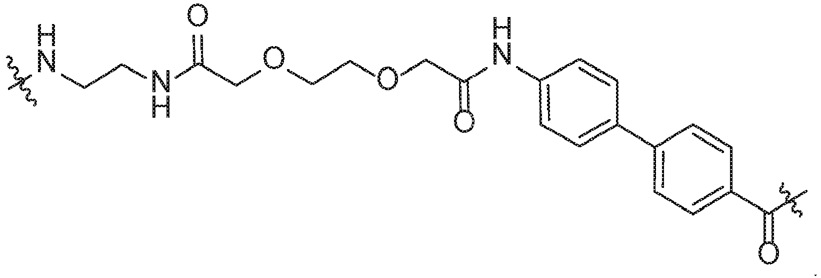

In some instances, at least one linker group of Formula Ix and/or Formula II is

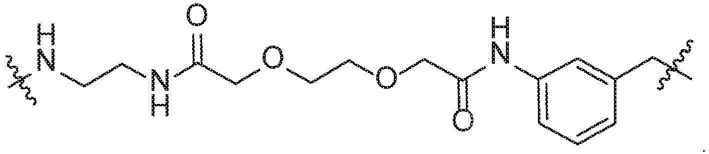

In some instances, at least one linker group of Formula Ix and/or Formula II is chosen from -C(=O)NH(CH₂)₂NH-, -CH₂NHCH₂-, and -C(=O)NHCH₂-. In some instances, at least one linker group is -C(=O)NH(CH₂)₂NH-.

As disclosed herein, an E-selectin antagonist may be chosen from Compound B: 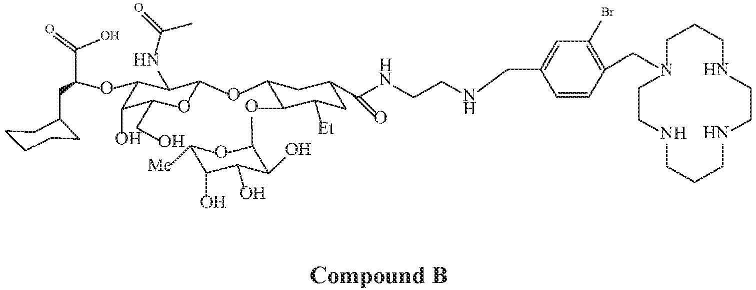 and pharmaceutically acceptable salts thereof.

As disclosed herein, an E-selectin antagonist may be chosen from compounds of Formula III: 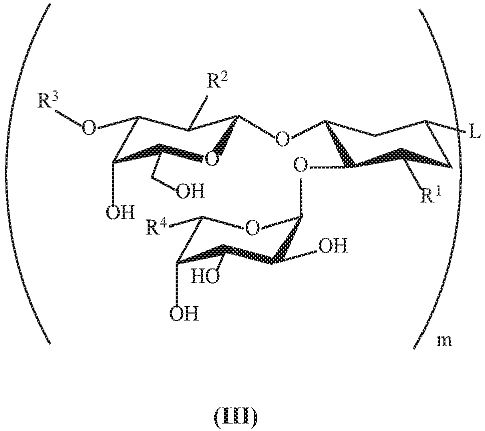 prodrugs of compounds of Formula III, and pharmaceutically acceptable salts of any of the foregoing, wherein:
each R¹, which may be identical or different, is independently chosen from H, C₁₋₁₂ alkyl, C₂₋₁₂ alkenyl, C₂₋₁₂ alkynyl, and -NHC(=O)R⁵ groups, wherein each R⁵, which may be identical or different, is independently chosen from C₁₋₁₂ alkyl, C₂₋₁₂ alkenyl, C₂₋₁₂ alkynyl, C₆₋₁₈ aryl, and C₁₋₁₃ heteroaryl groups;
each R², which may be identical or different, is independently chosen from halo,-OY¹, -NY¹Y², -OC(=O)Y¹, -NHC(=O)Y¹, and -NHC(=O)NY¹Y² groups, wherein each Y¹ and each Y², which may be identical or different, are independently chosen from H, C₁₋₁₂ alkyl, C₂₋₁₂ alkenyl, C₂₋₁₂ alkynyl, C₁₋₁₂ haloalkyl, C₂₋₁₂ haloalkenyl, C₂₋₁₂ haloalkynyl, C₆₋₁₈ aryl, and C₁₋₁₃ heteroaryl groups, wherein Y¹ and Y² may join together along with the nitrogen atom to which they are attached to form a ring;
each R³, which may be identical or different, is independently chosen from 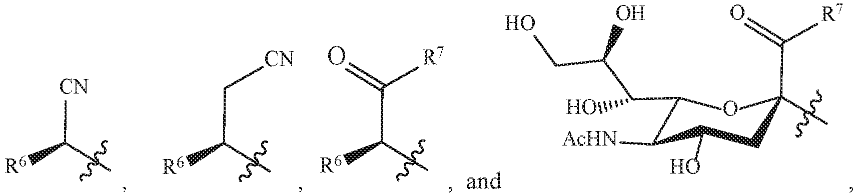 wherein each R⁶, which may be identical or different, is independently chosen from H, C₁₋₁₂ alkyl and C₁₋₁₂ haloalkyl groups, and wherein each R⁷, which may be identical or different, is independently chosen from C₁₋₈ alkyl, C₂₋₈ alkenyl, C₂₋₈ alkynyl, -OY³, -NHOH, -NHOCH₃, -NHCN, and -NY³Y⁴ groups, wherein each Y³ and each Y⁴, which may be identical or different, are independently chosen from H, C₁₋₈ alkyl, C₂₋₈ alkenyl, C₂₋₈ alkynyl, C₁₋₈ haloalkyl, C₂₋₈ haloalkenyl, and C₂₋₈ haloalkynyl groups, wherein Y³ and Y⁴ may join together along with the nitrogen atom to which they are attached to form a ring;
each R⁴, which may be identical or different, is independently chosen from -CN, C₁₋₄ alkyl, and C₁₋₄ haloalkyl groups;
m is chosen from integers ranging from 2 to 256; and
L is chosen from linker groups.

As disclosed herein, an E-selectin antagonist may be chosen from compounds of Formula IV: 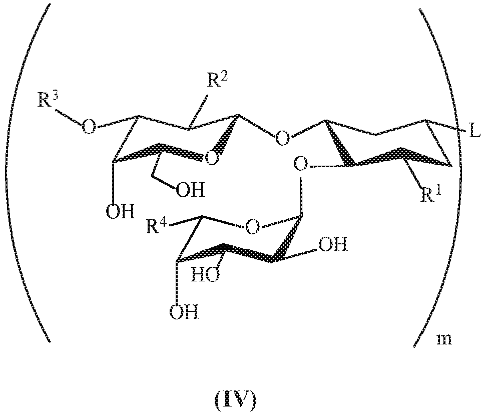 prodrugs of compounds of Formula IV, and pharmaceutically acceptable salts of any of the foregoing, wherein:
each R¹, which may be identical or different, is independently chosen from H, C₁₋₁₂ alkyl, C₂₋₁₂ alkenyl, C₂₋₁₂ alkynyl, and -NHC(=O)R⁵ groups, wherein each R⁵, which may be identical or different, is independently chosen from C₁₋₁₂ alkyl, C₂₋₁₂ alkenyl, C₂₋₁₂ alkynyl, C₆₋₁₈ aryl, and C₁₋₁₃ heteroaryl groups;
each R², which may be identical or different, is independently chosen from halo, - OY¹, -NY¹Y², -OC(=O)Y¹, -NHC(=O)Y¹, and -NHC(=O)NY¹Y² groups, wherein each Y¹ and each Y², which may be identical or different, are independently chosen from H, C₁₋₁₂ alkyl, C₂₋₁₂ alkenyl, C₂₋₁₂ alkynyl, C₁₋₁₂ haloalkyl, C₂₋₁₂ haloalkenyl, C₂₋₁₂ haloalkynyl, C₆₋₁₈ aryl, and C₁₋₁₃ heteroaryl groups, wherein Y¹ and Y² may join together along with the nitrogen atom to which they are attached to form a ring;
each R³, which may be identical or different, is independently chosen from 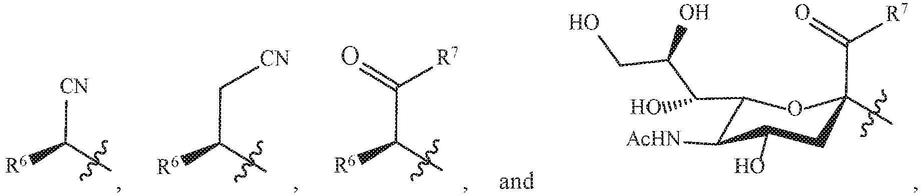 wherein each R⁶, which may be identical or different, is independently chosen from H, C₁₋₁₂ alkyl and C₁₋₁₂ haloalkyl groups, and wherein each R⁷, which may be identical or different, is independently chosen from C₁₋₈ alkyl, C₂₋₈ alkenyl, C₂₋₈ alkynyl, -OY³, --NHOH, -NHOCH₃, -NHCN, and -NY³Y⁴ groups, wherein each Y³ and each Y⁴, which may be identical or different, are independently chosen from H, C₁₋₈ alkyl, C₂₋₈ alkenyl, C₂₋₈ alkynyl, C₁₋₈ haloalkyl, C₂₋₈ haloalkenyl, and C₂₋₈ haloalkynyl groups, wherein Y³ and Y⁴ may join together along with the nitrogen atom to which they are attached to form a ring;
each R⁴, which may be identical or different, is independently chosen from -CN, C₁₋₄ alkyl, and C₁₋₄ haloalkyl groups;
m is 2; and
L is chosen from 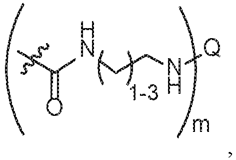
wherein Q is a chosen from 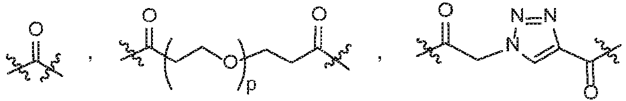 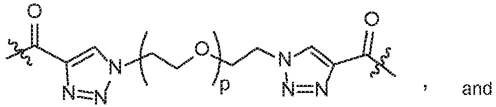 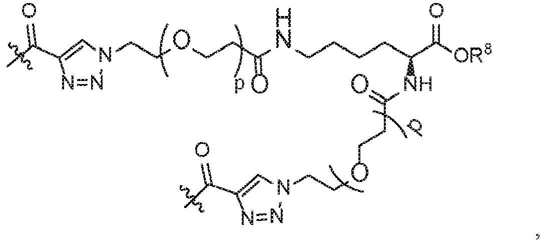
wherein R⁸ is chosen from H, C₁₋₈ alkyl, C₆₋₁₈ aryl, C₇₋₁₉ arylalkyl, and C₁₋₁₃ heteroaryl groups and each p, which may be identical or different, is independently chosen from integers ranging from 0 to 250.

In some instances, the E-selectin antagonist of Formula III or Formula IV is chosen from compounds of the following Formula IIIa/IVa (see definitions of L and m for Formula III or IV above):

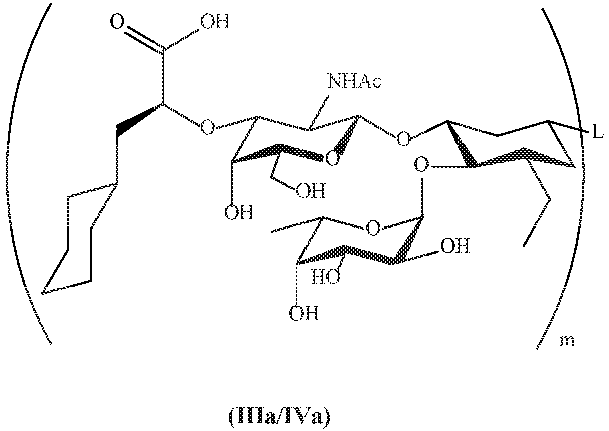

In some instances, the E-selectin antagonist of Formula III or Formula IV is chosen from compounds of the following Formula IIIb/IVb (see definitions of L and m for Formula III or IV above):

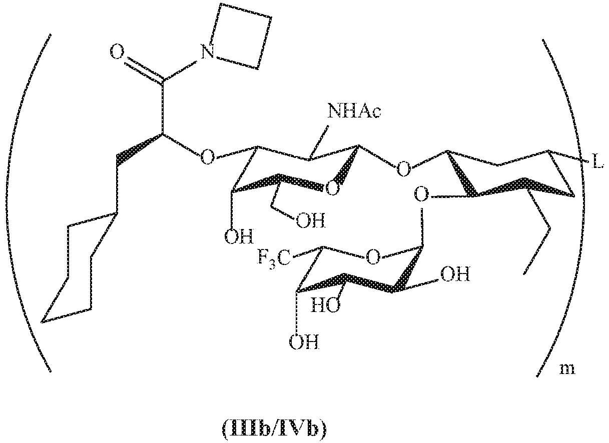

As disclosed herein, an E-selectin antagonist may be Compound C:

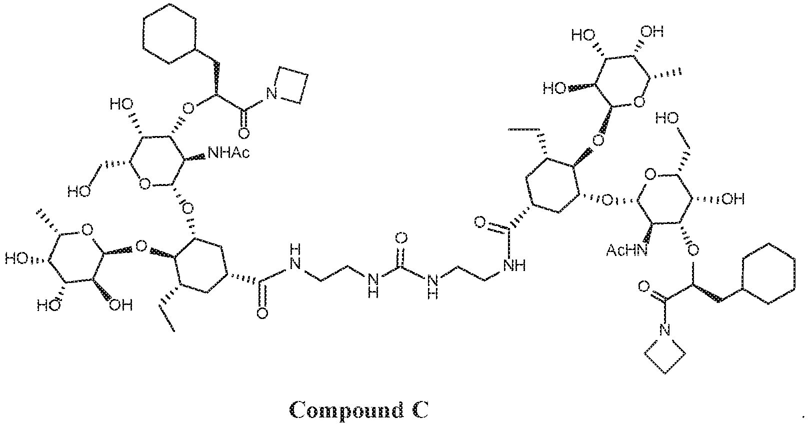

As disclosed herein, an E-selectin antagonist may be a heterobifunctional inhibitor of E-selectin and Galectin-3), chosen from compounds of Formula V: 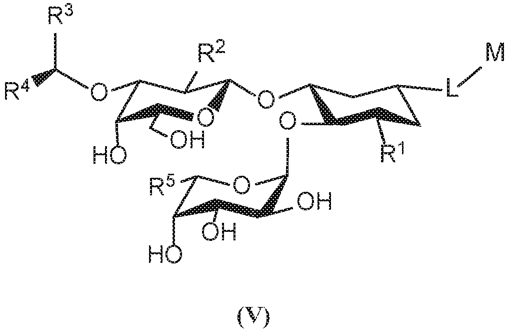 prodrugs of compounds of Formula V, and pharmaceutically acceptable salts of any of the foregoing, wherein:
R¹ is chosen from H, C₁₋₈ alkyl, C₂₋₈ alkenyl, C₂₋₈ alkynyl, C₁₋₈ haloalkyl, C₂₋₈ haloalkenyl, C₂₋₈ haloalkynyl, 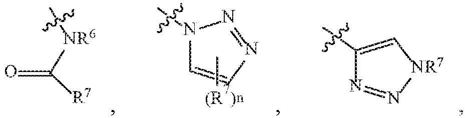 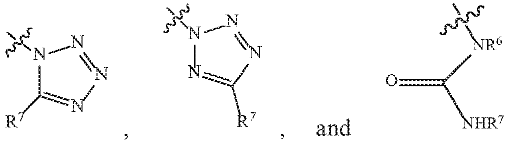 groups, wherein n is chosen from integers ranging from 0 to 2, R⁶ is chosen from H, C₁₋₈ alkyl, C₂₋₈ alkenyl, C₂₋₈ alkynyl, C₄₋₁₆ cycloalkylalkyl, and -C(=O)R⁷ groups, and each R⁷ is independently chosen from H, C₁₋₈ alkyl, C₂₋₈ alkenyl, C₂₋₈ alkynyl, C₄₋₁₆ cycloalkylalkyl, C₆₋₁₈ aryl, and C₁₋₁₃ heteroaryl groups;
R² is chosen from -OH, -OY¹, halo, -NH₂, -NY¹Y², -OC(=O)Y¹, -NHC(=O)Y¹, and -NHC(=O)NHY¹ groups, wherein Y¹ and Y², which may be the same or different, are independently chosen from C₁₋₈ alkyl, C₂₋₈ alkenyl, C₂₋₈ alkynyl, C₄₋₁₆ cycloalkylalkyl, C₂₋₁₂ heterocyclyl, C₆₋₁₈ aryl, and C₁₋₁₃ heteroaryl groups, wherein Y¹ and Y² may join together along with the nitrogen atom to which they are attached to form a ring;
R³ is chosen from -CN, -CH₂CN, and -C(=O)Y³ groups, wherein Y³ is chosen from C₁₋₈ alkyl, C₂₋₈ alkenyl, C₂₋₈ alkynyl, -OZ¹, -NHOH, -NHOCH₃, -NHCN, and-NZ¹Z² groups, wherein Z¹ and Z², which may be identical or different, are independently chosen from H, C₁₋₈ alkyl, C₂₋₈ alkenyl, C₂₋₈ alkynyl, C₁₋₈ haloalkyl, C₂₋₈ haloalkenyl, C₂₋₈ haloalkynyl, and C₇₋₁₂ arylalkyl groups, wherein Z¹ and Z² may join together along with the nitrogen atom to which they are attached to form a ring;
R⁴ is chosen from H, C₁₋₈ alkyl, C₂₋₈ alkenyl, C₂₋₈ alkynyl, C₁₋₈ haloalkyl, C₂₋₈ haloalkenyl, C₂₋₈ haloalkynyl, C₄₋₁₆ cycloalkylalkyl, and C₆₋₁₈ aryl groups;
R⁵ is chosen from -CN, C₁₋₈ alkyl, and C₁₋₄ haloalkyl groups;
M is chosen from 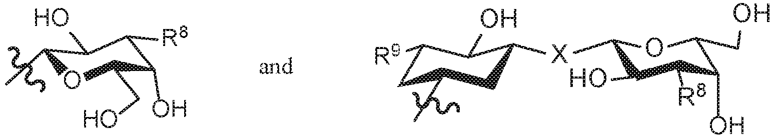 groups, wherein X is chosen from O and S, and R⁸ and R⁹, which may be identical or different, are independently chosen from C₆₋₁₈ aryl, C₁₋₁₃ heteroaryl, C₇₋₁₉ arylalkyl, C₇₋₁₉ arylalkoxy, C₂₋₁₄ heteroarylalkyl, C₂₋₁₄ heteroarylalkoxy, and -NBC(=O)Y⁴ groups, wherein Y⁴ is chosen from C₁₋₈ alkyl, C₂₋₁₂ heterocyclyl, C₆₋₁₈ aryl, and C₁₋₁₃ heteroaryl groups; and
L is chosen from linker groups.

As disclosed herein, an E-selectin antagonist may be chosen from compounds having the following Formulae: 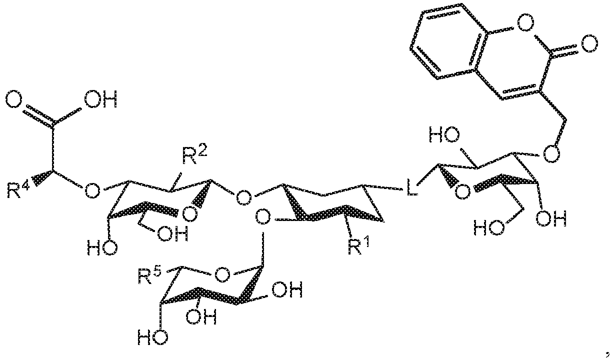 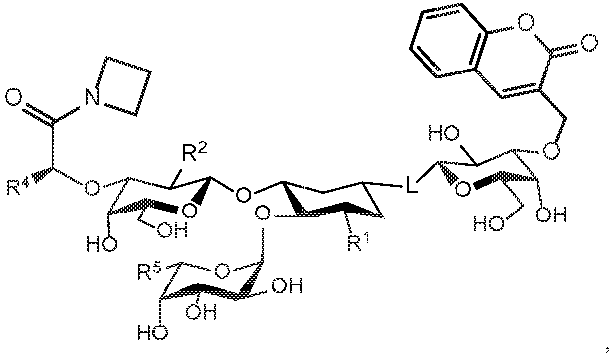 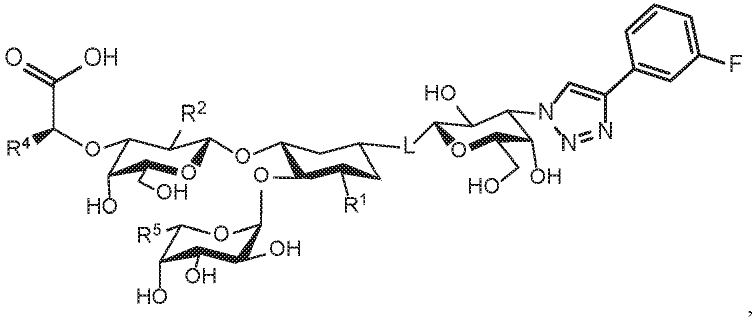 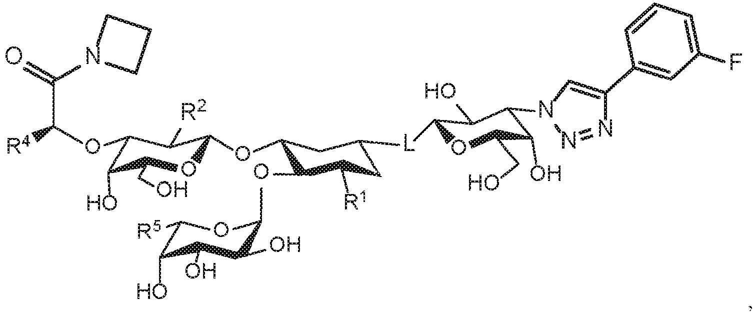 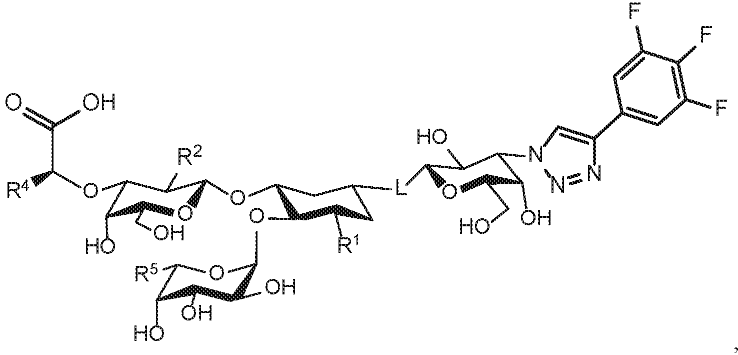 and

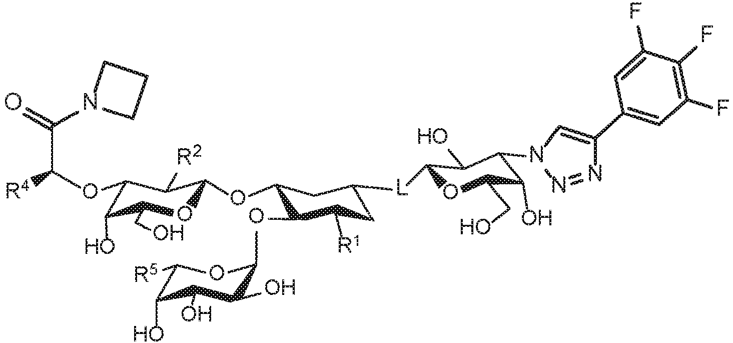

As disclosed herein, an E-selectin antagonist may be chosen from compounds having the following Formulae: 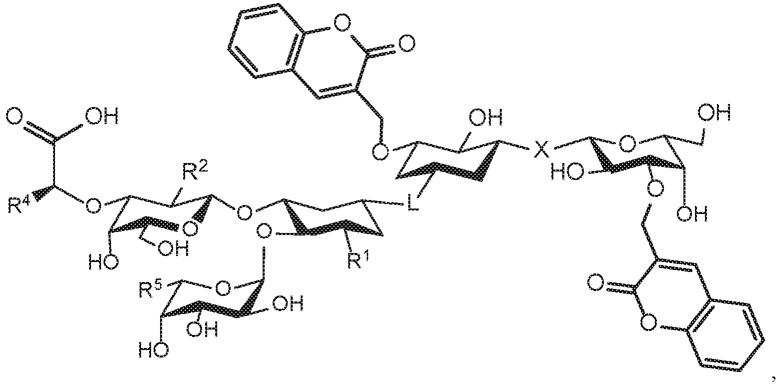 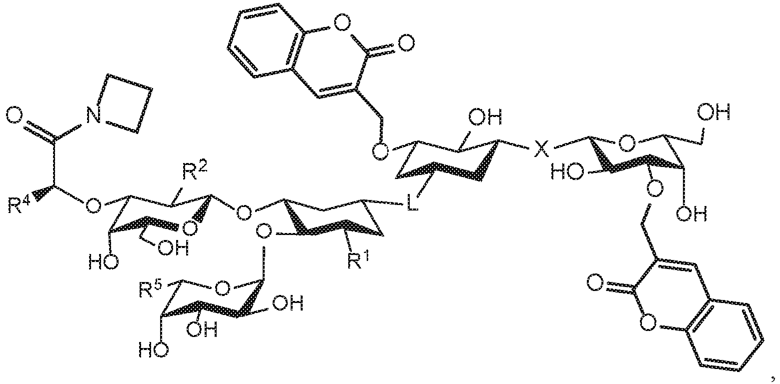 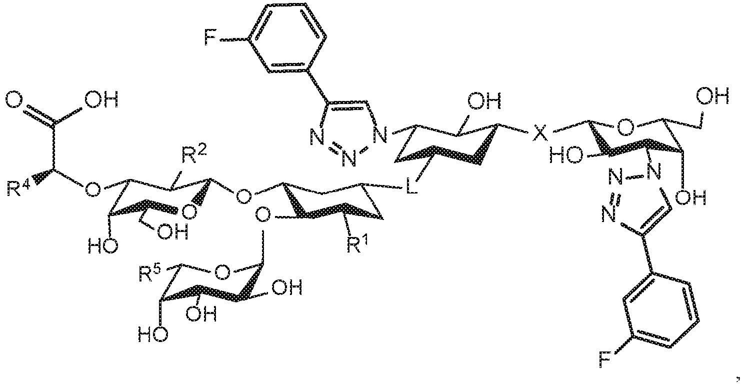 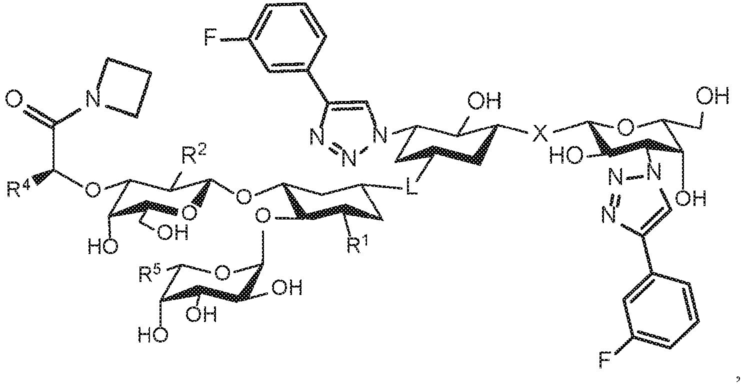 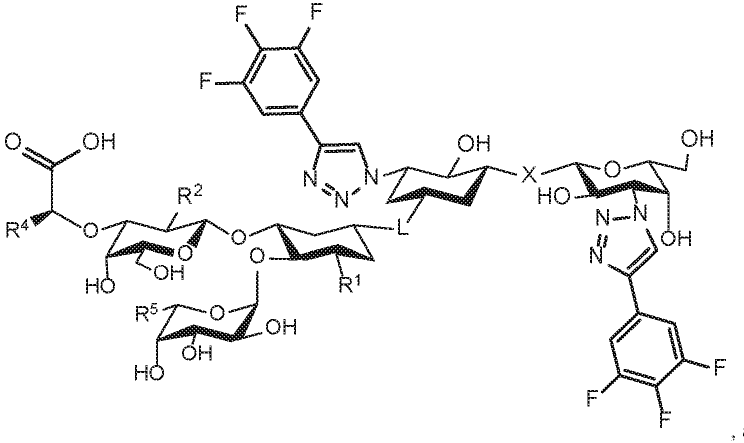 and

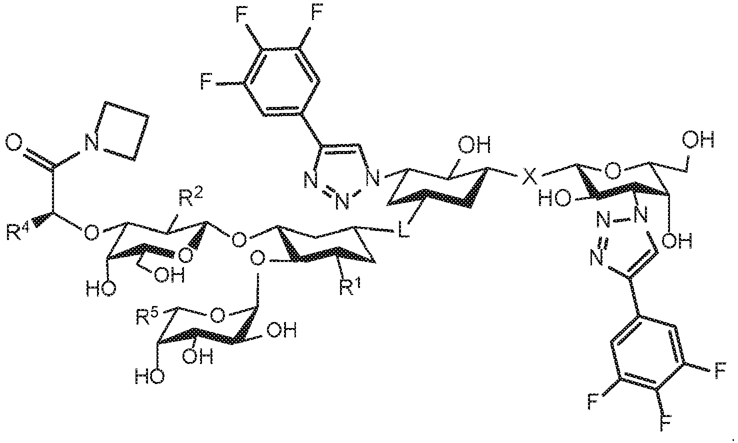

As disclosed herein, an E-selectin antagonist may be Compound D:

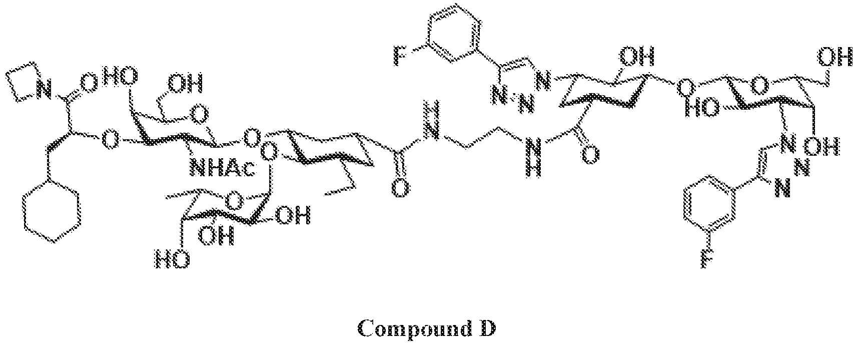

As disclosed herein, an E-selectin antagonist may be chosen from compounds of Formula VI: 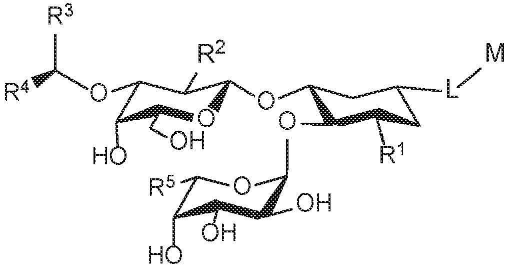 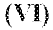 prodrugs of compounds of Formula VI, and pharmaceutically acceptable salts of any of the foregoing, wherein:
R¹ is chosen from H, C₁₋₈ alkyl, C₂₋₈ alkenyl, C₂₋₈ alkynyl, C₁₋₈ haloalkyl, C₂₋₈ haloalkenyl, C₂₋₈ haloalkynyl, 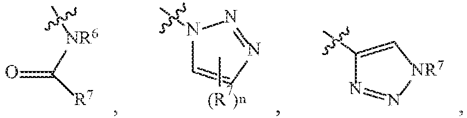 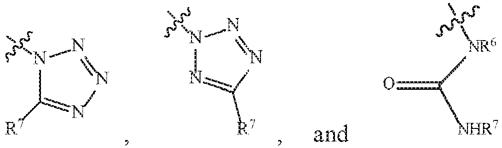 groups, wherein n is chosen from integers ranging from 0 to 2, R⁶ is chosen from H, C₁₋₈ alkyl, C₂₋₈ alkenyl, C₂₋₈ alkynyl, C₄₋₁₆ cycloalkylalkyl, and -C(=O)R⁷ groups, and each R⁷ is independently chosen from H, C₁₋₈ alkyl, C₂₋₈ alkenyl, C₂₋₈ alkynyl, C₄₋₁₆ cycloalkylalkyl, C₆₋₁₈ aryl, and C₁₋₁₃ heteroaryl groups;
R² is chosen from -OH, -OY¹, halo, -NH₂, -NY¹Y², -OC(=O)Y¹, -NHC(=O)Y¹, and -NHC(=O)NHY¹ groups, wherein Y¹ and Y², which may be the same or different, are independently chosen from C₁₋₈ alkyl, C₂₋₈ alkenyl, C₂₋₈ alkynyl, C₄₋₁₆ cycloalkylalkyl, C₂₋₁₂ heterocyclyl, C₆₋₁₈ aryl, and C₁₋₁₃ heteroaryl groups, or Y¹ and Y² join together along with the nitrogen atom to which they are attached to form a ring;
R³ is chosen from -CN, -CH₂CN, and -C(=O)Y³ groups, wherein Y³ is chosen from C₁₋₈ alkyl, C₂₋₈ alkenyl, C₂₋₈ alkynyl, -OZ¹, -NHOH, -NHOCH₃, -NHCN, and-NZ¹Z² groups, wherein Z¹ and Z², which may be identical or different, are independently chosen from H, C₁₋₈ alkyl, C₂₋₈ alkenyl, C₂₋₈ alkynyl, C₁₋₈ haloalkyl, C₂₋₈ haloalkenyl, C₂₋₈ haloalkynyl, and C₇₋₁₂ arylalkyl groups, or Z¹ and Z² join together along with the nitrogen atom to which they are attached to form a ring;
R⁴ is chosen from H, C₁₋₈ alkyl, C₂₋₈ alkenyl, C₂₋₈ alkynyl, C₁₋₈ haloalkyl, C₂₋₈ haloalkenyl, C₂₋₈ haloalkynyl, C₄₋₁₆ cycloalkylalkyl, and C₆₋₁₈ aryl groups;
R⁵ is chosen from -CN, C₁₋₈ alkyl, and C₁₋₄ haloalkyl groups;
M is chosen from 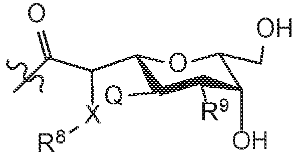 groups, wherein
   X is chosen from -O-, -S-, -C-, and -N(R¹⁰)-, wherein R¹⁰ is chosen from H, C₁₋₈ alkyl, C₂₋₈ alkenyl, C₂₋₈ alkynyl, C₁₋₈ haloalkyl, C₂₋₈ haloalkenyl, and C₂₋₈ haloalkynyl groups,
   Q is chosen from H, halo, and -OZ³ groups, wherein Z³ is chosen from H and C₁₋₈ alkyl groups,
   R⁸ is chosen from H, C₁₋₈ alkyl, C₂₋₈ alkenyl, C₂₋₈ alkynyl, C₁₋₈ haloalkyl, C₂₋₈ haloalkenyl, C₂₋₈ haloalkynyl, C₄₋₁₆ cycloalkylalkyl, C₆₋₁₈ aryl, C₁₋₁₃ heteroaryl, C₇₋₁₉ arylalkyl, and C₂₋₁₄ heteroarylalkyl groups, wherein the C₁₋₈ alkyl, C₂₋₈ alkenyl, C₂₋₈ alkynyl, C₁₋₈ haloalkyl, C₂₋₈ haloalkenyl, C₂₋₈ haloalkynyl, C₄₋₁₆ cycloalkylalkyl, C₆₋₁₈ aryl, C₁₋₁₃ heteroaryl, C₇₋₁₉ arylalkyl, and C₂₋₁₄ heteroarylalkyl groups are optionally substituted with one or more groups independently chosen from halo, C₁₋₈ alkyl, C₁₋₈ hydroxyalkyl, C₁₋₈ haloalkyl, C₆₋₁₈ aryl, -OZ⁴, -C(=O)OZ⁴, -C(=O)NZ⁴Z⁵, and -SO₂Z⁴ groups, wherein Z⁴ and Z⁵, which may be identical or different, are independently chosen from H, C₁₋₈ alkyl, and C₁₋₈ haloalkyl groups, or Z⁴ and Z⁵ join together along with the nitrogen atom to which they are attached to form a ring,
   R⁹ is chosen from C₆₋₁₈ aryl and C₁₋₁₃ heteroaryl groups, wherein the C₆₋₁₈ aryl and C₁₋₁₃ heteroaryl groups are optionally substituted with one or more groups independently chosen from R¹¹, C₁₋₈ alkyl, C₁₋₈ haloalkyl, -C(=O)OZ⁶, and-C(=O)NZ⁶Z⁷ groups, wherein R¹¹ is independently chosen from C₆₋₁₈ aryl groups optionally substituted with one or more groups independently chosen from halo, C₁₋₈ alkyl, -OZ³, -C(=O)OZ⁸, and -C(=O)NZ⁸Z⁹ groups, wherein Z⁶, Z⁷, Z⁸ and Z⁹, which may be identical or different, are independently chosen from H and C₁₋₈ alkyl groups, or Z⁶ and Z⁷ join together along with the nitrogen atom to which they are attached to form a ring and/or Z⁸ and Z⁹ join together along with the nitrogen atom to which they are attached to form a ring, and
   wherein each of Z³, Z⁴, Z⁵, Z⁶, Z⁷, Z⁸, and Z⁹ is optionally substituted with one or more groups independently chosen from halo and -OR¹² groups, wherein R¹² is independently chosen from H and C₁₋₈ alkyl groups; and
   L is chosen from linker groups.

In some instances of Formula VI, M is chosen from 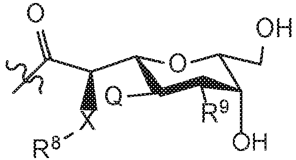 groups.

In some instances of Formula VI, M is chosen from 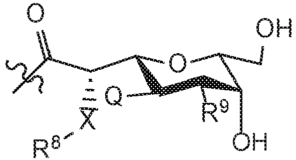 groups.

In some instances of Formula VI, linker groups may be chosen from groups comprising spacer groups, such spacer groups as, for example, -(CH₂)ₜ- and -O(CH₂)ₜ-, wherein t is chosen from integers ranging from 1 to 20. Other non-limiting examples of spacer groups include carbonyl groups and carbonyl-containing groups such as, for example, amide groups. A non-limiting example of a spacer group is

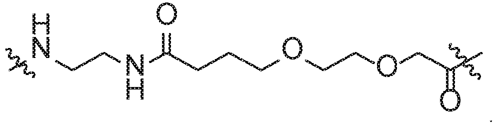

In some instances of Formula VI, the linker group is chosen from

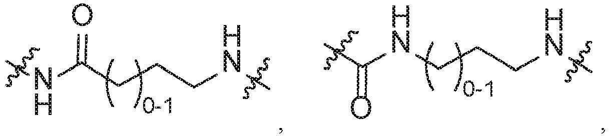

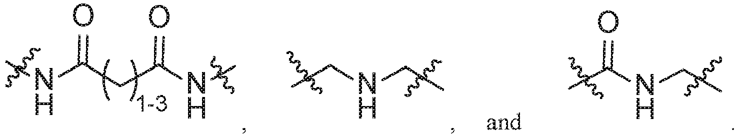

In some instances of Formula VI, the linker group is chosen from polyethylene glycols (PEGs), -C(=O)NH(CH₂)ᵥO-, -C(=O)NH(CH₂)ᵥNHC(=O) -, -C(=O)NHC(=O)(CH₂)NH-, and -C(=O)NH(CH₂)ᵥC(=O)NH- groups, wherein v is chosen from integers ranging from 2 to 20. In some instances, v is chosen from integers ranging from 2 to 4. In some instances, v is 2. In some instances, v is 3. In some instances, v is 4.

In some instances of Formula VI, the linker group is

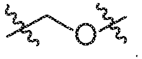

In some instances of Formula VI, the linker group is

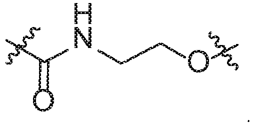

In some instances of Formula VI, the linker group is

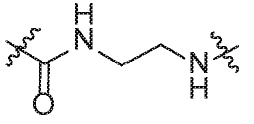

In some instances of Formula VI, the linker group is

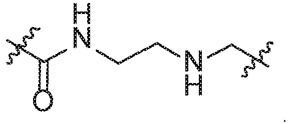

In some instances of Formula VI, the linker group is

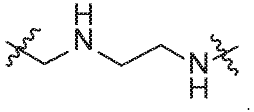

In some instances of Formula VI, the linker group is

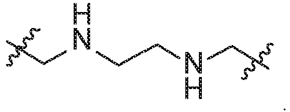

In some instances of Formula VI, the linker group is

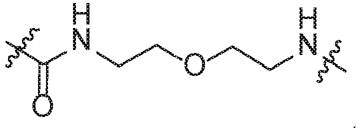

In some instances of Formula VI, the linker group is

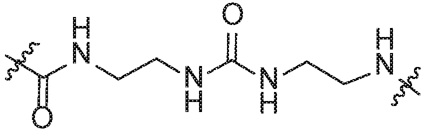

In some instances of Formula VI, the linker group is

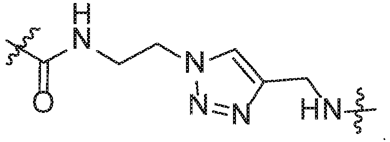

As disclosed herein, an E-selectin antagonist may be a multimeric inhibitor of E-selectin, Galectin-3, and/or CXCR4, chosen from compounds of Formula VII: 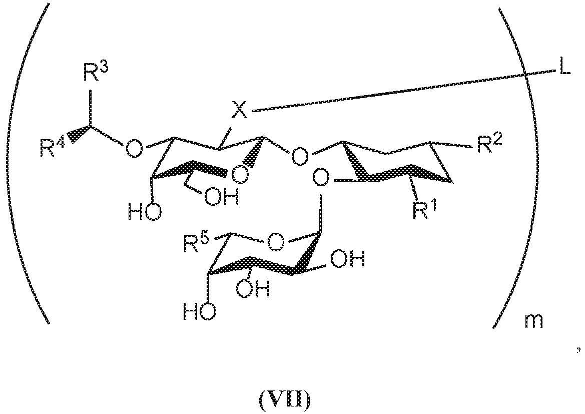 prodrugs of compounds of Formula VII, and pharmaceutically acceptable salts of any of the foregoing, wherein:
each R¹, which may be identical or different, is independently chosen from H, C₁₋₁₂ alkyl, C₂₋₁₂ alkenyl, C₂₋₁₂ alkynyl, C₁₋₈ haloalkyl, C₂₋₈ haloalkenyl, C₂₋₈ haloalkynyl, 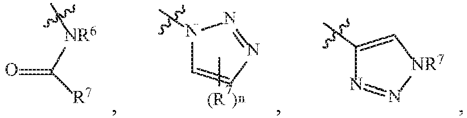 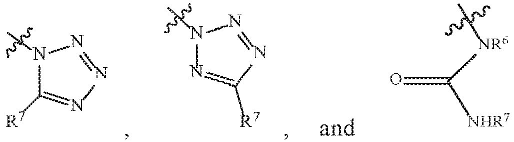 groups, wherein each n, which may be identical or different, is chosen from integers ranging from 0 to 2, each R⁶, which may be identical or different, is independently chosen from H, C₁₋₈ alkyl, C₂₋₈ alkenyl, C₂₋₈ alkynyl, C₄₋₁₆ cycloalkylalkyl, and -C(=O)R⁷ groups, and each R⁷, which may idential or different, is independently chosen from H, C₁₋₈ alkyl, C₂₋₈ alkenyl, C₂₋₈ alkynyl, C₄₋₁₆ cycloalkylalkyl, C₆₋₁₈ aryl, and C₁₋₁₃ heteroaryl groups;
each R², which may be identical or different, is independently chosen from H, a non-glycomimetic moiety, and a linker-non-glycomimetic moiety, wherein each non-glycomimetic moiety, which may be identical or different, is independently chosen from galectin-3 inhibitors, CXCR4 chemokine receptor inhibitors, polyethylene glycol, thiazolyl, chromenyl, C₁₋₈ alkyl, R⁸, C₆₋₁₈ aryl-R⁸, C₁₋₁₂ heteroaryl-R⁸, 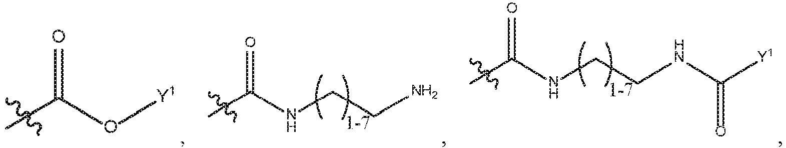 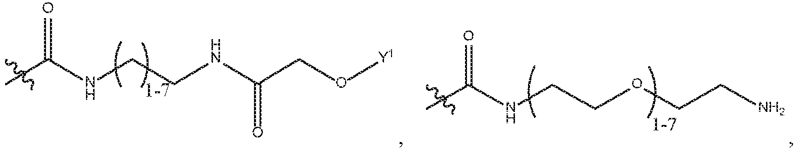 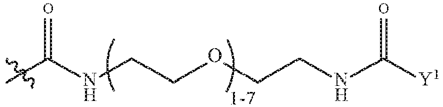 and 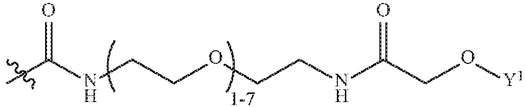 groups, wherein each Y¹, which may be identical or different, is independently chosen from C₁₋₄ alkyl, C₂-₄ alkenyl, and C₂₋₄ alkynyl groups and wherein each R³, which may be identical or different, is independently chosen from C₁₋₁₂ alkyl groups substituted with at least one substituent chosen from -OH, -OSO₃Q, -OPO₃Q₂, -CO₂Q, and -SO₃Q groups and C₂₋₁₂ alkenyl groups substituted with at least one substituent chosen from -OH, -OSO₃Q,-OPO₃Q₂, -CO₂Q, and -SO₃Q groups, wherein each Q, which may be identical or different, is independently chosen from H and pharmaceutically acceptable cations;
each R³, which may be identical or different, is independently chosen from -CN, -CH₂CN, and -C(=O)Y² groups, wherein each Y², which may be identical or different, is independently chosen from C₁₋₈ alkyl, C₂₋₈ alkenyl, C₂₋₈ alkynyl, -OZ¹, -NHOH, -NHOCH₃, -NHCN, and -NZ¹Z² groups, wherein each Z¹ and Z², which may be identical or different, are independently chosen from H, C₁₋₁₂ alkyl, C₂₋₁₂ alkenyl, C₂₋₁₂ alkynyl, C₁₋₁₂ haloalkyl, C₂₋₁₂ haloalkenyl, C₂₋₁₂ haloalkynyl, and C₇₋₁₂ arylalkyl groups, wherein Z¹ and Z² may join together along with the nitrogen atom to which they are attached to form a ring;
each R⁴, which may be identical or different, is independently chosen from H, C₁₋₁₂ alkyl, C₂₋₁₂ alkenyl, C₂₋₁₂ alkynyl, C₁₋₁₂ haloalkyl, C₂₋₁₂ haloalkenyl, C₂₋₁₂ haloalkynyl, C₄₋₁₆ cycloalkylalkyl, and C₆₋₁₈ aryl groups;
each R⁵, which may be identical or different, is independently chosen from -CN, C₁₋₁₂ alkyl, and C₁₋₁₂ haloalkyl groups;
each X, which may be identical or different, is independently chosen from -O- and -N(R⁹)-, wherein each R⁹, which may be identical or different, is independently chosen from H, C₁₋₈ alkyl, C₂₋₈ alkenyl, C₂₋₈ alkynyl, C₁₋₈ haloalkyl, C₂₋₈ haloalkenyl, and C₂₋₈ haloalkynyl groups;
m is chosen from integers ranging from 2 to 256; and
L is independently chosen from linker groups.

In some instances of Formula VII, at least one linker group is chosen from groups comprising spacer groups, such spacer groups as, for example, -(CH₂)_{z}- and -O(CH₂)_{z}-, wherein z is chosen from integers ranging from 1 to 250. Other non-limiting examples of spacer groups include carbonyl groups and carbonyl-containing groups such as, for example, amide groups. A non-limiting example of a spacer group is

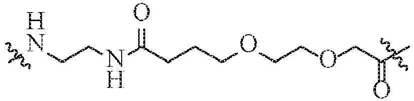

In some instances of Formula VII, at least one linker group is chosen from 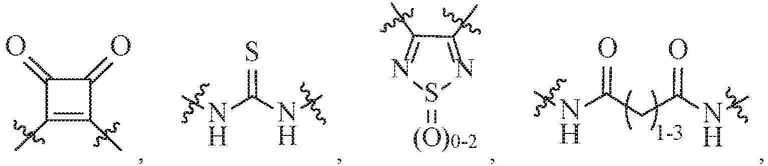 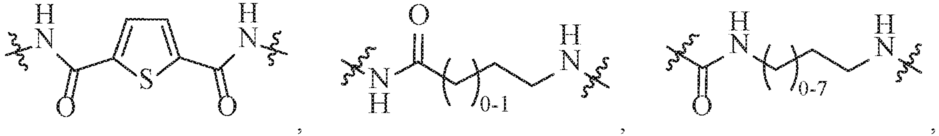 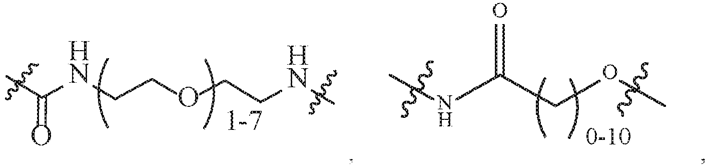 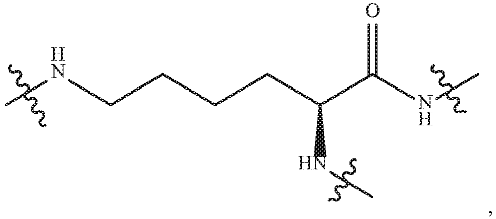 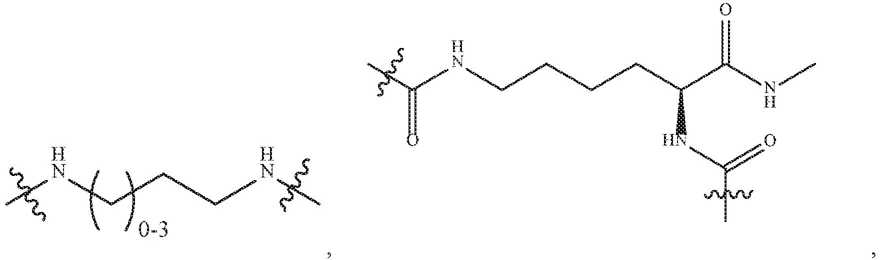 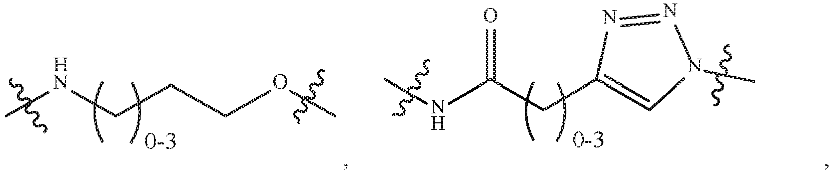 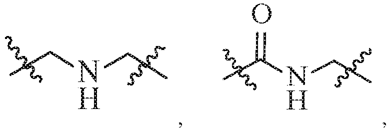 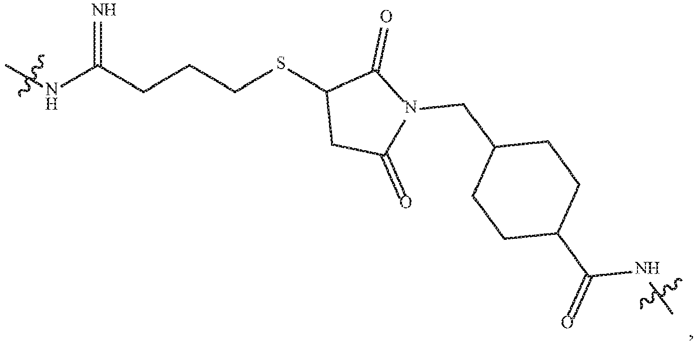 and 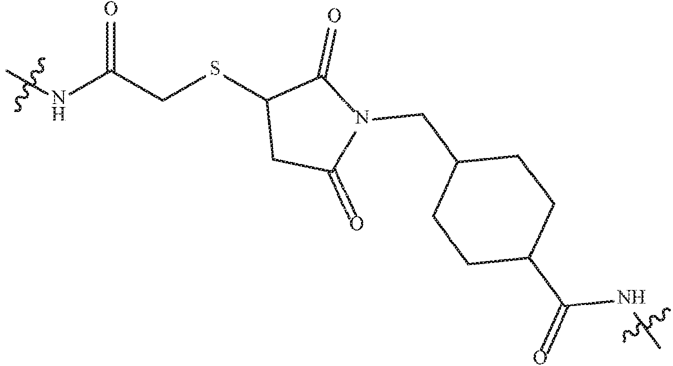 groups.

Other linker groups for certain instances of Formula VII, such as, for example, polyethylene glycols (PEGs) and -C(=O)-NH-(CH₂)_{z}-C(=O)-NH-, wherein z is chosen from integers ranging from 1 to 250, will be familiar to those of ordinary skill in the art and/or those in possession of the present disclosure.

In some instances of Formula VII, at least one linker group is

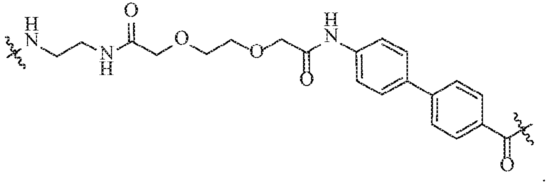

In some instances of Formula VII, at least one linker group is

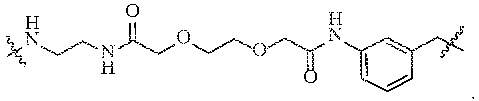

In some instances of Formula VII, at least one linker group is chosen from -C(=O)NH(CH₂)₂NH-, -CH₂NHCH₂-, and -C(=O)NHCH₂-. In some instances of Formula VII, at least one linker group is -C(=O)NH(CH₂)₂NH-.

In some instances of Formula VII, L is chosen from dendrimers. In some instances of Formula VII, L is chosen from polyamidoamine ("PAMAM") dendrimers. In some instances of Formula VII, L is chosen from PAMAM dendrimers comprising succinamic. In some instances of Formula VII, L is PAMAM GO generating a tetramer. In some instances of Formula VII, L is PAMAM G1 generating an octamer. In some instances of Formula VII, L is PAMAM G2 generating a 16-mer. In some instances of Formula VII, L is PAMAM G3 generating a 32-mer. In some instances of Formula VII, L is PAMAM G4 generating a 64-mer. In some instances, L is PAMAM G5 generating a 128-mer.

In some instances of Formula VII, m is 2 and L is chosen from 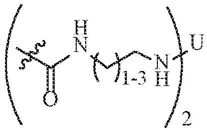 groups,
wherein U is chosen from 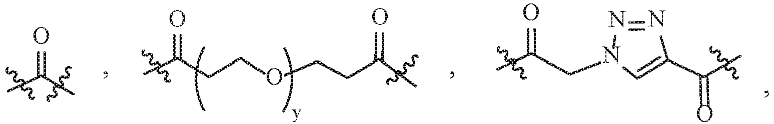 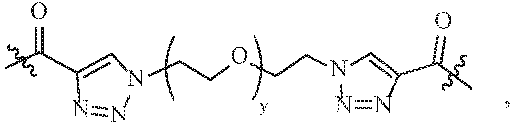 and 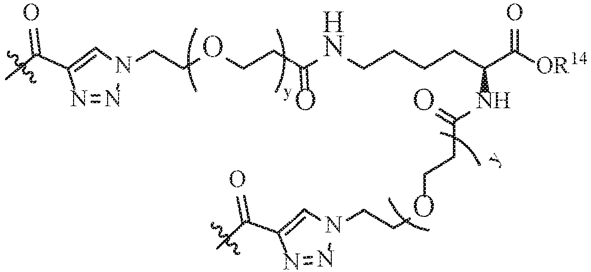 groups,
wherein R¹⁴ is chosen from H, C₁₋₈ alkyl, C₆₋₁₈ aryl, C₇₋₁₉ arylalkyl, and C₁₋₁₃ heteroaryl groups and each y, which may be identical or different, is independently chosen from integers ranging from 0 to 250. In some embodiments of Formula VII, R¹⁴ is chosen from C₁₋₈ alkyl. In some embodiments of Formula VII, R¹⁴ is chosen from C₇₋₁₉ arylalkyl. In some embodiments of Formula VII, R¹⁴ is H. In some embodiments of Formula VII, R¹⁴ is benzyl.

In some instances of Formula VII, L is chosen from 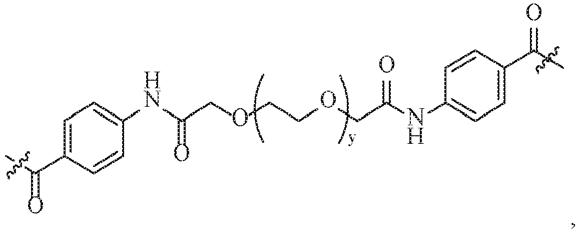 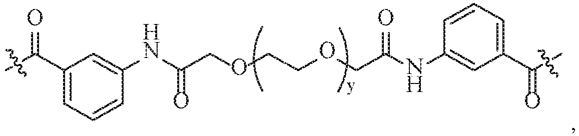 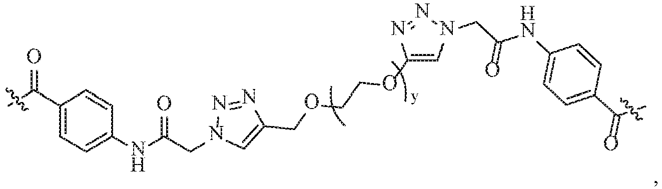 and 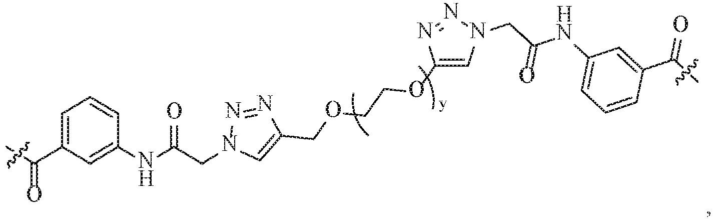 wherein y is chosen from integers ranging from 0 to 250.

In some instances of Formula VII, L is chosen from 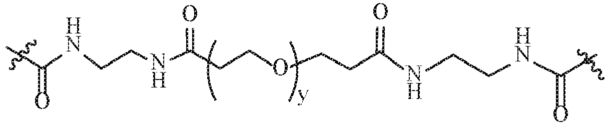 groups,
wherein y is chosen from integers ranging from 0 to 250.

In some instances of Formula VII, L is

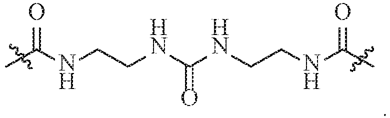

In some instances of Formula VII, L is chosen from 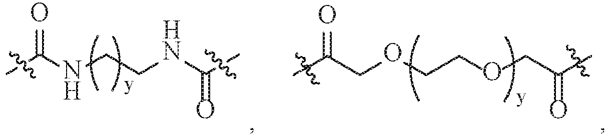 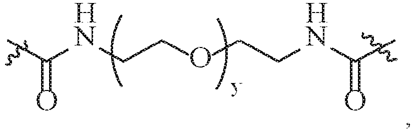 and 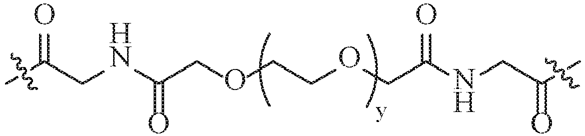 groups, wherein y is chosen from integers ranging from 0 to 250.

In some instances of Formula VII, L is chosen from 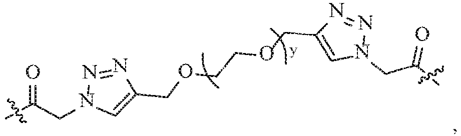 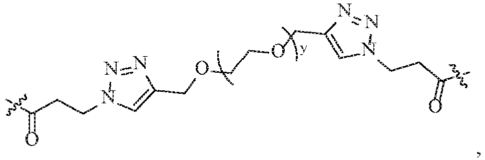 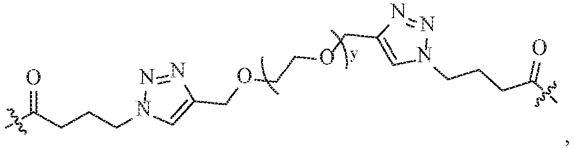 and 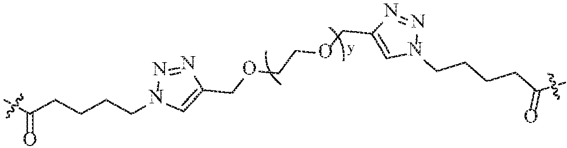 groups, wherein y is chosen from integers ranging from 0 to 250.

In some instances of Formula VII, L is chosen from

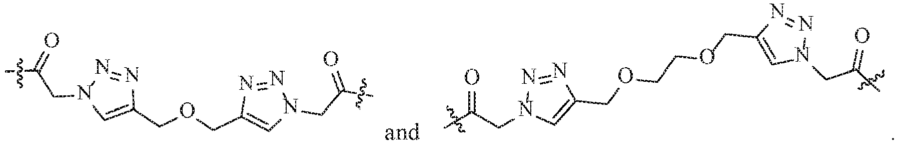

In some instances of Formula VII, L is

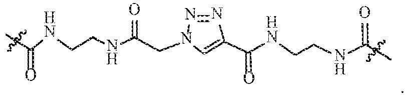

In some instances of Formula VII, L is chosen from 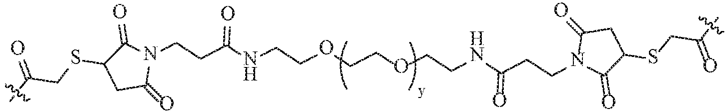 groups,
wherein y is chosen from integers ranging from 0 to 250.

In some instances of Formula VII, L is

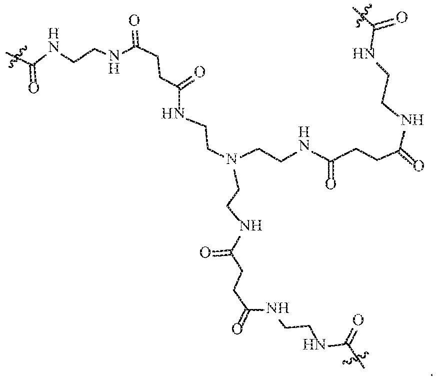

In some instances of Formula VII, L is

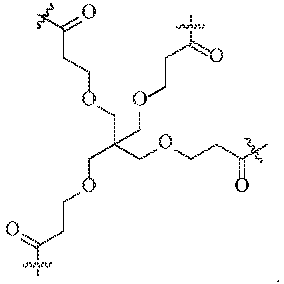

In some instances of Formula VII, L is

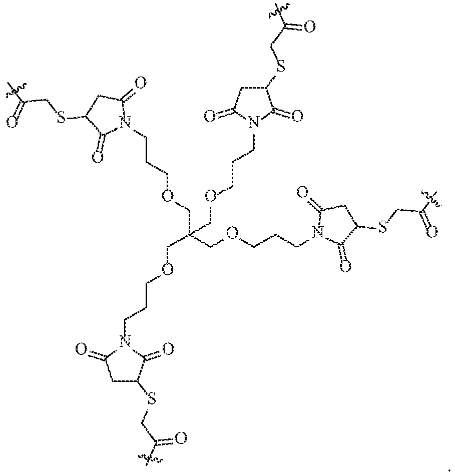

In some instances of Formula VII, L is chosen from 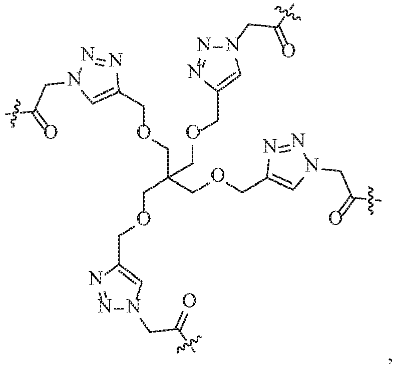 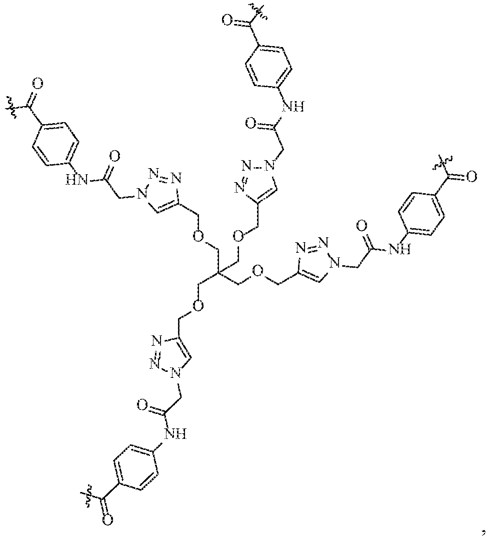 and

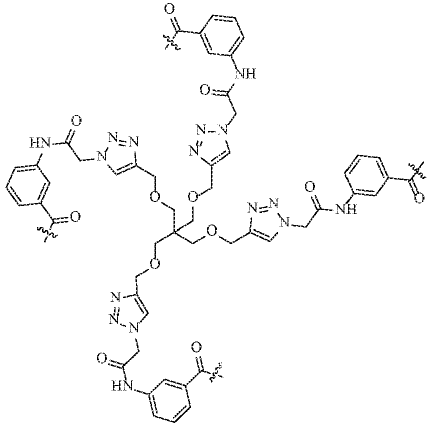

In some instances of Formula VII, L is

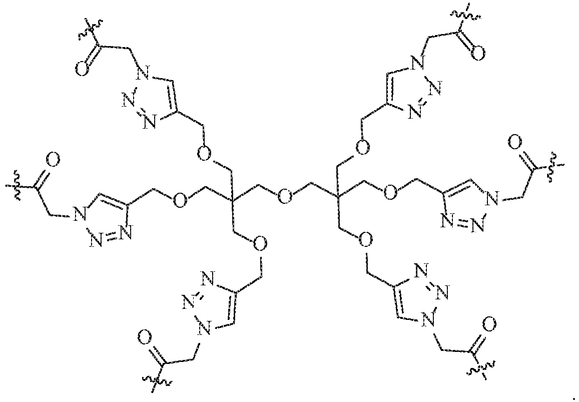

In some instances of Formula VII, L is chosen from 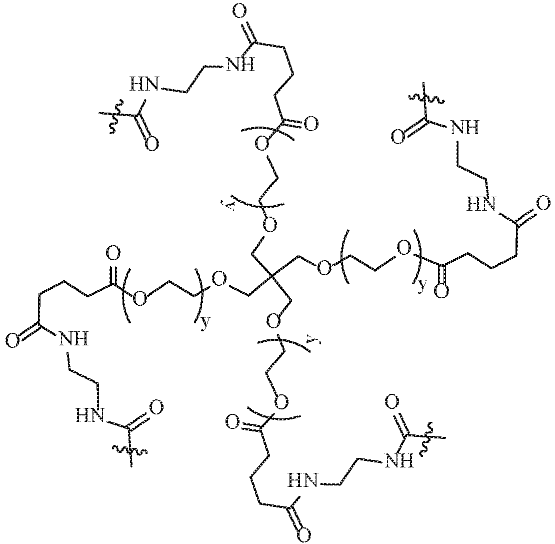 groups,
wherein each y, which may be identical or different, is independently chosen from integers ranging from 0 to 250.

In some instances of Formula VII, L is chosen from 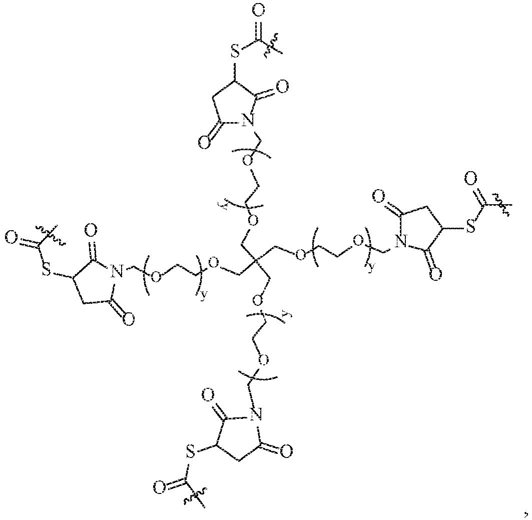 wherein each y, which may be identical or different, is independently chosen from integers ranging from 0 to 250.

In some instances of Formula VII, L is chosen from

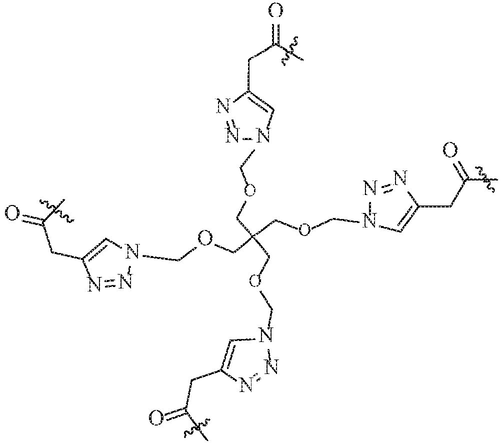

In some instances, at least one compound is chosen from compounds of Formula VII, wherein each R¹ is identical, each R² is identical, each R³ is identical, each R⁴ is identical, each R⁵ is identical, and each X is identical. In some instances, at least one compound is chosen from compounds of Formula VII, wherein said compound is symmetrical.

Disclosed herein are pharmaceutical compositions comprising at least one compound chosen from compounds of Formula Ix, Ia, II, IIa, III, IV, IIIa/IVa, IIIb/IVb, V, VI, and VII, and pharmeutically acceptable salts of any of the foregoing. Also disclosed are pharmaceutical compositions comprising at least one compound chosen from compound A, compound B, compound C, and compound D, and pharmentically acceptable salts of any of the foregoing. In some instances, the pharmaceutically acceptable salts is a sodium salt. These compounds and compositions may be used in the methods described herein.

### EXAMPLES

Examples 1-252 are reference examples; example 253 is relevant for understanding the invention; and example 254 is according to the invention.

### EXAMPLE 1

### PROPHETIC SYNTHESIS OF MULTIMERIC COMPOUND 21

**Compound 3:** A mixture of compound 1 (preparation described in WO 2007/028050) and compound **2** (preparation described in WO 2013/096926) (1.7 eq) is azeotroped 3 times from toluene. The mixture is dissolved in DCM under argon and cooled on an ice bath. To this solution is added boron trifluoride etherate (1.5 eq). The reaction mixture is stirred 12 hours at room temperature. The reaction is quenched by the addition of triethylamine (2 eq). The reaction mixture is transferred to a separatory funnel and washed 1 time with half saturated sodium bicarbonate solution and 1 time with water. The organic phase is dried over sodium sulfate, filtered, and concentrated. The residue is purified by flash chromatography to afford compound 3.

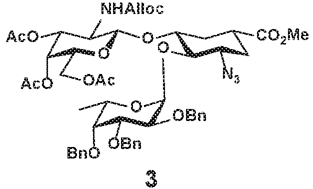

**Compound 4:** Compound 3 is dissolved in methanol at room temperature. A solution of sodium methoxide in methanol (0.1 eq) is added and the reaction mixture stirred overnight at room temperature. The reaction mixture is quenched by the addition of acetic acid. The reaction mixture is diluted with ethyl acetate, transferred to a separatory funnel and washed 2 times with water. The organic phase is dried over magnesium sulfate, filtered and concentrated. The residue is separated by flash chromatography to afford compound 4.

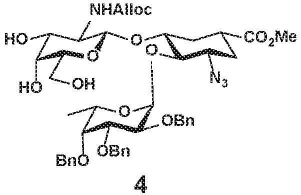

**Compound 5:** To a solution of compound 4 in dichloromethane cooled on an ice bath is added DABCO (1.5 eq) followed by monomethyoxytrityl chloride (1.2 eq). The reaction mixture is stirred overnight allowing to warm to room temperature. The reaction mixture is transferred to a separatory funnel and washed 2 times with water. The organic phase is concentrated and the residue is purified by flash chromatography to afford compound 5.

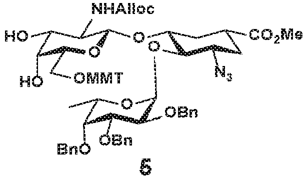

**Compound 7:** To a solution of compound 5 in methanol is added dibutyltin oxide (1.1 eq). The reaction mixture is refluxed for 3 hours then concentrated. The residue is suspended in DME. To this suspension is added compound 6 (preparation described in Thoma et. al. J. Med. Chem., 1999, 42, 4909) (1.5 eq) followed by cesium fluoride (1.2 eq). The reaction mixture is stirred at room temperature overnight. The reaction mixture is diluted with ethyl acetate, transferred to a separatory funnel, and washed with water. The organic phase is dried over sodium sulfate, filtered and concentrated. The residue is purified by flash chromatography to afford compound 7.

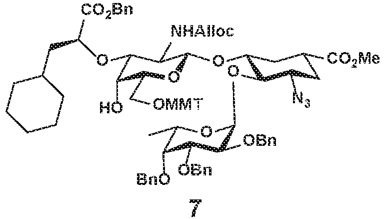

**Compound 8:** To a degassed solution of compound **7** in anhydrous DCM at 0 °C is added Pd(PPh₃)₄ (0.1 eq), Bu₃SnH (1.1 eq) and N-trifluoroacetyl glycine anhydride (2.0 eq) (preparation described in Chemische Berichte (1955), 88(1), 26). The resulting solution is stirred for 12 hrs allowing the temperature to increase to room temperature. The reaction mixture is diluted with DCM, transferred to a separatory funnel, and washed with water. The organic phase is dried over Na₂SO₄, then filtered and concentrated. The residue is purified by flash chromatography to afford compound **8.**

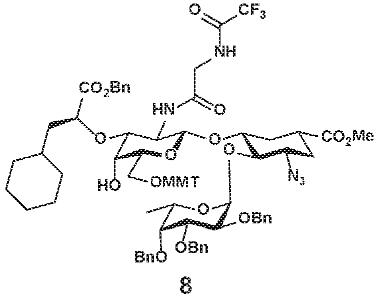

**Compound 9:** To a stirred solution of compound **8** in DCM/MeOH (25/1) at room temperature is added orotic acid chloride (5 eq) and triphenylphosphine (5 eq). The reaction mixture is stirred 24 hours. The solvent is removed and the residue is separated by column chromatography to afford compound **9.**

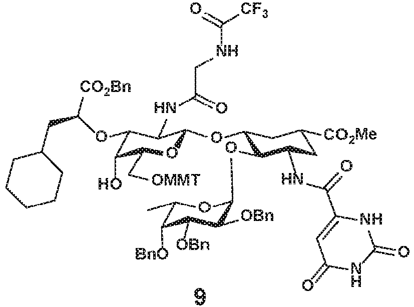

**Compound 10:** Compound **9** is dissolved in methanol and degassed. To this solution is added Pd(OH)₂/C. The reaction mixture is vigorously stirred under a hydrogen atmosphere for 12 hours. The reaction mixture is filtered through a Celite pad. The filtrate is concentrated under reduced pressure to give compound **10.**

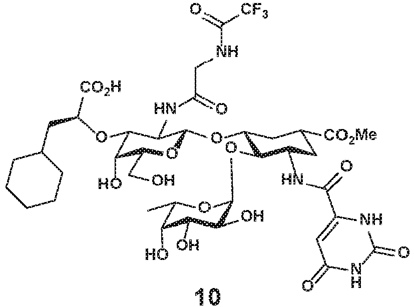

**Compound 11:** Compound **10** is dissolved in methanol at room temperature. A solution of sodium methoxide in methanol (1.1 eq) is added and the reaction mixture stirred overnight at room temperature. The reaction mixture is quenched by the addition of acetic acid. The reaction mixture is concentrated. The residue is separated by C-18 reverse phase chromatography to afford compound **11.**

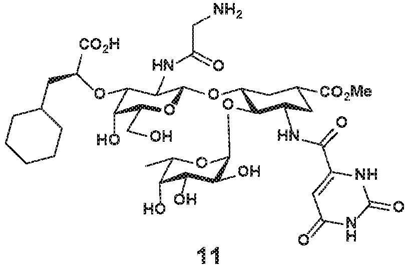

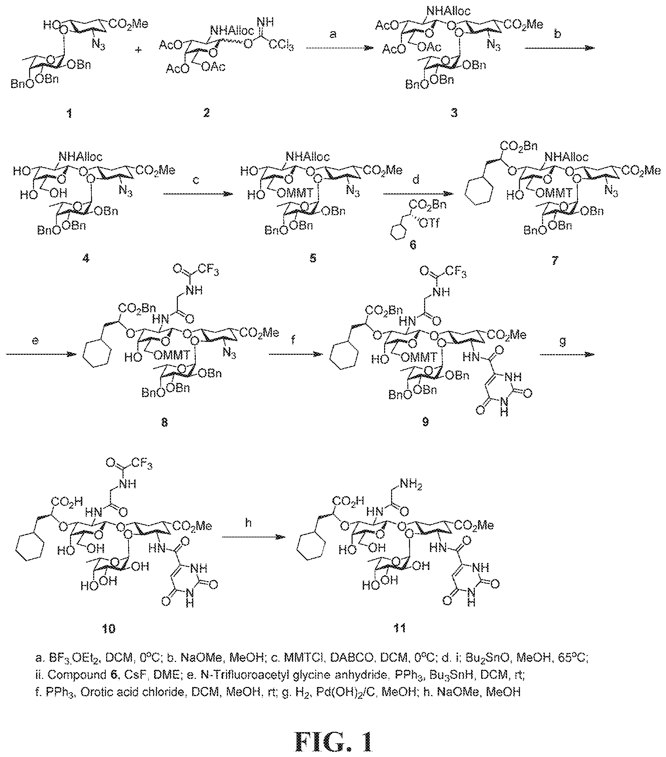

**Compound 12:** Compound **12** can be prepared in an analogous fashion to Figure 1 by substituting (acetylthio)acetyl chloride for N-trifluoroacetyl glycine anhydride in step e.

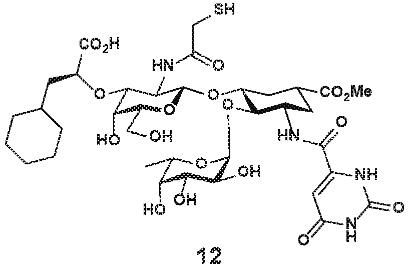

**Compound 13:** Compound **10** is dissolved in DMF and cooled on an ice bath. Diisopropylethylamine (1.5 eq) is added followed by HATU (1.1 eq). The reaction mixture is stirred 15 minutes on the ice bath then azetidine (2 eq) is added. The ice bath is removed and the reaction mixture is stirred overnight at room temperature. The solvent is removed under reduced pressure and the residue is separated by flash chromatography to afford compound **13.**

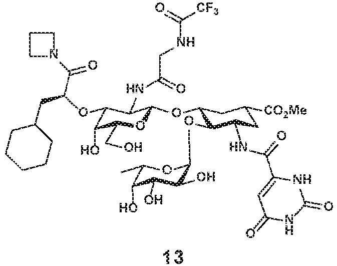

**Compound 14:** Compound **13** is dissolved in methanol at room temperature. A solution of sodium methoxide in methanol (0.3 eq) is added and the reaction mixture stirred overnight at room temperature. The reaction mixture is quenched by the addition of acetic acid. The reaction mixture is concentrated. The residue is separated by C-18 reverse phase chromatography to afford compound **14.**

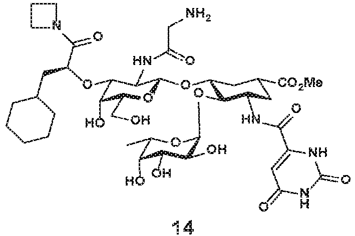

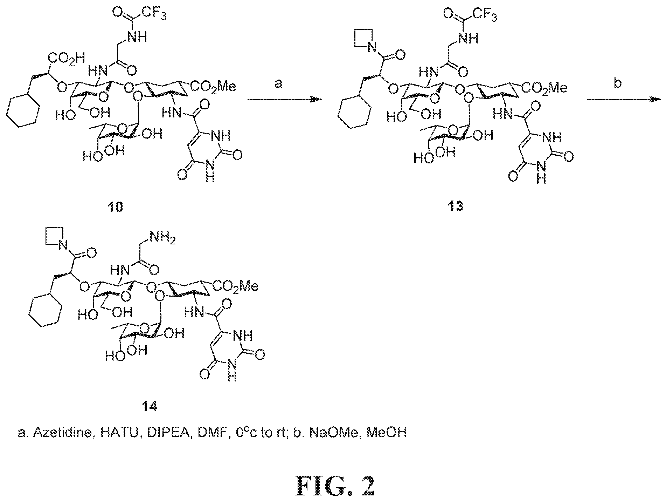

**Compound 15:** Compound **15** can be prepared in an analogous fashion to Figure 2 by using methylamine in place of azetidine in step a.

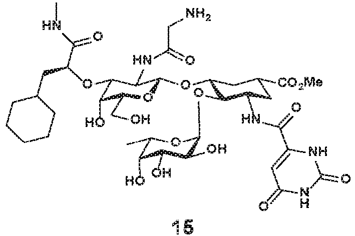

**Compound 16:** Compound **16** can be prepared in an analogous fashion to Figure 2 by using dimethylamine in place of azetidine in step a.

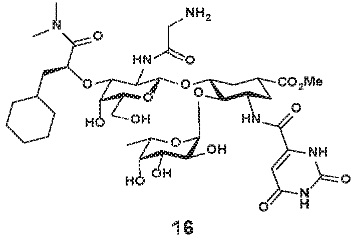

**Compound 17:** Compound **17** can be prepared in an analogous fashion to Figure 2 by using 2-methoxyethylamine in place of azetidine in step a.

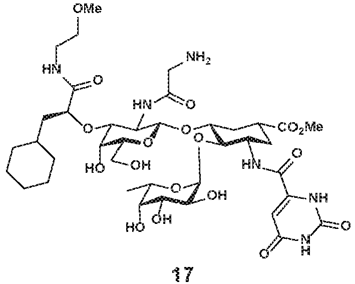

**Compound 18:** Compound **18** can be prepared in an analogous fashion to Figure 2 by using piperidine in place of azetidine in step a.

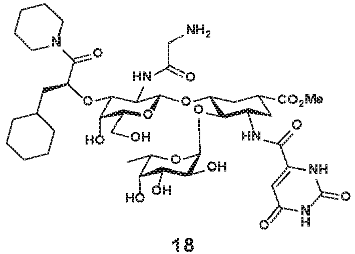

**Compound 19:** Compound **19** can be prepared in an analogous fashion to Figure 2 by using morpholine in place of azetidine in step a.

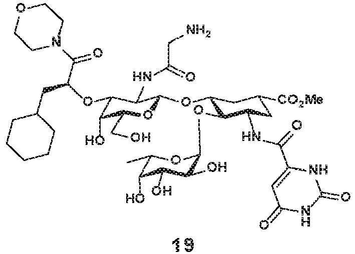

**Compound 21:** A solution of compound **20** (0.4 eq) in DMSO is added to a solution of compound **11** (1 eq) and DIPEA (10 eq) in anhydrous DMSO at room temperature. The resulting solution is stirred overnight. The solution is dialyzed against distilled water for 3 days with dialysis tube MWCO 1000 while distilled water is changed every 12 hours. The solution in the tube is lyophilized to give compound **21.**

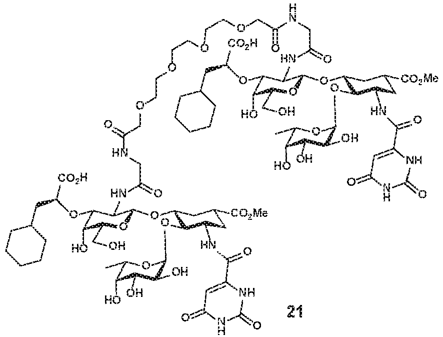

### EXAMPLE 2

### PROPHETIC SYNTHESIS OF MULTIMERIC COMPOUND 22

**Compound 22:** A solution of compound **21** in ethylenediamine is stirred overnight at 70 °C. The reaction mixture is concentrated under reduced pressure and the residue is purified by reverse phase chromatography to give compound **22.**

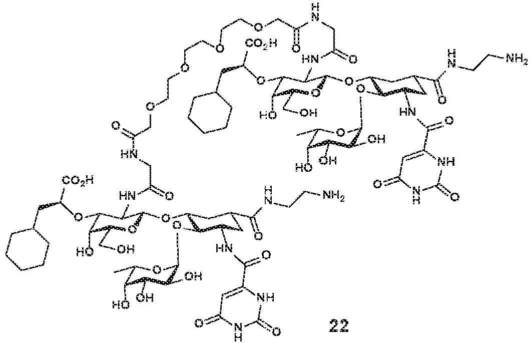

### EXAMPLE 3

### PROPHETIC SYNTHESIS OF MULTIMERIC COMPOUND 23

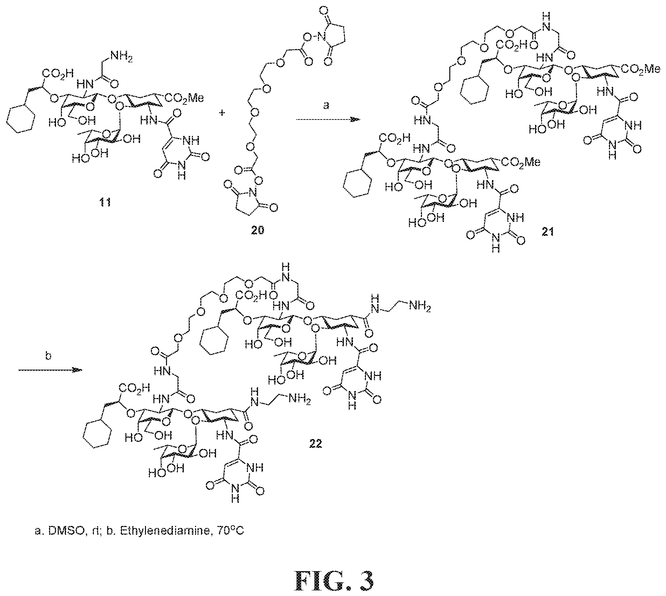

**Compound 23:** Compound **23** can be prepared in an analogous fashion to Figure 3 by replacing compound **20** with PEG-11 diacetic acid di-NHS ester in step a.

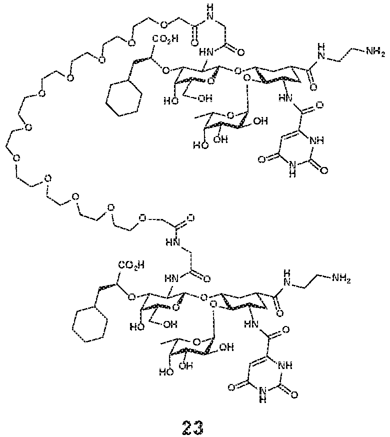

### EXAMPLE 4

### PROPHETIC SYNTHESIS OF MULTIMERIC COMPOUND 24

**Compound 24:** Compound **24** can be prepared in an analogous fashion to Figure 3 by replacing compound **20** with PEG-15 diacetic acid di-NHS ester in step a.

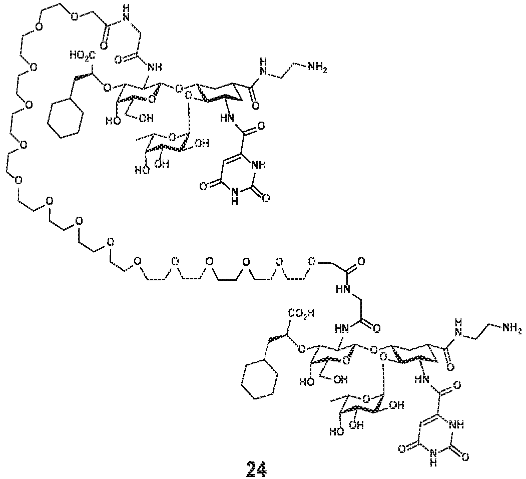

### EXAMPLE 5

### PROPHETIC SYNTHESIS OF MULTIMERIC COMPOUND 25

**Compound 25:** Compound **25** can be prepared in an analogous fashion to Figure 3 by replacing compound **20** with ethylene glycol diacetic acid di-NHS ester in step a.

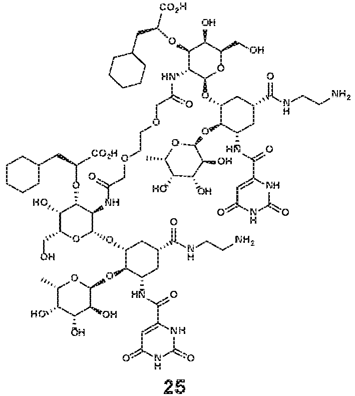

### EXAMPLE 6

### PROPHETIC SYNTHESIS OF MULTIMERIC COMPOUND 26

**Compound 26:** Compound **26** can be prepared in an analogous fashion to Figure 3 by replacing compound **20** with 3,3'-[[2,2-bis[[3-[(2,5-dioxo-1-pyrrolidinyl)oxy]-3-oxopropoxy]methyl]-1,3-propanediyl]bis(oxy)]bis-, 1,1'-bis(2,5-dioxo-1-pyrrolidinyl)-propanoic acid ester in step a.

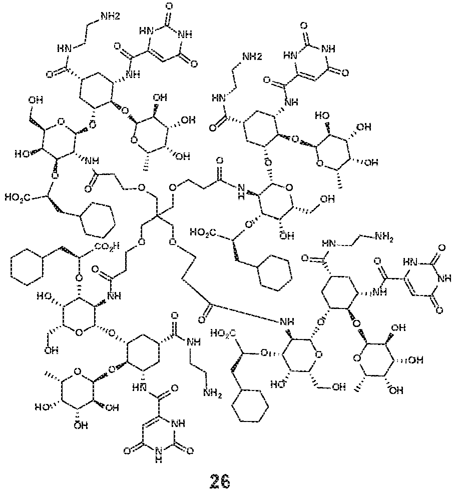

### EXAMPLE 7

### PROPHETIC SYNTHESIS OF MULTIMERIC COMPOUND 27

**Compound 27:** Compound **27** can be prepared in an analogous fashion to Figure 3 by replacing ethylenediamine with 2-aminoethyl ether in step b.

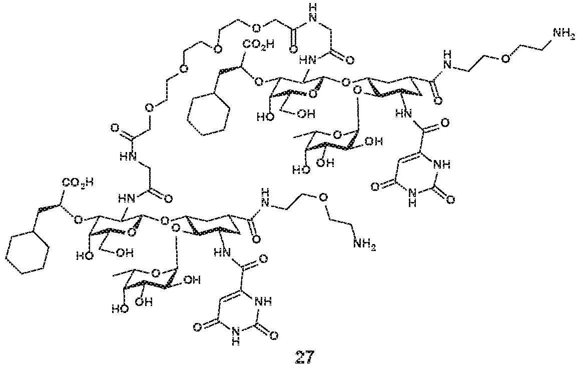

### EXAMPLE 8

### PROPHETIC SYNTHESIS OF MULTIMERIC COMPOUND 28

**Compound 28:** Compound **28** can be prepared in an analogous fashion to Figure 3 by replacing ethylenediamine with 1,5-diaminopentane in step b.

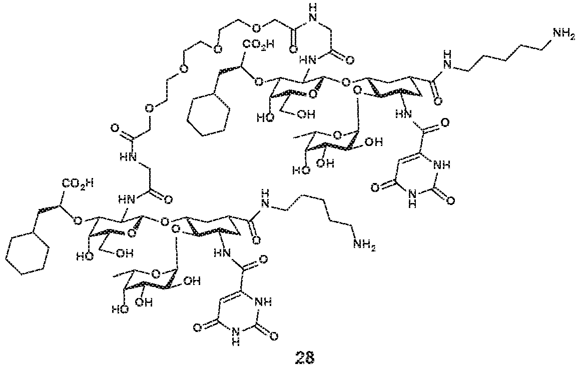

### EXAMPLE 9

### PROPHETIC SYNTHESIS OF MULTIMERIC COMPOUND 29

**Compound 29:** Compound **29** can be prepared in an analogous fashion to Figure 3 by replacing ethylenediamine with 1,2-bis(2-aminoethoxy)ethane in step b.

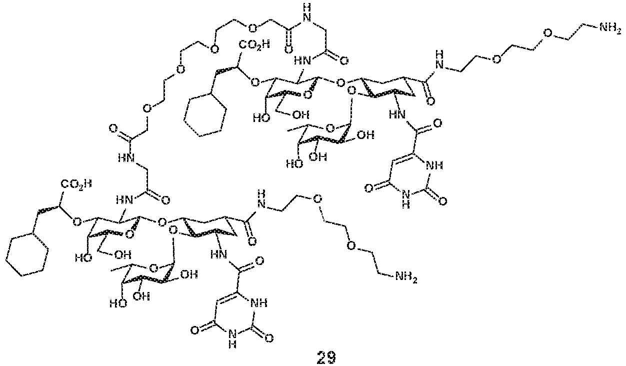

### EXAMPLE 10

### PROPHETIC SYNTHESIS OF MULTIMERIC COMPOUND 30

**Compound 30:** Compound **30** can be prepared in an analogous fashion to Figure 3 by replacing compound **11** with compound **14** and compound **20** with PEG-11 diacetic acid di-NHS ester in step a.

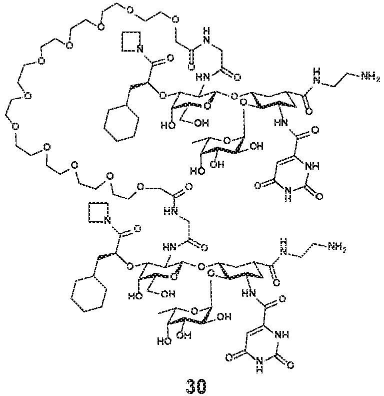

### EXAMPLE 11

### PROPHETIC SYNTHESIS OF MULTIMERIC COMPOUND 31

**Compound 31:** Compound **31** can be prepared in an analogous fashion to Figure 3 by replacing compound **11** with compound **15** in step a.

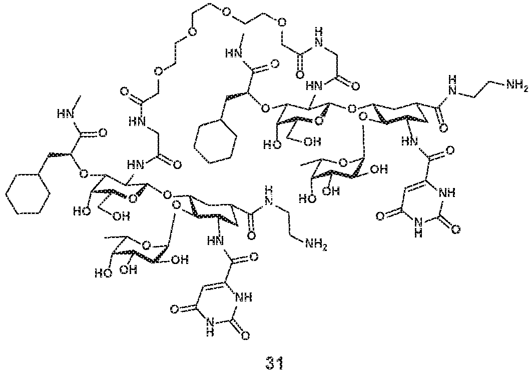

### EXAMPLE 12

### PROPHETIC SYNTHESIS OF MULTIMERIC COMPOUND 32

**Compound 32: Compound 32** can be prepared in an analogous fashion to Figure 3 by replacing compound **11** with compound **17** and compound **20** with PEG-15 diacetic acid di-NHS ester in step a.

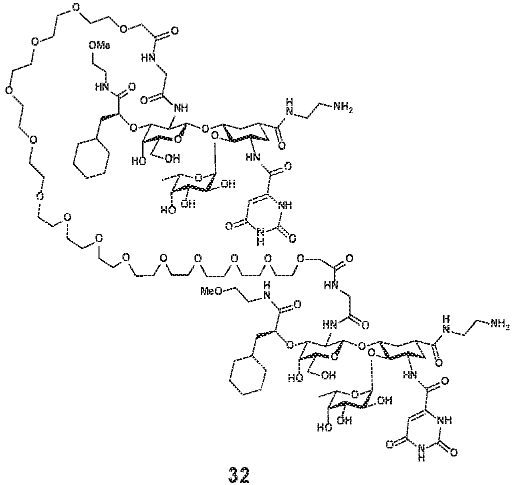

### EXAMPLE 13

### PROPHETIC SYNTHESIS OF MULTIMERIC COMPOUND 33

**Compound 33:** Compound **33** can be prepared in an analogous fashion to Figure 3 by replacing compound **11** with compound **16** and compound **20** with ethylene glycol diacetic acid di-NHS ester in step a.

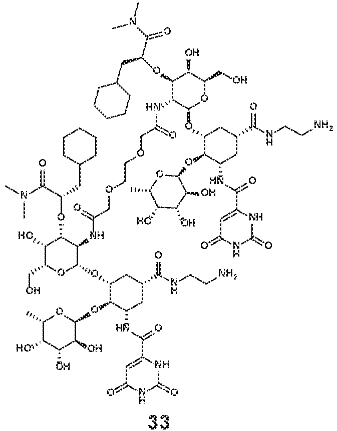

### EXAMPLE 14

### PROPHETIC SYNTHESIS OF MULTIMERIC COMPOUND 24

**Compound 34:** Compound **34** can be prepared in an analogous fashion to Figure 3 by replacing compound **11** with compound **18** in step a and replacing ethylenediamine with 2-aminoethyl ether in step b.

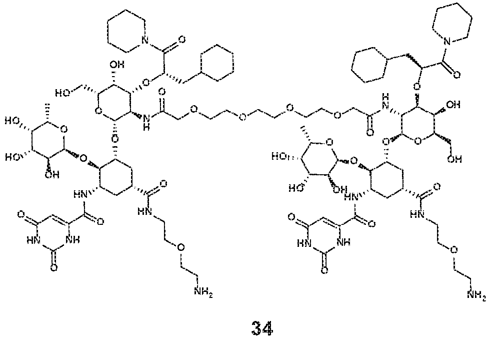

### EXAMPLE 15

### PROPHETIC SYNTHESIS OF MULTIMERIC COMPOUND 36

**Compound 36:** To a solution of compound **12** in MeOH at room temperature is added compound **35** followed by cesium acetate (2.5 eq). The reaction mixture is stirred at room temperature until completion. The solvent is removed under reduced pressure. The product is purified by reverse phase chromatography to give compound **36.**

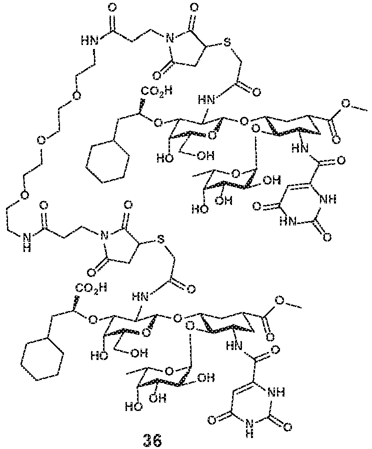

### EXAMPLE 16

### PROPHETIC SYNTHESIS OF MULTIMERIC COMPOUND 37

**Compound 37:** Compound **36** is dissolved in ethylenediamine and the reaction mixture is stirred overnight at 70 °C. The reaction mixture is concentrated under reduced pressure and the residue is purified by reverse phase chromatography to give compound **37.**

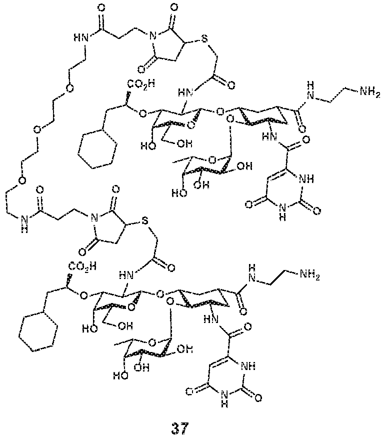

### EXAMPLE 17

### PROPHETIC SYNTHESIS OF MULTIMERIC COMPOUND 38

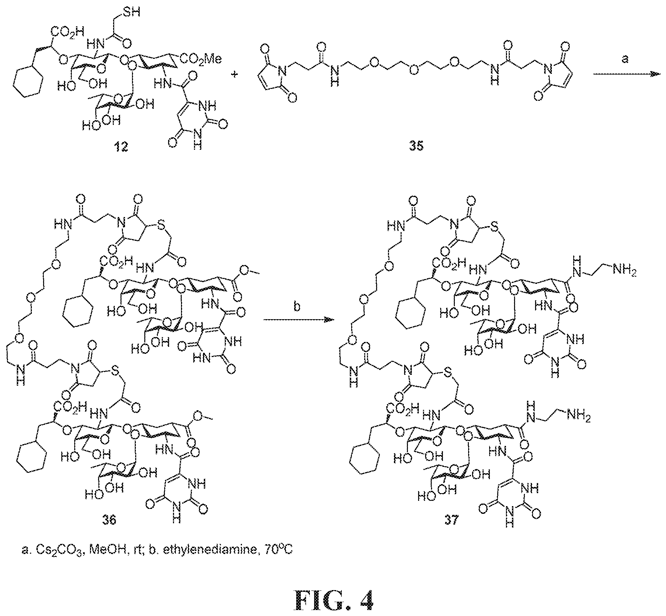

**Compound 38:** Compound **38** can be prepared in an analogous fashion to Figure 4 by substituting PEG-6-bis maleimidoylpropionamide for compound **35** in step a.

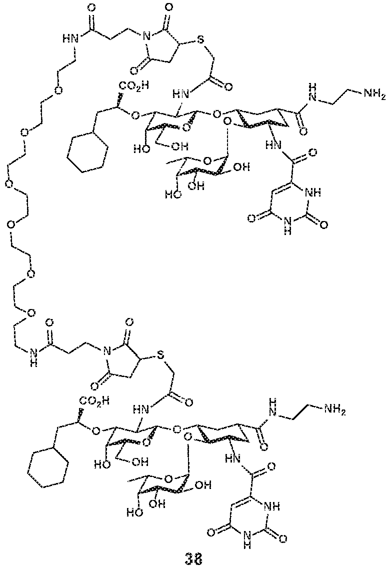

### EXAMPLE 18

### PROPHETIC SYNTHESIS OF MULTIMERIC COMPOUND 39

**Compound 39:** Compound **39** can be prepared in an analogous fashion to Figure 4 by substituting compound 35 for, 1,1'-[[2,2-bis[[3-(2,5-dihydro-2,5-dioxo-1H-pyrrol-1-yl) propoxy]methyl]-1,3-propanediyl]bis(oxy-3,1-propanediyl)]bis-1*H*- one in step a.

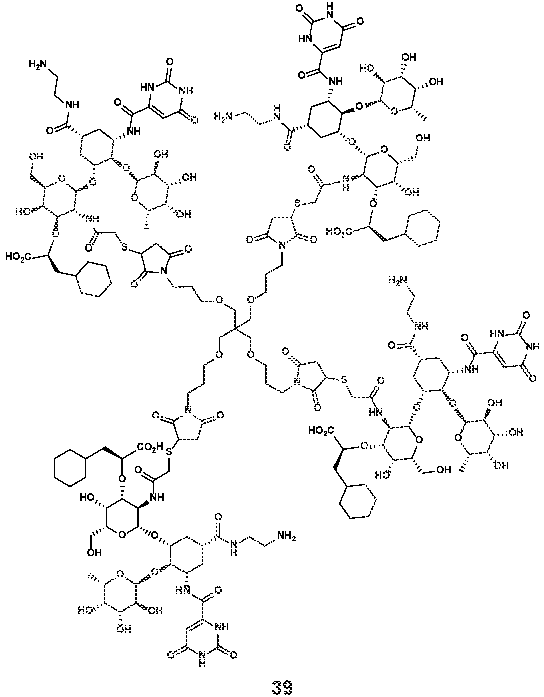

### EXAMPLE 19

### PROPHETIC SYNTHESIS OF MULTIMERIC COMPOUND 40

**Compound 40:** Compound **40** can be prepared in an analogous fashion to Figure 4 by substituting propylenediamine for ethylenediamine in step b.

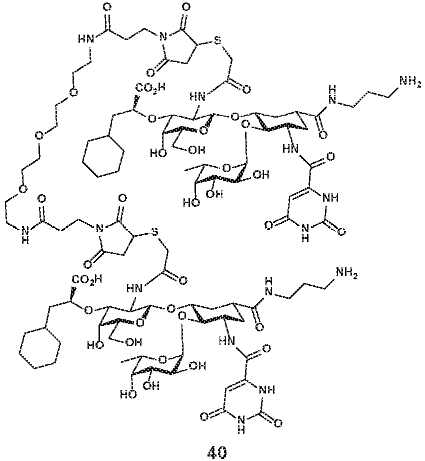

### EXAMPLE 20

### PROPHETIC SYNTHESIS OF MULTIMERIC COMPOUND 44

**Compound 41:** To a stirred solution of compound 7 in DCM/MeOH (25/1) at room temperature is added orotic acid chloride (5 eq) and triphenylphosphine (5 eq). The reaction mixture is stirred 24 hours. The solvent is removed and the residue is separated by column chromatography to afford compound **41.**

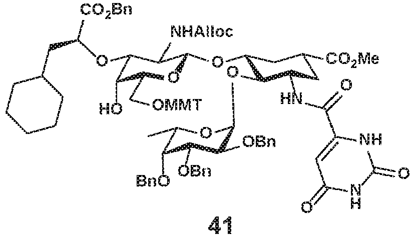

**Compound 42:** To a degassed solution of compound **41** in anhydrous DCM at 0 °C is added Pd(PPh₃)₄ (0.1 eq), Bu₃SnH (1.1 eq) and azidoacetic anhydride (2.0 eq). The ice bath is removed and the solution is stirred for 12 hrs under a N₂ atmosphere at room temperature. The reaction mixture is diluted with DCM, washed with water, dried over Na₂SO₄, then concentrated. The crude product is purified by column chromatography to give compound **42.**

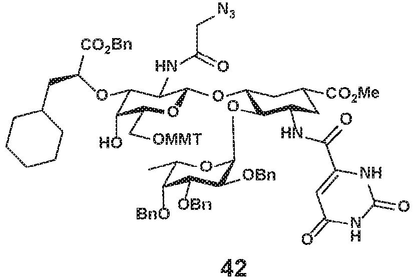

**Compound 44:** A solution of bispropagyl PEG-5 (compound 43) and compound **42** (2.4 eq) in MeOH is degassed at room temperature. A solution of CuSO₄/THPTA in distilled water (0.04 M) (0.2 eq) and sodium ascorbate (0.2 eq) are added successively and the resulting solution is stirred 12 hrs at 70 °C. The solution is cooled to room temperature and concentrated under reduced pressure. The crude product is purified by chromatography to give compound **44.**

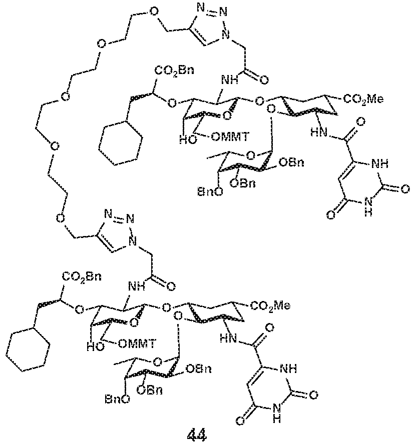

### EXAMPLE 21

### PROPHETIC SYNTHESIS OF MULTIMERIC COMPOUND 45

**Compound 45:** Compound **44** is dissolved in MeOH/*i*-PrOH (2/1) and hydrogenated in the presence of Pd(OH)₂ (20 wt %) at 1 atm of H₂ gas pressure for 24 hrs at room temperature. The solution is filtered through a Celite pad. The filtrate is concentrated to give compound 45.

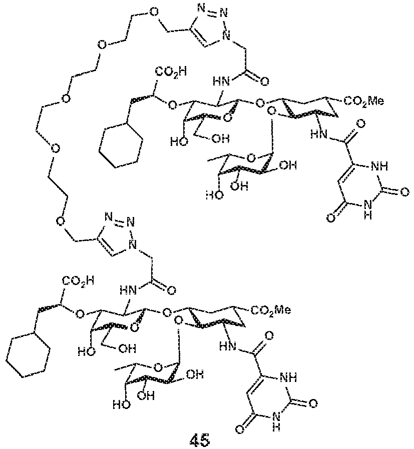

### EXAMPLE 22

### PROPHETIC SYNTHESIS OF MULTIMERIC COMPOUND 46

**Compound 46:** Compound **45** is dissolved in ethylenediamine and stirred for 12 hrs at 70 °C. The reaction mixture is concentrated under reduced pressure. The crude product is purified by C-18 column chromatography followed by lyophilization to give a compound **46.**

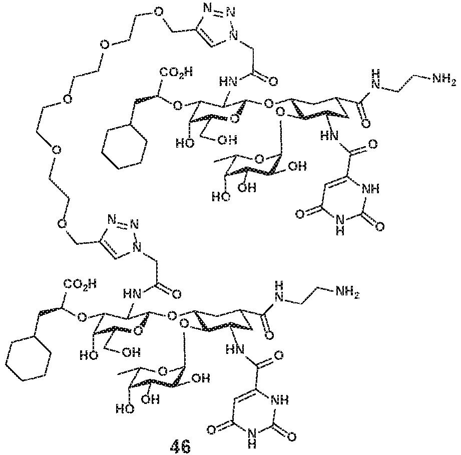

### EXAMPLE 23

### PROPHETIC SYNTHESIS OF MULTIMERIC COMPOUND 47

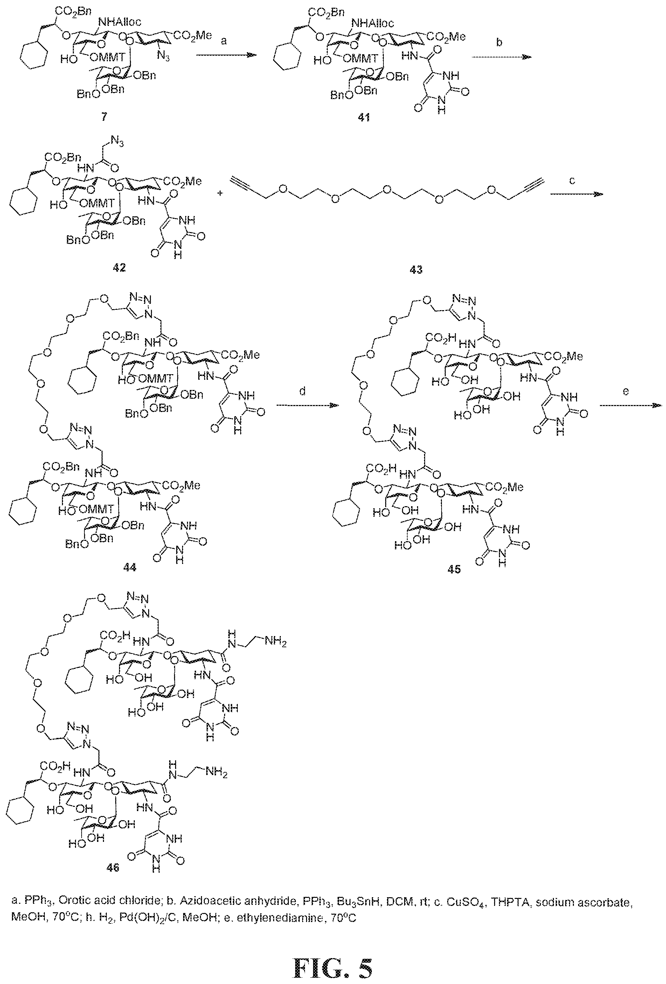

**Compound 47:** Compound **47** can be prepared in an analogous fashion to Figure 5 using 3-azidopropanoic anhydride (Yang, C. et. al. JACS, (2013) 135(21), 7791-7794) in place of azidoacetic anhydride in step b.

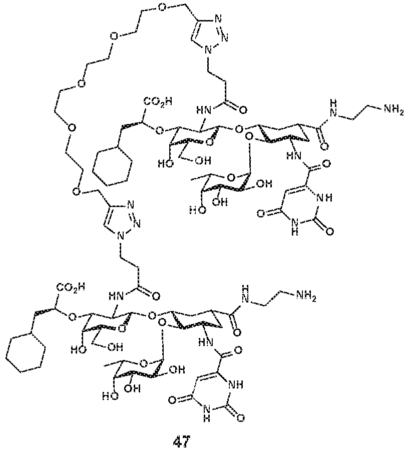

### EXAMPLE 24

### PROPHETIC SYNTHESIS OF MULTIMERIC COMPOUND 48

**Compound 48:** Compound **48** can be prepared in an analogous fashion to Figure 5 using 4-azidobutanoic anhydride (Yang, C. et. al. JACS, (2013) 135(21), 7791-7794) in place of azidoacetic anhydride in step b.

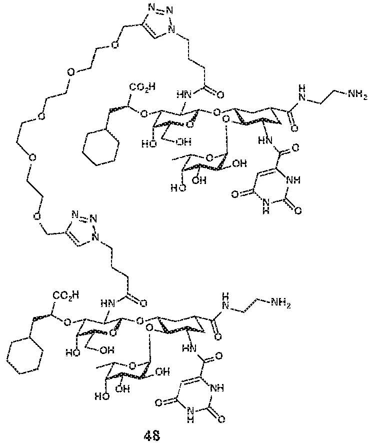

### EXAMPLE 25

### PROPHETIC SYNTHESIS OF MULTIMERIC COMPOUND 49

**Compound 49:** Compound **49** can be prepared in an analogous fashion to Figure 5 using 4-azidobutanoic anhydride (Yang, C. et. al. JACS, (2013) 135(21), 7791-7794) in place of azidoacetic anhydride in step b and using 1,2-bis(2-propynyloxy) ethane in place of compound **43** in step c.

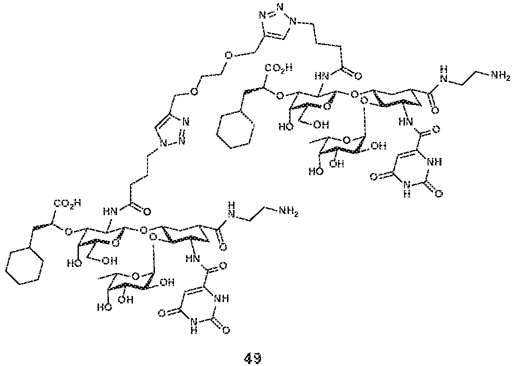

### EXAMPLE 26

### PROPHETIC SYNTHESIS OF MULTIMERIC COMPOUND 50

**Compound 50:** Compound **50** can be prepared in an analogous fashion to Figure 5 using 4,7,10,13,16, 19,22,25,28,31-decaoxatetratriaconta-1, 33-diyne in place of compound **43** in step c.

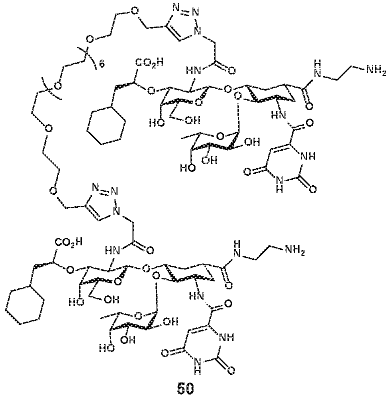

### EXAMPLE 27

### PROPHETIC SYNTHESIS OF MULTIMERIC COMPOUND 51

**Compound 51:** Compound **51** can be prepared in an analogous fashion to Figure 5 using 3,3'-[[2,2-bis[(2-propyn-1-yloxy)methyl]-1,3-propanediyl]bis(oxy)]bis-1-propyne in place of compound **43** in step c.

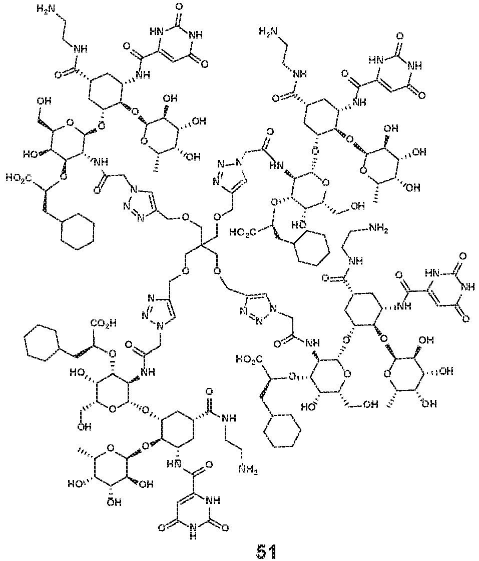

### EXAMPLE 28

### PROPHETIC SYNTHESIS OF MULTIMERIC COMPOUND 52

**Compound 52:** Compound **52** can be prepared in an analogous fashion to Figure 5 using 3,3'-[oxybis[[2,2-bis[(2-propyn-1-yloxy)methyl]-3,1-propanediyl]oxy]]bis-1-propyne in place of compound **43** in step c.

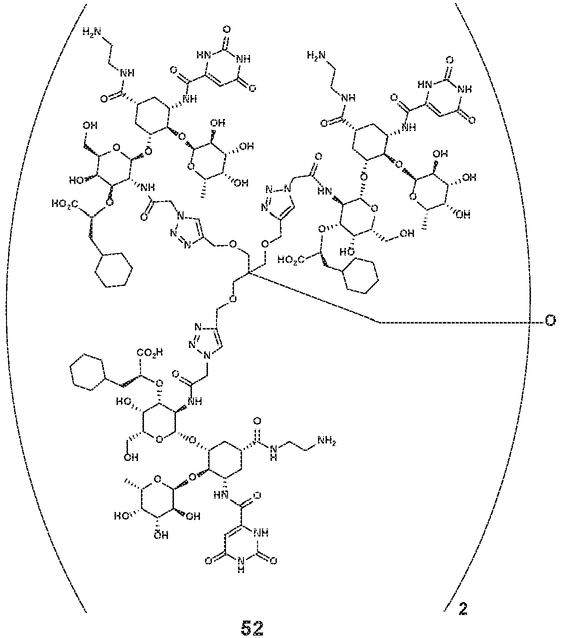

### EXAMPLE 29

### PROPHETIC SYNTHESIS OF MULTIMERIC COMPOUND 53

**Compound 53:** Compound **53** can be prepared in an analogous fashion to Figure 5 using butylenediamine in place of ethylenediamine in step e.

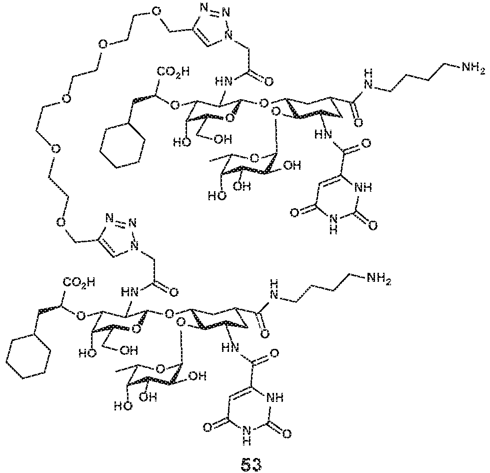

### EXAMPLE 30

### PROPHETIC SYNTHESIS OF MULTIMERIC COMPOUND 54

**Compound 54:** Compound **54** can be prepared in an analogous fashion to Figure 5 using 4-azidobutanoic anhydride (Yang, C. et. al. JACS, (2013) 135(21), 7791-7794) in place of azidoacetic anhydride in step b and using 1,2-bis(2-propynyloxy) ethane in place of compound **43** in step c and using 2-aminoethyl ether in step e.

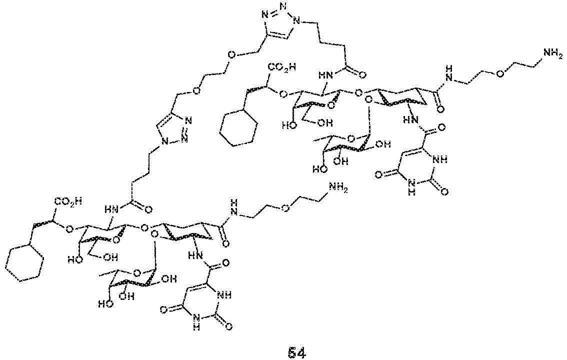

### EXAMPLE 31

### PROPHETIC SYNTHESIS OF MULTIMERIC COMPOUND 55

**Compound 55: Compound 54** is dissolved in DMF and cooled on an ice bath. Diisopropylethylamine (2.5 eq) is added followed by HATU (2.2 eq). The reaction mixture is stirred 15 minutes on the ice bath then azetidine (10 eq) is added. The ice bath is removed and the reaction mixture is stirred overnight at room temperature. The solvent is removed under reduced pressure and the residue is separated by flash chromatography to afford compound **55.**

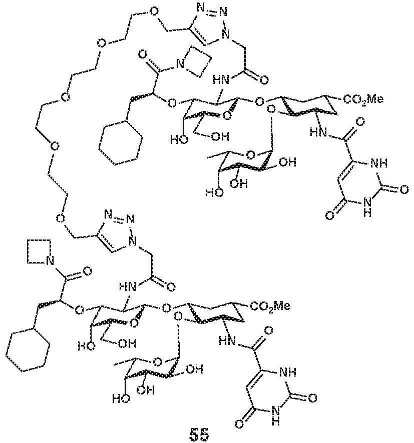

### EXAMPLE 32

### PROPHETIC SYNTHESIS OF MULTIMERIC COMPOUND 56

**Compound 56:** Compound **55** is dissolved in ethylenediamine and stirred for 12 hrs at 70 °C. The reaction mixture is concentrated under reduced pressure. The crude product is purified by C-18 column chromatography followed by lyophilization to give a compound **56.**

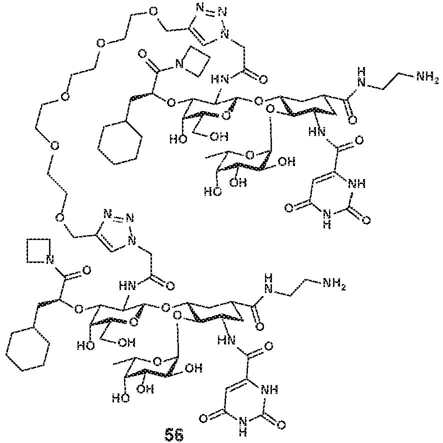

### EXAMPLE 33

### PROPHETIC SYNTHESIS OF MULTIMERIC COMPOUND 57

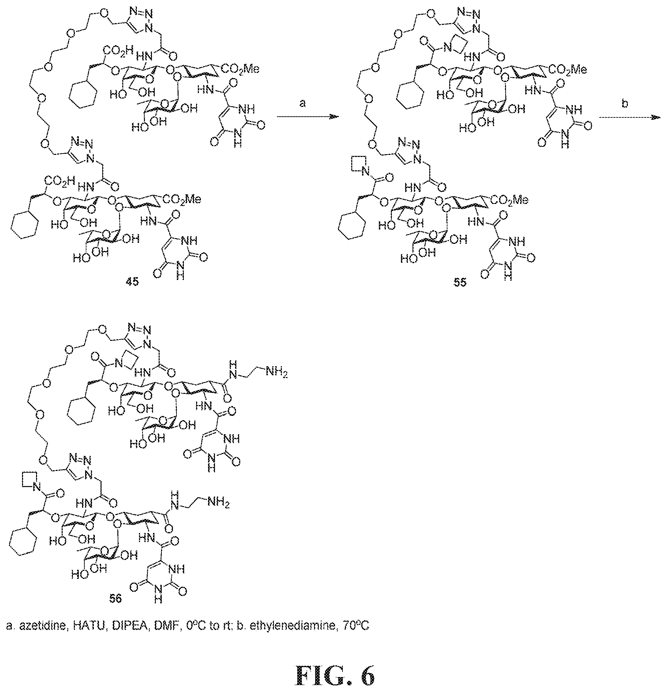

**Compound 57:** Compound **57** can be prepared in an analogous fashion to Figure 6 using ethylamine in place of azetidine in step a.

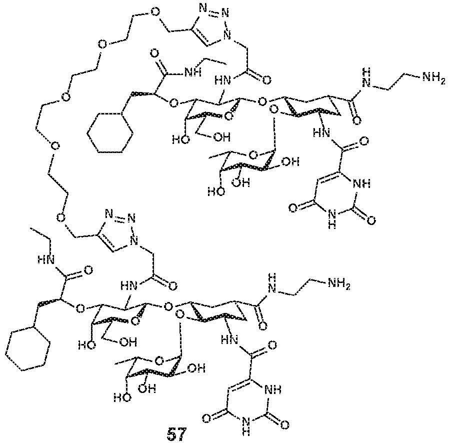

### EXAMPLE 34

### PROPHETIC SYNTHESIS OF MULTIMERIC COMPOUND 58

**Compound 58:** Compound **58** can be prepared in an analogous fashion to Figure 6 using dimethylamine in place of azetidine in step a.

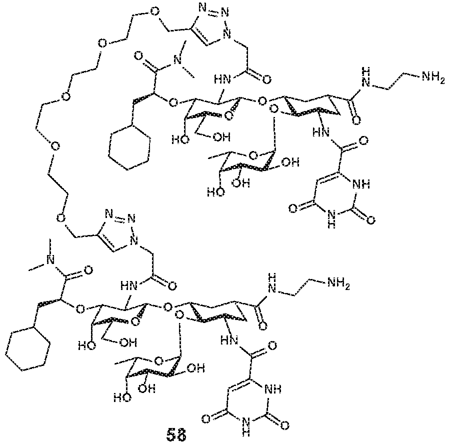

### EXAMPLE 35

### PROPHETIC SYNTHESIS OF MULTIMERIC COMPOUND 59

**Compound 59:** Compound **59** can be prepared in an analogous fashion to Figure 6 using 1,2 bis(2-aminoethoxy)ethane in place of ethylenediamine in step b.

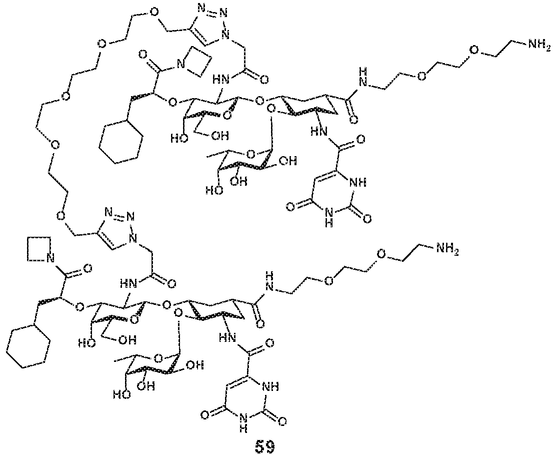

### EXAMPLE 36

### PROPHETIC SYNTHESIS OF MULTIMERIC COMPOUND 66

**Compound 60:** To a stirred solution of compound 1 in DCM/MeOH (25/1) at room temperature is added orotic acid chloride (5 eq) and triphenylphosphine (5 eq). The reaction mixture is stirred 24 hours. The solvent is removed and the residue is separated by column chromatography to afford compound **60.**

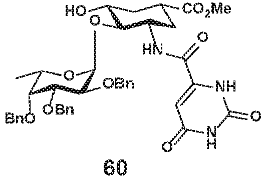

**Compound 62: Compound 61** is dissolved in acetonitrile at room temperature. Benzaldehyde dimethylacetal (1.1 eq) is added followed by camphorsulfonic acid (0.2 eq). The reaction mixture is stirred until completion. Triethylamine is added. The solvent is removed and the residue separated by flash chromatography to afford compound **62.**

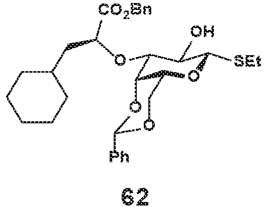

**Compound 63:** Compound **62** is dissolved in pyridine at room temperature. Dimethylaminopyridine (.01 eq) is added followed by chloroacetyl chloride (2 eq). The reaction mixture is stirred until completion. The solvent is removed under educed pressure. The residue is dissolved in ethyl acetate, transferred to a separatory funnel and washed two times with 0.1N HCl and two times with water. The organic phase is dried over sodium sulfate, filtered, and concentrated. The residue is separated by column chromatograph to afford compound **63.**

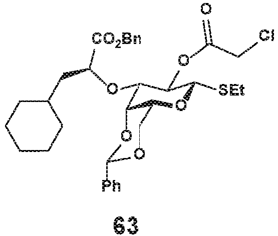

**Compound 64:** Activated powdered 4Å molecular sieves are added to a solution of compound **60** and compound **63** (2 eq) in dry DCM under argon. The mixture is stirred for 2 hours at room temperature. Solid DMTST (1.5 eq) is added in 4 portions over 1.5 hours. The reaction mixture is stirred overnight at room temperature. The reaction mixture is filtered through Celite, transferred to a separatory funnel and washed two times with half saturated sodium bicarbonate and two times with water. The organic phase is dried over sodium sulfate, filtered and concentrated. The residue is separated by flash chromatography to afford compound **64.**

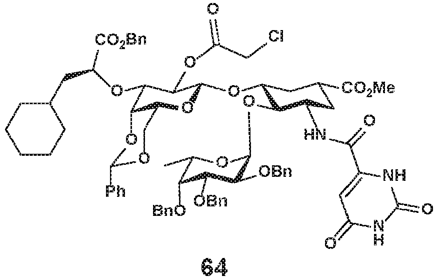

**Compound 65:** Compound **64** is dissolved in DMF. Sodium azide (1.5 eq) is added and the reaction mixture is stirred at 50°C until completion. The reaction mixture is cooled to room temperature, diluted with ethyl acetate and transferred to a separatory funnel. The organic phase is washed 4 times with water then dried over sodium sulfate and concentrated. The residue is separated by column chromatography to afford compound **65.**

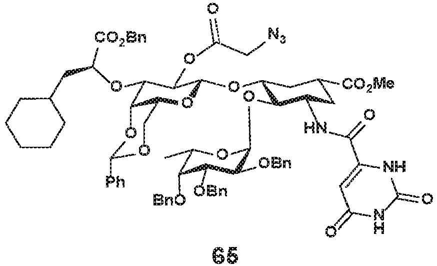

**Compound 66:** A solution of bispropagyl PEG-5 (compound **43**) and compound 65 (2.4 eq) in MeOH is degassed at room temperature. A solution of CuSO₄/THPTA in distilled water (0.04 M) (0.2 eq) and sodium ascorbate (0.2 eq) are added successively and the resulting solution is stirred 12 hrs at 50°C. The solution is concentrated under reduced pressure. The crude product is purified by chromatography to give a compound 66.

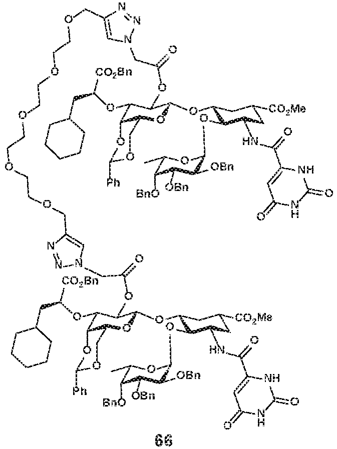

### EXAMPLE 37

### PROPHETIC SYNTHESIS OF MULTIMERIC COMPOUND 67

**Compound 67:** To a solution of compound **66** in dioxane/water (4/1) is added Pd(OH)₂/C. The reaction mixture is stirred vigorously overnight under a hydrogen atmosphere. The reaction mixture is filtered through Celite and concentrated. The residue is purified by C-19 reverse phase column chromatography to afford compound **67.**

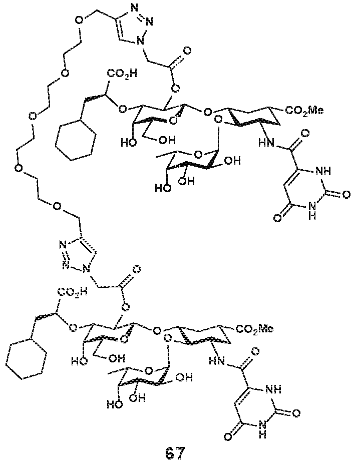

### EXAMPLE 38

### PROPHETIC SYNTHESIS OF MULTIMERIC COMPOUND 68

**Compound 68:** Compound **67** is dissolved in ethylenediamine and stirred for 12 hrs at 70 °C. The reaction mixture is concentrated under reduced pressure. The crude product is purified by C-18 column chromatography followed by lyophilization to afford compound **68.**

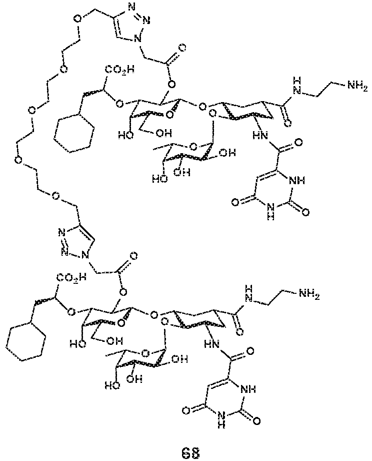

### EXAMPLE 39

### PROPHETIC SYNTHESIS OF MULTIMERIC COMPOUND 69

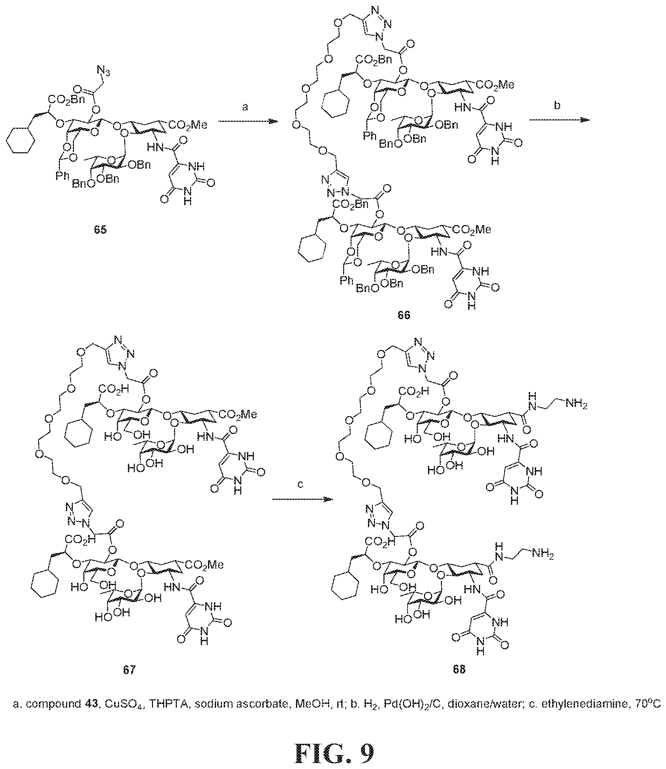

**Compound 69:** Compound **69** can be prepared in an analogous fashion to Figure 9 by replacing compound **43** with PEG-8 bis propargyl ether in step a.

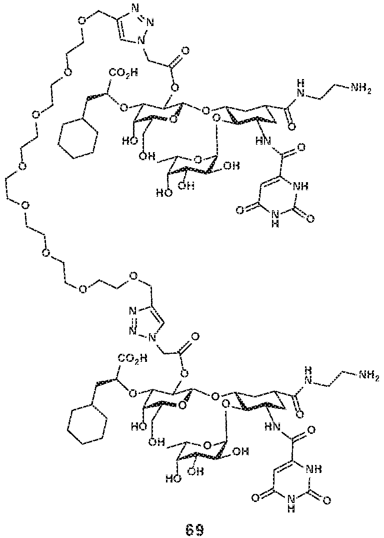

### EXAMPLE 40

### PROPHETIC SYNTHESIS OF MULTIMERIC COMPOUND 70

**Compound 70:** Compound 70 can be prepared in an analogous fashion to Figure 9 by replacing compound **43** with ethylene glycol bis propargyl ether in step a.

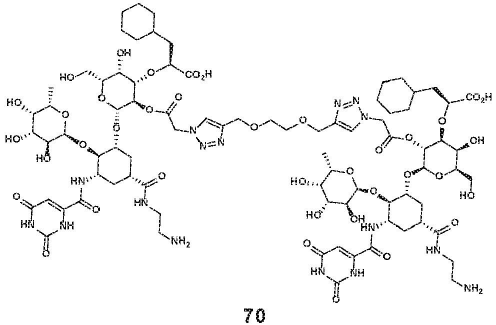

### EXAMPLE 41

### PROPHETIC SYNTHESIS OF MULTIMERIC COMPOUND 71

**Compound 71:** Compound **71** can be prepared in an analogous fashion to Figure 9 using 3,3'-[[2,2-bis[(2-propyn-1-yloxy)methyl]-1,3-propanediyl]bis(oxy)bis-1-propyne in place of compound **43** in step a.

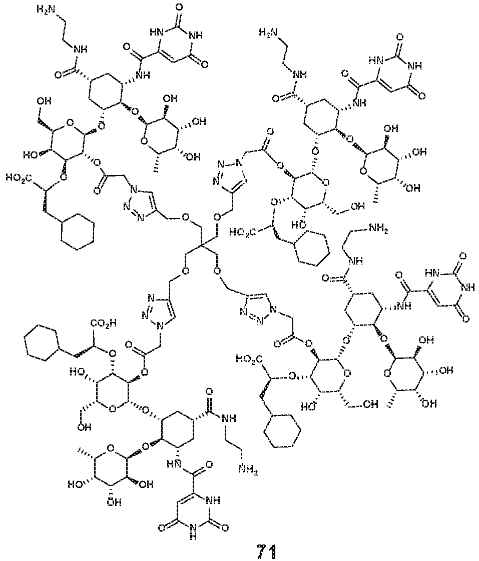

### EXAMPLE 42

### PROPHETIC SYNTHESIS OF MULTIMERIC COMPOUND 72

**Compound 72:** Compound **67** is dissolved in DMF and cooled on an ice bath. Diisopropylethylamine (2.5 eq) is added followed by HATU (2.2 eq). The reaction mixture is stirred 15 minutes on the ice bath then azetidine (10 eq) is added. The ice bath is removed and the reaction mixture is stirred overnight at room temperature. The solvent is removed under reduced pressure and the residue is separated by flash chromatography to afford compound 72.

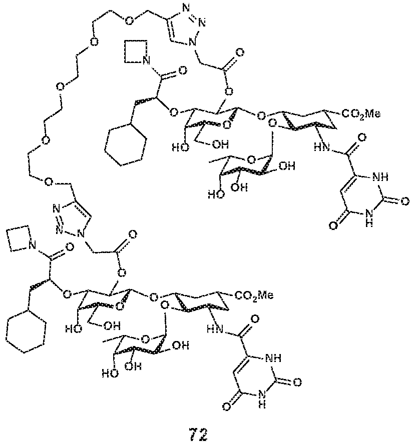

### EXAMPLE 43

### PROPHETIC SYNTHESIS OF MULTIMERIC COMPOUND 73

**Compound 73:** Compound **72** is dissolved in ethylenediamine and stirred for 12 hrs at 70 °C. The reaction mixture is concentrated under reduced pressure. The crude product is purified by C-18 column chromatography followed by lyophilization to afford compound **73.**

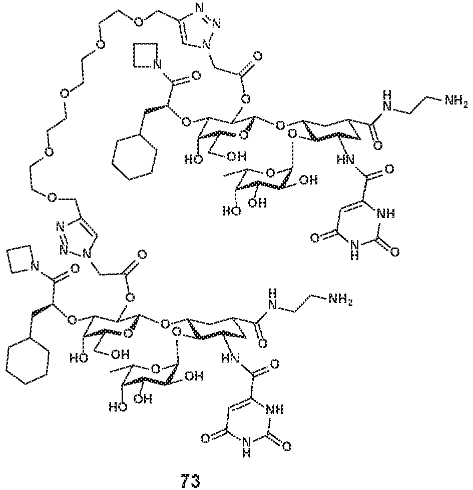

### EXAMPLE 44

### SYNTHESIS OF MULTIMERIC COMPOUND 76

**Compound 75:** To a degassed solution of compound **74** (synthesis described in WO 2013/096926) (0.5 g, 0.36 mmole) in anhydrous DCM (10 mL) at 0 °C was added Pd(PPh₃)₄ (42 mg, 36.3 µmole, 0.1 eq), Bu₃SnH (110 µL, 0.4 µmole, 1.1 eq) and azidoacetic anhydride (0.14 g, 0.73 mmole, 2.0 eq). The resulting solution was stirred for 12 hrs under N₂ atmosphere while temperature was gradually increased to room temperature. After the reaction was completed, the solution was diluted with DCM (20 mL), washed with distilled water, dried over Na₂SO₄, then concentrated. The crude product was purified by combi-flash (EtOAc/Hex, Hex only - 3/2, v/v) to give compound **75** (0.33 g, 67%). MS: Calculated (C₈₁H₉₅N₄O₁₆, 1376.6), ES-Positive (1400.4, M-1-Na)).

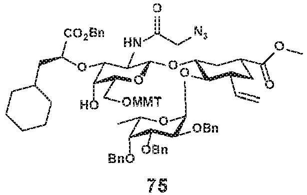

**Compound 76:** A solution of bispropargyl PEG-5 (compound **43,** 27 mg, 0.1 mmole) and compound **75** (0.33 g, 0.24 mmole, 2.4 eq) in a mixed solution (MeOH/1,4 dioxane, 2/1, v/v, 12 mL) was degassed at room temperature. A solution of CuSO₄/THPTA in distilled water (0.04 M) (0.5 mL, 20 µmole, 0.2 eq) and sodium ascorbate (4.0 mg, 20 µmole, 0.2 eq) were added successively and the resulting solution was stirred 12 hrs at 70 °C. The solution was cooled to room temperature and concentrated under reduced pressure. The crude product was purified by combi-flash (EtOAc/MeOH, EtOAc only - 4/1, v/v) to give a compound 76 as a white foam (0.23 g, 70%).

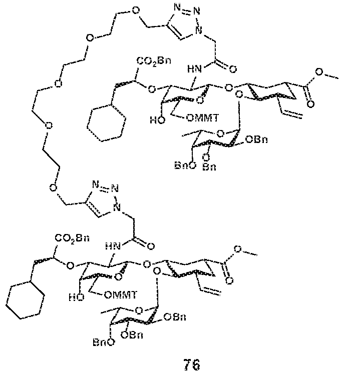

### EXAMPLE 45

### SYNTHESIS OF MULTIMERIC COMPOUND 77

**Compound 77:** A solution of compound **76** (0.23 g, 0.76 µmole) in solution of MeOH/*i*-PrOH (2/1, v/v, 12 mL) was hydrogenated in the presence of Pd(OH)₂ (0.2 g) and 1 atm of H₂ gas pressure for 24 hrs at room temperature. The solution was filtered through a Celite pad and the cake was washed with MeOH. The combined filtrate was concentrated under reduced pressure. The crude product was washed with hexane and dried under high vacuum to give compound **77** as a white solid (0.14 g, quantitative). MS: Calculated (C₈₀H₁₃₀N₈O₃₅, 1762.8), ES- positive (1785.4, M+Na), ES - Negative (1761.5, M-1, 879.8).

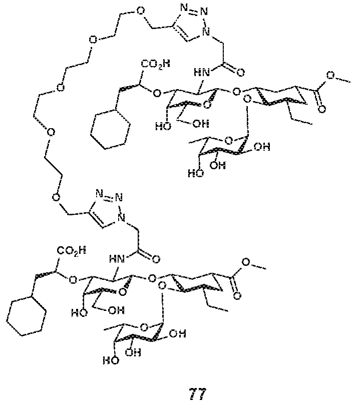

### EXAMPLE 46

### PROPHETIC SYNTHESIS OF MULTIMERIC COMPOUND 78

**Compound 78:** Compound **77** (60 mg, 34.0 µmole) was dissolved in ethylenediamine (3 mL) and the homogeneous solution was stirred for 12 hrs at 70 °C. The reaction mixture was concentrated under reduced pressure and the residue was dialyzed against distilled water with MWCO 500 dialysis tube. The crude product was further purified by C-18 column chromatography with water/MeOH (9/1 - 1/9, v/v) followed by lyophilization to give a compound **78** as a white solid (39 mg, 63%).

1H NMR (400 MHz, Deuterium Oxide) δ 8.00 (s, 2H), 5.26 - 5.14 (two d, J = 16.0 Hz, 4H), 4.52 (d, J = 4.0 Hz, 2H), 4.84 (dd, J = 8.0 Hz, J = 4.0 Hz, 2H), 4.66 (s, 4H), 4.54 (broad d, J = 12 Hz, 2H), 3.97 (broad t, 2H), 3.91 - 3.78 (m, 6H), 3.77 - 3.58 (m, 28H), 3.57-3.46(m, 4H), 3.42 (t, J = 8.0 Hz, 6H), 3.24 (t, J = 12.0 Hz, 2H),3.02 (t, J = 6.0Hz, 4H), 2.67 (s, 2H), 2.32 (broad t, J = 12 Hz, 2H), 2.22 - 2.06 (m, 2H), 1.96 - 1.74 (m, 4H), 1.73 - 1.39 (m, 18H), 1.38 - 1.21 (m, 6H), 1.20 - 0.99 (m, J = 8.0 Hz, 14H), 0.98 - 0.73 (m, J = 8.0 Hz, 10H).

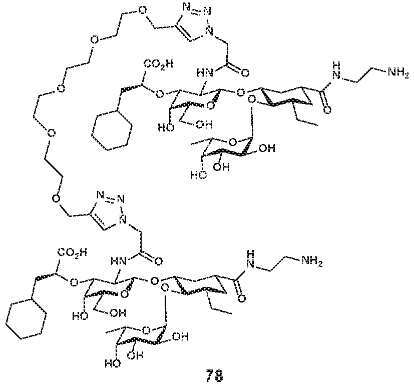

### EXAMPLE 47

### PROPHETIC SYNTHESIS OF MULTIMERIC COMPOUND 79

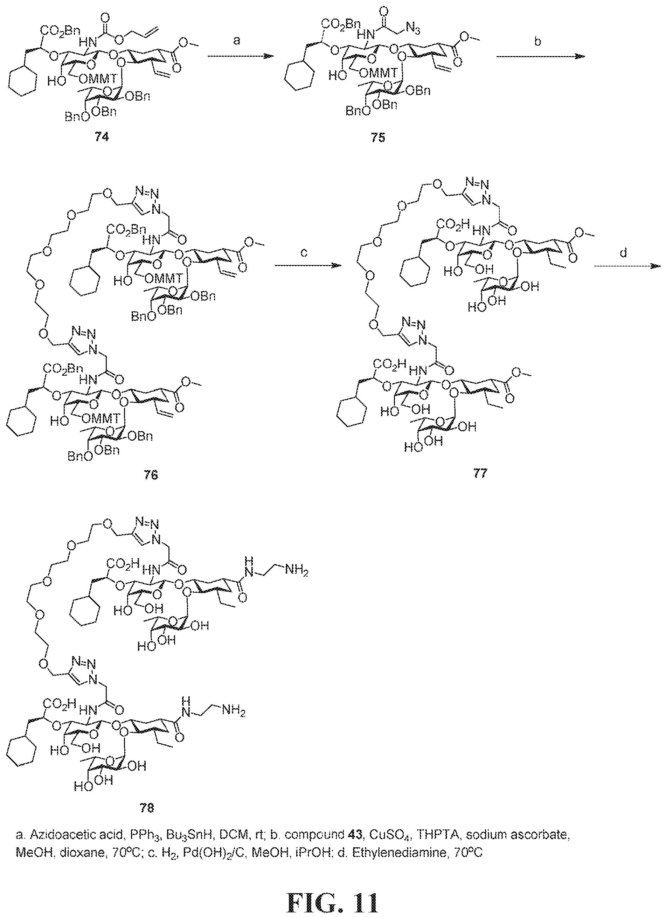

**Compound 79:** Compound **79** can be prepared in an analogous fashion to Figure 11 using 3-azidopropanoic anhydride (Yang, C. et. al. JACS, (2013) 135(21), 7791-7794) in place of azidoacetic anhydride in step a.

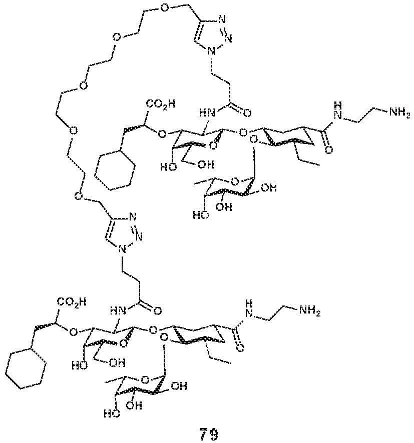

### EXAMPLE 48

### PROPHETIC SYNTHESIS OF MULTIMERIC COMPOUND 80

**Compound 80:** Compound **80** can be prepared in an analogous fashion to Figure 11 using 4-azidobutanoic anhydride (Yang, C. et. al. JACS, (2013) 135(21), 7791-7794) in place of azidoacetic anhydride in step a.

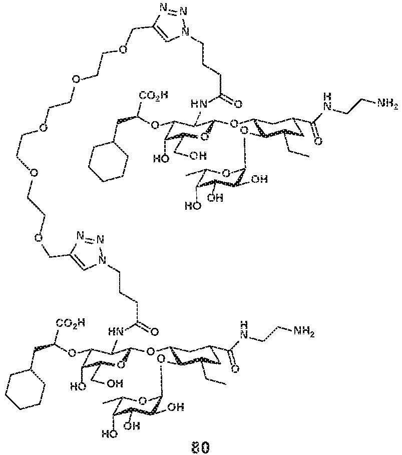

### EXAMPLE 49

### PROPHETIC SYNTHESIS OF MULTIMERIC COMPOUND 81

**Compound 81:** Compound **81** can be prepared in an analogous fashion to Figure 11 using 4-azidobutanoic anhydride (Yang, C. et. al. JACS, (2013) 135(21), 7791-7794) in place of azidoacetic anhydride in step a and using 1,2-bi(2-propynyloxy) ethane in place of compound 43 in step b.

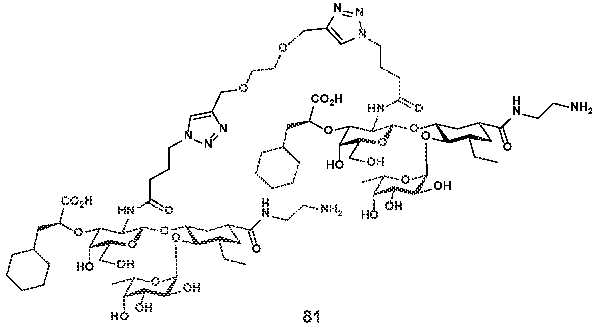

### EXAMPLE 50

### PROPHETIC SYNTHESIS OF MULTIMERIC COMPOUND 82

Compound **82:** Compound **82** can be prepared in an analogous fashion to Figure 11 using 4,7,10,13,16,19,22,25,28,31-decaoxatetratriaconta-1, 33-diyne in place of compound **43** in step b.

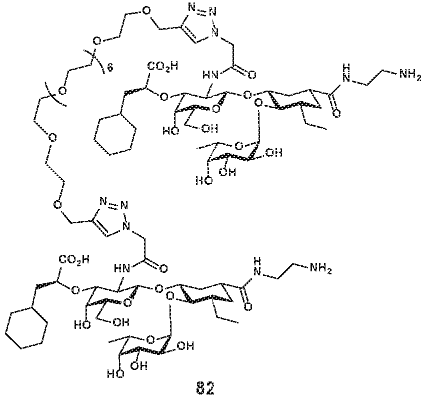

### EXAMPLE 51

### PROPHETIC SYNTHESIS OF MULTIMERIC COMPOUND 83

**Compound 83:** Compound **83** can be prepared in an analogous fashion to Figure 11 using 2-aminoethylether in place of ethylenediamine in step d.

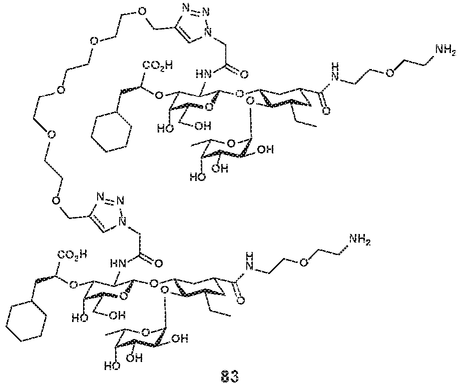

### EXAMPLE 52

### PROPHETIC SYNTHESIS OF MULTIMERIC COMPOUND 84

**Compound 84:** Compound **84** can be prepared in an analogous fashion to Figure 11 using 1,2-bi(2-propynyloxy) ethane in place of compound **43** in step b.

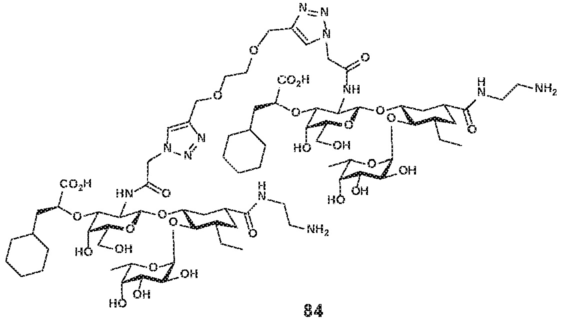

### EXAMPLE 53

### PROPHETIC SYNTHESIS OF MULTIMERIC COMPOUND 85

**Compound 85:** Compound **85** can be prepared in an analogous fashion to Figure 11 using PEG-8 dipropargyl ether in place of compound **43** in step b and 1,5-diaminopentane in place of ethylenediamine in step d.

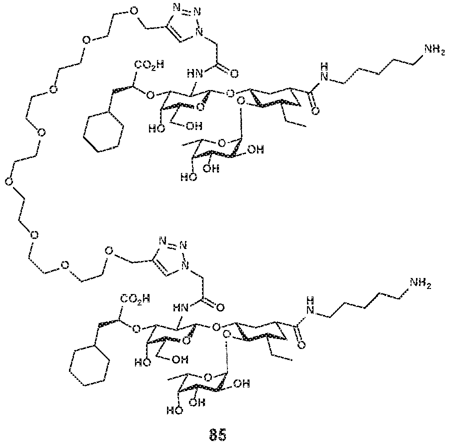

### EXAMPLE 54

### PROPHETIC SYNTHESIS OF MULTIMERIC COMPOUND 86

**Compound 86:** Compound **77** is dissolved in DMF and cooled on an ice bath. Diisopropylethylamine (2.5 eq) is added followed by HATU (2.2 eq). The reaction mixture is stirred 15 minutes on the ice bath then azetidine (10 eq) is added. The ice bath is removed and the reaction mixture is stirred overnight at room temperature. The solvent is removed under reduced pressure and the residue is separated by flash chromatography to afford compound **86.**

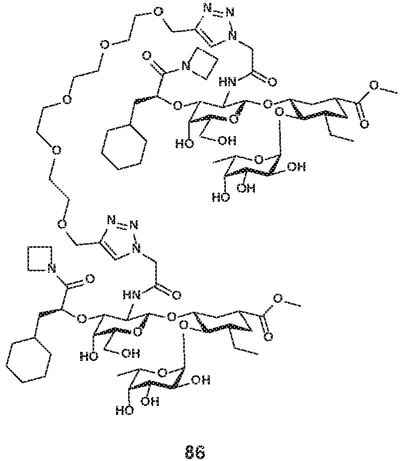

### EXAMPLE 55

### PROPHETIC SYNTHESIS OF MULTIMERIC COMPOUND 87

**Compound 87:** Compound **86** is dissolved in ethylenediamine stirred for 12 hrs at 70 °C. The reaction mixture was concentrated under reduced pressure. The residue was purified by C-18 column chromatography followed by lyophilization to give a compound **87.**

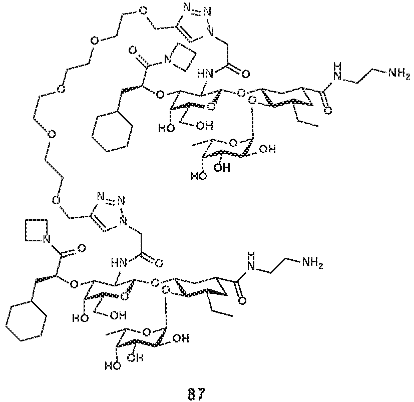

### EXAMPLE 56

### PROPHETIC SYNTHESIS OF MULTIMERIC COMPOUND 88

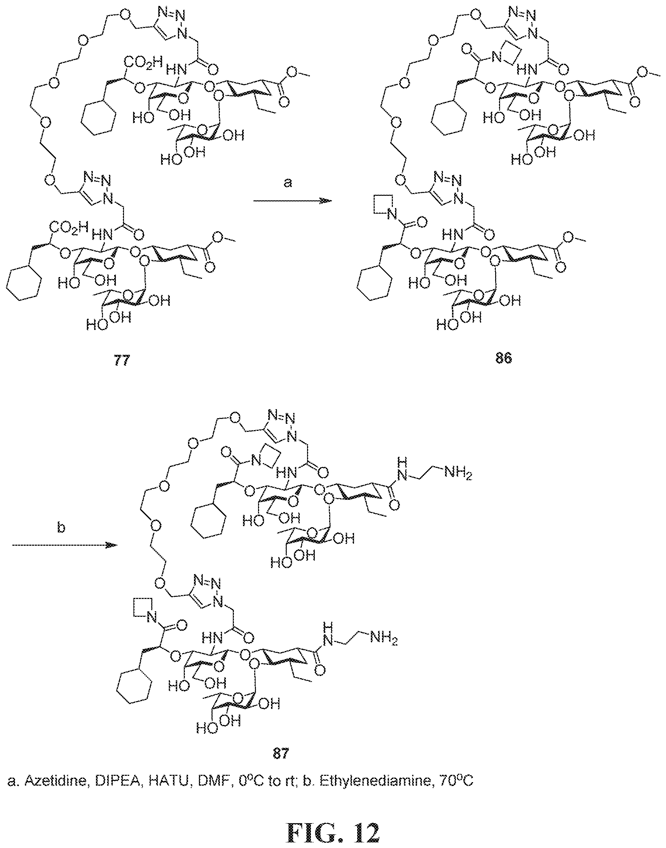

**Compound 88:** Compound **88** can be prepared in an analogous fashion to Figure 12 using 2-aminoethylether in place of ethylenediamine in step b.

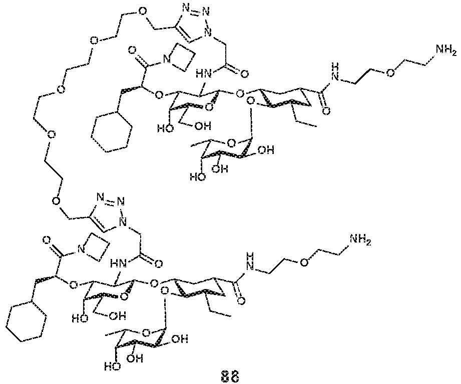

### EXAMPLE 57

### PROPHETIC SYNTHESIS OF MULTIMERIC COMPOUND 89

**Compound 89:** Compound **89** can be prepared in an analogous fashion to Figure 12 using dimethylamine in place of azetidine in step a and 2-aminoethylether in place of ethylenediamine in step b.

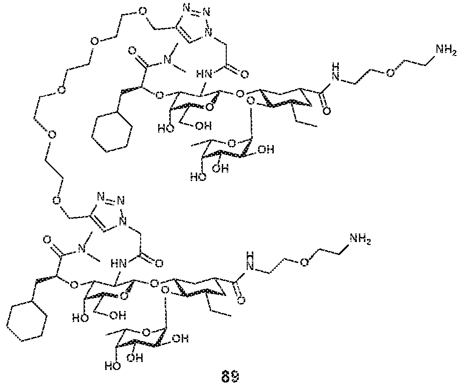

### EXAMPLE 58

### PROPHETIC SYNTHESIS OF MULTIMERIC COMPOUND 90

**Compound 90:** Compound **90** can be prepared in an analogous fashion to Figure 12 using piperidine in place of azetidine in step a.

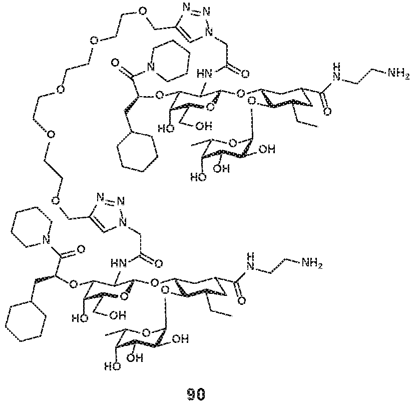

### EXAMPLE 59

### PROPHETIC SYNTHESIS OF MULTIMERIC COMPOUND 91

**Compound 91:** Compound **91** can be prepared in an analogous fashion to Figures 11 and 12 using in PEG-9 bis-propargyl ether in place of compound **43** in step b of Scheme 11.

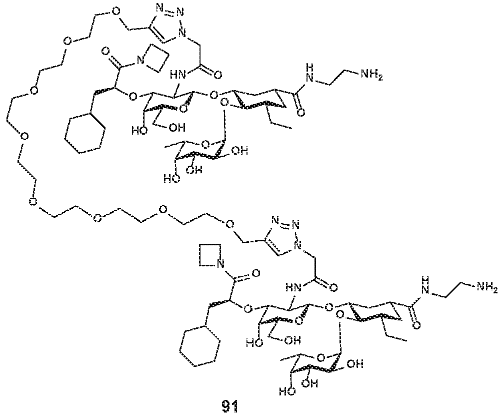

### EXAMPLE 60

### PROPHETIC SYNTHESIS OF MULTIMERIC COMPOUND 92

**Compound 92:** Compound **92** can be prepared in an analogous fashion to Figures 11 and 12 using 1,2-bi(2-propynyloxy) ethane in place of compound **43** in step b in Scheme 11.

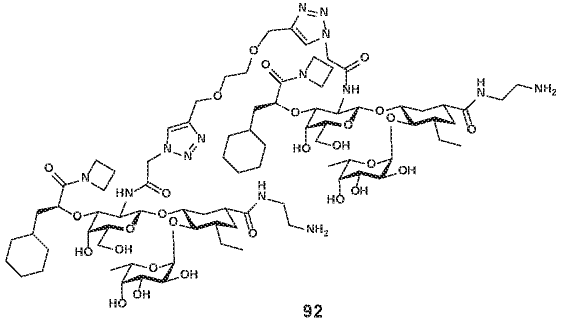

### EXAMPLE 61

### PROPHETIC SYNTHESIS OF MULTIMERIC COMPOUND 93

**Compound 93:** Compound **93** can be prepared in an analogous fashion to Figures 11 and 12 using 1,2-bi(2-propynyloxy) ethane in place of compound **43** in step b in Scheme 11 and using 2-aminoethyl ether in place of ethylenediamine in step b of Scheme 12.

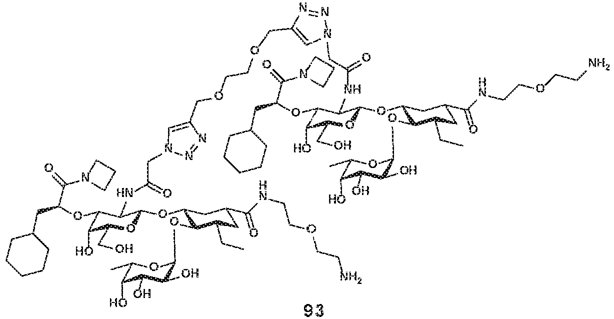

### EXAMPLE 62

### SYNTHESIS OF MULTIMERIC COMPOUND 95

**Compound 95:** Compound **22** and compound **94** (5 eq)(preparation described in WO/2016089872) is co-evaporated 3 times from methanol and stored under vacuum for 1 hour. The mixture is dissolved in methanol under an argon atmosphere and stirred for 1 hour at room temperature. Sodium triacetoxyborohydride (15 eq) is added and the reaction mixture is stirred overnight at room temperature. The solvent is removed and the residue is separated by C-18 reverse phase chromatography.

The purified material is dissolved in methanol at room temperature. The pH is adjusted to 12 with 1N NaOH. The reaction mixture is stirred at room temperature until completion. The pH is adjusted to 9. The solvent is removed under vacuum and the residue is separated by C-18 reverse phase chromatography to afford compound **95.**

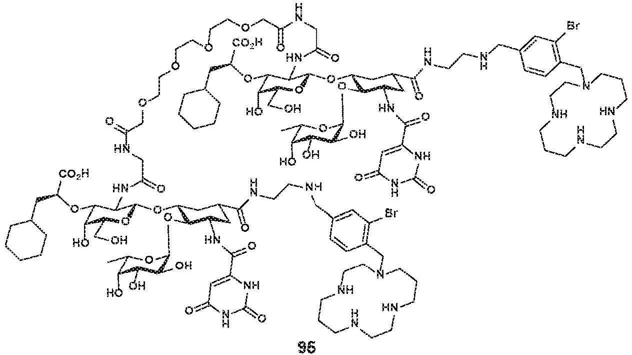

### EXAMPLE 63

### PROPHETIC SYNTHESIS OF MULTIMERIC COMPOUND 96

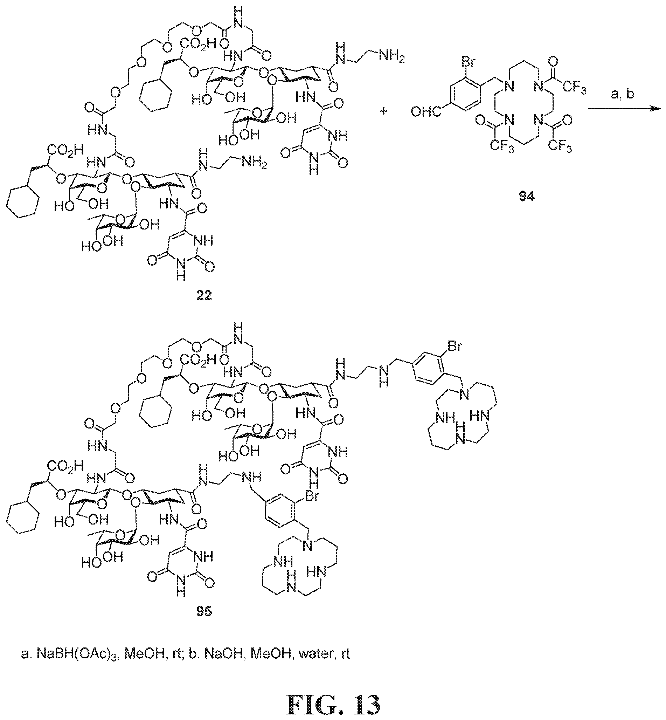

**Compound 96:** Compound **96** can be prepared in an analogous fashion to Figure 13 by replacing compound **22** with compound **23** in step a.

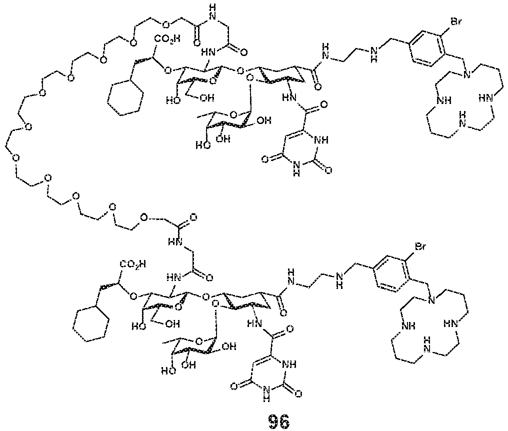

### EXAMPLE 64

### PROPHETIC SYNTHESIS OF MULTIMERIC COMPOUND 97

**Compound 97:** Compound **97** can be prepared in an analogous fashion to Figure 13 by replacing compound **22** with compound **24** in step a.

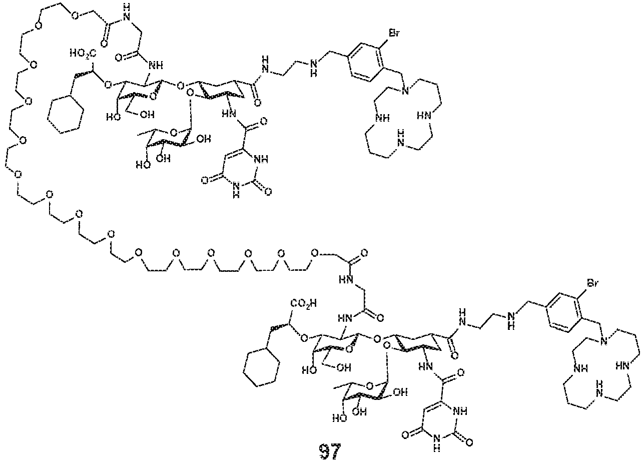

### EXAMPLE 65

### PROPHETIC SYNTHESIS OF MULTIMERIC COMPOUND 98

**Compound 98:** Compound **98** can be prepared in an analogous fashion to Figure 13 by replacing compound **22** with compound **25** in step a.

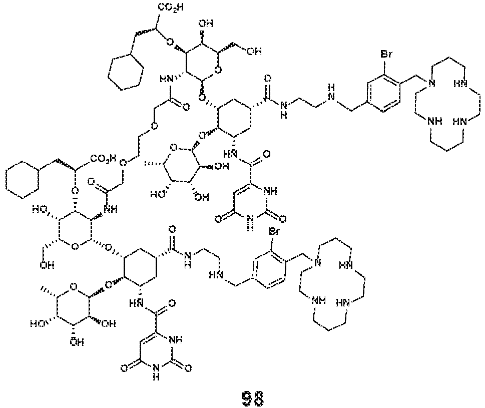

### EXAMPLE 66

### PROPHETIC SYNTHESIS OF MULTIMERIC COMPOUND 99

**Compound 99:** Compound **99** can be prepared in an analogous fashion to Figure 13 by replacing compound **22** with compound **26** in step a.

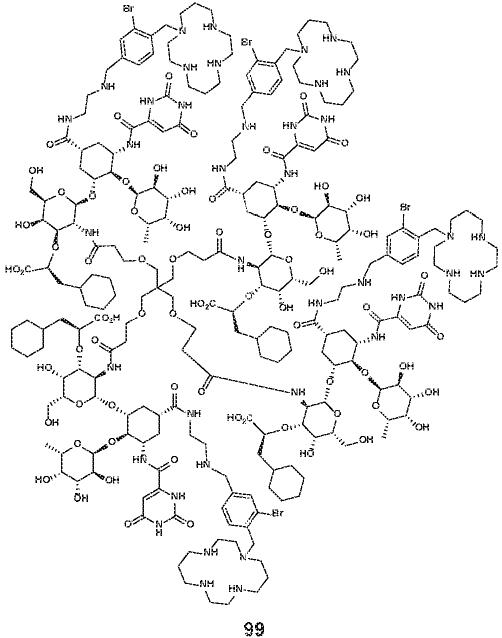

### EXAMPLE 67

### PROPHETIC SYNTHESIS OF MULTIMERIC COMPOUND 100

**Compound 100:** Compound **100** can be prepared in an analogous fashion to Figure 13 by replacing compound **22** with compound **27** in step a.

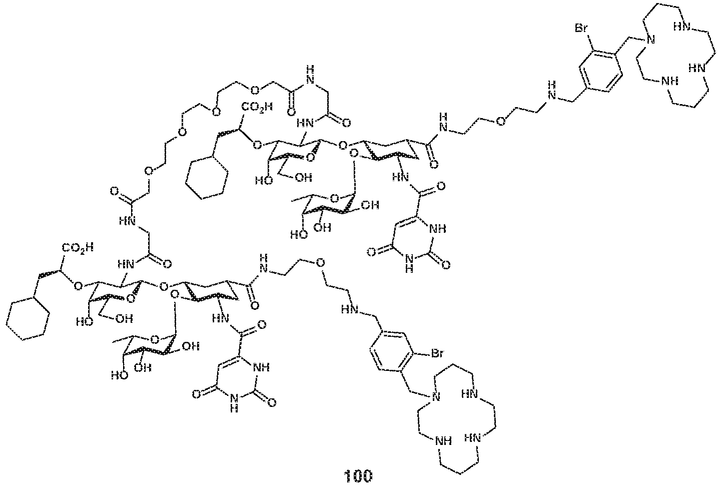

### EXAMPLE 68

### PROPHETIC SYNTHESIS OF MULTIMERIC COMPOUND 101

**Compound 101:** Compound **101** can be prepared in an analogous fashion to Figure 13 by replacing compound **22** with compound **28** in step a.

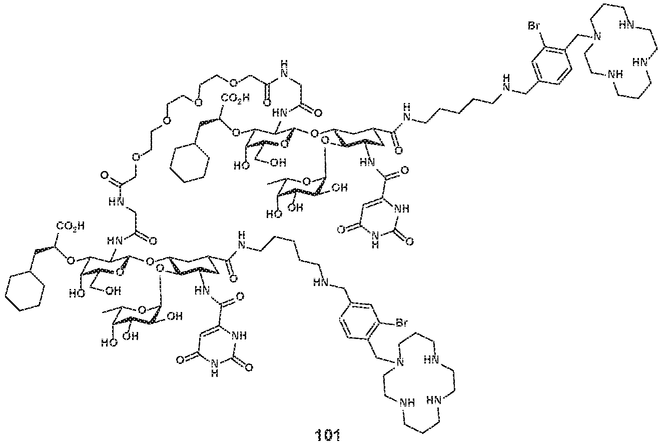

### EXAMPLE 69

### PROPHETIC SYNTHESIS OF MULTIMERIC COMPOUND 102

**Compound 102:** Compound **102** can be prepared in an analogous fashion to Figure 13 by replacing compound **22** with compound **29** in step a.

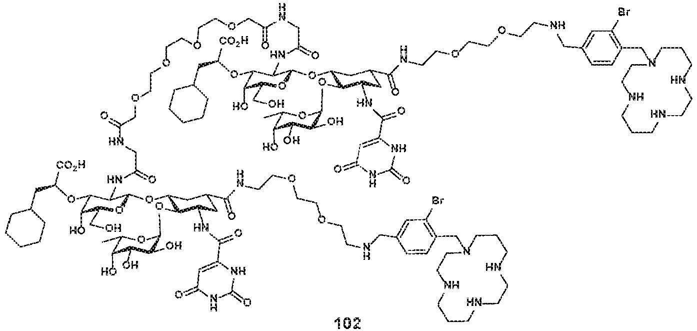

### EXAMPLE 70

### PROPHETIC SYNTHESIS OF MULTIMERIC COMPOUND 103

**Compound 103:** Compound **103** can be prepared in an analogous fashion to Figure 13 by replacing compound **22** with compound **30** in step a.

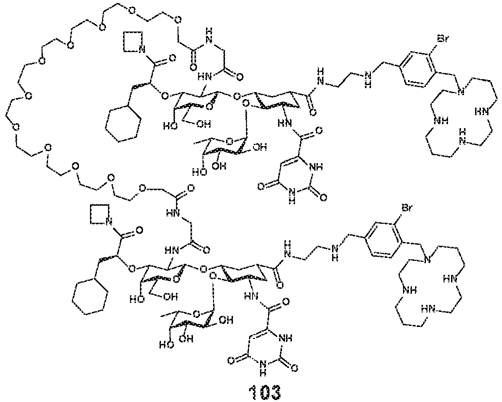

### EXAMPLE 71

### PROPHETIC SYNTHESIS OF MULTIMERIC COMPOUND 104

**Compound 104:** Compound **104** can be prepared in an analogous fashion to Figure 13 by replacing compound **22** with compound **31** in step a.

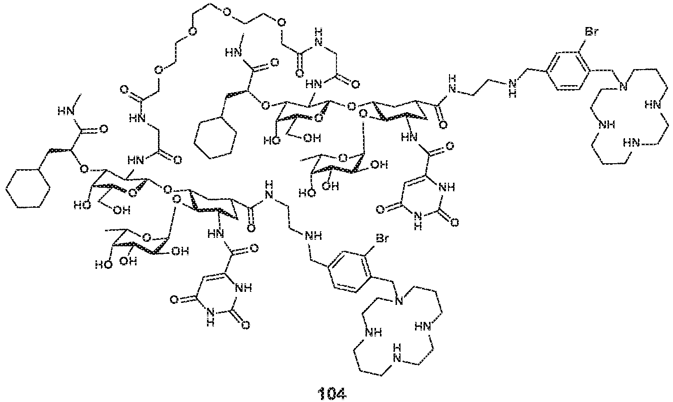

### EXAMPLE 72

### PROPHETIC SYNTHESIS OF MULTIMERIC COMPOUND 105

**Compound 105:** Compound **105** can be prepared in an analogous fashion to Figure 13 by replacing compound **22** with compound **32** in step a.

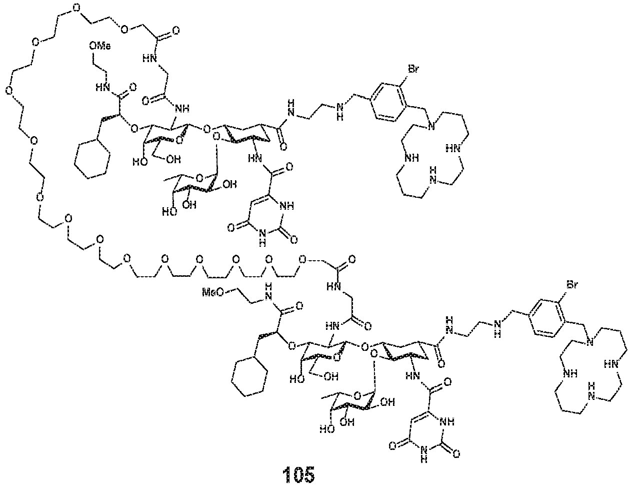

### EXAMPLE 73

### PROPHETIC SYNTHESIS OF MULTIMERIC COMPOUND 106

**Compound 106:** Compound **106** can be prepared in an analogous fashion to Figure 13 by replacing compound **22** with compound **33** in step a.

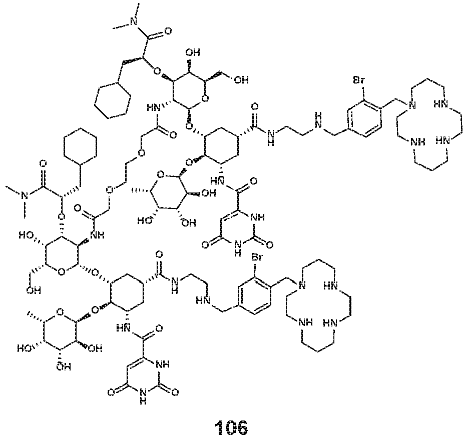

### EXAMPLE 74

### PROPHETIC SYNTHESIS OF MULTIMERIC COMPOUND 107

**Compound 107**: Compound **107** can be prepared in an analogous fashion to Figure 13 by replacing compound **22** with compound **34** in step a.

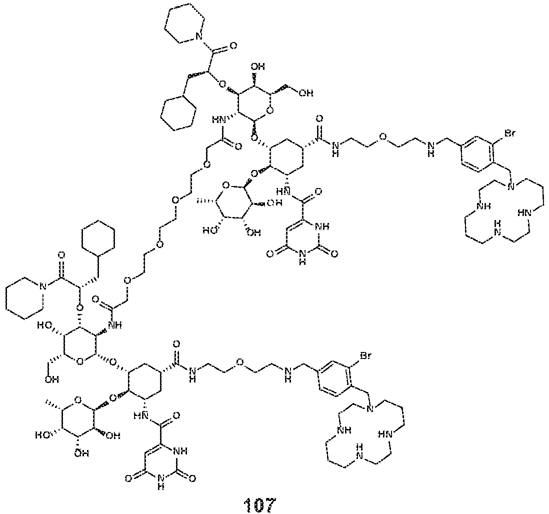

### EXAMPLE 75

### PROPHETIC SYNTHESIS OF MULTIMERIC COMPOUND 108

**Compound 108:** Compound **108** can be prepared in an analogous fashion to Figure 13 by replacing compound **22** with compound **37** in step a.

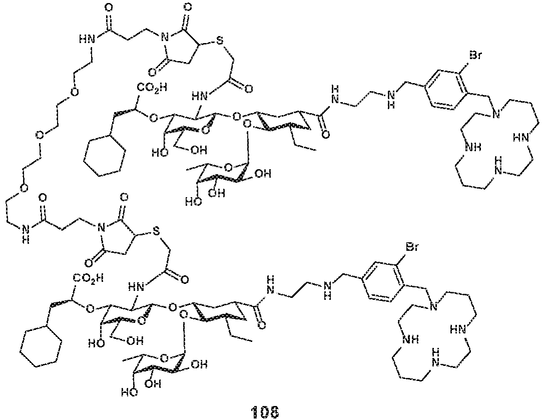

### EXAMPLE 76

### PROPHETIC SYNTHESIS OF MULTIMERIC COMPOUND 109

**Compound 109:** Compound **109** can be prepared in an analogous fashion to Figure 13 by replacing compound **22** with compound **38** in step a.

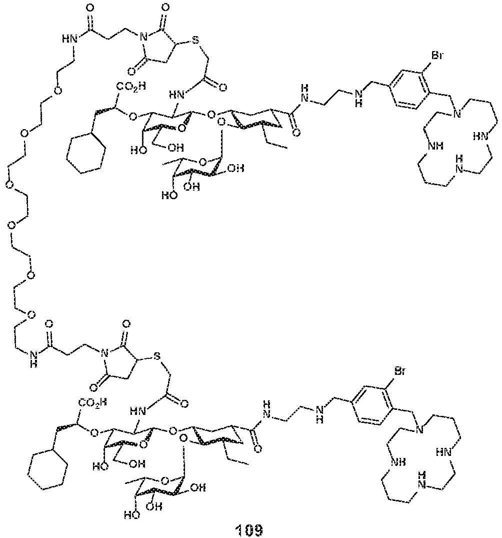

### EXAMPLE 77

### PROPHETIC SYNTHESIS OF MULTIMERIC COMPOUND 110

**Compound 110:** Compound **110** can be prepared in an analogous fashion to Figure 13 by replacing compound **22** with compound **39** in step a.

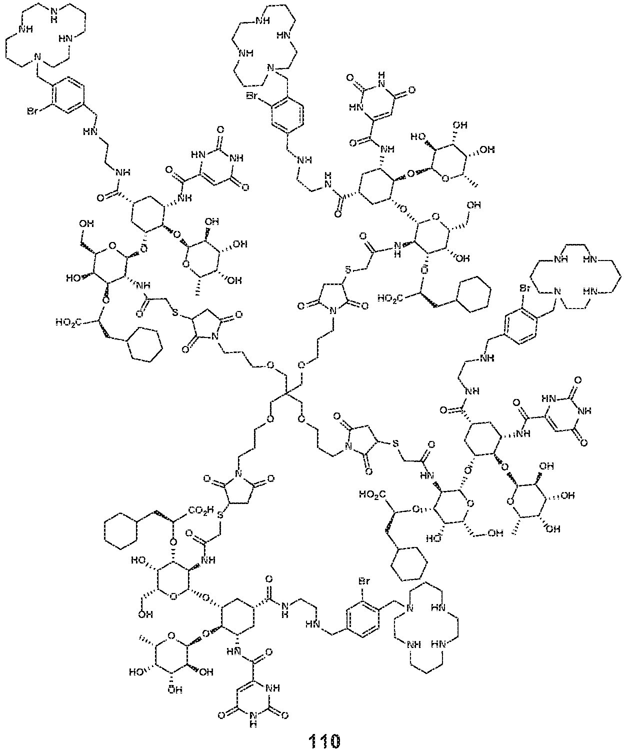

### PROPHETIC SYNTHESIS OF MULTIMERIC COMPOUND 111

**Compound 111:** Compound **111** can be prepared in an analogous fashion to Figure 13 by replacing compound **22** with compound **40** in step a.

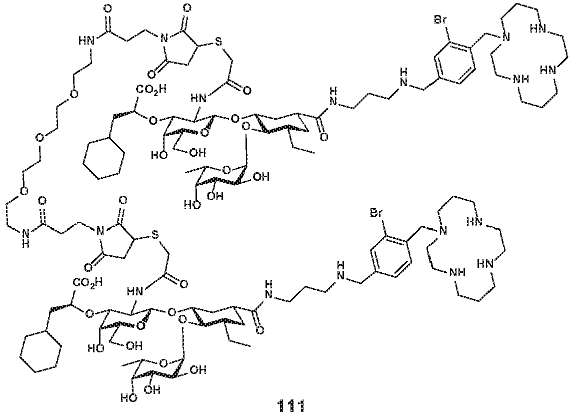

### EXAMPLE 78

### PROPHETIC SYNTHESIS OF MULTIMERIC COMPOUND 112

**Compound 112:** Compound **112** can be prepared in an analogous fashion to Figure 13 by replacing compound **22** with compound **46** in step a.

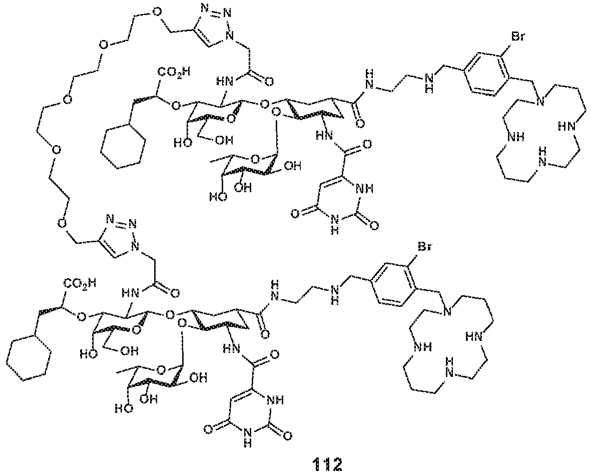

### EXAMPLE 79

### PROPHETIC SYNTHESIS OF MULTIMERIC COMPOUND 113

**Compound 113:** Compound **113** can be prepared in an analogous fashion to Figure 13 by replacing compound **22** with compound **47** in step a.

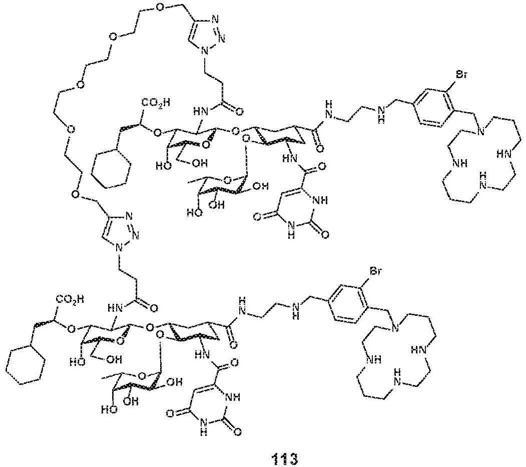

### EXAMPLE 80

### PROPHETIC SYNTHESIS OF MULTIMERIC COMPOUND 114

**Compound 114:** Compound **114** can be prepared in an analogous fashion to Figure 13 by replacing compound **22** with compound **48** in step a.

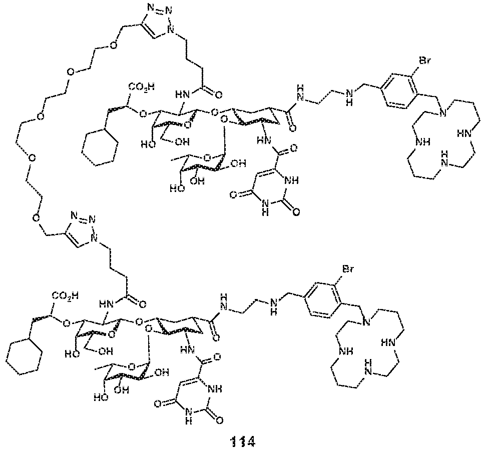

### EXAMPLE 81

### PROPHETIC SYNTHESIS OF MULTIMERIC COMPOUND 115

**Compound 115:** Compound **115** can be prepared in an analogous fashion to Figure 13 by replacing compound **22** with compound **49** in step a.

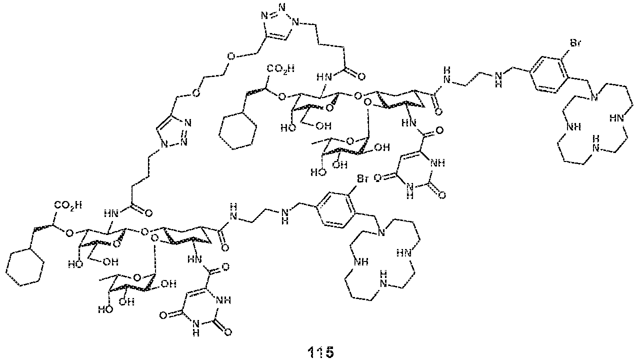

### EXAMPLE 82

### PROPHETIC SYNTHESIS OF MULTIMERIC COMPOUND 116

**Compound 116:** Compound **116** can be prepared in an analogous fashion to Figure 13 by replacing compound **22** with compound **50** in step a.

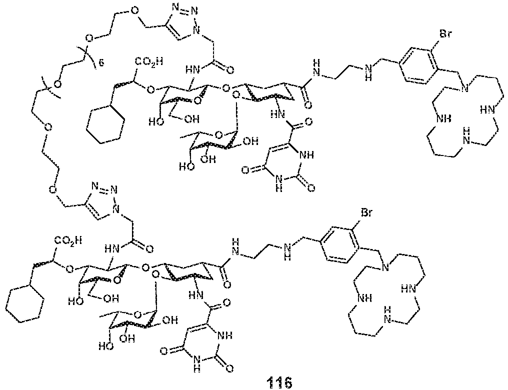

### EXAMPLE 83

### PROPHETIC SYNTHESIS OF MULTIMERIC COMPOUND 117

**Compound 117:** Compound **117** can be prepared in an analogous fashion to Figure 13 by replacing compound **22** with compound **51** in step a.

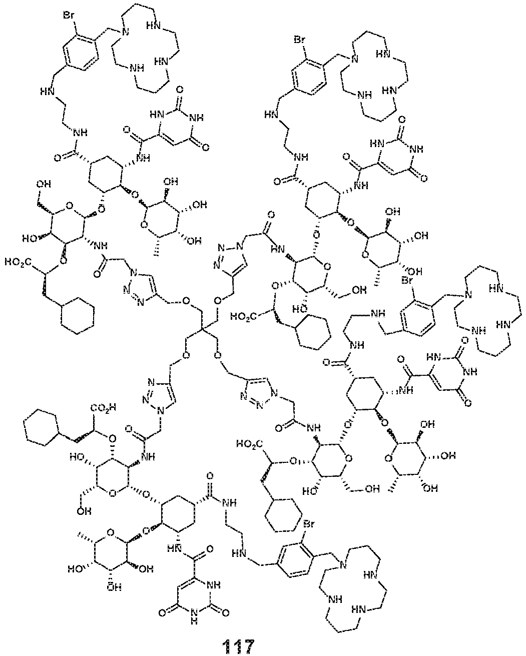

### EXAMPLE 84

### PROPHETIC SYNTHESIS OF MULTIMERIC COMPOUND 118

**Compound 118:** Compound **118** can be prepared in an analogous fashion to Figure 13 by replacing compound **22** with compound **52** in step a.

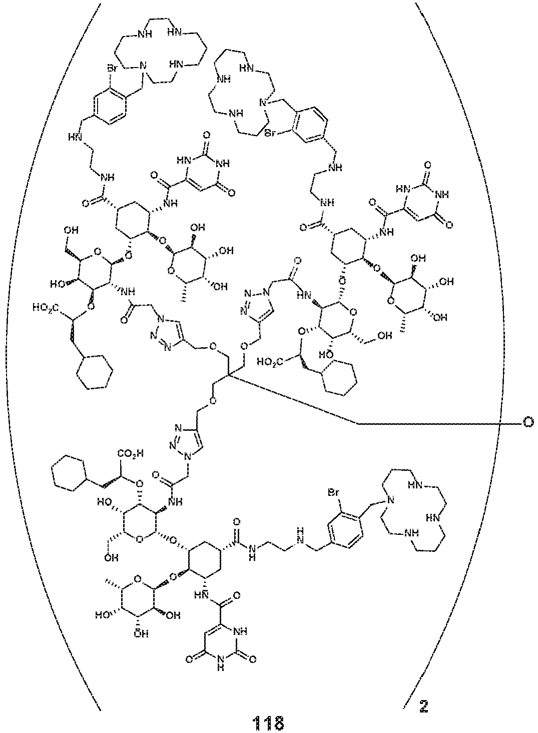

### EXAMPLE 85

### PROPHETIC SYNTHESIS OF MULTIMERIC COMPOUND 119

**Compound 119:** Compound **119** can be prepared in an analogous fashion to Figure 13 by replacing compound **22** with compound **53** in step a.

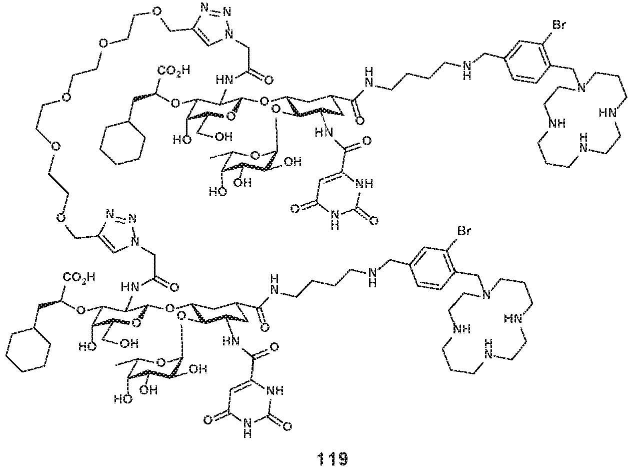

### EXAMPLE 86

### PROPHETIC SYNTHESIS OF MULTIMERIC COMPOUND 120

**Compound 120:** Compound **120** can be prepared in an analogous fashion to Figure 13 by replacing compound **22** with compound **54** in step a.

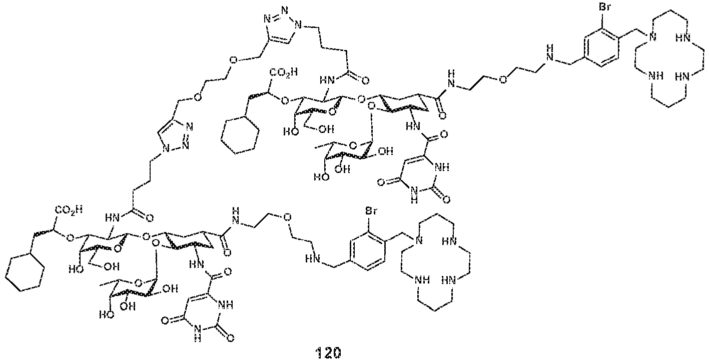

### EXAMPLE 87

### PROPHETIC SYNTHESIS OF MULTIMERIC COMPOUND 121

**Compound 121:** Compound **121** can be prepared in an analogous fashion to Figure 13 by replacing compound **22** with compound **56** in step a.

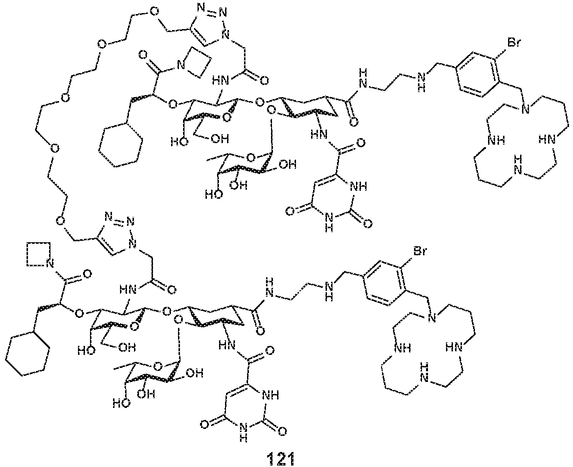

### EXAMPLE 88

### PROPHETIC SYNTHESIS OF MULTIMERIC COMPOUND 122

**Compound 122:** Compound **122** can be prepared in an analogous fashion to Figure 13 by replacing compound **22** with compound **57** in step a.

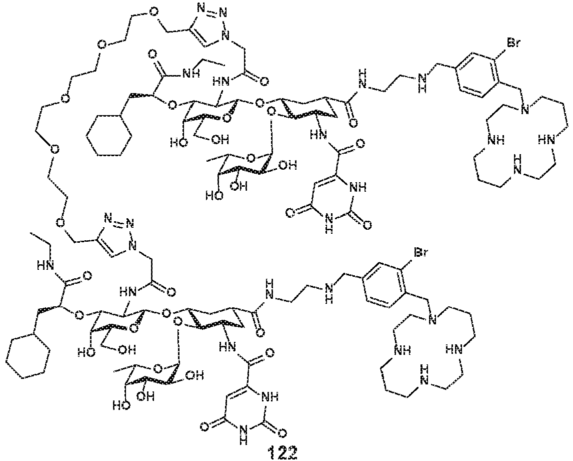

### EXAMPLE 89

### PROPHETIC SYNTHESIS OF MULTIMERIC COMPOUND 123

**Compound 123:** Compound **123** can be prepared in an analogous fashion to Figure 13 by replacing compound **22** with compound **58** in step a.

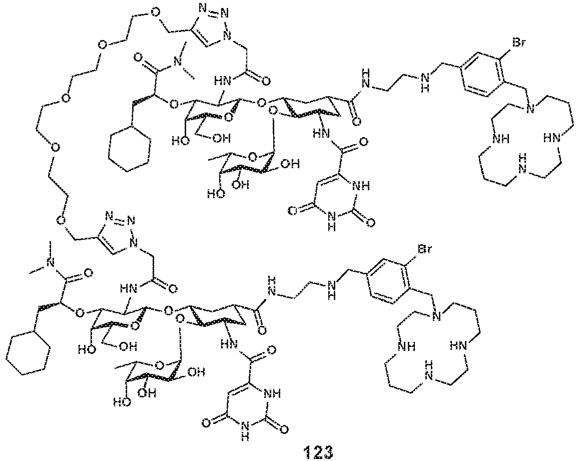

### EXAMPLE 90

### PROPHETIC SYNTHESIS OF MULTIMERIC COMPOUND 124

**Compound 124:** Compound **124** can be prepared in an analogous fashion to Figure 13 by replacing compound **22** with compound **59** in step a.

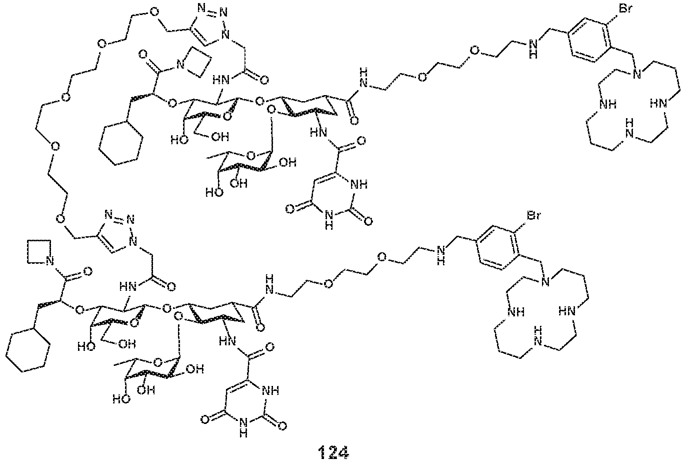

### EXAMPLE 91

### PROPHETIC SYNTHESIS OF MULTIMERIC COMPOUND 125

**Compound 125:** Compound **125** can be prepared in an analogous fashion to Figure 13 by replacing compound **22** with compound **68** in step a.

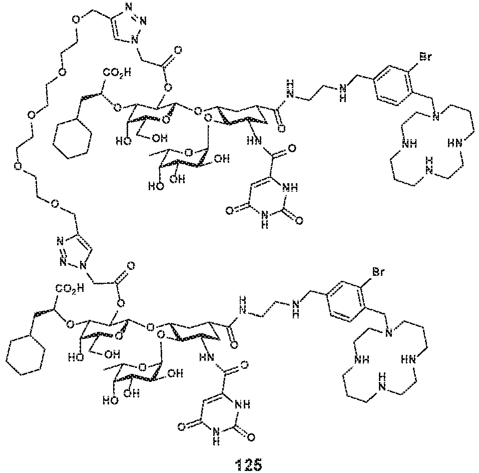

### EXAMPLE 92

### PROPHETIC SYNTHESIS OF MULTIMERIC COMPOUND 126

**Compound 126:** Compound **126** can be prepared in an analogous fashion to Figure 13 by replacing compound **22** with compound **69** in step a.

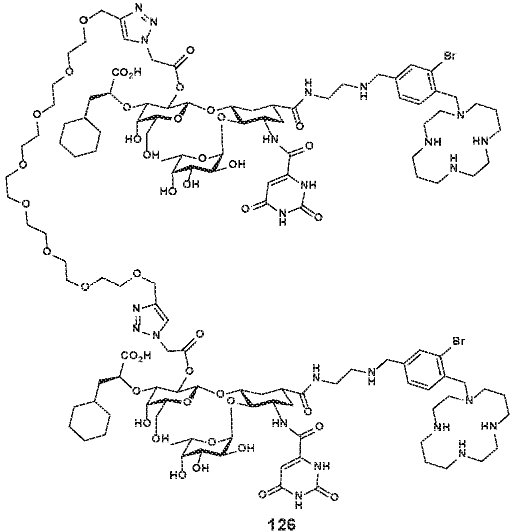

### EXAMPLE 93

### PROPHETIC SYNTHESIS OF MULTIMERIC COMPOUND 127

**Compound 127:** Compound **127** can be prepared in an analogous fashion to Figure 13 by replacing compound **22** with compound **70** in step a.

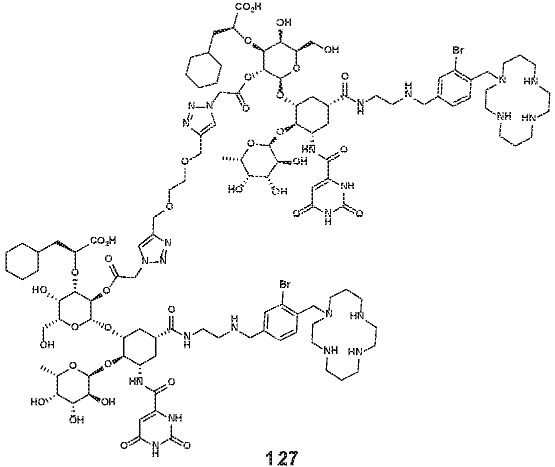

### EXAMPLE 94

### PROPHETIC SYNTHESIS OF MULTIMERIC COMPOUND 128

**Compound 128:** Compound **128** can be prepared in an analogous fashion to Figure 13 by replacing compound **22** with compound **71** in step a.

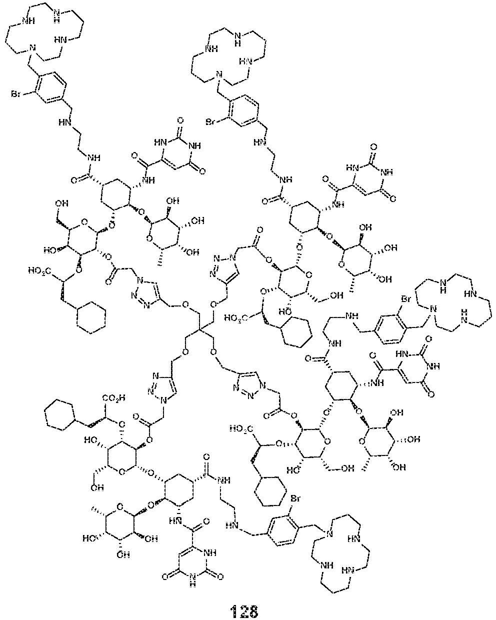

### EXAMPLE 95

### PROPHETIC SYNTHESIS OF MULTIMERIC COMPOUND 129

**Compound 129:** Compound **129** can be prepared in an analogous fashion to Figure 13 by replacing compound **22** with compound **73** in step a.

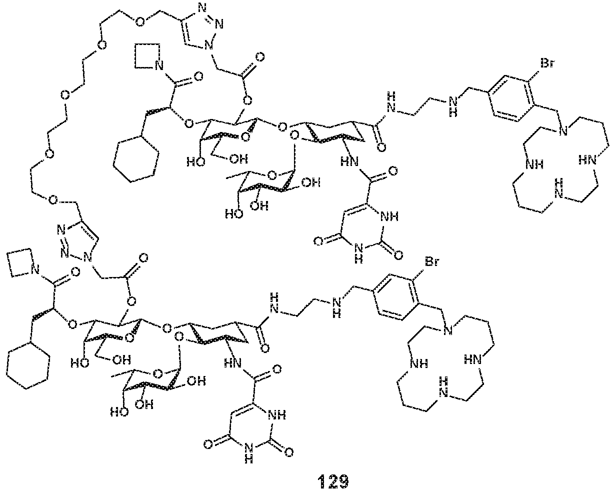

### EXAMPLE 96

### PROPHETIC SYNTHESIS OF MULTIMERIC COMPOUND 130

**Compound 130:** Compound **130** can be prepared in an analogous fashion to Figure 13 by replacing compound **22** with compound **78** in step a.

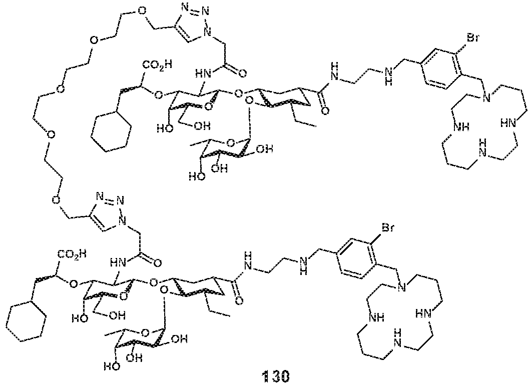

### EXAMPLE 97

### PROPHETIC SYNTHESIS OF MULTIMERIC COMPOUND 131

**Compound 131:** Compound **131** can be prepared in an analogous fashion to Figure 13 by replacing compound **22** with compound **79** in step a.

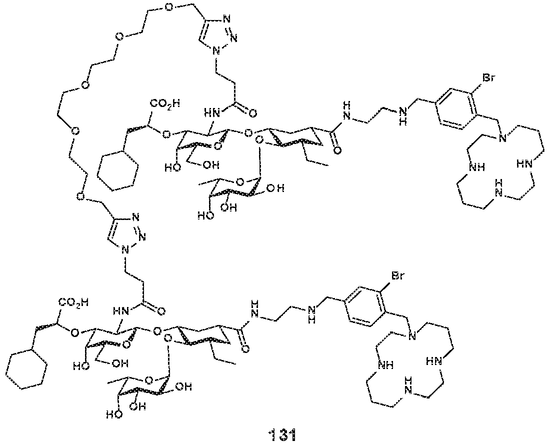

### EXAMPLE 98

### PROPHETIC SYNTHESIS OF MULTIMERIC COMPOUND 132

**Compound 132:** Compound **132** can be prepared in an analogous fashion to Figure 13 by replacing compound **22** with compound **80** in step a.

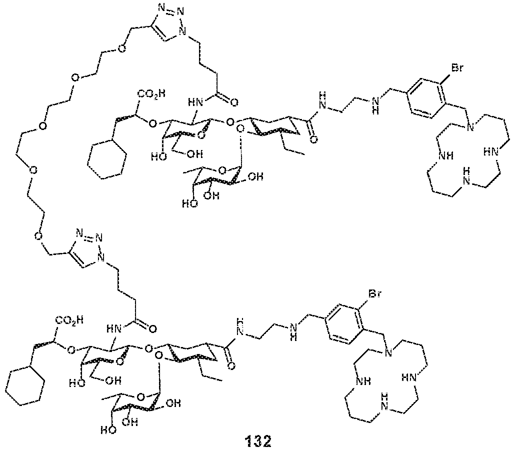

### EXAMPLE 99

### PROPHETIC SYNTHESIS OF MULTIMERIC COMPOUND 133

**Compound 133:** Compound **133** can be prepared in an analogous fashion to Figure 13 by replacing compound **22** with compound **81** in step a.

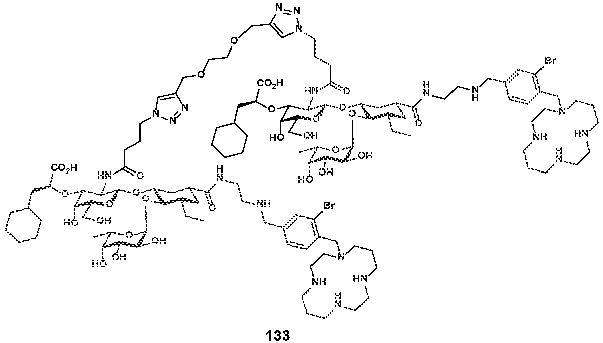

### EXAMPLE 100

### PROPHETIC SYNTHESIS OF MULTIMERIC COMPOUND 134

**Compound 134:** Compound **134** can be prepared in an analogous fashion to Figure 13 by replacing compound **22** with compound **82** in step a.

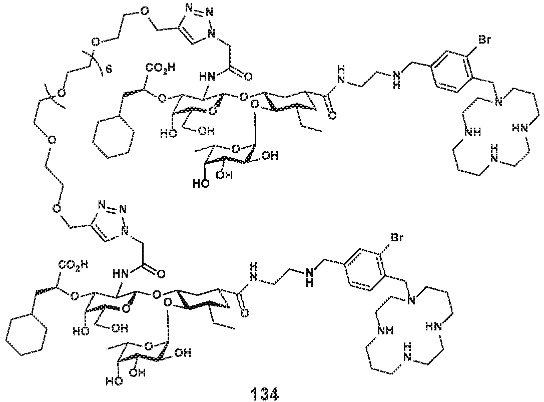

### EXAMPLE 101

### PROPHETIC SYNTHESIS OF MULTIMERIC COMPOUND 135

**Compound 135:** Compound **135** can be prepared in an analogous fashion to Figure 13 by replacing compound **22** with compound **83** in step a.

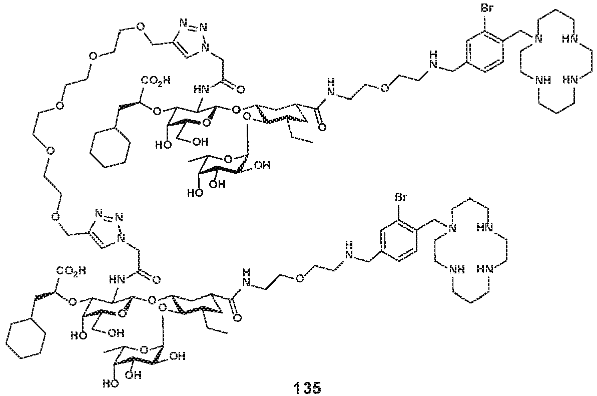

### EXAMPLE 102

### PROPHETIC SYNTHESIS OF MULTIMERIC COMPOUND 136

**Compound 136:** Compound **136** can be prepared in an analogous fashion to Figure 13 by replacing compound **22** with compound **84** in step a.

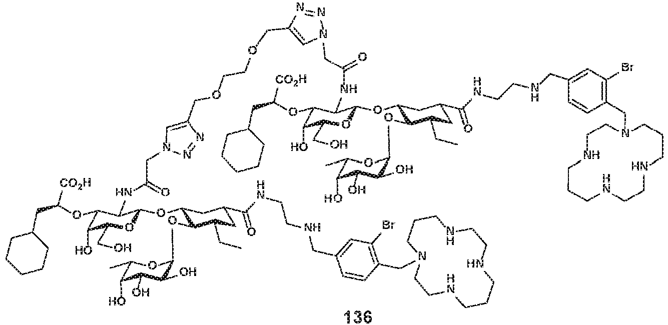

### EXAMPLE 103

### PROPHETIC SYNTHESIS OF MULTIMERIC COMPOUND 137

**Compound 137:** Compound **137** can be prepared in an analogous fashion to Figure 13 by replacing compound **22** with compound **85** in step a.

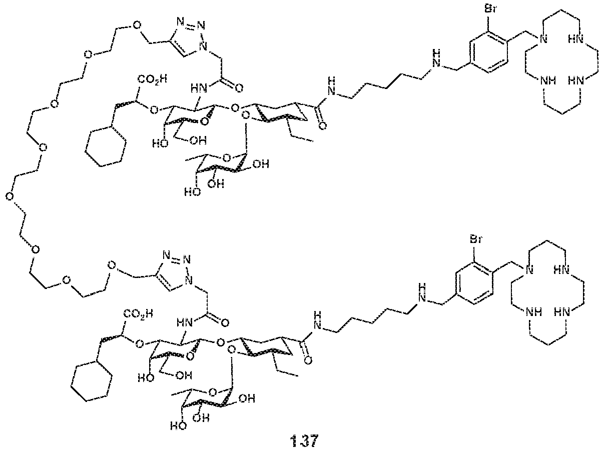

### EXAMPLE 104

### PROPHETIC SYNTHESIS OF MULTIMERIC COMPOUND 138

**Compound 138:** Compound **138** can be prepared in an analogous fashion to Figure 13 by replacing compound **22** with compound **87** in step a.

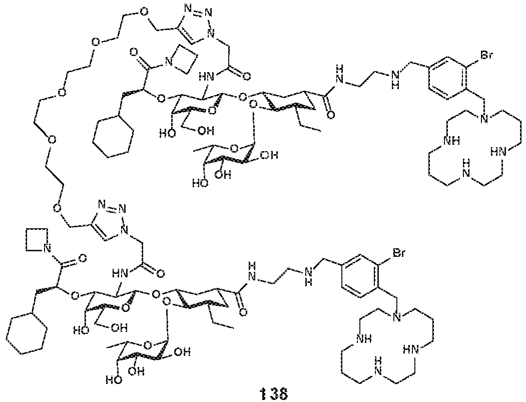

### EXAMPLE 105

### PROPHETIC SYNTHESIS OF MULTIMERIC COMPOUND 139

**Compound 139:** Compound **139** can be prepared in an analogous fashion to Figure 13 by replacing compound **22** with compound **88** in step a.

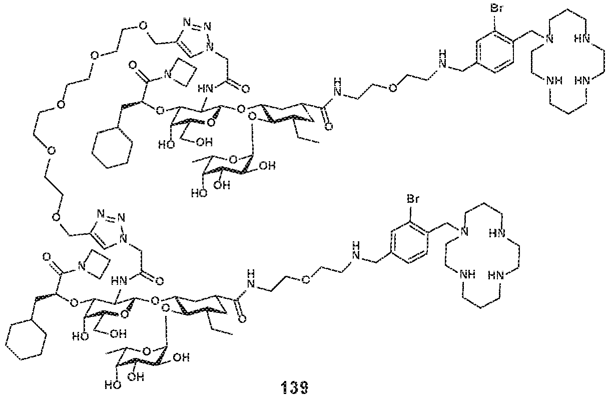

### EXAMPLE 106

### PROPHETIC SYNTHESIS OF MULTIMERIC COMPOUND 140

**Compound 140:** Compound **140** can be prepared in an analogous fashion to Figure 13 by replacing compound **22** with compound **89** in step a.

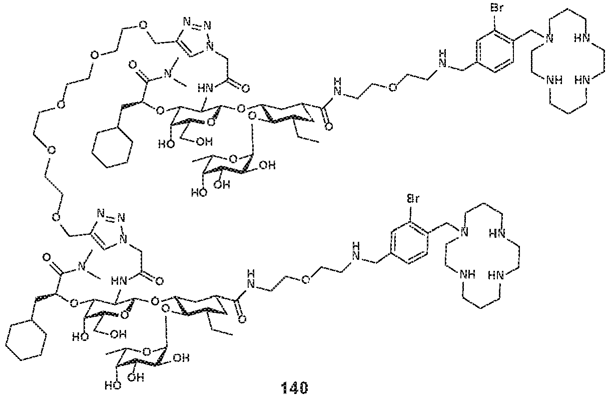

### EXAMPLE 107

### PROPHETIC SYNTHESIS OF MULTIMERIC COMPOUND 141

**Compound 141:** Compound **141** can be prepared in an analogous fashion to Figure 13 by replacing compound **22** with compound **90** in step a.

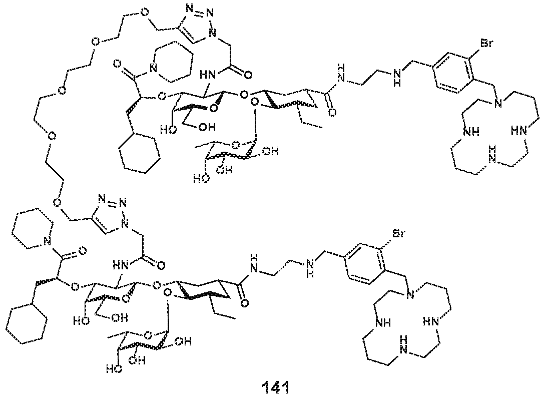

### EXAMPLE 108

### PROPHETIC SYNTHESIS OF MULTIMERIC COMPOUND 142

**Compound 142:** Compound **142** can be prepared in an analogous fashion to Figure 13 by replacing compound **22** with compound **91** in step a.

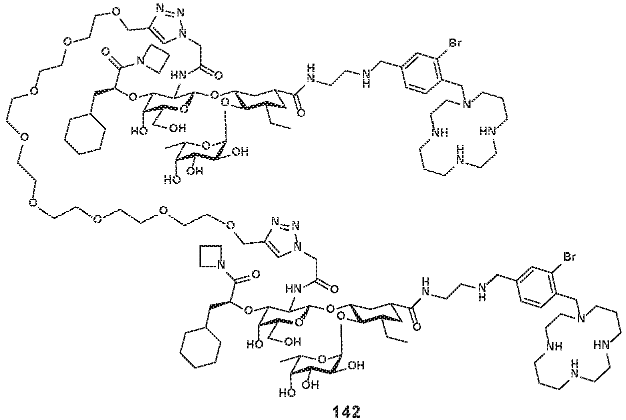

### EXAMPLE 109

### PROPHETIC SYNTHESIS OF MULTIMERIC COMPOUND 143

**Compound 143:** Compound **143** can be prepared in an analogous fashion to Figure 13 by replacing compound **22** with compound **92** in step a.

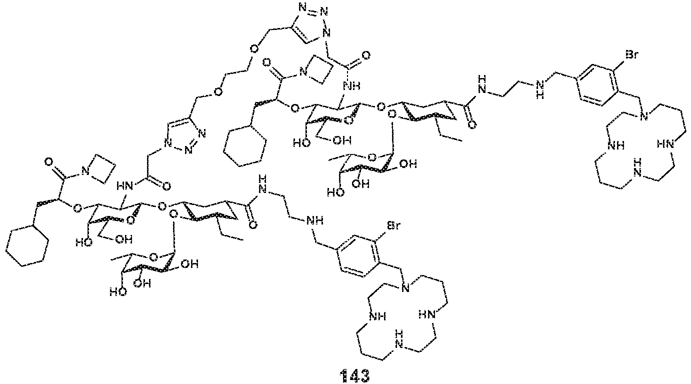

### EXAMPLE 110

### PROPHETIC SYNTHESIS OF MULTIMERIC COMPOUND 144

**Compound 144:** Compound **144** can be prepared in an analogous fashion to Figure 13 by replacing compound **22** with compound **93** in step a.

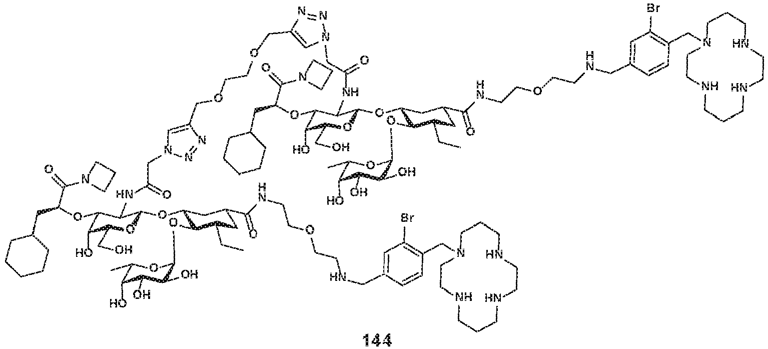

### EXAMPLE 111

### PROPHETIC SYNTHESIS OF MULTIMERIC COMPOUND 146

**Compound 315:** To a solution of compound **314** (1 gm, 3.89 mmol) (preparation described in WO 2007/028050) and benzyl trichloroacetaimidate (1.1 ml, 5.83 mmol) in anhydrous dichloromethane (10 ml) was added trimethylsilyl trifluoromethanesulfonate (70 uL, 0.4 mmol). The mixture was stirred at ambient temperature for 12 h. After this period the reaction was diluted with dichloromethane, washed with saturated NaHCO₃, dried over MgSO₄ and concentrated. The residue was purified by column chromatography to give compound **315** (0.8 gm, 60 %).

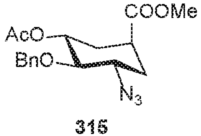

**Compound 316:** To a solution of compound **315** (800 mg, 2.3 mmol) in anhydrous methanol (1 ml) and anhydrous methyl acetate (5 ml) was added 0.5M sodium methoxide solution in methanol (9.2 ml). The mixture was stirred at 40 °C for 4h. The reaction was quenched with acetic acid and concentrated. The residue was purified by column chromatography to afford compound **316** as mixture of epimers at the methyl ester with 75% equatorial and 25% axial epimer (242 mg, 35 %). ¹H NMR (400 MHz, Chloroform-d) δ 7.48 - 7.32 (m, 6H), 4.97 (d, J = 11.1 Hz, 1H), 4.72 (dd, J = 11.1, 5.7 Hz, 1H), 3.77 - 3.65 (m, 6H), 3.22 - 3.15 (m, 1H), 2.92 - 2.82 (m, 1H), 2.39 (dddd, J = 15.7, 10.6, 5.1, 2.7 Hz, 2H), 1.60 (dtd, J = 13.9, 11.2, 5.4 Hz, 3H). MS: Calculated for C₁₅H₁₉N₃O₄ = 305.3, Found ES- positive *m*/*z* =306.1 (M+Na⁺).

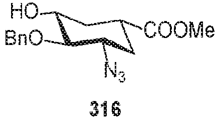

**Compound 318:** A solution of compound **317** (5 gm, 11.8 mmol) (preparation described in WO 2009/139719) in anhydrous methanol (20 ml) was treated with 0.5 M solution of sodium methoxide in methanol (5 ml) for 3h. Solvent was removed *in vacuo* and the residue was co-evaporated with toluene (20 ml) three times. The residue was dissolved in pyridine (20 ml) followed by addition of benzoyl chloride (4.1 ml, 35.4 mmol) over 10 minutes. The reaction mixture was stirred at ambient temperature under an atmosphere of argon for 22h. The reaction mixture was concentrated to dryness, dissolved in dichloromethane, washed with cold 1N hydrochloric acid and cold water, dried over MgSO₄, filtered, and concentrated. The residue was purified by column chromatography to give compound **318.** MS: Calculated for C₃₃H₂₇N₃O₇S = 609.2, Found ES- positive *m*/*z* =610.2 (M+Na⁺).

**Compound 319:** A mixture of compound **318** (2.4 gm, 3.93 mmol), diphenyl sulfoxide (1.5 gm, 7.3 mmol) and 2,6-di-tert-butyl pyridine (1.8 gm, 7.8 mmol) was dissolved in anhydrous dichloromethane (10 ml) at room temperature. The reaction mixture was cooled to - 60 °C. Triflic anhydride (0.62 ml, 3.67 mmol) was added dropwise and the mixture was stirred for 15 minutes at the same temperature. A solution of compound **316** (0.8 gm, 2.6 mmol) in anhydrous dichloromethane (10 ml) was added dropwise to the reaction mixture. The mixture was allowed to warm to 0 °C over 2h. The reaction mixture was diluted with dichloromethane, transferred toa separatory funnel and washed with saturated sodium bicarbonate solution followed by brine. The organic phase was dried over MgSO₄, filtered, and concentrated. The residue was separated by column chromatography to afford compound **319** as a white solid (1.2 gm, 57%). MS: Calculated for C₄₂H₄₀N₆O₁₁ = 804.3, Found ES- positive *m*/*z* = 805.3 (M+Na⁺).

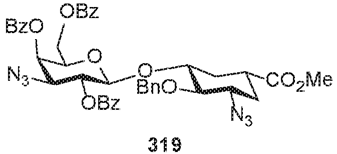

**Compound 320:** To a solution of compound **319** (1.2 gm 2.067 mmol) and 2-fluorophenyl acetylene (1.2 ml, 10.3 mmol) in methanol (30 ml) was added a stock solution of copper sulfate and tris(3-hydroxypropyltriazolylmethyl) amine in water (2.58 ml). The reaction was initiated by addition of an aqueous solution of sodium ascorbate (0.9 gm, 4.5 mmol) and the mixture was stirred at ambient temperature for 16 hours. The mixture was co-evaporated with dry silica gel and purified by column chromatography to afford compound **320** as a white solid (1.2 gm, 77%).
**Stock solution of Copper Sulfate/THPTA** - (100 mg of copper sulfate pentahydrate and 200 mg of tris(3-hydroxypropyltriazolylmethyl)amine were dissolved in 10 ml of water).
¹H NMR (400 MHz, Chloroform-d) δ 8.07 - 8.00 (m, 2H), 7.96 (ddd, J = 9.8, 8.2, 1.3 Hz, 4H), 7.79 (d, J = 5.4 Hz, 2H), 7.65 - 7.53 (m, 5H), 7.43 (ddt, J = 22.4, 10.7, 5.0 Hz, 7H), 7.25 - 7.01 (m, 9H), 6.92 (td, J = 7.6, 7.1, 2.2 Hz, 1H), 6.13 - 6.02 (m, 2H), 5.58 (dd, J = 11.6, 3.2 Hz, 1H), 5.15 (d, J = 7.5 Hz, 1H), 4.98 (d, J = 10.3 Hz, 1H), 4.68 (dd, J = 11.2, 5.7 Hz, 1H), 4.52 (dq, J = 22.1, 6.6, 5.6 Hz, 2H), 4.35 (dd, J = 11.1, 7.6 Hz, 1H), 4.28 - 4.18 (m, 1H), 4.11 (d, J = 10.3 Hz, 1H), 3.87 (t, J = 9.1 Hz, 1H), 3.71 (s, 3H), 2.95 (s, 1H), 2.62 - 2.43 (m, 3H), 1.55 (dt, J = 12.7, 6.1 Hz, 1H). MS: Calculated for C₅₈H₅₀N₆O₁₁ = 1044.4, Found ES- positive *m*/*z =* 1045.5 (M+Na⁺).

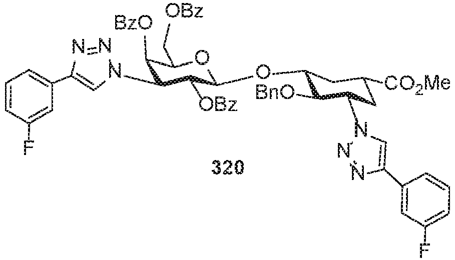

**Compound 145:** To a solution of compound **320** (1.2 gm, 1.1 mmol) in *iso*propanol (40 ml) was added Na-metal (80 mg, 3.4 mmol) at ambient temperature and the mixture was stirred for 12 hours at 50 °C. 10% aqueous sodium hydroxide (2 ml) was added to the reaction mixture and stirring continued for another 6 hours at 50 °C. The reaction mixture was cooled to room temperature and neutralized with 50% aqueous hydrochloric acid. To the mixture was added 10% Pd(OH)₂ on carbon (0.6 gm) and the reaction mixture was stirred under an atmosphere of hydrogen for 12 hours. The reaction mixture was filtered through a Celite pad and concentrated. The residue was separated by HPLC to give compound **145** as a white solid (0.5 gm, 70%). HPLC Conditions - Waters preparative HPLC system was used with ELSD & PDA detectors. Kinetex XB- C18, 100 A, 5 uM, 250 x 21.2 mm column (from Phenomenex) was used with 0.2% formic acid in water as solvent A and acetonitrile as solvent B at a flow rate of 20 mL/min.
¹H NMR (400 MHz, DMSO-d6) δ 8.77 (s, 1H), 8.68 (s, 1H), 7.77 - 7.60 (m, 5H), 7.49 (tdd, J = 8.3, 6.1, 2.6 Hz, 3H), 7.15 (tt, J = 8.6, 3.2 Hz, 3H), 4.83 (dd, J = 10.9, 3.1 Hz, 1H), 4.63 (d, J = 7.5 Hz, 1H), 4.53 - 4.41 (m, 1H), 4.10 (dd, J = 10.9, 7.5 Hz, 1H), 3.92 (d, J = 3.2 Hz, 1H), 3.74 (h, J = 6.0, 5.6 Hz, 3H), 3.65 - 3.24 (m, 5H), 2.37 (d, J = 13.4 Hz, 1H), 2.24 - 2.04 (m, 2H), 1.93 (q, J = 12.5 Hz, 1H), 1.46 (t, J = 12.1 Hz, 1H). MS: Calculated for C₂₉H₃₀F₂N₆O₈ = 628.2, Found ES- positive *m*/*z* = 629.2 (M+Na⁺)

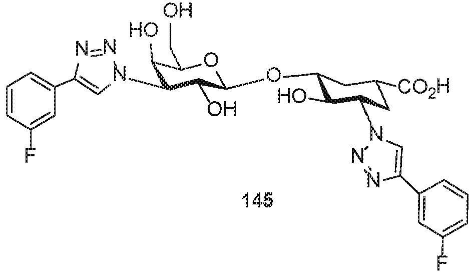

**Compound 146:** To a solution of compound **145** (3 eq) in anhydrous DMF was added HATU (3.3 eq) and DIPEA (5 eq). The mixture was stirred at ambient temperature for 15 minutes followed by addition of compound **22** (1 eq). The mixture was stirred at ambient temperature for 12h. The solvent was removed *in vacuo* and the residue was purified by HPLC to afford compound **146.**

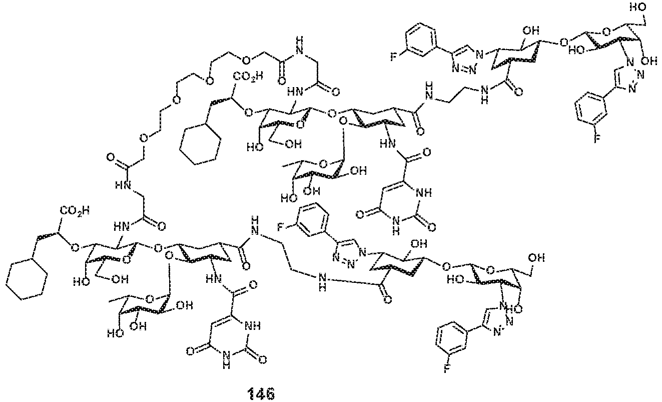

### EXAMPLE 112

### PROPHETIC SYNTHESIS OF MULTIMERIC COMPOUND 147

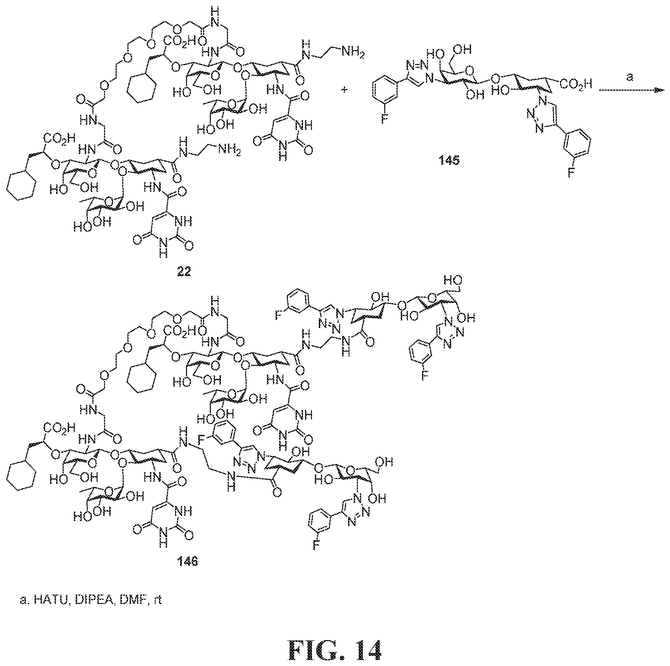

**Compound 147:** Compound **147** can be prepared in an analogous fashion to Figure 14 by replacing compound **22** with compound **23.**

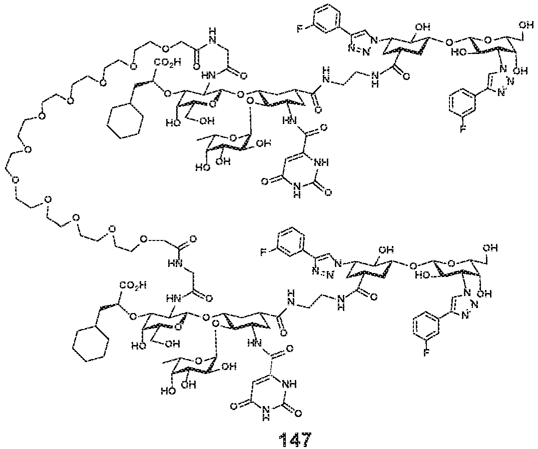

### EXAMPLE 113

### PROPHETIC SYNTHESIS OF MULTIMERIC COMPOUND 148

**Compound 148:** Compound **148** can be prepared in an analogous fashion to Figure 14 by replacing compound **22** with compound **24.**

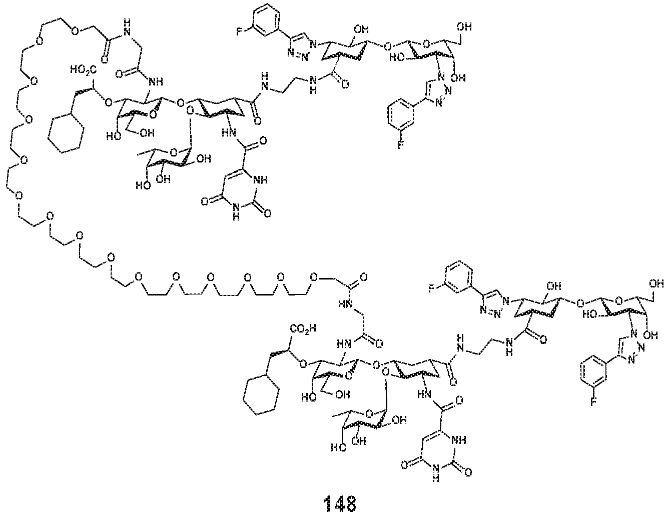

### EXAMPLE 114

### PROPHETIC SYNTHESIS OF MULTIMERIC COMPOUND 149

**Compound 149:** Compound **149** can be prepared in an analogous fashion to Figure 14 by replacing compound **22** with compound **25.**

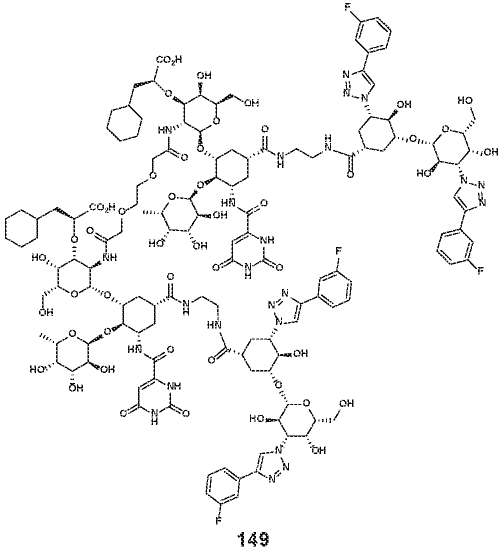

### EXAMPLE 115

### PROPHETIC SYNTHESIS OF MULTIMERIC COMPOUND 150

**Compound 150:** Compound **150** can be prepared in an analogous fashion to Figure 14 by replacing compound **22** with compound **26.**

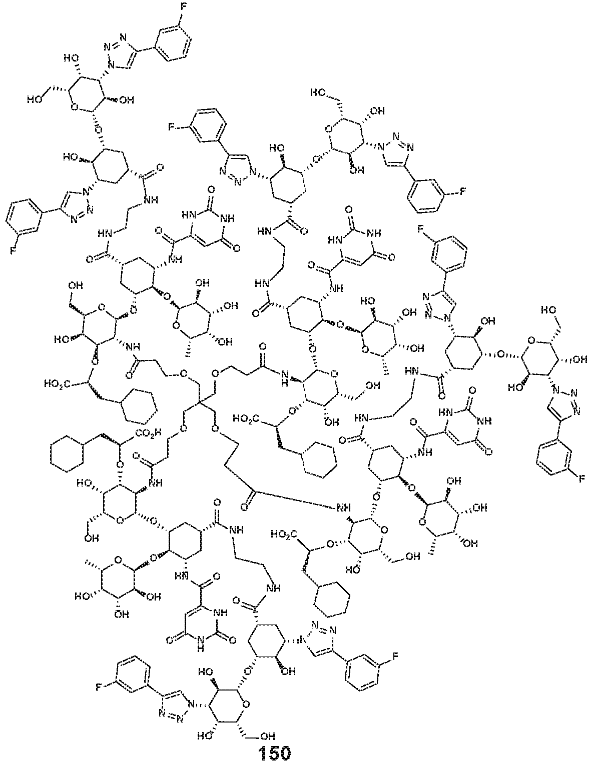

### EXAMPLE 116

### PROPHETIC SYNTHESIS OF MULTIMERIC COMPOUND 151

**Compound 151:** Compound **151** can be prepared in an analogous fashion to Figure 14 by replacing compound **22** with compound **27.**

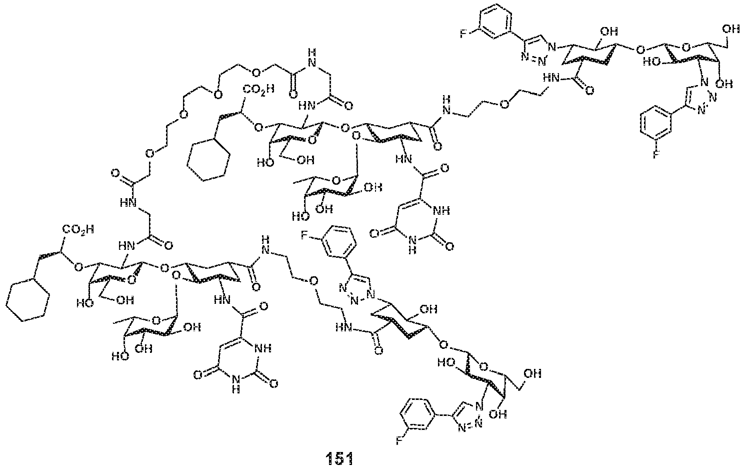

### EXAMPLE 117

### PROPHETIC SYNTHESIS OF MULTIMERIC COMPOUND 152

**Compound 152:** Compound **152** can be prepared in an analogous fashion to Figure 14 by replacing compound **22** with compound **28.**

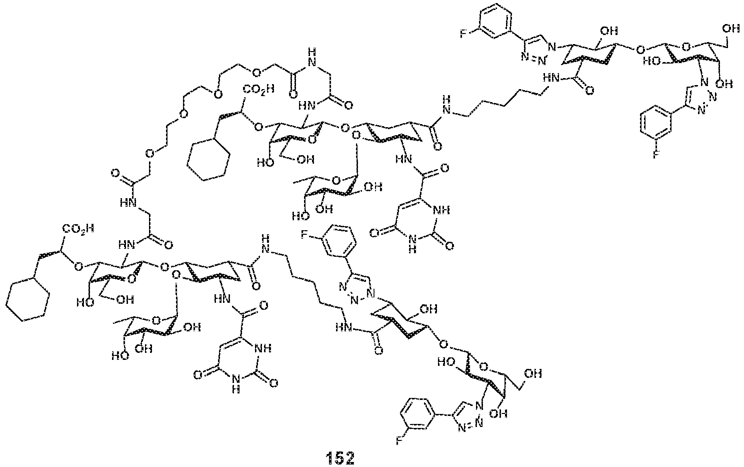

### EXAMPLE 118

### PROPHETIC SYNTHESIS OF MULTIMERIC COMPOUND 153

**Compound 153:** Compound **153** can be prepared in an analogous fashion to Figure 14 by replacing compound **22** with compound **29.**

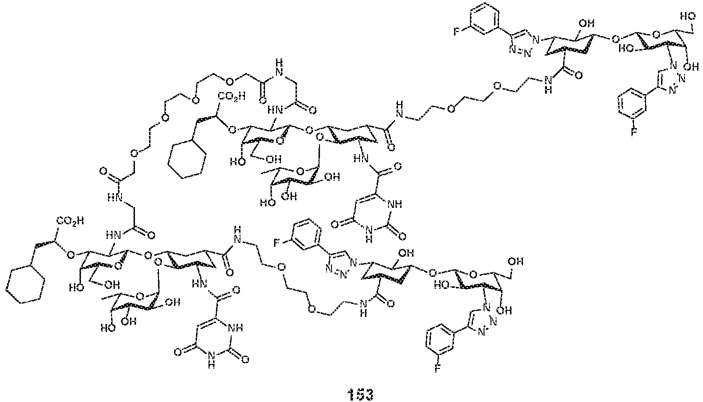

### EXAMPLE 119

### PROPHETIC SYNTHESIS OF MULTIMERIC COMPOUND 154

**Compound 154:** Compound **154** can be prepared in an analogous fashion to Figure 14 by replacing compound **22** with compound **30.**

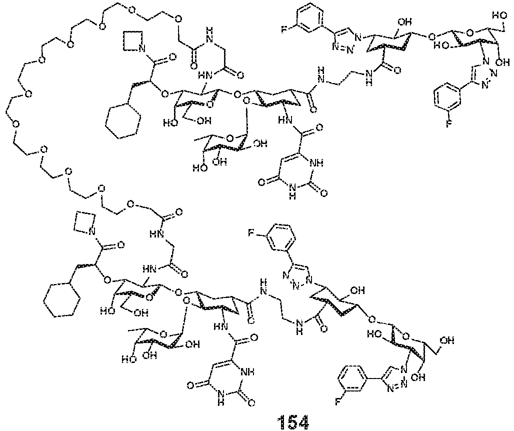

### EXAMPLE 120

### PROPHETIC SYNTHESIS OF MULTIMERIC COMPOUND 155

**Compound 155:** Compound **155** can be prepared in an analogous fashion to Figure 14 by replacing compound **22** with compound **31.**

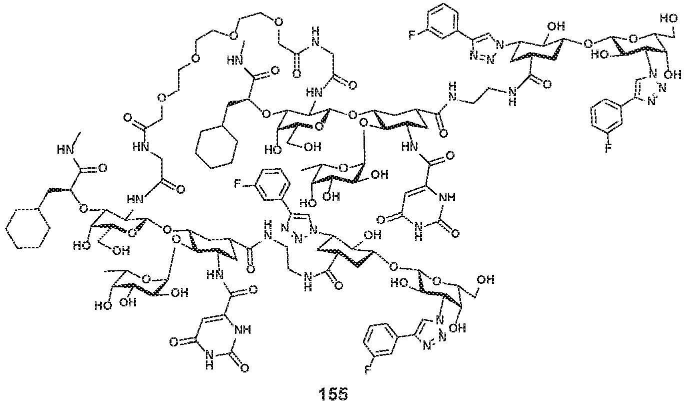

### EXAMPLE 121

### PROPHETIC SYNTHESIS OF MULTIMERIC COMPOUND 156

**Compound 156:** Compound **156** can be prepared in an analogous fashion to Figure 14 by replacing compound **22** with compound **32.**

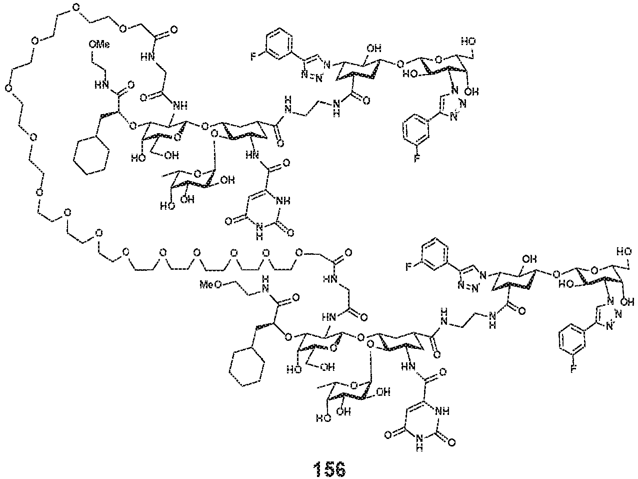

### EXAMPLE 122

### PROPHETIC SYNTHESIS OF MULTIMERIC COMPOUND 157

**Compound 157:** Compound **157** can be prepared in an analogous fashion to Figure 14 by replacing compound **22** with compound **33.**

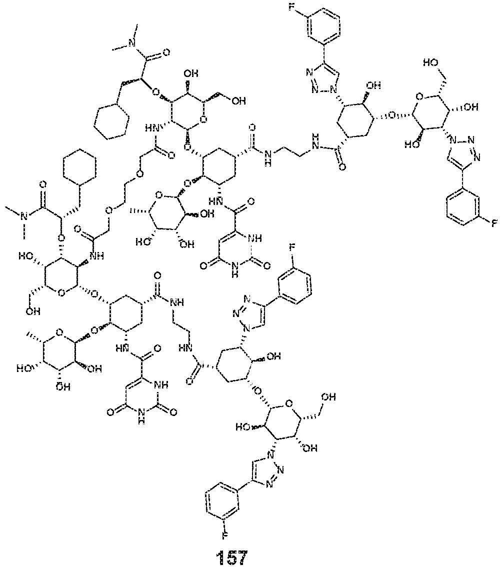

### EXAMPLE 123

### PROPHETIC SYNTHESIS OF MULTIMERIC COMPOUND 158

**Compound 158:** Compound **158** can be prepared in an analogous fashion to Figure 14 by replacing compound **22** with compound **34.**

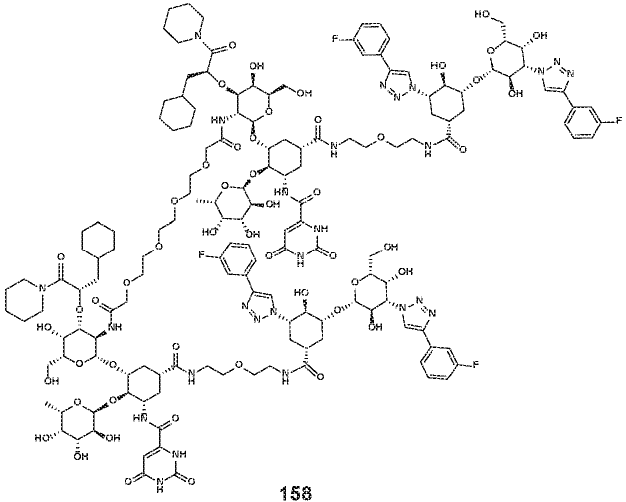

### EXAMPLE 124

### PROPHETIC SYNTHESIS OF MULTIMERIC COMPOUND 159

**Compound 159:** Compound **159** can be prepared in an analogous fashion to Figure 14 by replacing compound **22** with compound **37.**

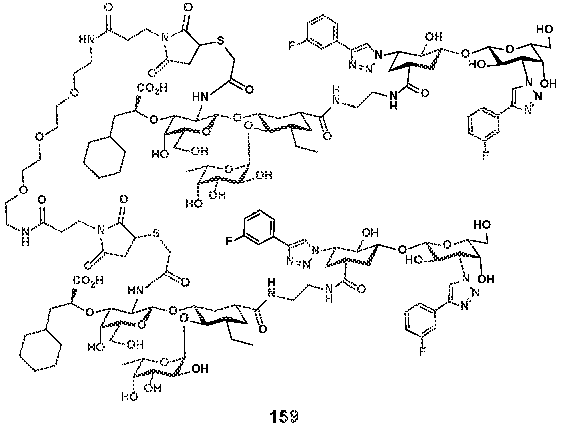

### EXAMPLE 125

### PROPHETIC SYNTHESIS OF MULTIMERIC COMPOUND 160

**Compound 160:** Compound **160** can be prepared in an analogous fashion to Figure 14 by replacing compound **22** with compound **38.**

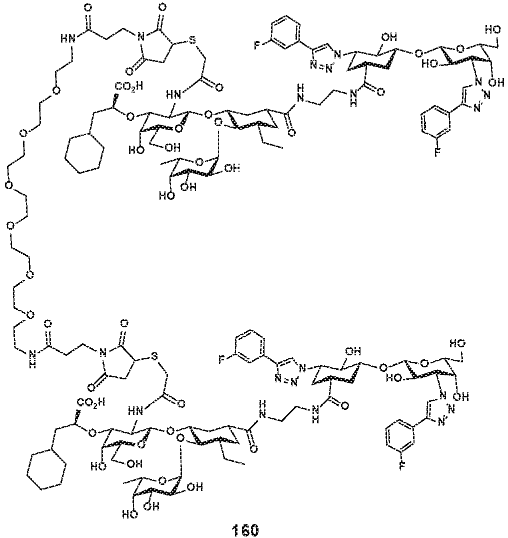

### EXAMPLE 126

### PROPHETIC SYNTHESIS OF MULTIMERIC COMPOUND 161

**Compound 161:** Compound **161** can be prepared in an analogous fashion to Figure 14 by replacing compound **22** with compound **39.**

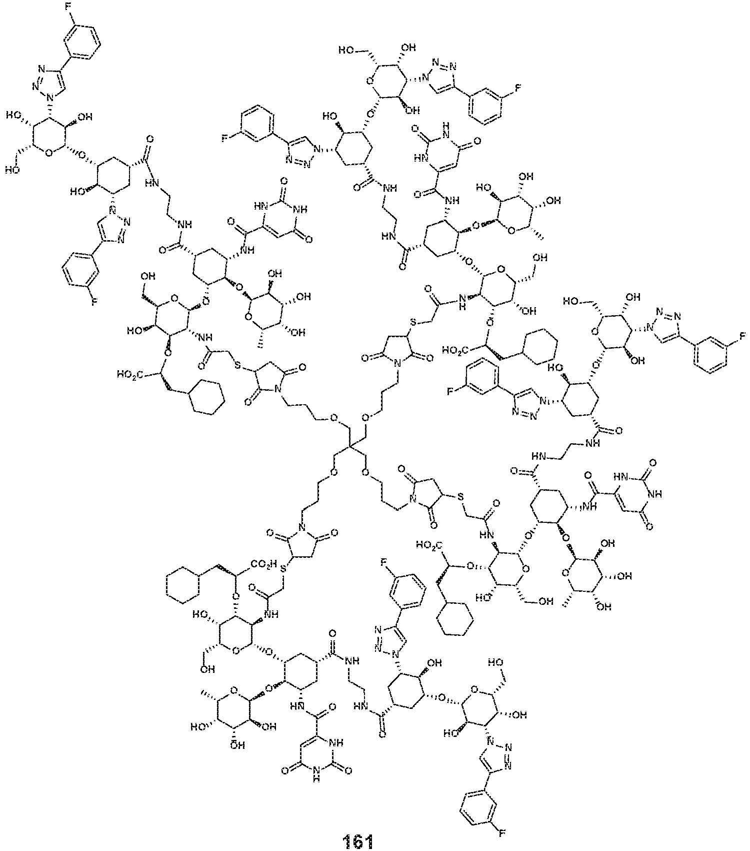

### EXAMPLE 127

### PROPHETIC SYNTHESIS OF MULTIMERIC COMPOUND 162

**Compound 162:** Compound **162** can be prepared in an analogous fashion to Figure 14 by replacing compound **22** with compound **40.**

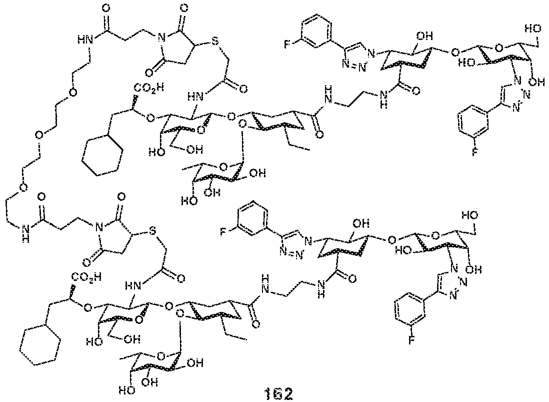

### EXAMPLE 128

### PROPHETIC SYNTHESIS OF MULTIMERIC COMPOUND 163

**Compound 163:** Compound **163** can be prepared in an analogous fashion to Figure 14 by replacing compound **22** with compound **46.**

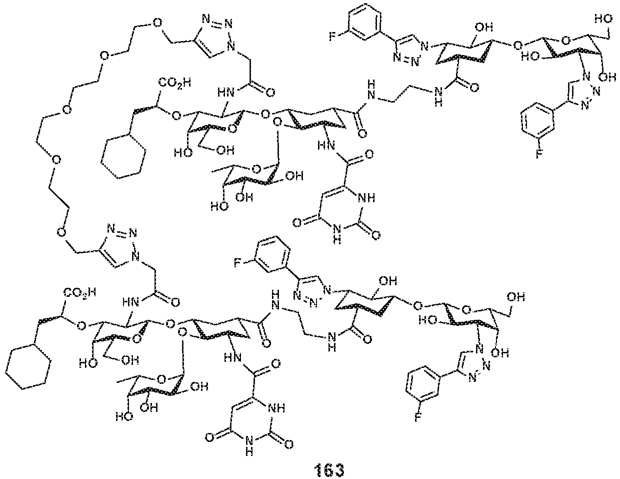

### EXAMPLE 129

### PROPHETIC SYNTHESIS OF MULTIMERIC COMPOUND 164

**Compound 164:** Compound **164** can be prepared in an analogous fashion to Figure 14 by replacing compound **22** with compound **47.**

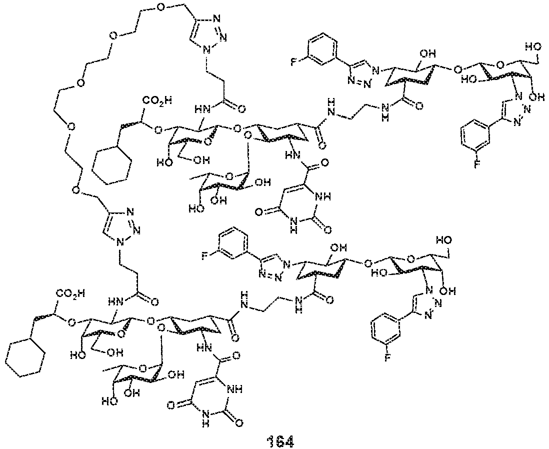

### EXAMPLE 130

### PROPHETIC SYNTHESIS OF MULTIMERIC COMPOUND 165

**Compound 165:** Compound **165** can be prepared in an analogous fashion to Figure 13 by replacing compound **22** with compound **48.**

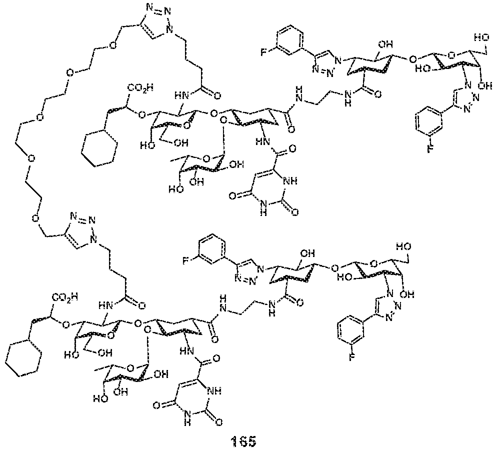

### EXAMPLE 131

### PROPHETIC SYNTHESIS OF MULTIMERIC COMPOUND 166

**Compound 166:** Compound **166** can be prepared in an analogous fashion to Figure 14 by replacing compound **22** with compound **49.**

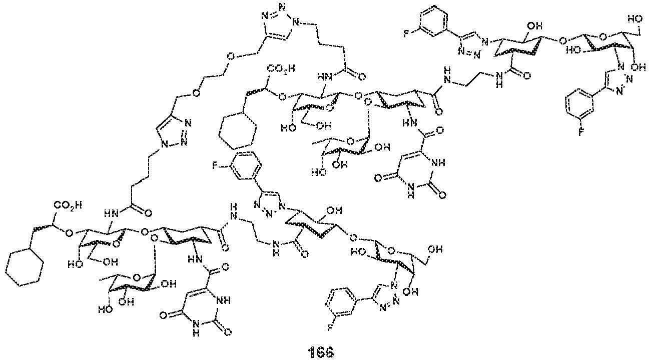

### EXAMPLE 132

### PROPHETIC SYNTHESIS OF MULTIMERIC COMPOUND 167

**Compound 167:** Compound **167** can be prepared in an analogous fashion to Figure 14 by replacing compound **22** with compound **50.**

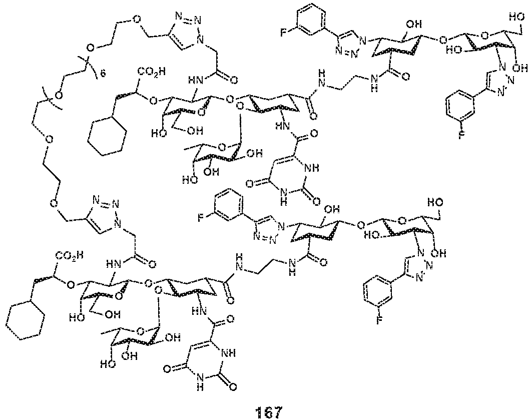

### EXAMPLE 133

### PROPHETIC SYNTHESIS OF MULTIMERIC COMPOUND 168

**Compound 168:** Compound **168** can be prepared in an analogous fashion to Figure 14 by replacing compound **22** with compound **51.**

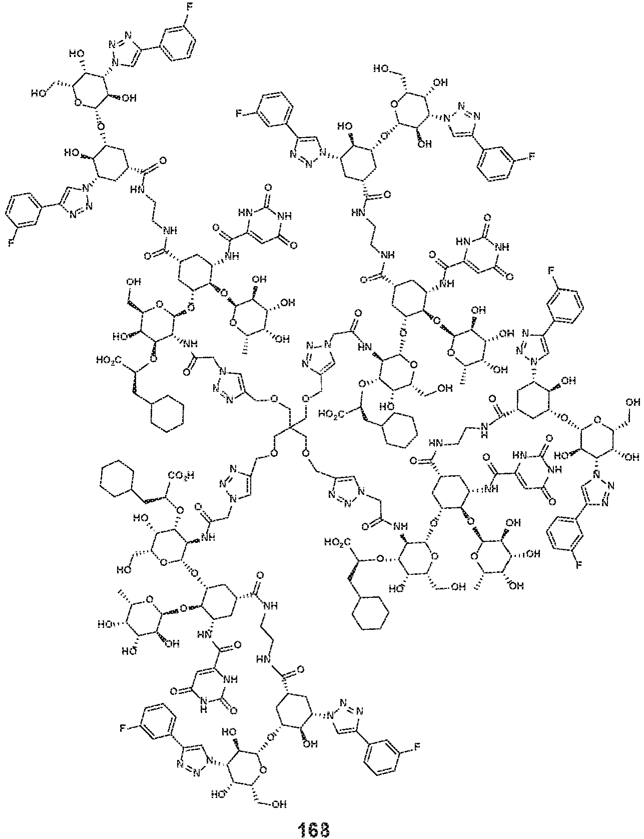

### EXAMPLE 134

### PROPHETIC SYNTHESIS OF MULTIMERIC COMPOUND 169

**Compound 169:** Compound **169** can be prepared in an analogous fashion to Figure 14 by replacing compound **22** with compound **52.**

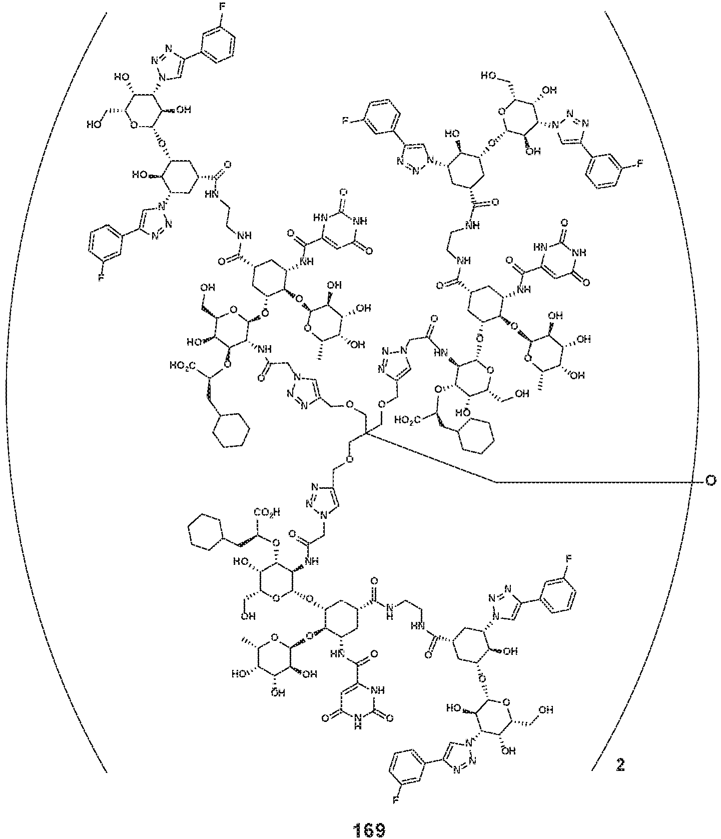

### EXAMPLE 135

### PROPHETIC SYNTHESIS OF MULTIMERIC COMPOUND 170

**Compound 170:** Compound **170** can be prepared in an analogous fashion to Figure 14 by replacing compound **22** with compound **53.**

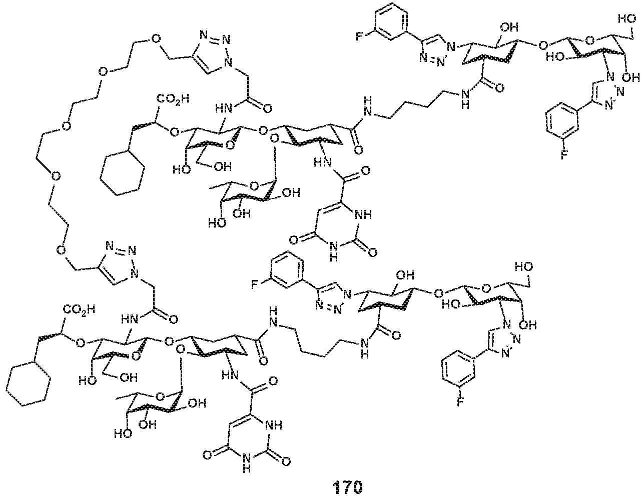

### EXAMPLE 136

### PROPHETIC SYNTHESIS OF MULTIMERIC COMPOUND 171

**Compound 171: Compound 171** can be prepared in an analogous fashion to Figure 14 by replacing compound **22** with compound **54.**

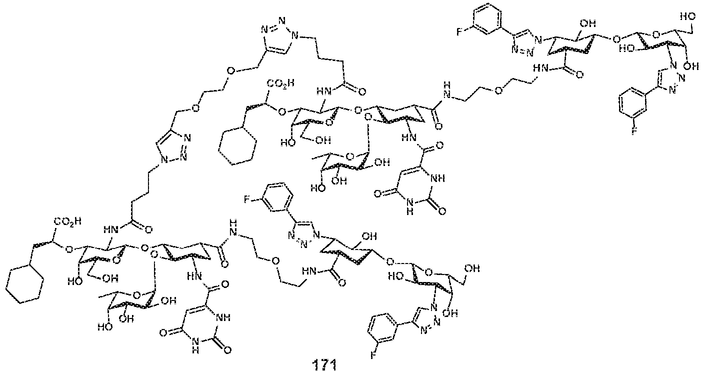

### EXAMPLE 137

### PROPHETIC SYNTHESIS OF MULTIMERIC COMPOUND 172

**Compound 172:** Compound **172** can be prepared in an analogous fashion to Figure 14 by replacing compound **22** with compound **56.**

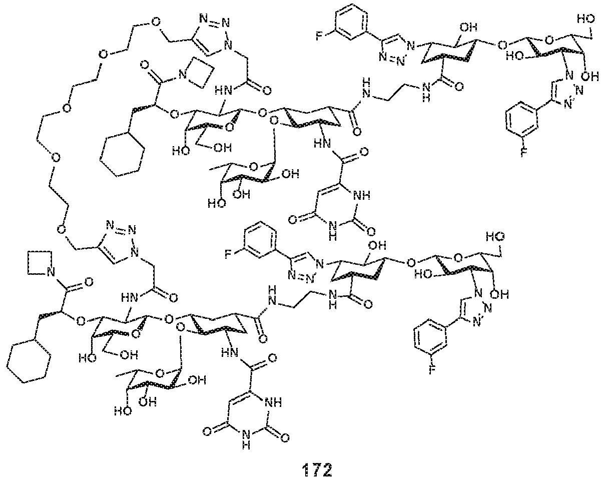

### EXAMPLE 138

### PROPHETIC SYNTHESIS OF MULTIMERIC COMPOUND 173

**Compound 173: Compound 173** can be prepared in an analogous fashion to Figure 14 by replacing compound **22** with compound **57.**

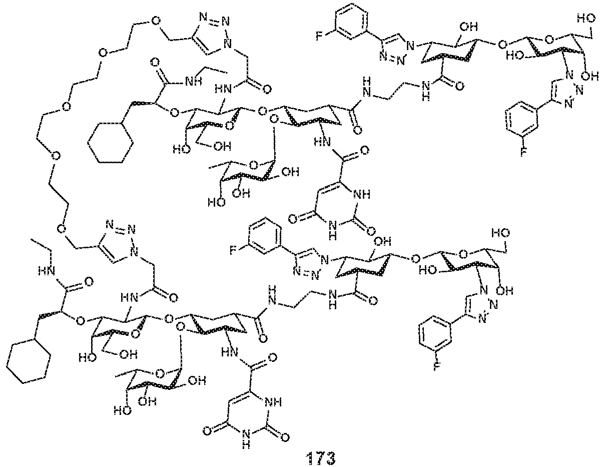

### EXAMPLE 139

### PROPHETIC SYNTHESIS OF MULTIMERIC COMPOUND 174

**Compound 174:** Compound **174** can be prepared in an analogous fashion to Figure 14 by replacing compound **22** with compound **58.**

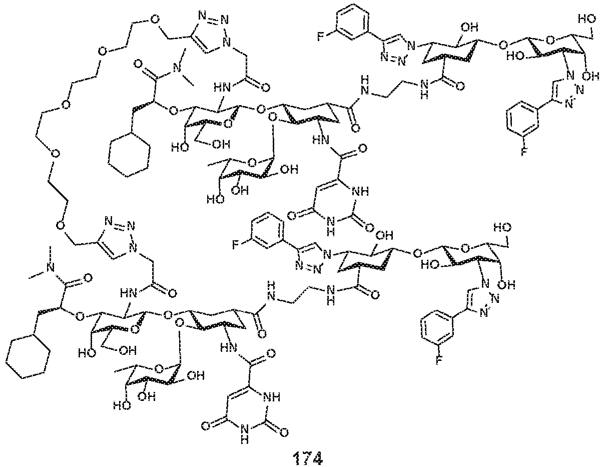

### EXAMPLE 140

### PROPHETIC SYNTHESIS OF MULTIMERIC COMPOUND 175

**Compound 175: Compound 175** can be prepared in an analogous fashion to Figure 14 by replacing compound **22** with compound **59.**

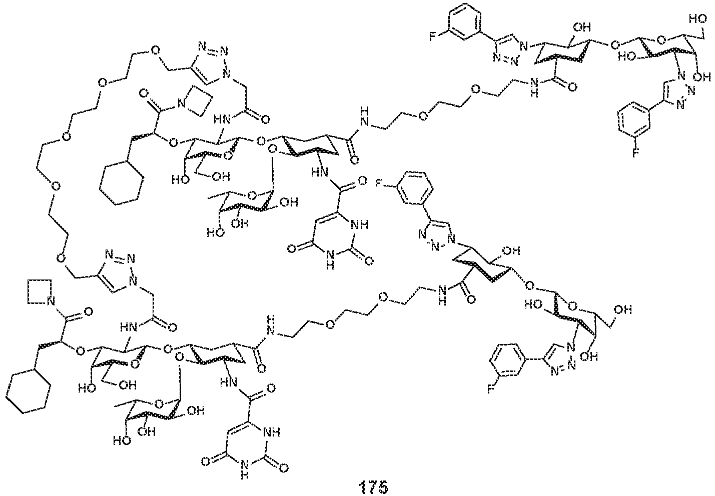

### EXAMPLE 141

### PROPHETIC SYNTHESIS OF MULTIMERIC COMPOUND 176

**Compound 176: Compound 176** can be prepared in an analogous fashion to Figure 14 by replacing compound **22** with compound **68.**

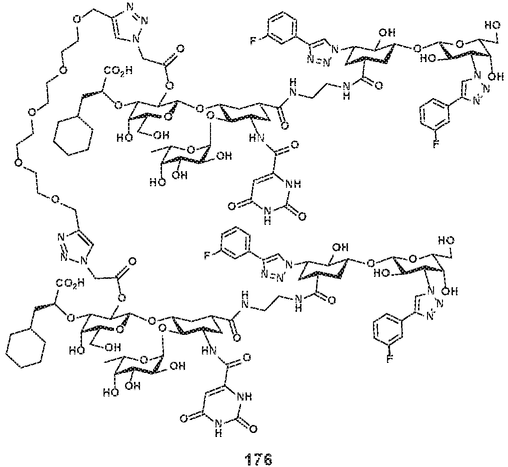

### EXAMPLE 142

### PROPHETIC SYNTHESIS OF MULTIMERIC COMPOUND 177

**Compound 177:** Compound **177** can be prepared in an analogous fashion to Figure 14 by replacing compound **22** with compound **69.**

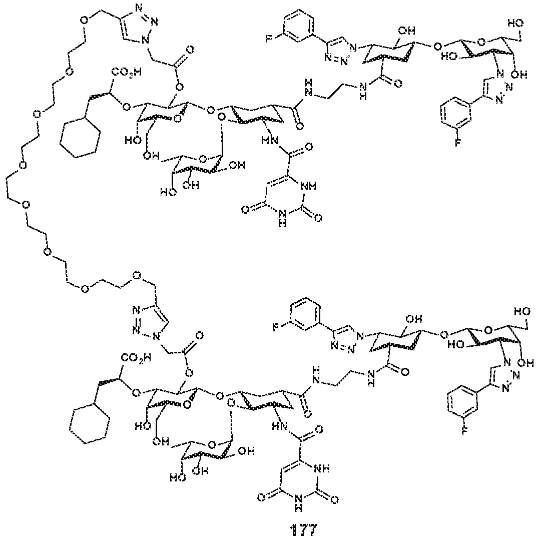

### EXAMPLE 143

### PROPHETIC SYNTHESIS OF MULTIMERIC COMPOUND 178

**Compound 178: Compound 178** can be prepared in an analogous fashion to Figure 14 by replacing compound **22** with compound **70.**

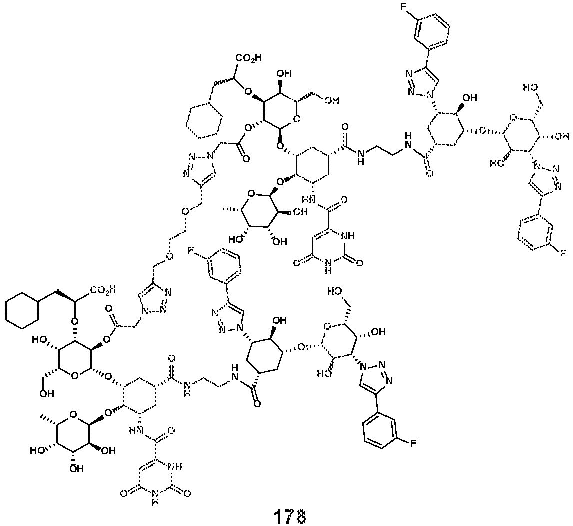

### EXAMPLE 144

### PROPHETIC SYNTHESIS OF MULTIMERIC COMPOUND 179

Compound 179: Compound 179 can be prepared in an analogous fashion to Figure 14 by replacing compound 22 with compound 71.

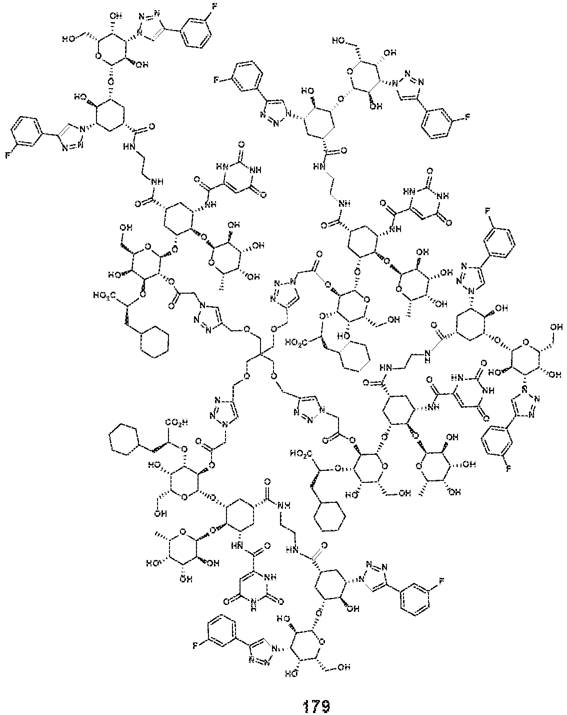

### EXAMPLE 145

### PROPHETIC SYNTHESIS OF MULTIMERIC COMPOUND 180

**Compound 180:** Compound **180** can be prepared in an analogous fashion to Figure 14 by replacing compound **22** with compound **73.**

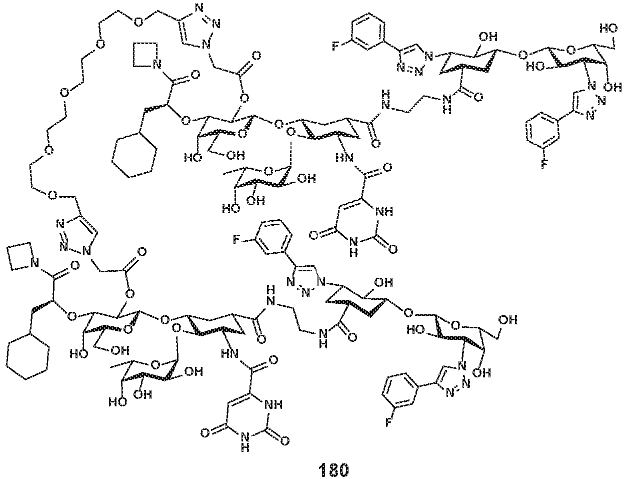

### EXAMPLE 146

### PROPHETIC SYNTHESIS OF MULTIMERIC COMPOUND 181

**Compound 181:** Compound **181** can be prepared in an analogous fashion to Figure 14 by replacing compound **22** with compound **78.**

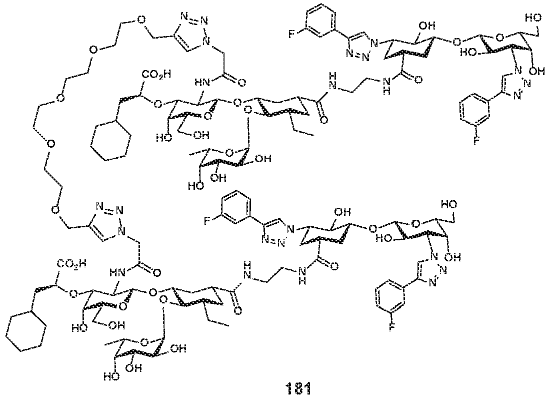

### EXAMPLE 147

### PROPHETIC SYNTHESIS OF MULTIMERIC COMPOUND 182

**Compound 182:** Compound **182** can be prepared in an analogous fashion to Figure 14 by replacing compound **22** with compound **79.**

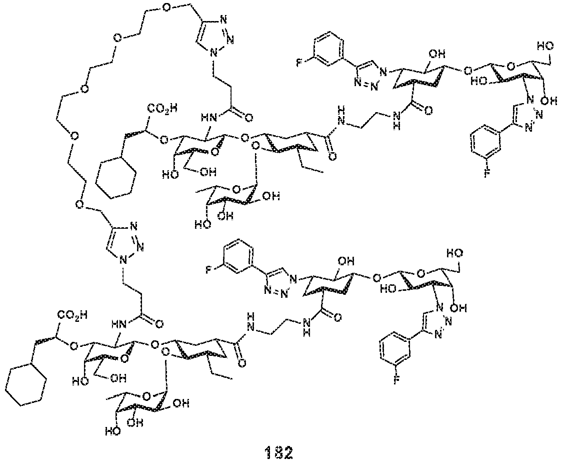

### EXAMPLE 148

### PROPHETIC SYNTHESIS OF MULTIMERIC COMPOUND 183

**Compound 183:** Compound **183** can be prepared in an analogous fashion to Figure 14 by replacing compound **22** with compound **80.**

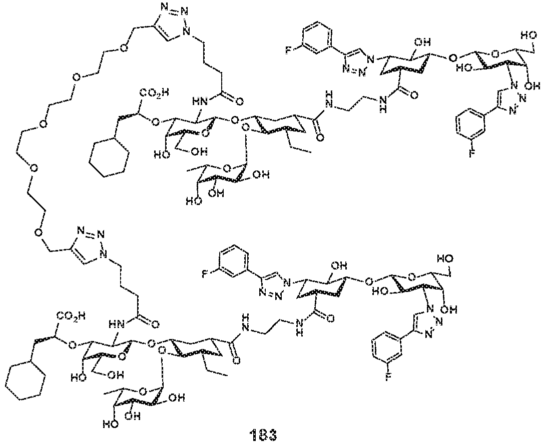

### EXAMPLE 149

### PROPHETIC SYNTHESIS OF MULTIMERIC COMPOUND 184

**Compound 184: Compound 184** can be prepared in an analogous fashion to Figure 14 by replacing compound **22** with compound **81.**

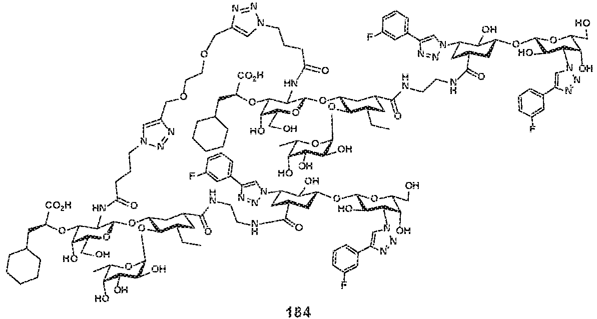

### EXAMPLE 150

### PROPHETIC SYNTHESIS OF MULTIMERIC COMPOUND 185

**Compound 185:** Compound **185** can be prepared in an analogous fashion to Figure 14 by replacing compound **22** with compound **82.**

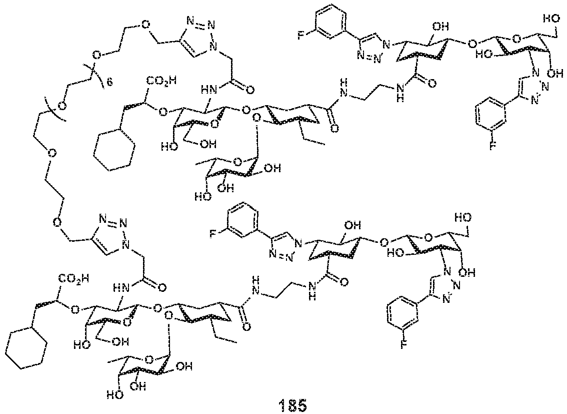

### EXAMPLE 151

### PROPHETIC SYNTHESIS OF MULTIMERIC COMPOUND 186

**Compound 186:** Compound **186** can be prepared in an analogous fashion to Figure 14 by replacing compound **22** with compound **83.**

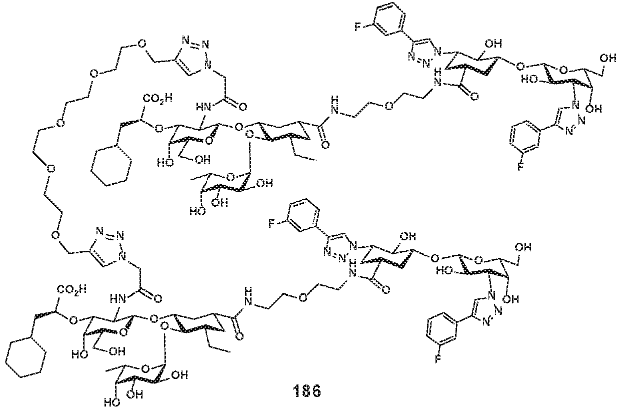

### EXAMPLE 152

### PROPHETIC SYNTHESIS OF MULTIMERIC COMPOUND 187

**Compound 187:** Compound **187** can be prepared in an analogous fashion to Figure 14 by replacing compound **22** with compound **84.**

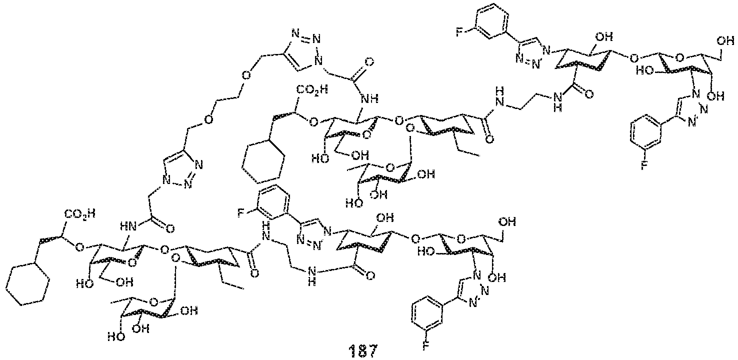

### EXAMPLE 153

### PROPHETIC SYNTHESIS OF MULTIMERIC COMPOUND 188

**Compound 188:** Compound **188** can be prepared in an analogous fashion to Figure 14 by replacing compound **22** with compound **85.**

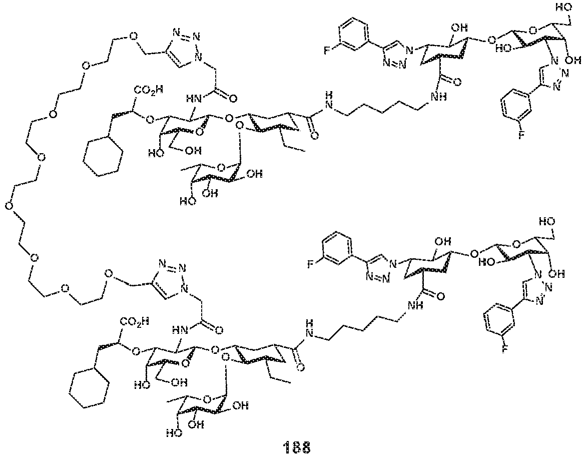

### EXAMPLE 154

### PROPHETIC SYNTHESIS OF MULTIMERIC COMPOUND 189

**Compound 189:** Compound **189** can be prepared in an analogous fashion to Figure 14 by replacing compound **22** with compound **87.**

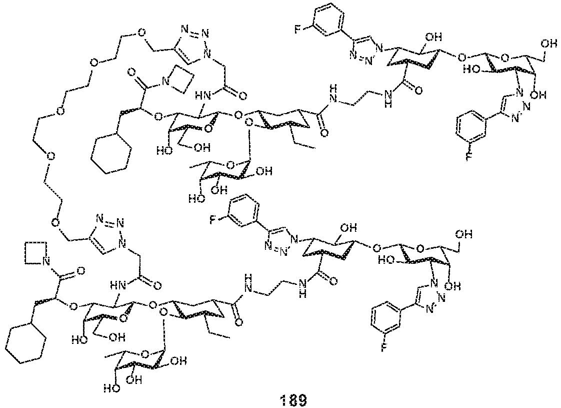

### EXAMPLE 155

### PROPHETIC SYNTHESIS OF MULTIMERIC COMPOUND 190

**Compound 190:** Compound **190** can be prepared in an analogous fashion to Figure 14 by replacing compound **22** with compound **88.**

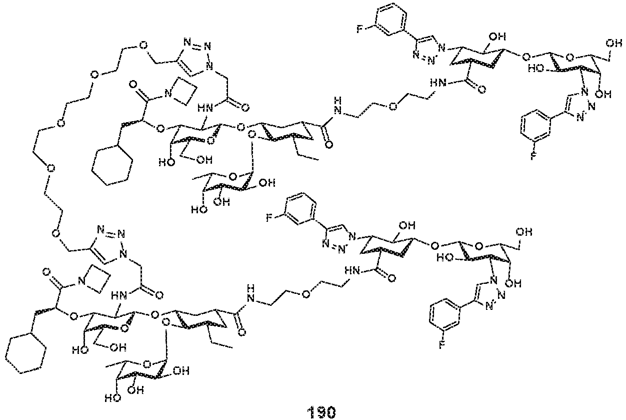

### EXAMPLE 156

### PROPHETIC SYNTHESIS OF MULTIMERIC COMPOUND 191

**Compound 191:** Compound **191** can be prepared in an analogous fashion to Figure 14 by replacing compound **22** with compound **89.**

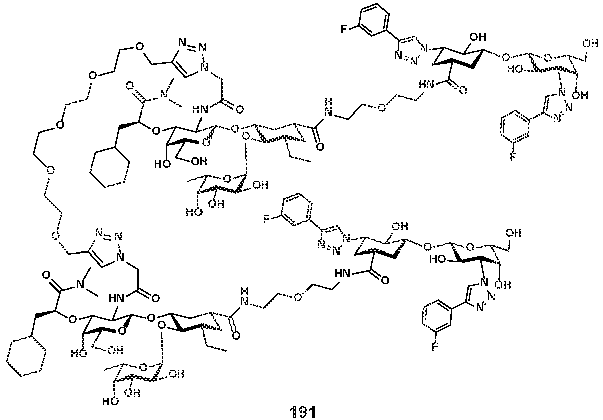

### EXAMPLE 157

### PROPHETIC SYNTHESIS OF MULTIMERIC COMPOUND 192

**Compound 192:** Compound **192** can be prepared in an analogous fashion to Figure 14 by replacing compound **22** with compound **90.**

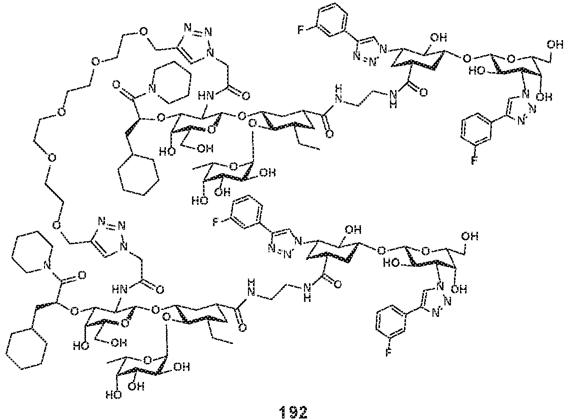

### EXAMPLE 158

### PROPHETIC SYNTHESIS OF MULTIMERIC COMPOUND 193

**Compound 193:** Compound **193** can be prepared in an analogous fashion to Figure 14 by replacing compound **22** with compound **91.**

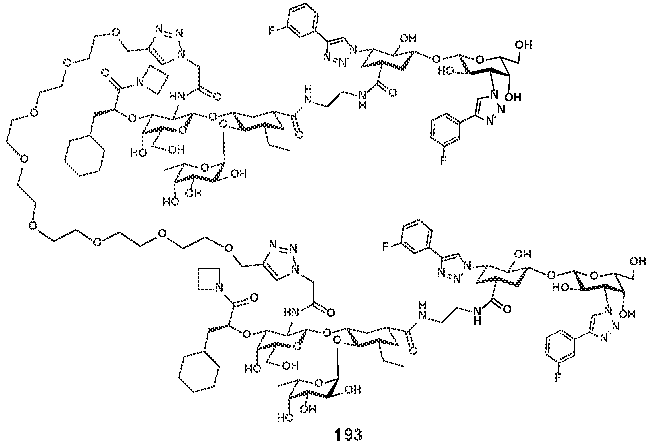

### EXAMPLE 159

### PROPHETIC SYNTHESIS OF MULTIMERIC COMPOUND 194

**Compound 194:** Compound **194** can be prepared in an analogous fashion to Figure 14 by replacing compound **22** with compound **92.**

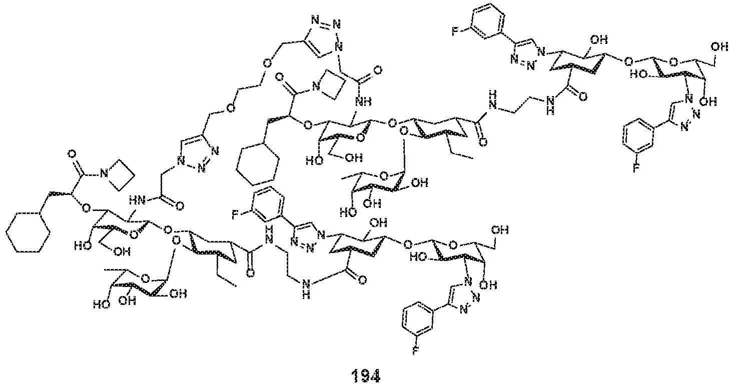

### EXAMPLE 160

### PROPHETIC SYNTHESIS OF MULTIMERIC COMPOUND 195

**Compound 195:** Compound **195** can be prepared in an analogous fashion to Figure 14 by replacing compound **22** with compound **93.**

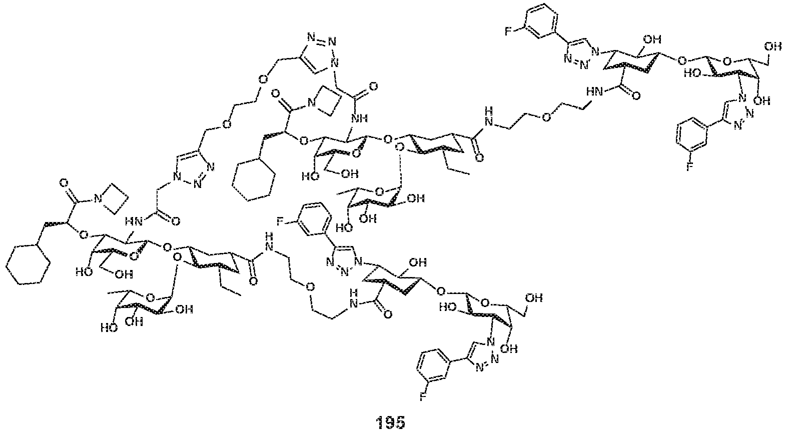

### EXAMPLE 161

### PROPHETIC SYNTHESIS OF MULTIMERIC COMPOUND 197

**Compound 197**: To a solution of compound **22** (1 eq) in anhydrous DMSO was acetic acid NHS ester (compound **196)** (5 eq). The mixture was stirred at ambient temperature for 12 hours. The solvent was removed *in vacuo* and the residue was purified by HPLC to afford compound 197.

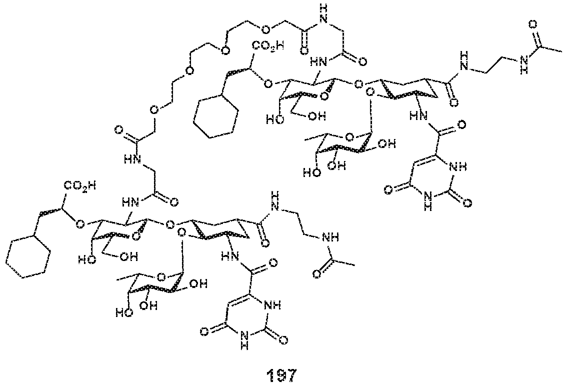

### EXAMPLE 162

### PROPHETIC SYNTHESIS OF MULTIMERIC COMPOUND 198

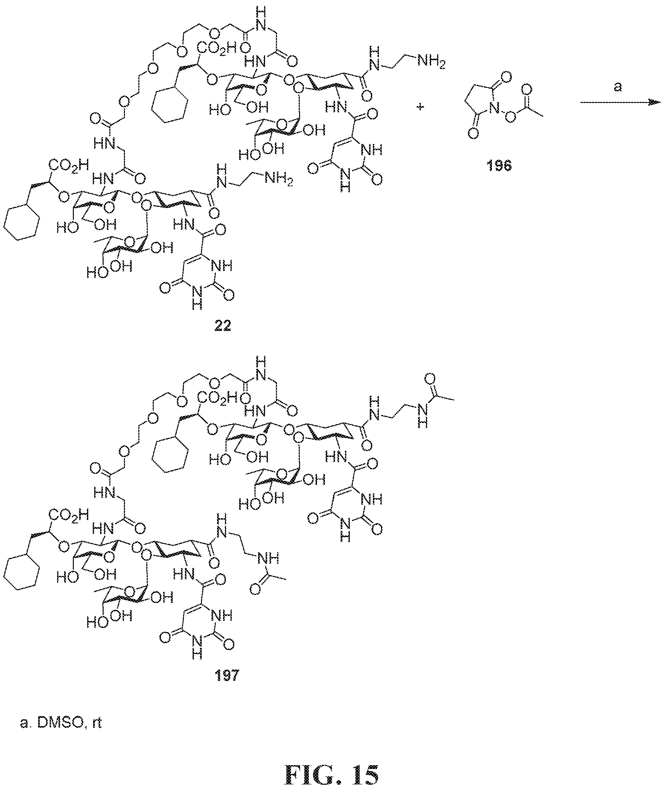

**Compound 198:** Compound **198** can be prepared in an analogous fashion to Figure 15 by replacing compound **196** with NHS-methoxyacetate.

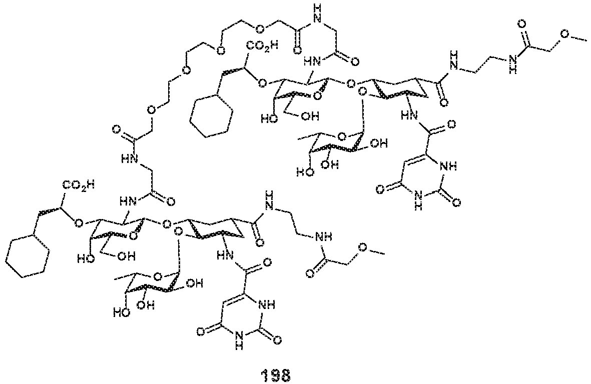

### EXAMPLE 163

### PROPHETIC SYNTHESIS OF MULTIMERIC COMPOUND 199

**Compound 199: Compound 199** can be prepared in an analogous fashion to Figure 15 by replacing compound **196** with PEG-12 propionic acid NHS ester.

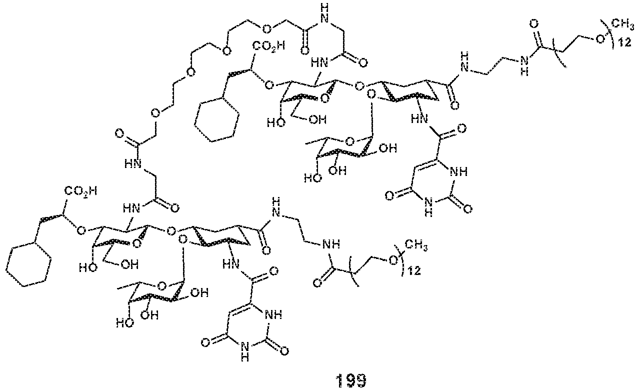

### EXAMPLE 164

### PROPHETIC SYNTHESIS OF MULTIMERIC COMPOUND 200

**Compound 200:** Compound **200** can be prepared in an analogous fashion to Figure 15 by replacing compound **22** with compound **78.**

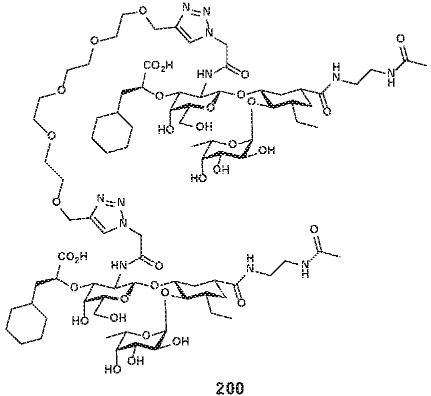

### EXAMPLE 165

### PROPHETIC SYNTHESIS OF MULTIMERIC COMPOUND 201

**Compound 201:** Compound **201** can be prepared in an analogous fashion to Figure 15 by replacing compound **22** with compound **78** and replacing compound **196** with NHS-methoxyacetate.

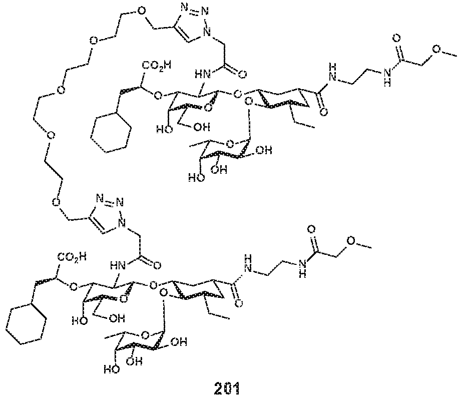

### EXAMPLE 166

### PROPHETIC SYNTHESIS OF MULTIMERIC COMPOUND 202

**Compound 202:** Compound **202** can be prepared in an analogous fashion to Figure 15 by replacing compound **22** with compound **78** and replacing compound **196** with PEG-12 propionic acid NHS ester.

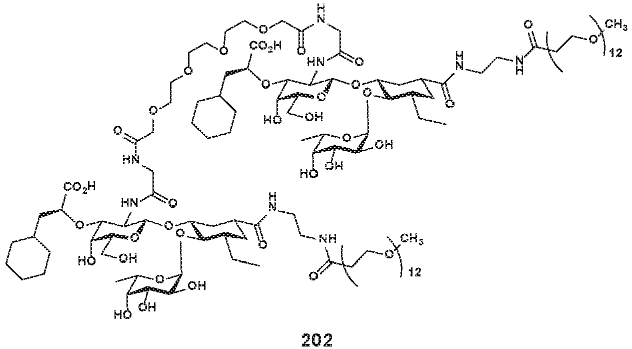

### PROPHETIC SYNTHESIS OF MULTIMERIC COMPOUND 203

**Compound 203:** Compound **203** can be prepared in an analogous fashion to Figure 15 by replacing compound **22** with compound **78.**

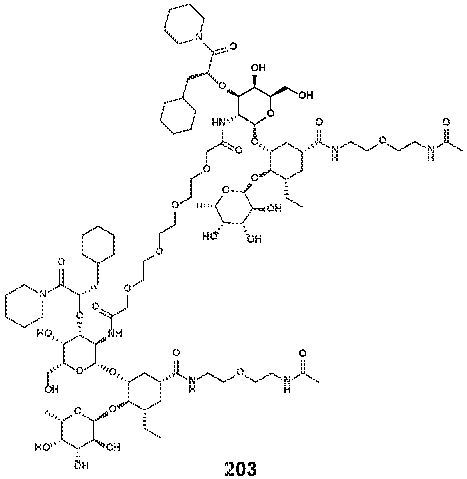

### EXAMPLE 167

### SYNTHESIS OF MULTIMERIC COMPOUND 206

**Compound 205:** A solution of compound **204** (synthesis described in Mead, G. et. al., Bioconj. Chem., 2015, 25, 1444 - 1452) (0.25 g, 0.53 mmole) and propiolic acid (0.33 mL, 5.30 mmole, 10 eq) in distilled water (1.5 mL) was degassed. A solution of CuSO₄/THPTA in distilled water (0.04 M) (1.3 mL, 53 µmole, 0.1 eq) and sodium ascorbate (21 mg, 0.11 mmole, 0.2 eq) were added successively and the resulting solution was stirred 3 hrs at room temperature. The reaction mixture was concentrated under reduced pressure and partially purified by C-18 column chromatography (water/MeOH, water only - 5/5, v/v). The resulting material was further purified by C-18 column chromatography eluting with water to afford compound **205** (0.16 g, 0.34 mmole, 64%). MS: (Calculated for C₈H₁₀₃N₃Na₃O₁₄S₃, 537.34), ES-Negative (513.5, M-Na-1).

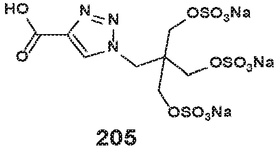

**Compound 206:** To a solution of compound **205** (7.5 mg, 14 µmole), DIPEA (2.4 µL, 14 µmole) and a catalytic amount of DMAP in DMF/DMSO (3/1, v/v, 0.15 mL) at 0 °C was added EDCI (1.6 mg, 8.22 µmole). The solution was stirred for 20 min. This solution was slowly added to a solution of compound **78** (5.0 mg, 2.7 µmole) in DMF/DMSO (3/1, v/v, 0.2 mL) cooled at 0 °C. The resulting solution was stirred 12 hrs allowing the reaction temperature to increase to room temperature. The reaction mixture was purified directly by HPLC. The product portions were collected, concentrated under reduced pressure, then lyophilized to give compound 206 as a white solid (0.4 mg, 1.15 µmole, 1.1%). MS: Calculated (C₉₈H₁₅₄N₁₈Na₆O₅₉S₆, 2856.7), ES-Negative (907.7, M/3; 881.0, M-1SO₃/3; 854.1 M-2SO₃/3; 685.8 M+1Na/4; 680.5 M/4); Fraction of RT = 10.65 min, 1399.4, M+7Na-1SO₃/2; 959.3 M+7Na/3; M+7Na-1SO₃/3; 724.8, M+8Na/4; 549.M+1Na/5; 460.9 M+2Na/6; 401.M+4Na/7).

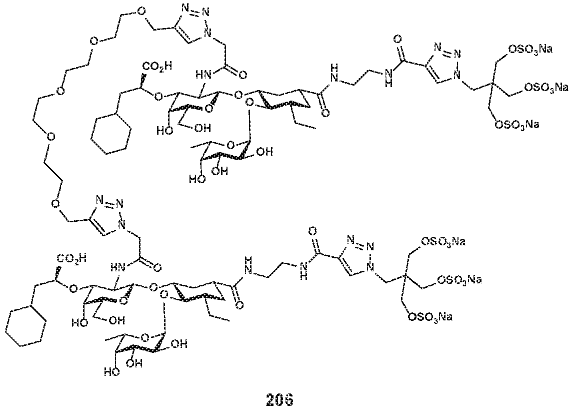

### EXAMPLE 168

### PROPHETIC SYNTHESIS OF MULTIMERIC COMPOUND 207

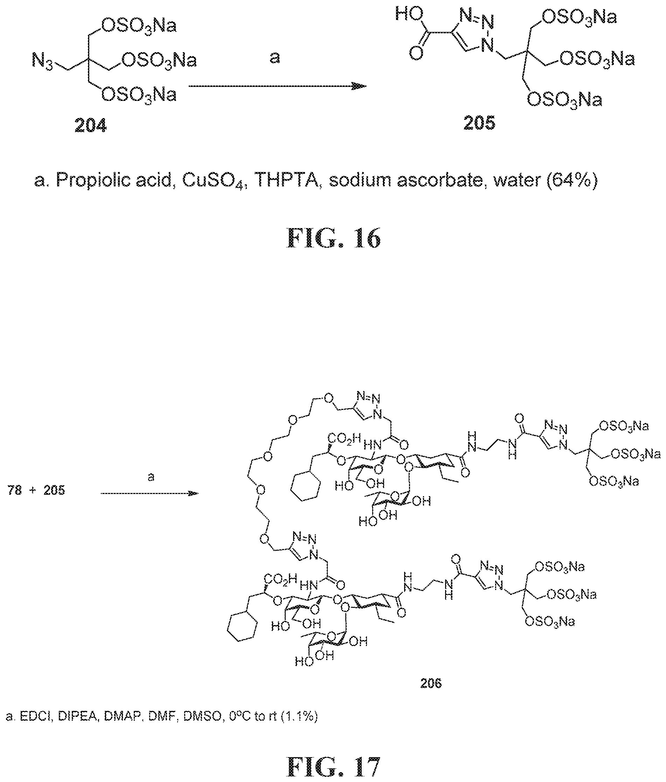

**Compound 207:** Compound **207** can be prepared in an analogous fashion to Figure 17 by replacing compound **78** with compound **22.**

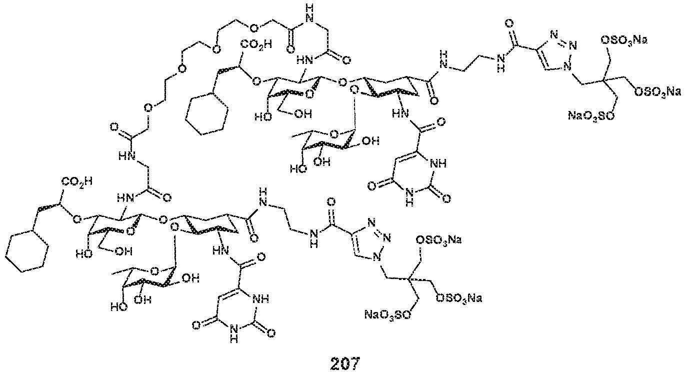

### EXAMPLE 169

### PROPHETIC SYNTHESIS OF MULTIMERIC COMPOUND 208

**Compound 208:** Compound **208** can be prepared in an analogous fashion to Figure 17 using compound **83** in place of compound **78.**

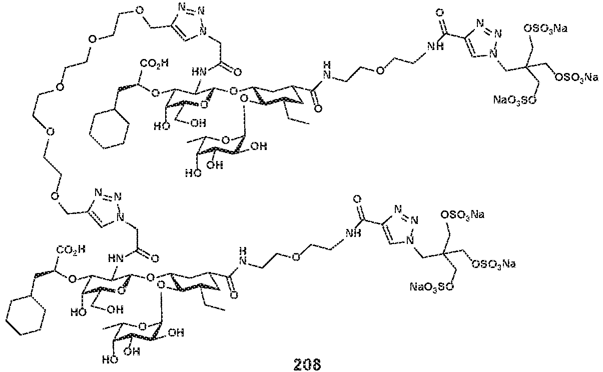

### EXAMPLE 170

### PROPHETIC SYNTHESIS OF MULTIMERIC COMPOUND 209

**Compound 209:** Compound **209** can be prepared in an analogous fashion to Figure 17 using compound **87** in place of compound **78.**

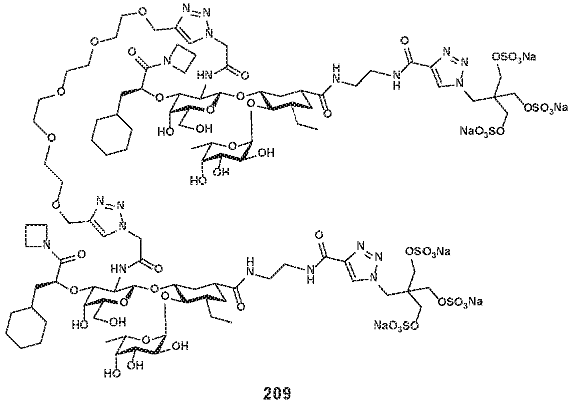

### EXAMPLE 171

### PROPHETIC SYNTHESIS OF MULTIMERIC COMPOUND 210

**Compound 210:** Compound **210** can be prepared in an analogous fashion to Figure 17 using compound **93** in place of compound **78.**

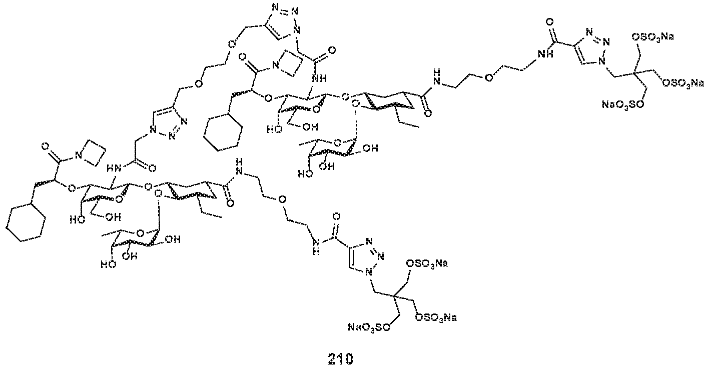

### EXAMPLE 172

### PROPHETIC SYNTHESIS OF MULTIMERIC COMPOUND 211

**Compound 211:** Compound **211** can be prepared in an analogous fashion to Figure 17 using compound **37** in place of compound **78.**

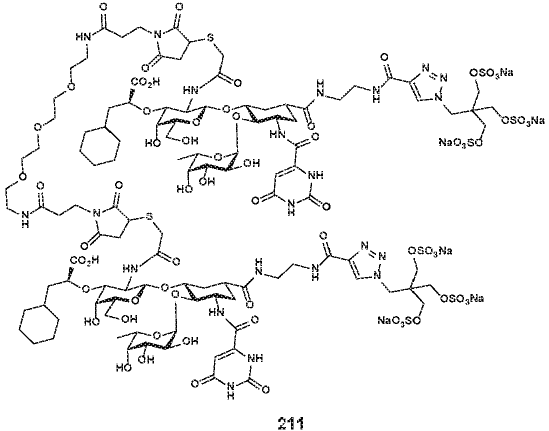

### EXAMPLE 173

### SYNTHESIS OF MULTIMERIC COMPOUND 218

**Compound 213:** Prepared according to Bioorg. Med. Chem. Lett. 1995, 5, 2321-2324 starting with D-threonolactone.

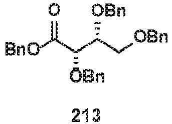

**Compound 214: Compound 213** (500 mg, 1 mmol) was dissolved in 9 mL acetonitrile. Potassium hydroxide (1 mL of a 2M solution) was added and the reaction mixture was stirred at 50°C for 12 hours. The reaction mixture was partitioned between dichloromethane and water. The phases were separated and the aqueous phase was extracted 3 times with dichloromethane. The aqueous phase was acidified with 1N HCl until pH ~ 1 and extracted 3 times with dichloromethane. The combined dichloromethane extracts from after acidification of the aqueous phase were concentrated in vacuo to give compound **214** as a yellow oil (406 mg). LCMS (C-18; 5-95 H₂O/MeCN): UV (peak at 4.973 min), positive mode: *m*/*z=* 407 [M+H]⁺; negative mode: *m*/*z=* 405 [M-H]⁻C₂₅H₂₆O₅ (406).

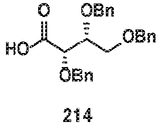

**Compound 215:** Prepared in an analogous fashion to compound **214** using L-erythronolactone as the starting material. LCMS (C-18; 5-95 H₂O/MeCN): ELSD (5.08 min), UV (peak at 4.958 min), positive mode: *m*/*z=* 407 [M+H]⁺; negative mode: *m*/*z=* 405 [M-H]⁻ C₂₅H₂₆O₅ (406).

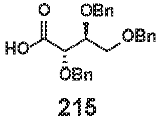

**Compound 216:** Prepared in an analogous fashion to compound **214** using L-threonolactone as the starting material. LCMS (C-18; 5-95 H₂O/MeCN). ELSD (5.08 min), UV (peak at 4.958 min), positive mode: *m*/*z=* 407 [M+H]⁺; negative mode: *m*/*z=* 405 [M-H]⁻ C₂₅H₂₆O₅ (406).

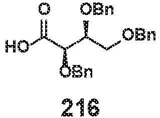

**Compound 217:** Prepared in an analogous fashion to compound **214** using D-erythronolactone as the starting material. LCMS (C-18; 5-95 H₂O/MeCN): ELSD (5.08 min), UV (peak at 4.958 min), positive mode: *m*/*z=* 407 [M+H]⁺; negative mode: *m*/*z=* 405 [M-H]⁻ C₂₅H₂₆O₅ (406).

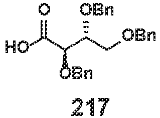

**Compound** 218: To a solution of compound 214 (3 eq) in anhydrous DMF was added HATU (3.3 eq) and DIPEA (5 eq). The mixture was stirred at ambient temperature for 15 minutes followed by addition of compound 78 (1 eq). The mixture was stirred at ambient temperature for 12h. The solvent was removed *in vacuo* and the residue was purified by HPLC to afford compound 218.

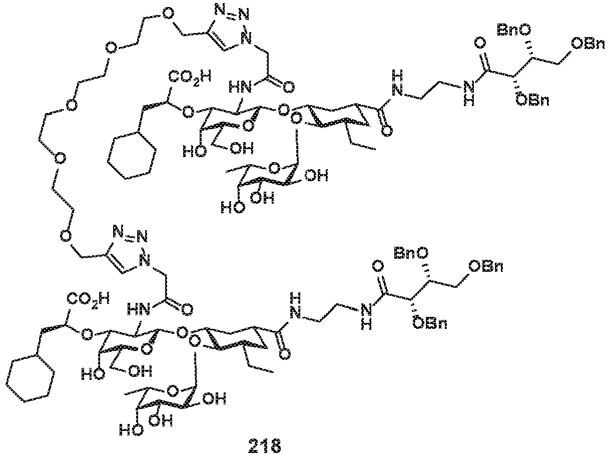

### EXAMPLE 174

### PROPHETIC SYNTHESIS OF MULTIMERIC COMPOUND 219

**Compound 219:** Compound **218** is dissolved in methanol and degassed. To this solution is added Pd(OH)₂/C. The reaction mixture is vigorously stirred under a hydrogen atmosphere for 12 hours. The reaction mixture is filtered through a Celite pad. The filtrate is concentrated under reduced pressure to give compound **219.**

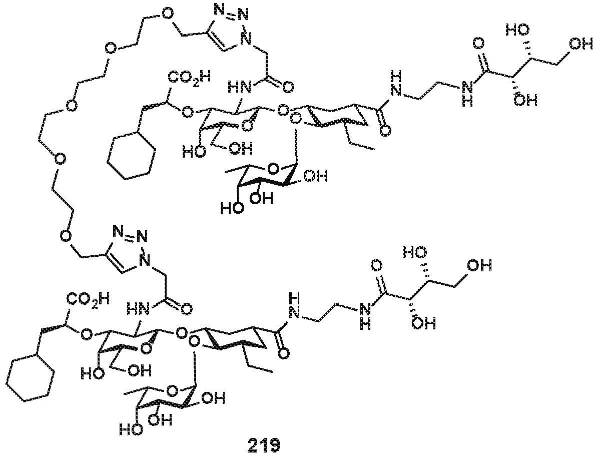

### EXAMPLE 175

### SYNTHESIS OF MULTIMERIC COMPOUND 220

**Compound 220:** A solution of the sulfur trioxide pyridine complex (100 eq) and compound **219** (1 eq) in pyridine was stirred at 67 °C for 1h. The reaction mixture was concentrated under vacuum. The resulting solid was dissolved in water and cooled to 0 °C. A 1N solution of NaOH was then added slowly until pH~10 and the latter was freeze dried. The resulting residue was purified by Gel Permeation (water as eluent). The collected fractions were lyophilised to give compound **220.**

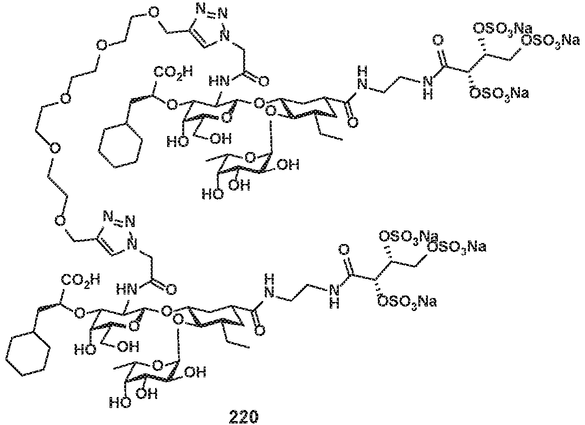

### EXAMPLE 176

### PROPHETIC SYNTHESIS OF MULTIMERIC COMPOUND 221

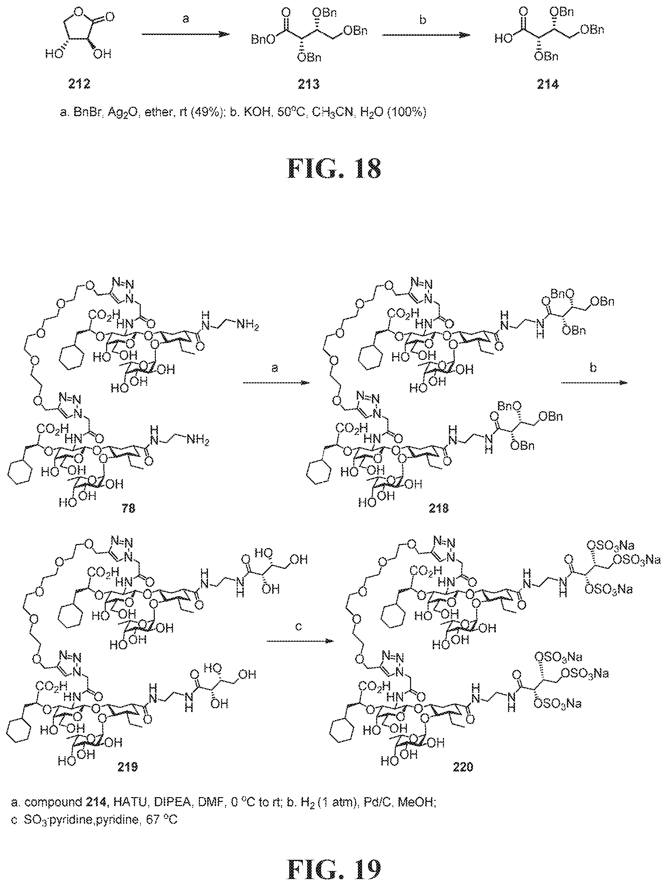

**Compound 221:** Compound **221** can be prepared in an analogous fashion to Figure 19 by replacing compound **214** with compound **215.**

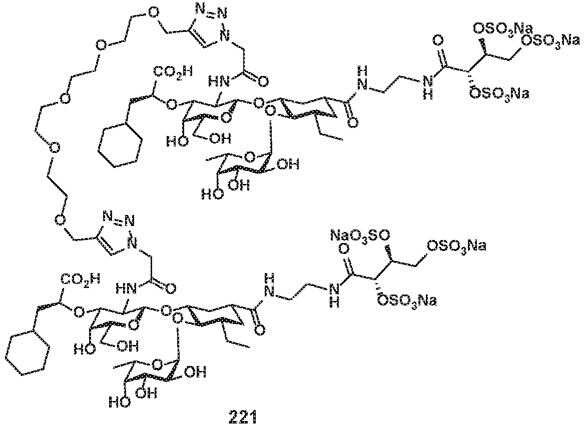

### EXAMPLE 177

### PROPHETIC SYNTHESIS OF MULTIMERIC COMPOUND 222

**Compound 222:** Compound **222** can be prepared in an analogous fashion to Figure 19 by replacing compound **214** with compound **216.**

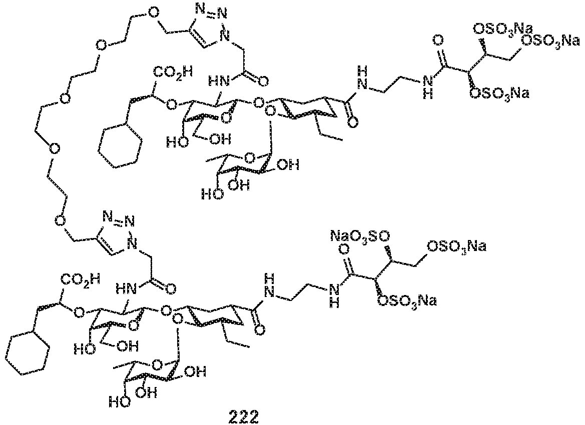

### EXAMPLE 178

### PROPHETIC SYNTHESIS OF MULTIMERIC COMPOUND 223

**Compound 223:** Compound **223** can be prepared in an analogous fashion to Figure 19 by replacing compound **214** with compound **217.**

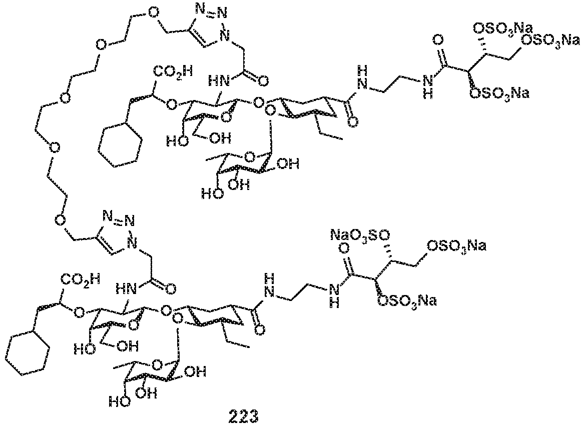

### EXAMPLE 179

### SYNTHESIS OF MULTIMERIC COMPOUND 224

**Compound 224:** To a solution of compound **78** in anhydrous DMSO was added a drop of DIPEA and the solution was stirred at room temperature until a homogeneous solution was obtained. A solution of succinic anhydride (2.2 eq) in anhydrous DMSO was added and the resulting solution was stirred at room temperature overnight. The solution was lyophilized to dryness and the crude product was purified by HPLC to give compound **224.**

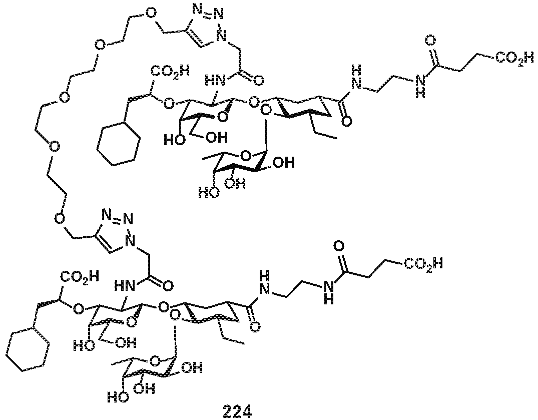

### EXAMPLE 180

### PROPHETIC SYNTHESIS OF MULTIMERIC COMPOUND 225

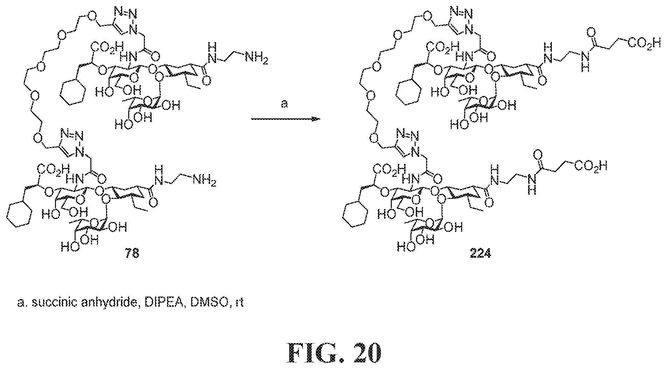

**Compound 225:** Compound **225** can be prepared in an analogous fashion to Figure 20 substituting glutaric anhydride for succinic anhydride.

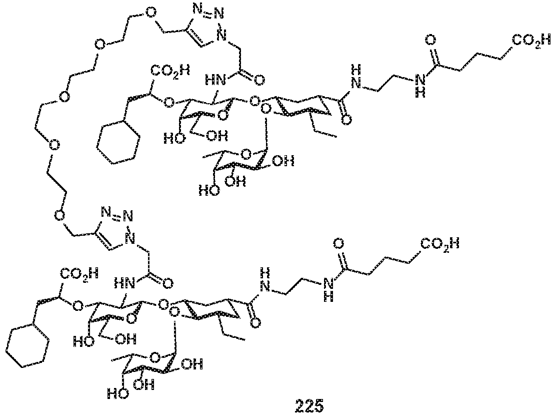

### EXAMPLE 181

### PROPHETIC SYNTHESIS OF MULTIMERIC COMPOUND 226

**Compound 226:** Compound **226** can be prepared in an analogous fashion to Figure 20 substituting compound **87** for compound **78.**

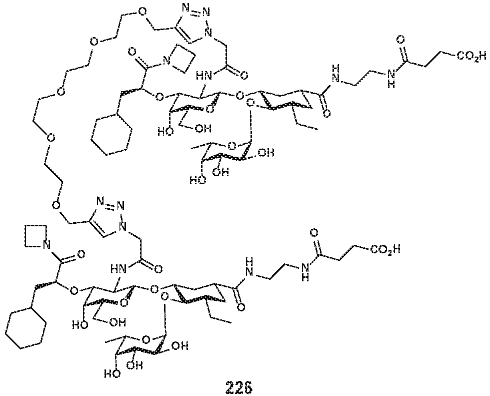

### EXAMPLE 182

### PROPHETIC SYNTHESIS OF MULTIMERIC COMPOUND 227

**Compound 227:** Compound **227** can be prepared in an analogous fashion to Figure 20 substituting phthalic anhydride for succinic anhydride.

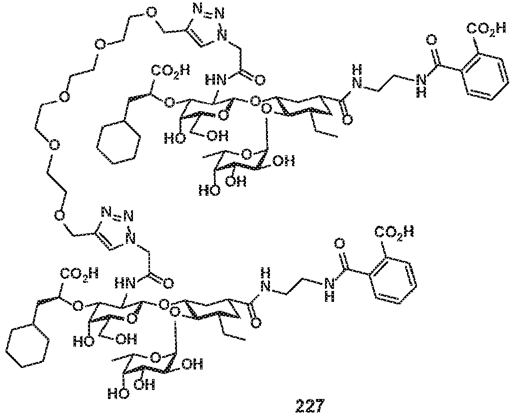

### EXAMPLE 183

### PROPHETIC SYNTHESIS OF MULTIMERIC COMPOUND 228

**Compound 228:** Compound **228** can be prepared in an analogous fashion to Figure 20 using compound **83** in place of compound **78.**

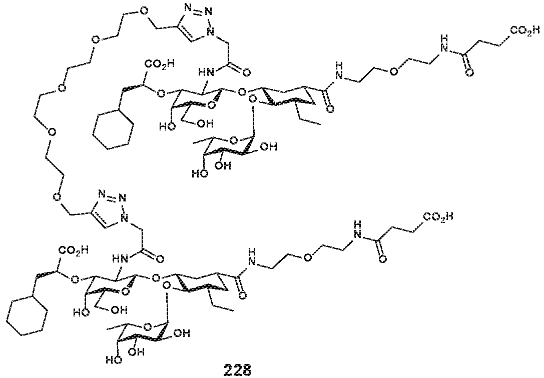

### EXAMPLE 184

### PROPHETIC SYNTHESIS OF MULTIMERIC COMPOUND 229

**Compound 229:** Compound **229** can be prepared in an analogous fashion to Figure 20 using compound **87** in place of compound **78.**

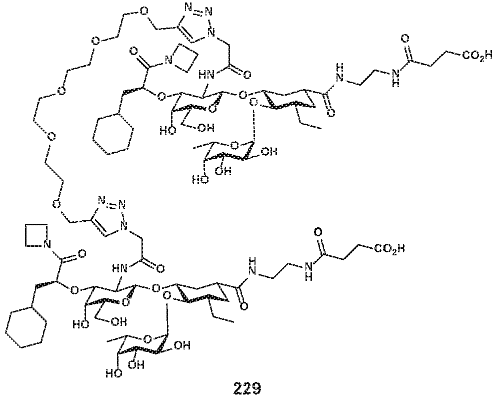

### EXAMPLE 185

### PROPHETIC SYNTHESIS OF MULTIMERIC COMPOUND 245

**Compound 231:** A mixture of compound **230** (preparation described in Schwizer, et. al., Chem. Eur. J., 2012, 18, 1342) and compound **2** (preparation described in WO 2013/096926) (1.7 eq) is azeotroped 3 times from toluene. The mixture is dissolved in DCM under argon and cooled on an ice bath. To this solution is added boron trifluoride etherate (1.5 eq). The reaction mixture is stirred 12 hours at room temperature. The reaction is quenched by the addition of triethylamine (2 eq). The reaction mixture is transferred to a separatory funnel and washed 1 time with half saturated sodium bicarbonate solution and 1 time with water. The organic phase is dried over sodium sulfate, filtered, and concentrated. The residue is purified by flash chromatography to afford compound **231.**

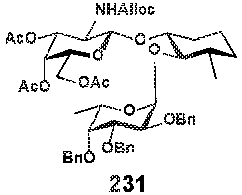

**Compound 232:** Compound **231** is dissolved in methanol at room temperature. A solution of sodium methoxide in methanol (0.1 eq) is added and the reaction mixture stirred overnight at room temperature. The reaction mixture is quenched by the addition of acetic acid. The reaction mixture is diluted with ethyl acetate, transferred to a separatory funnel and washed 2 times with water. The organic phase is dried over magnesium sulfate, filtered and concentrated. The residue is separated by flash chromatography to afford compound **232.**

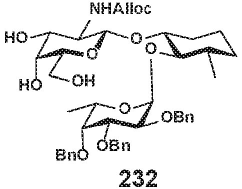

**Compound 233:** To a solution of compound **232** in dichloromethane cooled on an ice bath is added DABCO (1.5 eq) followed by monomethyoxytrityl chloride (1.2 eq). The reaction mixture is stirred overnight allowing to warm to room temperature. The reaction mixture is concentrated and the residue is purified by flash chromatography to afford compound 233.

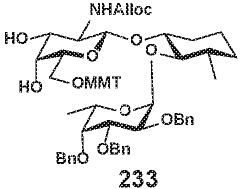

**Compound 234:** To a solution of compound **233** in methanol is added dibutyltin oxide (1.1 eq). The reaction mixture is refluxed for 3 hours then concentrated. The residue is suspended in DME. To this suspension is added compound 6 (preparation described in Thoma et. al. J. Med. Chem., 1999, 42, 4909) (1.5 eq) followed by cesium fluoride (1.2 eq). The reaction mixture is stirred at room temperature overnight. The reaction mixture is diluted with ethyl acetate, transferred to a separatory funnel, and washed with water. The organic phase is dried over sodium sulfate, filtered and concentrated. The residue is purified by flash chromatography to afford compound **234.**

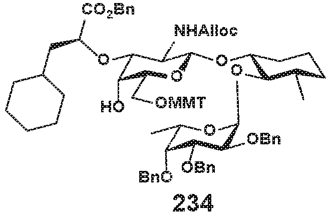

**Compound 235:** To a degassed solution of compound **234** in anhydrous DCM at 0 °C is added Pd(PPh₃)₄ (0.1 eq), Bu₃SnH (1.1 eq) and N-trifluoroacetyl glycine anhydride (2.0 eq) (preparation described in Chemische Berichte (1955), 88(1), 26). The resulting solution is stirred for 12 hrs allowing the temperature to increase to room temperature. The reaction mixture is diluted with DCM, transferred to a separatory funnel, and washed with water. The organic phase is dried over Na₂SO₄, then filtered and concentrated. The residue is purified by flash chromatography to afford compound **235.**

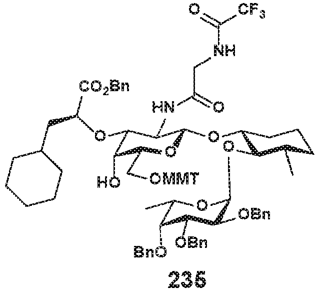

**Compound 236:** Compound **235** is dissolved in methanol and degassed. To this solution is added Pd(OH)₂/C. The reaction mixture is vigorously stirred under a hydrogen atmosphere for 12 hours. The reaction mixture is filtered through a Celite pad. The filtrate is concentrated under reduced pressure to give compound **236.**

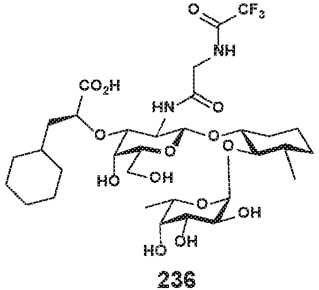

**Compound 237:** Compound **236** is dissolved in methanol at room temperature. A solution of sodium methoxide in methanol (1.1 eq) is added and the reaction mixture stirred overnight at room temperature. The reaction mixture is quenched by the addition of acetic acid. The reaction mixture is concentrated. The residue is separated by C-18 reverse phase chromatography to afford compound **237.**

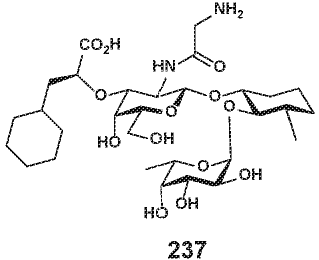

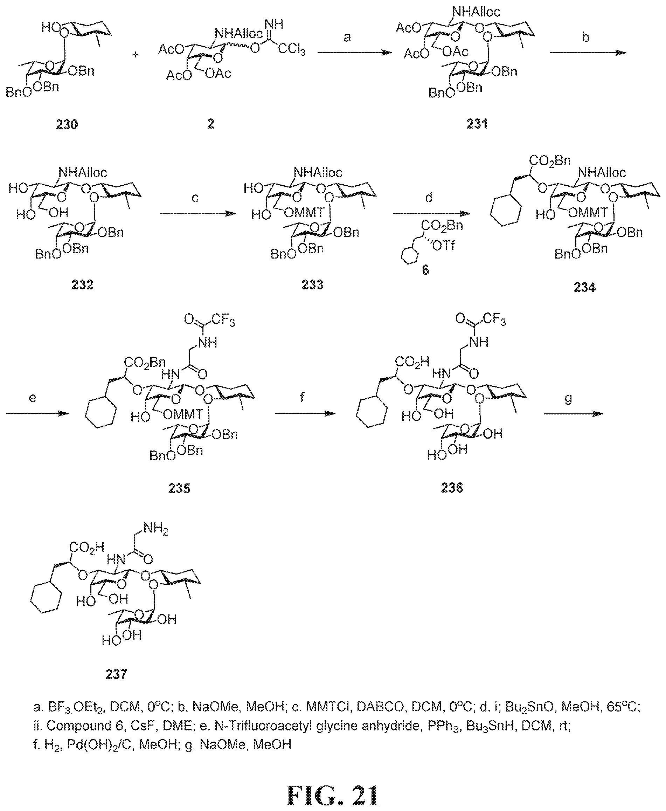

**Compound 238:** Compound **238** can be prepared in an analogous fashion to Figure 21 by substituting (acetylthio)acetyl chloride for N-trifluoroacetyl glycine anhydride in step e.

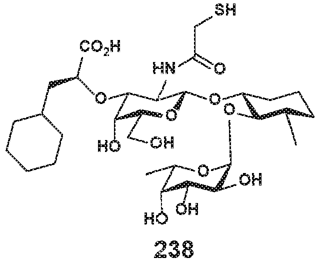

**Compound 239:** Compound **239** can be prepared in an analogous fashion to Figure 21 by substituting the vinylcyclohexyl analog of compound **230** (preparation described in Schwizer, et. al., Chem. Eur. J., 2012, 18, 1342) for compound **230** in step a.

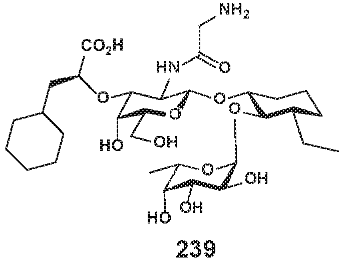

**Compound 240:** Compound **236** is dissolved in DMF and cooled on an ice bath. Diisopropylethylamine (1.5 eq) is added followed by HATU (1.1 eq). The reaction mixture is stirred 15 minutes on the ice bath then azetidine (2 eq) is added. The ice bath is removed and the reaction mixture is stirred overnight at room temperature. The solvent is removed under reduced pressure and the residue is separated by flash chromatography to afford compound **240.**

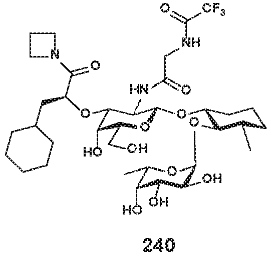

**Compound 241:** Compound **240** is dissolved in methanol at room temperature. A solution of sodium methoxide in methanol (0.3 eq) is added and the reaction mixture stirred overnight at room temperature. The reaction mixture is quenched by the addition of acetic acid. The reaction mixture is concentrated. The residue is separated by C-18 reverse phase chromatography to afford compound **241.**

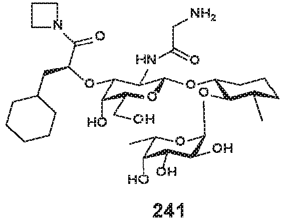

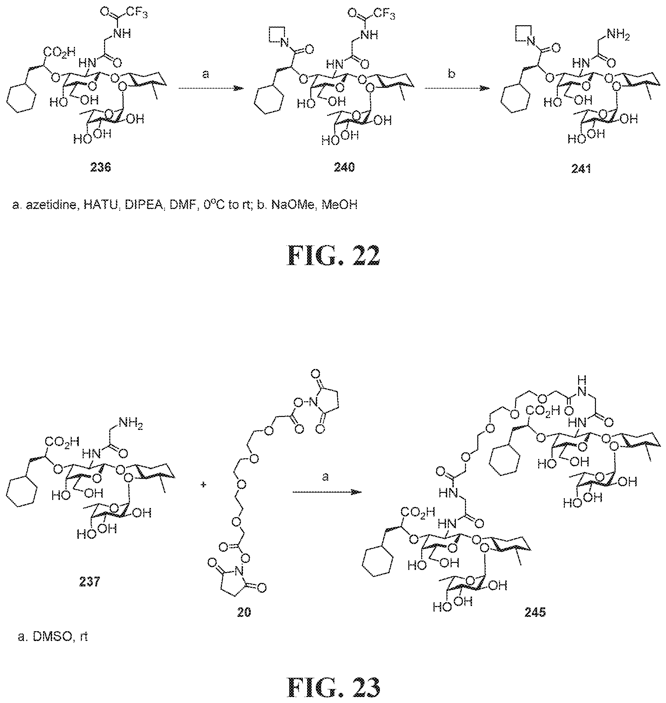

**Compound 242:** Compound **242** can be prepared in an analogous fashion to Figure 22 by using methylamine in place of azetidine in step a.

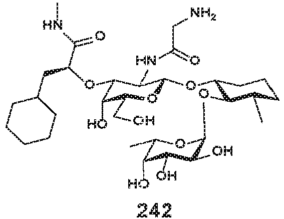

**Compound 243:** Compound **243** can be prepared in an analogous fashion to Figure 22 by using dimethylamine in place of azetidine in step a.

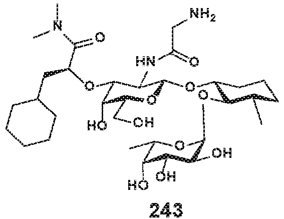

**Compound 244:** Compound **244** can be prepared in an analogous fashion to Figure 22 by using the ethylcyclohexyl analog of compound **236** in place of compound **236** in step a.

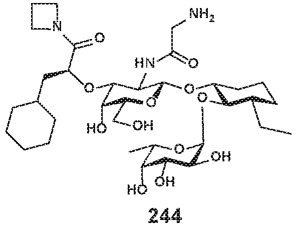

**Compound 245:** A solution of compound **20** (0.4 eq) in DMSO is added to a solution of compound **237** (1 eq) and DIPEA (10 eq) in anhydrous DMSO at room temperature. The resulting solution is stirred overnight. The reaction mixture is separated by reverse phase chromatography and the product lyophilized to give compound **245.**

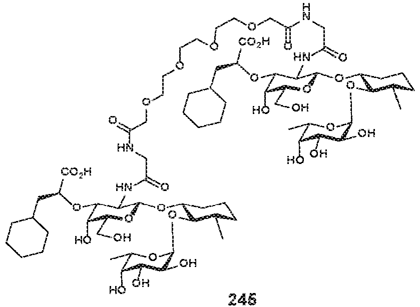

### EXAMPLE 186

### PROPHETIC SYNTHESIS OF MULTIMERIC COMPOUND 246

**Compound 246:** Compound **246** can be prepared in an analogous fashion to Figure 23 by replacing compound **20** with PEG-11 diacetic acid di-NHS ester.

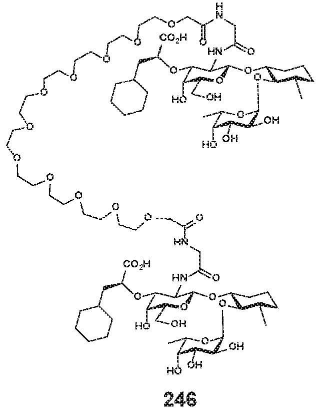

### EXAMPLE 187

### PROPHETIC SYNTHESIS OF MULTIMERIC COMPOUND 247

**Compound 247:** Compound **247** can be prepared in an analogous fashion to Figure 23 by replacing compound **20** with PEG-15 diacetic acid di-NHS ester.

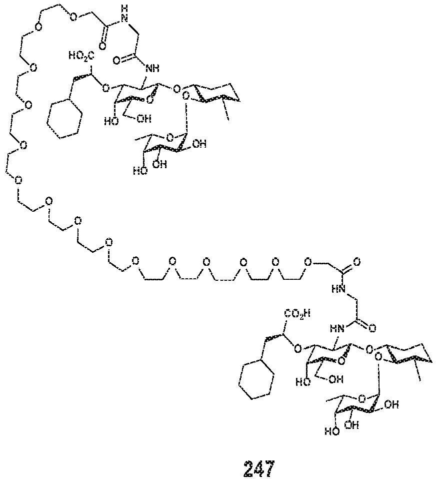

### EXAMPLE 188

### PROPHETIC SYNTHESIS OF MULTIMERIC COMPOUND 248

**Compound 248:** Compound **248** can be prepared in an analogous fashion to Figure 23 by replacing compound **20** with ethylene glycol diacetic acid di-NHS ester.

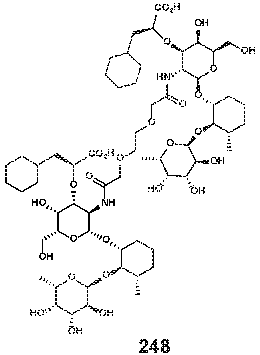

### EXAMPLE 189

### PROPHETIC SYNTHESIS OF MULTIMERIC COMPOUND 249

**Compound 249:** Compound **249** can be prepared in an analogous fashion to Figure 23 by replacing compound **20** with 3,3'-[[2,2-bis[[3-[(2,5-dioxo-1-pyrrolidinyl)oxy]-3-oxopropoxy]methyl]-1,3-propanediyl]bis(oxy)]bis-, 1,1'-bis(2,5-dioxo-1-pyrrolidinyl)-propanoic acid ester.

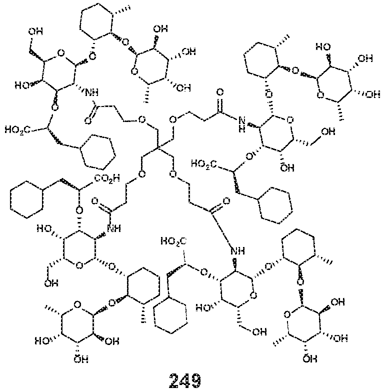

### EXAMPLE 190

### PROPHETIC SYNTHESIS OF MULTIMERIC COMPOUND 250

**Compound 250:** Compound **250** can be prepared in an analogous fashion to Figure 23 by replacing compound **237** with compound **239.**

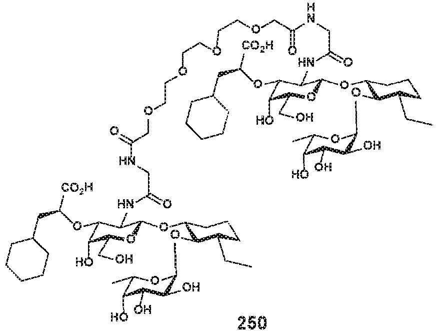

### EXAMPLE 191

### PROPHETIC SYNTHESIS OF MULTIMERIC COMPOUND 251

**Compound 251:** Compound **251** can be prepared in an analogous fashion to Figure 23 by replacing compound **237** with compound **241** and compound **20** with PEG-11 diacetic acid di-NHS ester.

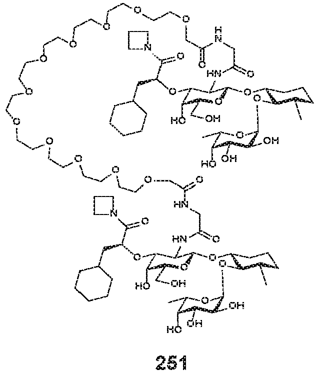

### EXAMPLE 192

### PROPHETIC SYNTHESIS OF MULTIMERIC COMPOUND 252

**Compound 252:** Compound **252** can be prepared in an analogous fashion to Figure 23 by replacing compound **237** with compound **242.**

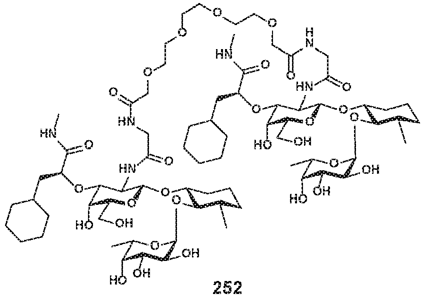

### EXAMPLE 193

### PROPHETIC SYNTHESIS OF MULTIMERIC COMPOUND 253

**Compound 253:** Compound **253** can be prepared in an analogous fashion to Figure 23 by replacing compound **237** with compound **243** and compound **20** with ethylene glycol diacetic acid di-NHS ester.

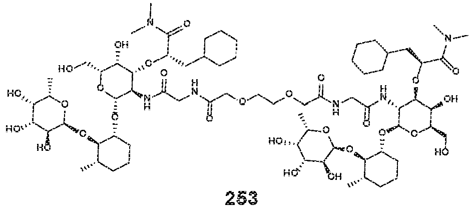

### EXAMPLE 194

### PROPHETIC SYNTHESIS OF MULTIMERIC COMPOUND 254

**Compound 254:** Compound **254** can be prepared in an analogous fashion to Figure 23 by replacing compound **237** with compound **244** and compound **20** with PEG-11 diacetic acid di-NHS ester.

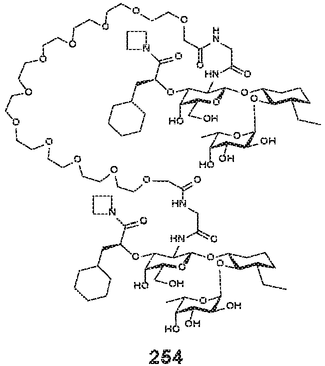

### EXAMPLE 195

### PROPHETIC SYNTHESIS OF MULTIMERIC COMPOUND 255

**Compound 255:** Compound **255** can be prepared in an analogous fashion to Figure 23 by replacing compound **237** with compound **241** and compound **20** with 1,1'-[oxybis[(1-oxo-2,1-ethanediyl)oxy]]bis-2,5-pyrrolidinedione.

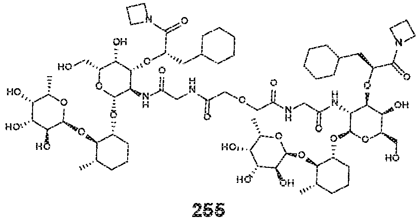

### EXAMPLE 196

### PROPHETIC SYNTHESIS OF MULTIMERIC COMPOUND 256

**Compound 256:** Compound **256** can be prepared in an analogous fashion to Figure 23 by replacing compound **237** with compound **244** and compound **20** with 1,1'-[oxybis[(1-oxo-2,1-ethanediyl)oxy]]bis-2,5-pyrrolidinedione.

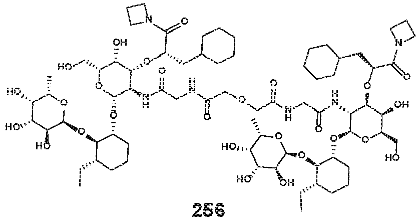

### EXAMPLE 197

### PROPHETIC SYNTHESIS OF MULTIMERIC COMPOUND 257

**Compound 257:** To a solution of compound **238** in MeOH at room temperature is added compound **35** followed by cesium acetate (2.5 eq). The reaction mixture is stirred at room temperature until completion. The solvent is removed under reduced pressure. The product is purified by reverse phase chromatography to give compound **257.**

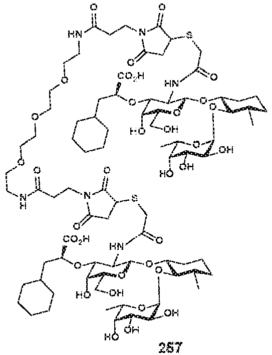

### EXAMPLE 198

### PROPHETIC SYNTHESIS OF MULTIMERIC COMPOUND 258

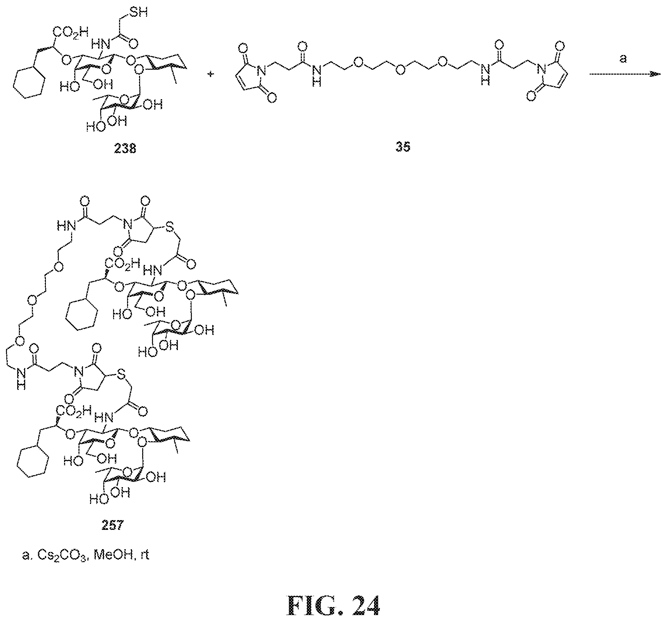

**Compound 258:** Compound **258** can be prepared in an analogous fashion to Figure 24 by substituting PEG-6-bis maleimidoylpropionamide for compound **35.**

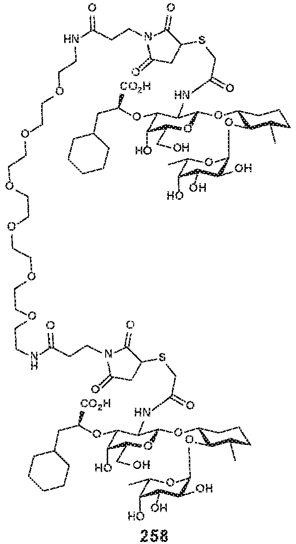

### EXAMPLE 199

### PROPHETIC SYNTHESIS OF MULTIMERIC COMPOUND 259

**Compound 259:** Compound **259** can be prepared in an analogous fashion to Figure 24 by substituting compound **35** for, 1,1'-[[2,2-bis[[3-(2,5-dihydro-2,5-dioxo-1*H*-pyrrol-1-yl)propoxy]methyl]-1,3-propanediyl]bis(oxy-3,1-propanediyl)]bis-1*H*-pyrrole-2,5-dione.

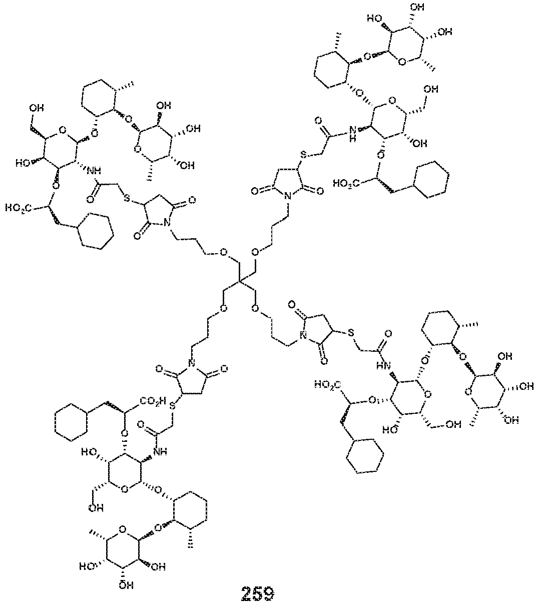

### EXAMPLE 200

### PROPHETIC SYNTHESIS OF MULTIMERIC COMPOUND 261

**Compound 260:** To a degassed solution of compound **234** in anhydrous DCM at 0 °C is added Pd(PPh₃)₄ (0.1 eq), Bu₃SnH (1.1 eq) and azidoacetic anhydride (2.0 eq). The ice bath is removed and the solution is stirred for 12 hrs under a N₂ atmosphere at room temperature. The reaction mixture is diluted with DCM, washed with water, dried over Na₂SO₄, then concentrated. The crude product is purified by column chromatography to give compound **260.**

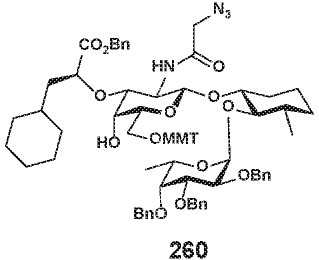

**Compound 261:** A solution of bis-propagyl PEG-5 (compound **43)** and compound 260 (2.4 eq) in MeOH is degassed at room temperature. A solution of CuSO₄/THPTA in distilled water (0.04 M) (0.2 eq) and sodium ascorbate (0.2 eq) are added successively and the resulting solution is stirred 12 hrs at 70°C. The solution is cooled to room temperature and concentrated under reduced pressure. The crude product is purified by chromatography to give compound 261.

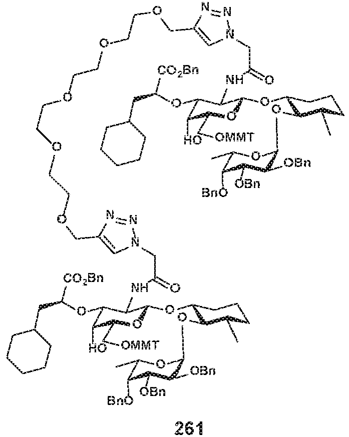

### EXAMPLE 201

### PROPHETIC SYNTHESIS OF MULTIMERIC COMPOUND 262

**Compound 262:** Compound **261** is dissolved in MeOH and hydrogenated in the presence of Pd(OH)₂ (20 wt %) at 1 atm of H₂ gas pressure for 24 hrs at room temperature. The solution is filtered through a Celite pad. The filtrate is concentrated to give compound **262.**

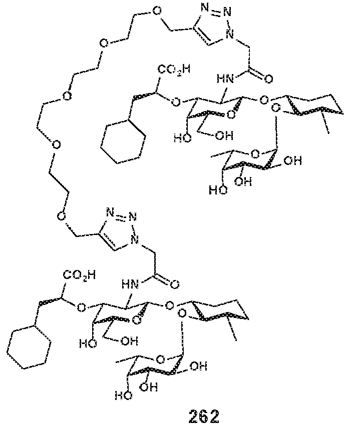

### EXAMPLE 202

### PROPHETIC SYNTHESIS OF MULTIMERIC COMPOUND 263

**Compound 263:** Compound **262** is dissolved in DMF and cooled on an ice bath. Diisopropylethylamine (2.5 eq) is added followed by HATU (2.2 eq). The reaction mixture is stirred 15 minutes on the ice bath then azetidine (10 eq) is added. The ice bath is removed and the reaction mixture is stirred overnight at room temperature. The solvent is removed under reduced pressure and the residue is separated by reverse phase chromatography to afford compound **263.**

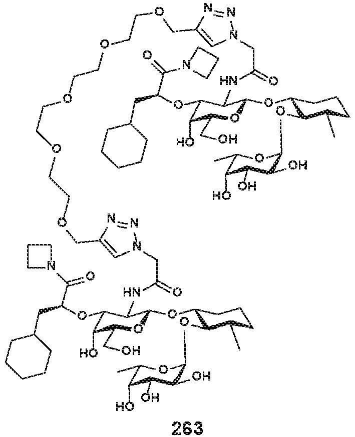

### EXAMPLE 203

### PROPHETIC SYNTHESIS OF MULTIMERIC COMPOUND 264

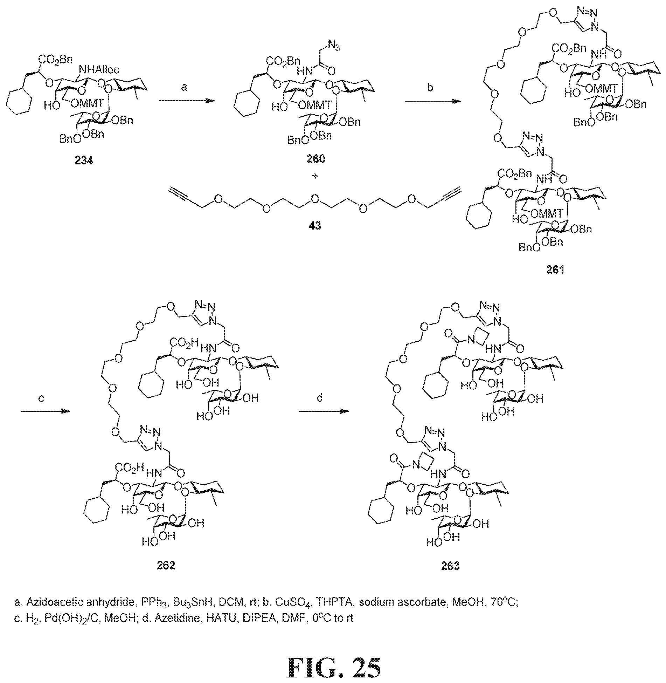

**Compound 264:** Compound **264** can be prepared in an analogous fashion to Figure 25 using 4,7,10,13,16,19,22,25,28,31-decaoxatetratriaconta-1, 33-diyne in place of compound **43** in step b.

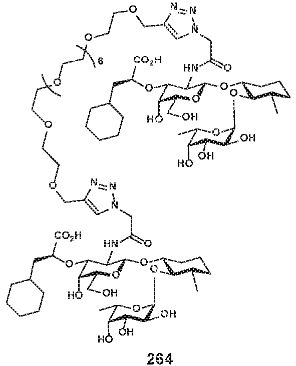

### EXAMPLE 204

### PROPHETIC SYNTHESIS OF MULTIMERIC COMPOUND 265

**Compound 265:** Compound **265** can be prepared in an analogous fashion to Figure 25 using 3,3'-[[2,2-bis[(2-propyn-1-yloxy)methyl]-1,3-propanediyl]bis(oxy)]bis-1-propyne in place of compound **43** in step b.

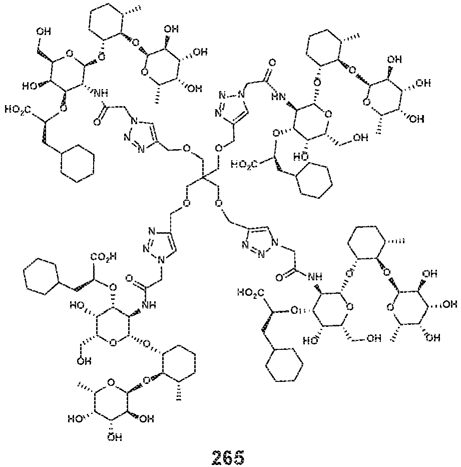

### EXAMPLE 205

### PROPHETIC SYNTHESIS OF MULTIMERIC COMPOUND 266

**Compound 266:** Compound **266** can be prepared in an analogous fashion to Figure 25 using 3,3'-[oxybis[[2,2-bis[(2-propyn-1-yloxy)rnethyl]-3,1-propanediyl]oxy]]bis-1-propyne in place of compound **43** in step b.

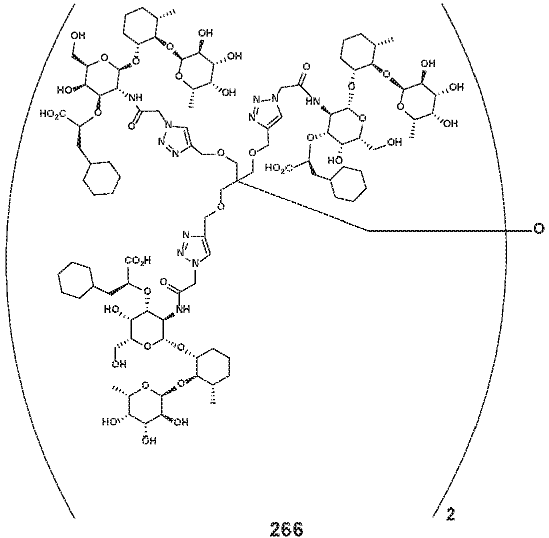

### EXAMPLE 206

### PROPHETIC SYNTHESIS OF MULTIMERIC COMPOUND 267

**Compound 267:** Compound **267** can be prepared in an analogous fashion to Figure 25 using ethylamine in place of azetidine in step d.

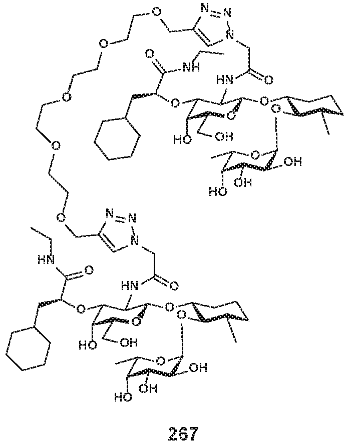

### EXAMPLE 207

### PROPHETIC SYNTHESIS OF MULTIMERIC COMPOUND 268

**Compound 268:** Compound **268** can be prepared in an analogous fashion to Figure 25 using dimethylamine in place of azetidine in step d.

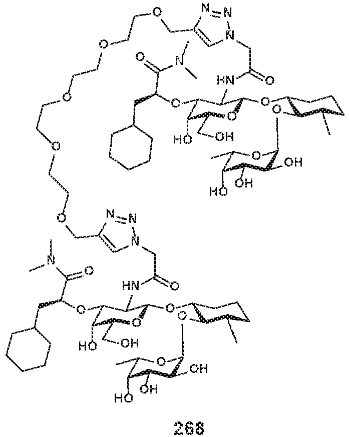

### EXAMPLE 208

### PROPHETIC SYNTHESIS OF MULTIMERIC COMPOUND 269

**Compound 269:** Compound **269** can be prepared in an analogous fashion to Figure 25 using the analog of compound 234 prepared from vinylcyclohexane in place of compound **234** in step a.

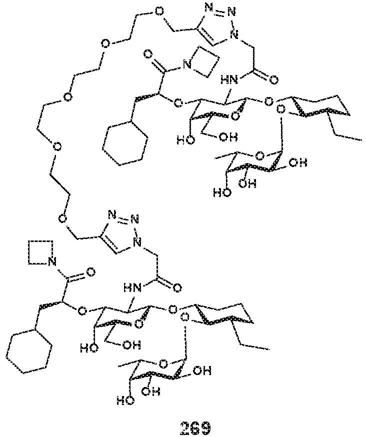

### EXAMPLE 209

### PROPHETIC SYNTHESIS OF MULTIMERIC COMPOUND 270

**Compound 270:** Compound **270** can be prepared in an analogous fashion to Figure 25 using propargyl ether in place of compound **43** in step b.

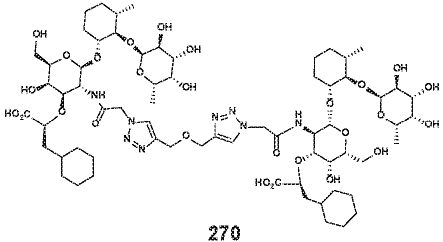

### EXAMPLE 210

### PROPHETIC SYNTHESIS OF MULTIMERIC COMPOUND 271

**Compound 271:** Compound **271** can be prepared in an analogous fashion to Figure 25 using propargyl ether in place of compound **43** in step b.

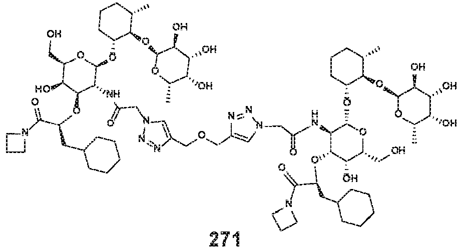

### EXAMPLE 211

### PROPHETIC SYNTHESIS OF MULTIMERIC COMPOUND 274

**Compound 272**: Activated powdered 4Å molecular sieves are added to a solution of compound **230** and compound **63** (2 eq) in dry DCM under argon. The mixture is stirred for 2 hours at room temperature. Solid DMTST (1.5 eq) is added in 4 portions over 1.5 hours. The reaction mixture is stirred overnight at room temperature. The reaction mixture is filtered through Celite, transferred to a separatory funnel and washed two times with half saturated sodium bicarbonate and two times with water. The organic phase is dried over sodium sulfate, filtered and concentrated. The residue is separated by flash chromatography to afford compound **272.**

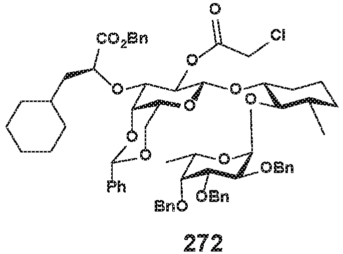

**Compound 273:** Compound **272** is dissolved in DMF. Sodium azide (1.5 eq) is added and the reaction mixture is stirred at 50°C until completion. The reaction mixture is cooled to room temperature, diluted with ethyl acetate and transferred to a separatory funnel. The organic phase is washed 4 times with water then dried over sodium sulfate and concentrated. The residue is separated by column chromatography to afford compound **273.**

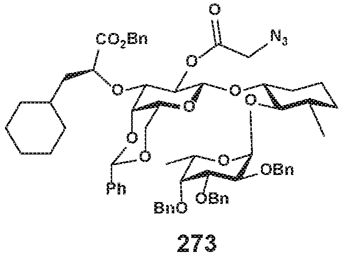

**Compound 274:** A solution of bispropagyl PEG-5 (compound 43) and compound 273 (2.4 eq) in MeOH is degassed at room temperature. A solution of CuSO₄/THPTA in distilled water (0.04 M) (0.2 eq) and sodium ascorbate (0.2 eq) are added successively and the resulting solution is stirred 12 hrs at 50°C. The solution is concentrated under reduced pressure. The crude product is purified by chromatography to give a compound **274.**

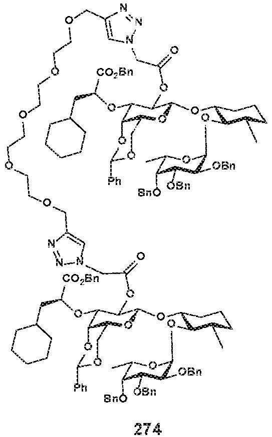

### EXAMPLE 212

### PROPHETIC SYNTHESIS OF MULTIMERIC COMPOUND 275

**Compound 275:** To a solution of compound **274** in dioxane/water (4/1) is added Pd(OH)₂/C. The reaction mixture is stirred vigorously overnight under a hydrogen atmosphere. The reaction mixture is filtered through Celite and concentrated. The residue is purified by C-18 reverse phase column chromatography to afford compound **275.**

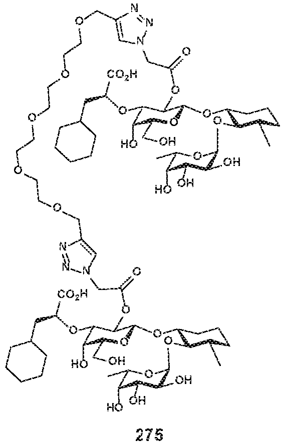

### EXAMPLE 213

### PROPHETIC SYNTHESIS OF MULTIMERIC COMPOUND 276

**Compound 276:** Compound **275** is dissolved in DMF and cooled on an ice bath. Diisopropylethylamine (2.5 eq) is added followed by HATU (2.2 eq). The reaction mixture is stirred 15 minutes on the ice bath then azetidine (10 eq) is added. The ice bath is removed and the reaction mixture is stirred overnight at room temperature. The solvent is removed under reduced pressure and the residue is separated by reverse phase chromatography to afford compound **276.**

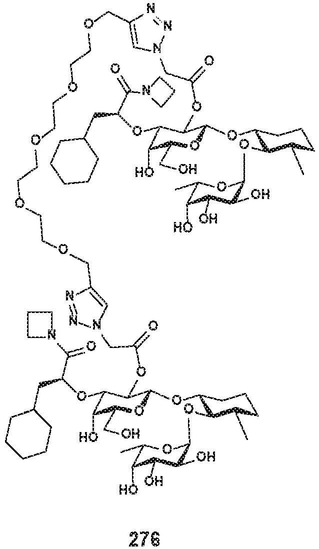

### EXAMPLE 214

### PROPHETIC SYNTHESIS OF MULTIMERIC COMPOUND 277

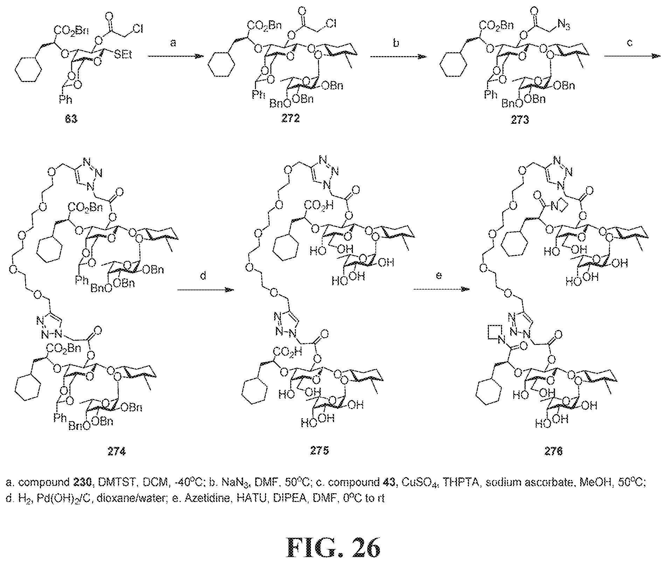

**Compound 277:** Compound **277** can be prepared in an analogous fashion to Figure 26 by replacing compound **43** with PEG-8 bis propargyl ether in step c.

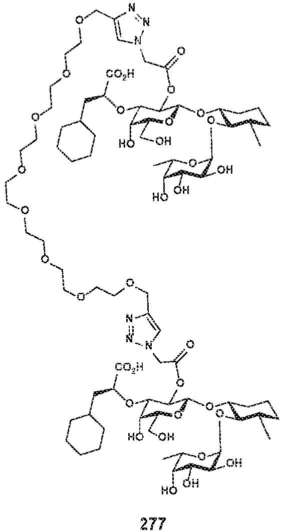

### EXAMPLE 215

### PROPHETIC SYNTHESIS OF MULTIMERIC COMPOUND 278

**Compound 278:** Compound **278** can be prepared in an analogous fashion to Figure 26 by replacing compound **43** with ethylene glycol bis propargyl ether in step c.

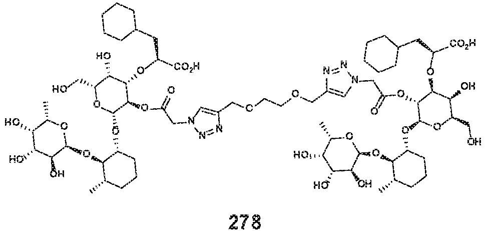

### EXAMPLE 216

### PROPHETIC SYNTHESIS OF MULTIMERIC COMPOUND 279

**Compound 279:** Compound **279** can be prepared in an analogous fashion to Figure 26 using 3,3'-[[2,2-bis[(2-propyn-1-yloxy)methyl]-1,3-propanediyl]bis(oxy)]bis-1-propyne in place of compound **43 in** step c.

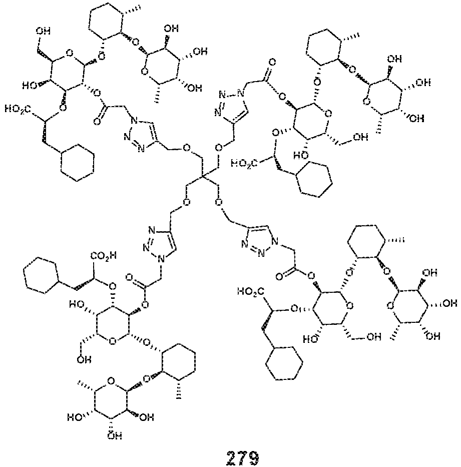

### EXAMPLE 217

### PROPHETIC SYNTHESIS OF MULTIMERIC COMPOUND 280

**Compound 280:** Compound **280** can be prepared in an analogous fashion to Figure 26 using propargyl ether in place of compound **43** in step c.

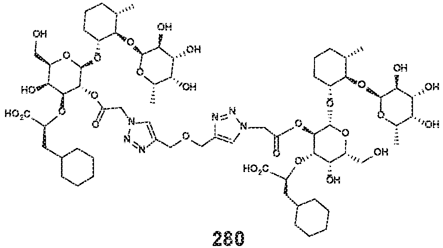

### EXAMPLE 218

### PROPHETIC SYNTHESIS OF MULTIMERIC COMPOUND 281

**Compound 281:** Compound **281** can be prepared in an analogous fashion to Figure 26 using propargyl ether in place of compound **36** in step c.

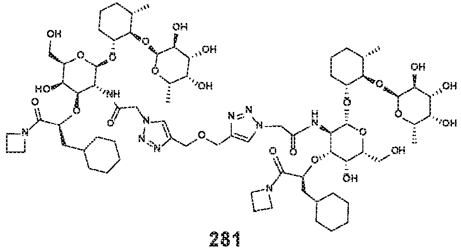

### EXAMPLE 219

### PROPHETIC SYNTHESIS OF MULTIMERIC COMPOUND 282

**Compound 282:** Compound **282** can be prepared in an analogous fashion to Figure 26 by replacing compound **43** with ethylene glycol bis propargyl ether in step c.

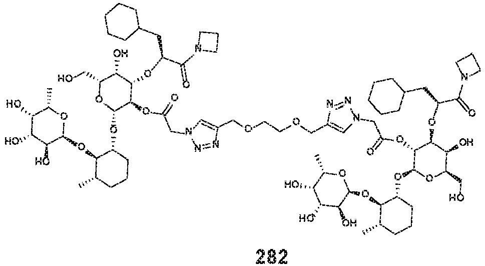

### EXAMPLE 220

### PROPHETIC SYNTHESIS OF MULTIMERIC COMPOUND 294

**Compound 284: A** mixture of compound **283** (preparation described in WO 2007/028050) and compound 2 (preparation described in WO 2013/096926) (1.7 eq) is azeotroped 3 times from toluene. The mixture is dissolved in DCM under argon and cooled on an ice bath. To this solution is added boron trifluoride etherate (1.5 eq). The reaction mixture is stirred 12 hours at room temperature. The reaction is quenched by the addition of triethylamine (2 eq). The reaction mixture is transferred to a separatory funnel and washed 1 time with half saturated sodium bicarbonate solution and 1 time with water. The organic phase is dried over sodium sulfate, filtered, and concentrated. The residue is purified by flash chromatography to afford compound **284.**

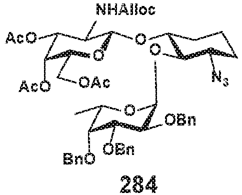

**Compound 285:** Compound **284** is dissolved in methanol at room temperature. A solution of sodium methoxide in methanol (0.1 eq) is added and the reaction mixture stirred overnight at room temperature. The reaction mixture is quenched by the addition of acetic acid. The reaction mixture is diluted with ethyl acetate, transferred to a separatory funnel and washed 2 times with water. The organic phase is dried over magnesium sulfate, filtered and concentrated. The residue is separated by flash chromatography to afford compound **285.**

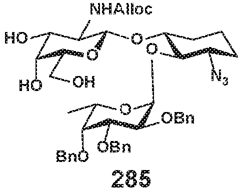

**Compound 286:** To a solution of compound **285** in dichloromethane cooled on an ice bath is added DABCO (1.5 eq) followed by monomethyoxytrityl chloride (1.2 eq). The reaction mixture is stirred overnight allowing to warm to room temperature. The reaction mixture is transferred to a separatory funnel and washed 2 times with water. The organic phase is concentrated and the residue is purified by flash chromatography to afford compound **286.**

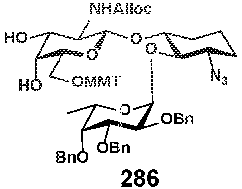

**Compound 287:** To a solution of compound **286** in methanol is added dibutyltin oxide (1.1 eq). The reaction mixture is refluxed for 3 hours then concentrated. The residue is suspended in DME. To this suspension is added compound 6 (preparation described in Thoma et. al. J. Med. Chem., 1999, 42, 4909) (1.5 eq) followed by cesium fluoride (1.2 eq). The reaction mixture is stirred at room temperature overnight. The reaction mixture is diluted with ethyl acetate, transferred to a separatory funnel, and washed with water. The organic phase is dried over sodium sulfate, filtered and concentrated. The residue is purified by flash chromatography to afford compound **287.**

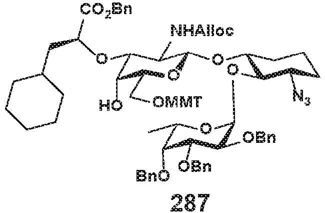

**Compound 288:** To a degassed solution of compound **287** in anhydrous DCM at 0 °C is added Pd(PPh₃)₄ (0.1 eq), Bu₃SnH (1.1 eq) and N-trifluoroacetyl glycine anhydride (2.0 eq) (preparation described in Chemische Berichte (1955), 88(1), 26). The resulting solution is stirred for 12 hrs allowing the temperature to increase to room temperature. The reaction mixture is diluted with DCM, transferred to a separatory funnel, and washed with water. The organic phase is dried over Na₂SO₄, then filtered and concentrated. The residue is purified by flash chromatography to afford compound **288.**

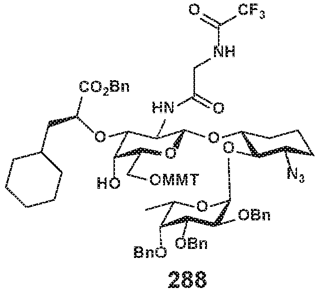

**Compound 289:** To a stirred solution of compound **288** in DCM/MeOH (25/1) at room temperature is added orotic acid chloride (5 eq) and triphenylphosphine (5 eq). The reaction mixture is stirred 24 hours. The solvent is removed and the residue is separated by column chromatography to afford compound **289**.

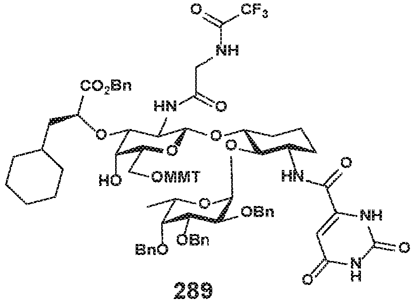

**Compound 290:** Compound **289** is dissolved in methanol and degassed. To this solution is added Pd(OH)₂/C. The reaction mixture is vigorously stirred under a hydrogen atmosphere for 12 hours. The reaction mixture is filtered through a Celite pad. The filtrate is concentrated under reduced pressure to give compound **290**.

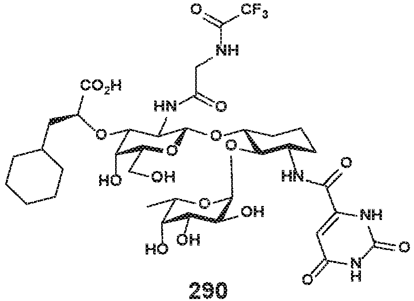

**Compound 291**: Compound **290** is dissolved in methanol at room temperature. A solution of sodium methoxide in methanol (1.1 eq) is added and the reaction mixture stirred overnight at room temperature. The reaction mixture is quenched by the addition of acetic acid. The reaction mixture is concentrated. The residue is separated by C-18 reverse phase chromatography to afford compound **291.**

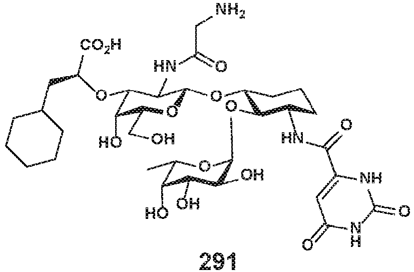

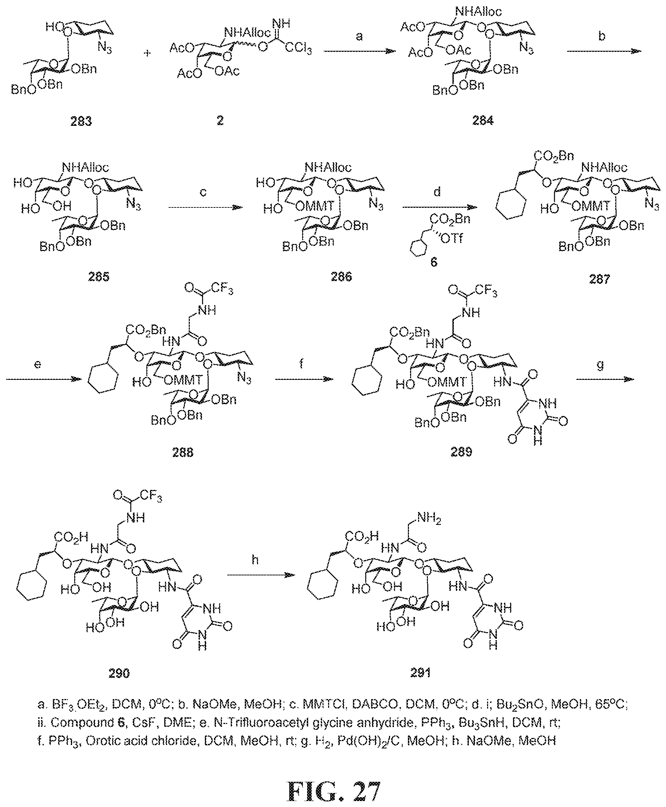

**Compound 292:** Compound **292** can be prepared in an analogous fashion to Figure 27 by replacing orotic acid chloride with acetyl chloride in step f.

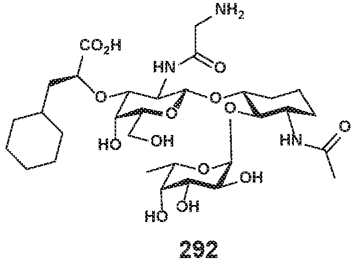

**Compound 293:** Compound **293** can be prepared in an analogous fashion to Figure 27 by replacing orotic acid chloride with benzoyl chloride in step f.

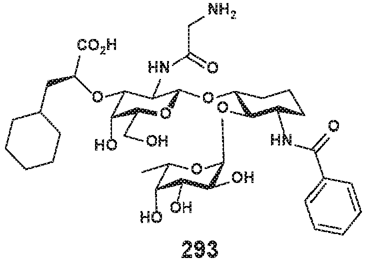

**Compound 294:** A solution of compound **291** (0.4 eq) in DMSO is added to a solution of compound 20 (1 eq) and DIPEA (10 eq) in anhydrous DMSO at room temperature. The resulting solution is stirred overnight. The reaction mixture is separated by reverse phase chromatography and the product lyophilized to give compound **294.**

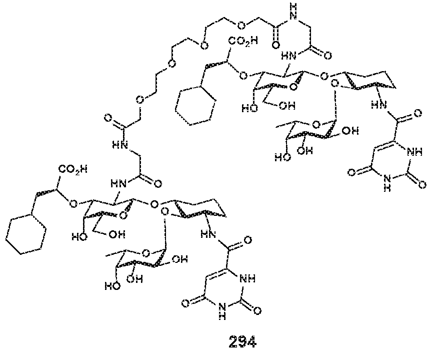

### EXAMPLE 221

### PROPHETIC SYNTHESIS OF MULTIMERIC COMPOUND 295

**Compound 295:** Compound **294** is dissolved in DMF and cooled on an ice bath. Diisopropylethylamine (2.5 eq) is added followed by HATU (2.2 eq). The reaction mixture is stirred 15 minutes on the ice bath then azetidine (10 eq) is added. The ice bath is removed and the reaction mixture is stirred overnight at room temperature. The solvent is removed under reduced pressure and the residue is separated by reverse phase chromatography to afford compound **295.**

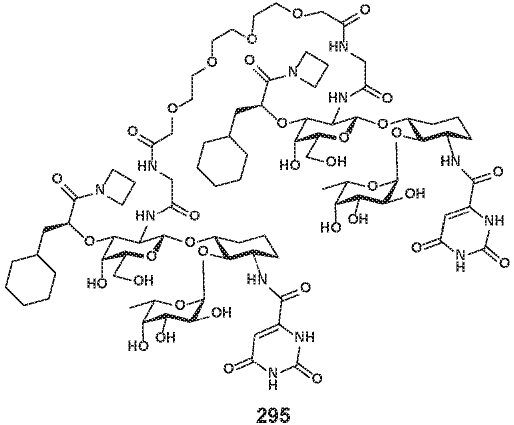

### EXAMPLE 222

### PROPHETIC SYNTHESIS OF MULTIMERIC COMPOUND 296

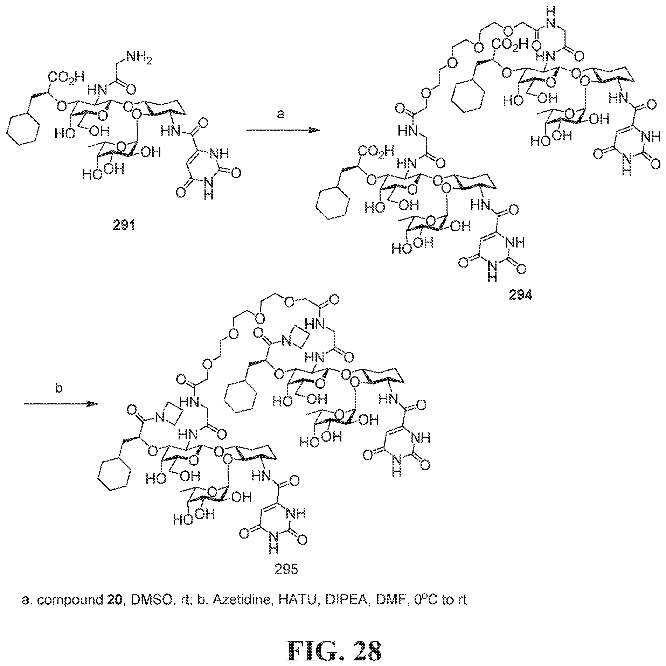

**Compound 296**: Compound **296** can be prepared in an analogous fashion to Figure 28 by replacing compound 20 with ethylene glycol diacetic acid di-NHS ester in step a.

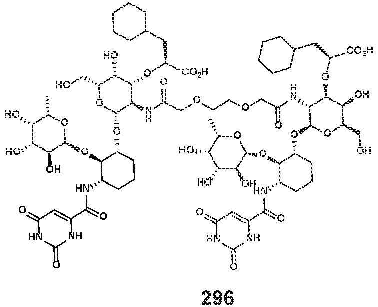

### EXAMPLE 223

### PROPHETIC SYNTHESIS OF MULTIMERIC COMPOUND 297

**Compound 297:** Compound **297** can be prepared in an analogous fashion to Figure 28 by replacing compound 20 with ethylene glycol diacetic acid di-NHS ester in step a.

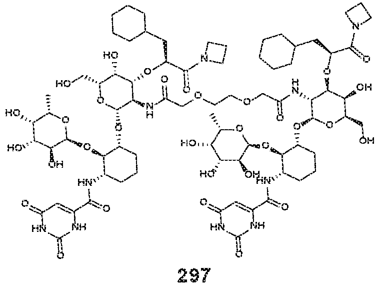

### EXAMPLE 224

### PROPHETIC SYNTHESIS OF MULTIMERIC COMPOUND 298

**Compound 298**: Compound **298** can be prepared in an analogous fashion to Figure 28 by replacing compound **291** with compound **292** and compound 20 with ethylene glycol diacetic acid di-NHS ester in step a.

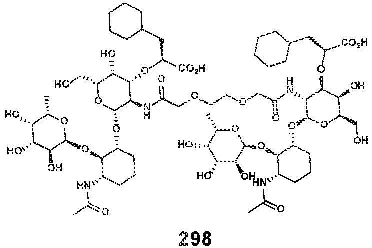

### EXAMPLE 225

### PROPHETIC SYNTHESIS OF MULTIMERIC COMPOUND 299

**Compound 299**: Compound **299** can be prepared in an analogous fashion to Figure 28 by replacing compound **291** with compound **292** and compound **20** with ethylene glycol diacetic acid di-NHS ester in step a.

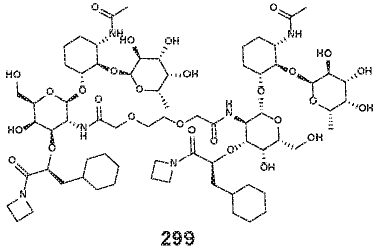

### EXAMPLE 226

### PROPHETIC SYNTHESIS OF MULTIMERIC COMPOUND 300

**Compound 300:** Compound **300** can be prepared in an analogous fashion to Figure 28 by replacing compound **291** with compound **293** and compound **20** with ethylene glycol diacetic acid di-NHS ester in step a.

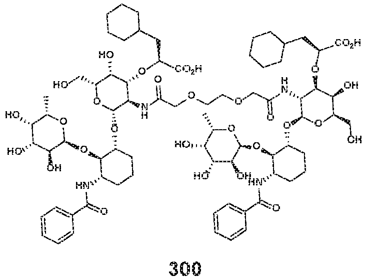

### EXAMPLE 227

### PROPHETIC SYNTHESIS OF MULTIMERIC COMPOUND 301

**Compound 301:** Compound **301** can be prepared in an analogous fashion to Figure 28 by replacing compound **291** with compound **293** and compound **20** with ethylene glycol diacetic acid di-NHS ester in step a.

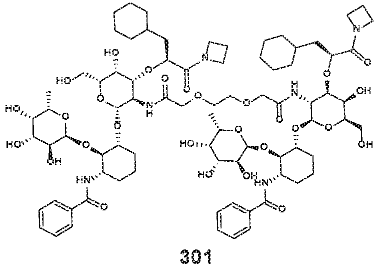

### EXAMPLE 228

### PROPHETIC SYNTHESIS OF MULTIMERIC COMPOUND 302

**Compound 302:** Compound **302** can be prepared in an analogous fashion to Figure 28 by replacing compound **20** with 3,3'-[[2,2-bis[[3-[(2,5-dioxo-1-pyrrolidinyl)oxy]-3 oxopropoxy]methyl]-1,3-propanediyl]bis(oxy)]bis-, 1,1'-bis(2,5-dioxo-1-pyrrolidinyl)-propanoic acid ester in step a.

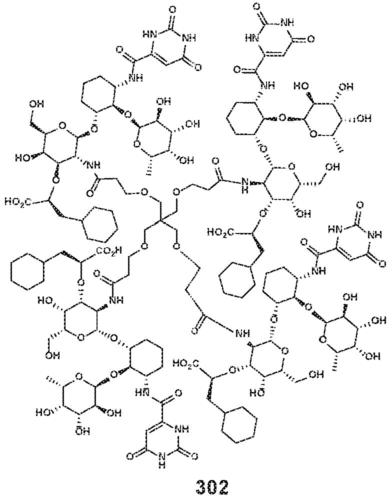

### EXAMPLE 229

### PROPHETIC SYNTHESIS OF MULTIMERIC COMPOUND 305

**Compound 303:** To a stirred solution of compound **287** in DCM/MeOH (25/1) at room temperature is added orotic acid chloride (5 eq) and triphenylphosphine (5 eq). The reaction mixture is stirred 24 hours. The solvent is removed and the residue is separated by column chromatography to afford compound **303.**

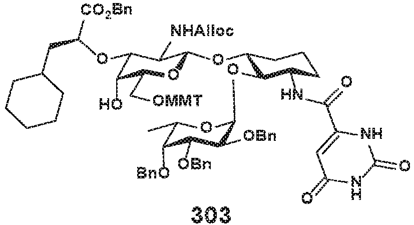

**Compound 304:** To a degassed solution of compound **303** in anhydrous DCM at 0 °C is added Pd(PPh₃)₄ (0.1 eq), Bu₃SnH (1.1 eq) and azidoacetic anhydride (2.0 eq). The ice bath is removed and the solution is stirred for 12 hrs under a N₂ atmosphere at room temperature. The reaction mixture is diluted with DCM, washed with water, dried over Na₂SO₄, then concentrated. The crude product is purified by column chromatography to give compound 304.

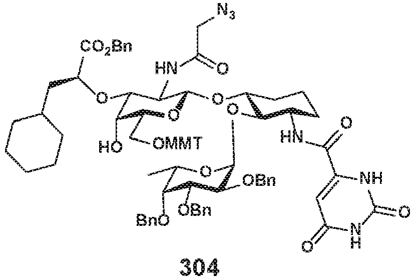

**Compound 305**: A solution of bispropagyl PEG-5 (compound 43) and compound 304 (2.4 eq) in MeOH is degassed at room temperature. A solution of CuSO₄/THPTA in distilled water (0.04 M) (0.2 eq) and sodium ascorbate (0.2 eq) are added successively and the resulting solution is stirred 12 hrs at 50 °C. The solution is cooled to room temperature and concentrated under reduced pressure. The crude product is purified by chromatography to give compound **305**.

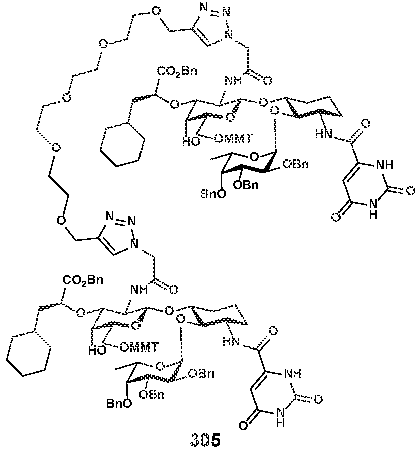

### EXAMPLE 230

### PROPHETIC SYNTHESIS OF MULTIMERIC COMPOUND 306

**Compound 306**: Compound **305** is dissolved in MeOH and hydrogenated in the presence of Pd(OH)₂ (20 wt %) at 1 atm of H₂ gas pressure for 24 hrs at room temperature. The solution is filtered through a Celite pad. The filtrate is concentrated to give compound **306.**

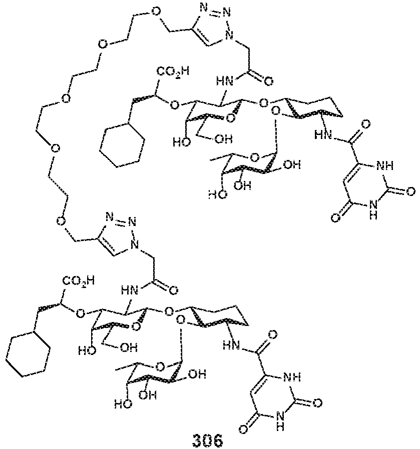

### EXAMPLE 231

### PROPHETIC SYNTHESIS OF MULTIMERIC COMPOUND 307

**Compound 307**: Compound **306** is dissolved in DMF and cooled on an ice bath. Diisopropylethylamine (2.5 eq) is added followed by HATU (2.2 eq). The reaction mixture is stirred 15 minutes on the ice bath then azetidine (10 eq) is added. The ice bath is removed and the reaction mixture is stirred overnight at room temperature. The solvent is removed under reduced pressure and the residue is separated by reverse phase chromatography to afford compound **307.**

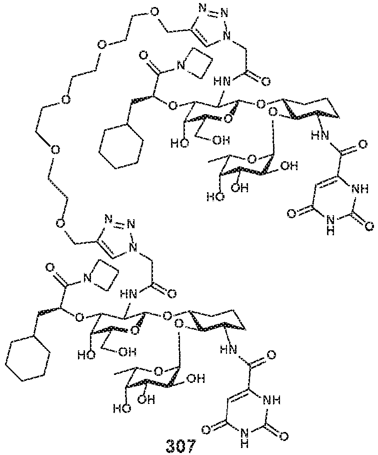

### EXAMPLE 232

### PROPHETIC SYNTHESIS OF MULTIMERIC COMPOUND 308

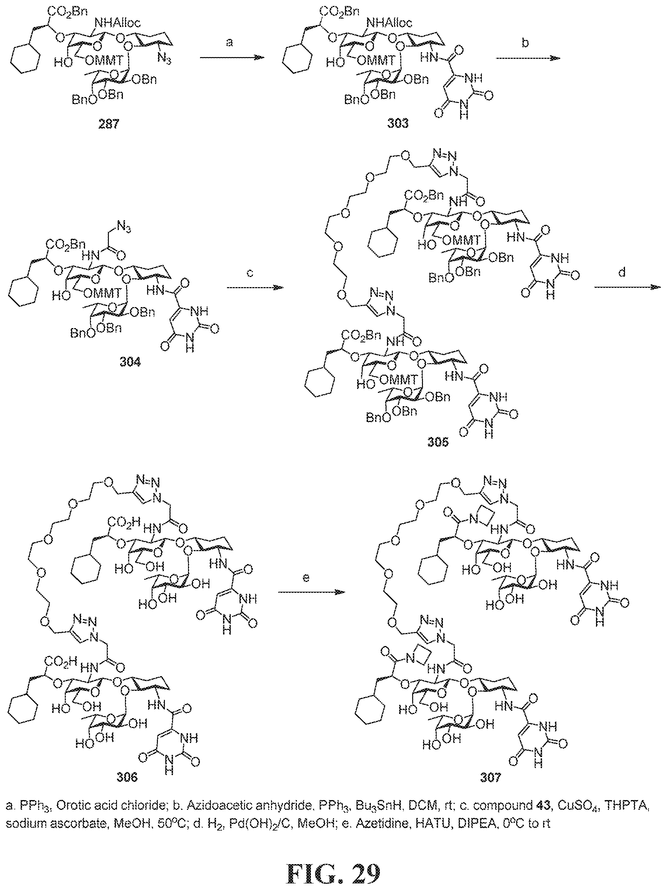

**Compound 308:** Compound **308** can be prepared in an analogous fashion to Figure 29 using 3,3'-[[2,2-bis[(2-propyn-1-yloxy)methyl]-1,3-propanediyl]bis(oxy)]bis-1-propyne in place of compound **43** in step c.

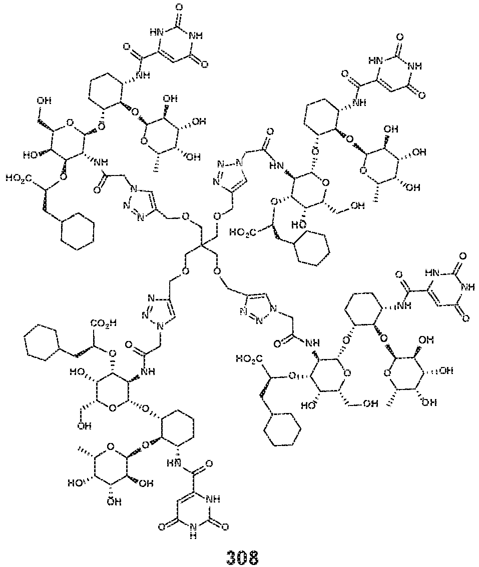

### EXAMPLE 233

### PROPHETIC SYNTHESIS OF MULTIMERIC COMPOUND 309

**Compound 309:** Compound **309** can be prepared in an analogous fashion to Figure 29 using 3,3'-[[2,2 bis[(2-propyn-1-yloxy)methyl]-1,3-propanedlyl]bis(oxy)]bis-1-propyne in place of compound **43** in step c.

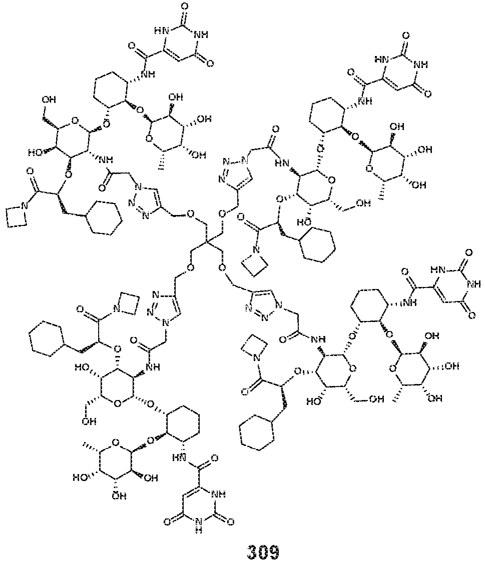

### EXAMPLE 234

### PROPHETIC SYNTHESIS OF MULTIMERIC COMPOUND 310

**Compound 310:** Compound **310** can be prepared in an analogous fashion to Figure 29 by replacing compound **43** with bis-propargyl ethylene glycol in step c.

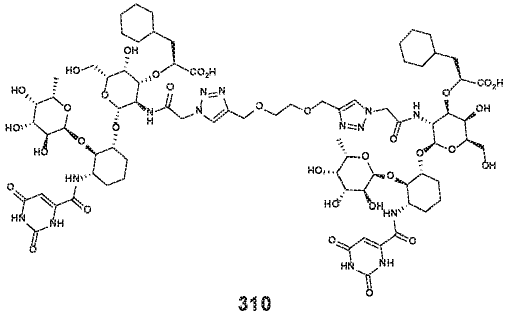

### EXAMPLE 235

### PROPHETIC SYNTHESIS OF MULTIMERIC COMPOUND 311

**Compound 311:** Compound **311** can be prepared in an analogous fashion to Figure 29 by replacing compound **43** with bis-propargyl ethylene glycol in step c.

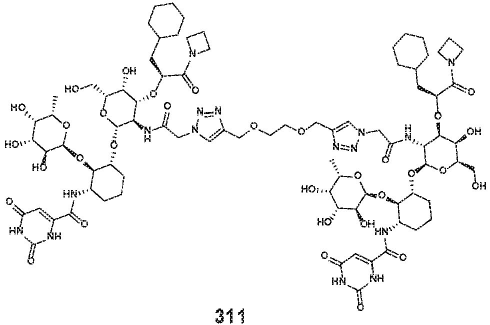

### EXAMPLE 236

### PROPHETIC SYNTHESIS OF MULTIMERIC COMPOUND 312

**Compound 312:** Compound **312** can be prepared in an analogous fashion to Figure 29 by replacing compound **43** with propargyl ether in step c.

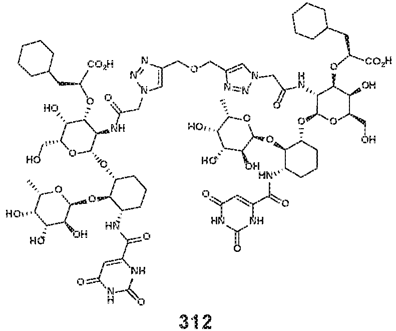

### EXAMPLE 237

### PROPHETIC SYNTHESIS OF MULTIMERIC COMPOUND 313

**Compound 313**: Compound **313** can be prepared in an analogous fashion to Figure 29 by replacing compound **43** with propargyl ether in step c.

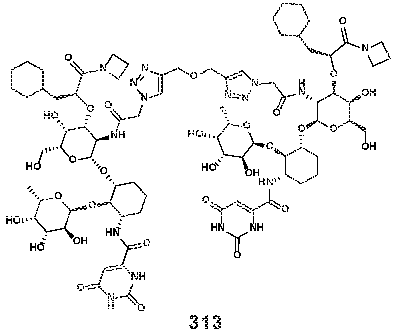

### EXAMPLE 238

### SYNTHESIS OF BUILDING BLOCK 332

**Compound 321:** Compound **317** (1.1 g, 2.60 mmoles) was dissolved in methanol (25 mL) at room temperature. Sodium methoxide (0.1 mL, 25% sol. in MeOH) was added and the reaction mixture was stirred at room temperature for 2 hours. The reaction mixture neutralized by the addition of Amberlyst acidic resin, filtered and concentrated to give crude **321,** which was used for the next step without further purification. LCMS (ESI): *m*/*z* calculated for C₁₂H₁₅N₃O₄S: 297.3, found 298.1 (M+1); 320.1 (M+Na).

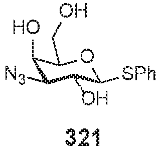

**Compound 322:** Crude compound **321** (2.60 mmoles), 3,4,5-trifluorophenyl-1-acetylene (2.5 equiv), THPTA (0.11 equiv), and copper (II) sulfate (0.1) were dissolved in methanol (15 mL) at room temperature. Sodium ascorbate (2.4 equiv) dissolved in water was added and the reaction mixture was stirred overnight at room temperature. The resultant precipitate was collected by filtration, washed with hexanes and water, and dried to give compound **322** as a pale yellow solid (1.2 g, 100% yield for 2 steps). LCMS (ESI): *m*/*z* calculated for C₂₀H₁₈F₃N₃O₄S: 4.53.1, found 454.2 (M+1); 476.2 (M+Na).

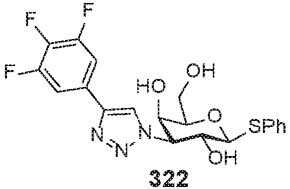

**Compound 323:** Compound **322** (1.2 g, 2.65 mmoles) was dissolved in DMF (15 mL) and cooled on an ice bath. Sodium hydride (60% oil dispersion, 477 mg, 11.93 mmoles) was added and the mixture stirred for 30 minutes. Benzyl bromide (1.42 mL, 11.93 mmoles) was added and the reaction was warmed to room temperature and stirred overnight. The reaction mixture was quenched by the addition of aqueous saturated ammonium chloride solution, transferred to a separatory funnel and extracted 3 times with ether. The combined organic phases were dried over magnesium sulfate, filtered, and concentrated. The residue was purified by flash chromatography to afford compound 323 (1.8 g, 94% yield). LCMS (ESI): *m*/*z* calculated for C₄₁H₃₆F₃N₃O₄S: 723.2, found 724.3 (M+1); 746.3 (M+Na).

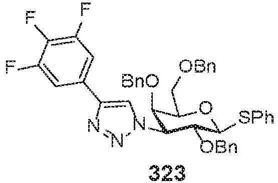

**Compound 324:** Compound **323** (1.8 g, 2.49 mmol) was dissolved in acetone (20 mL) and water (2 mL) and cooled on an ice bath. Trichloroisocyanuric acid (637 mg, 2.74 mmoles) was added and the reaction mixture stirred on the ice bath for 3 h. The acetone was removed in vacuo and the residue was diluted with DCM, transferred to a separatory funnel, and washed with saturated aqueous NaHCO₃. The organic phase was concentrated and the residue was purified by flash chromatography to afford compound **324** (1.5 g, 95%). LCMS (ESI): *m*/*z* calculated for C₃₅H₃₂F₃N₃O₅: 631.2, found 632.2 (M+1); 654.2 (M+Na).

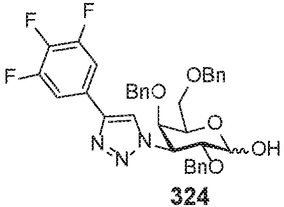

**Compound 325:** Compound **324** (1.0 g, 1.58 mmoles) was dissolved in DCM (20 mL) and cooled on an ice bath. Dess-Martin periodinane (1.0 g, 2.37 mmoles) was added and mixture was allowed to warm to room temperature and stirred overnight. The reaction mixture quenched by the addition of aqueous saturated NaHCO₃, transferred to a separatory funnel, and extracted 2 times with DCM. The combined organic phases were dried over sodium sulfate, filtered, and concentrated. The residue was purified by flash chromatography to afford compound **325** (520 mg, 52% yield). LCMS (ESI): *m*/*z* calculated for C₃₅H₃₀F₃N₃O₅: 629.2, found 652.2 (M+Na); 662.2 (M+MeOH+1); 684.2 (M+MeOH+Na).

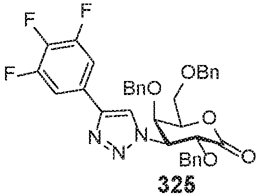

**Compound 326:** Methyl bromoacetate (253 mg, 1.65 mmoles) dissolved in 0.5 mL of THF was added dropwise to a solution of lithium bis(trimethylsilyl)amide (1.0 M in THF, 1.65 mL, 1.65 mmoles) cooled at -78 C. The reaction mixture was stirred for 30 minutes at -78 C. Compound **325** (260 mg, 0.41 mmoles) dissolved in THF (2.0 mL) was then added. The reaction mixture was stirred at -78 C for 30 minutes. The reaction was quenched by the addition of aqueous saturated NH₄Cl and warmed to rt. The reaction mixture was transferred to a separatory funnel and extracted 3 times with ethyl acetate. The combined organic phases were dried over sodium sulfate, filtered and concentrated. The residue was separated by flash chromatography to afford compound **326** (183 mg, 64% yield).

¹H NMR (400 MHz, Chloroform-*d*) δ 7.38 - 7.22 (m, 9H), 7.15 - 7.11 (m, 3H), 7.09 (dd, *J =* 8.4, 6.6 Hz, 1H), 7.06 - 7.00 (m, 2H), 6.98 - 6.93 (m, 2H), 5.11 (dd, *J =* 11.3, 3.2 Hz, 1H), 4.60 (d, *J* = 11.8 Hz, 1H), 4.57 - 4.49 (m, 2H), 4.49 - 4.42 (m, 2H), 4.35 (d, *J* = 11.8 Hz, 1H), 4.14 (d, *J* = 3.2 Hz*,* 1H), 4.05 (s, 1H), 4.02 (d, *J* = 7.0 Hz, 1H), 3.84 (d, *J* = 11.0 Hz, 1H), 3.81 (s, 3H), 3.70 (dd, *J* = 9.5, 7.7 Hz, 1H), 3.62 (dd, *J =* 9.4, 6.0 Hz, 1H). LCMS (ESI): *m*/*z* calculated for C₃₈H₃₄F₃N₃O₇: 701.2, found 702.3 (M+1); 724.3 (M+Na).

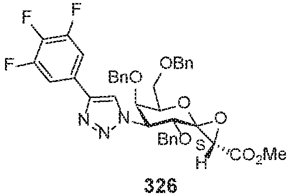

**Compound 327:** Compound **326** (5.0 g, 7.13 mmol) was azeotroped with toluene two times under reduced pressure, and then dried under high vacuum for 2 hours. It was then dissolved in anhydrous CH₂Cl₂ (125 mL) and cooled on an ice bath while stirring under an atmosphere of argon. Tributyltin hydride (15.1 mL, 56.1 mmol) was added dropwise and the solution was allowed to stir for 25 minutes on the ice bath. Trimethylsilyl triflate (2.1 mL, 11.6 mmol) dissolved in 20 mL of anhydrous CH₂Cl₂ was then added dropwise over the course of 5 minutes. The reaction was slowly warmed to ambient temperature and stirred for 16 hours. The reaction mixture was then diluted with CH₂Cl₂ (50 mL), transferred to a separatory funnel, and washed with saturated aqueous NaHCO₃ (50 mL). The aqueous phase was separated and extracted with CH₂Cl₂ (50 mL x 2). The combined organic phases were washed with saturated aqueous NaHCO₃ (50 mL), dried over Na₂SO₄, filtered, and concentrated. The residue was purified by flash chromatography (hexanes to 40% EtOAc in hexanes, gradient) to afford compound **327** (2.65 g, 48%).

¹H-NMR (400 MHz, CDCl₃): δ 7.65 (s, 1H), 7.36 - 7.22 (m, 8H), 7.16 - 7.06 (m, 7H), 6.96 - 6.90 (m, 2H), 5.03 (dd, *J* = 10.7, 3.2 Hz, 1H), 4.72 (d, *J* = 2.3 Hz, 1H), 4.51 (dt, *J =* 22.6, 11.4 Hz, 3H), 4.41 (d, *J* = 10.9 Hz, 114). 4.32 (dd, *J* = 10.7, 9.2 Hz, 114), 4.07 (d, *J* = 3.1 Hz, 1H), 3.94 (d, *J* = 10.9 Hz, 1H), 3.92 - 3.84 (m, 3H), 3.78 - 3.71 (m, 4H), 3.65 (dd, *J* = 9.1, 5.5 Hz, 1H), 0.24 (s, 9H). LCMS (ESI): *m*/*z* (M+Na) calculated for C₄₁H₄₄F₃N₃O₇SiNa: 798.87, found 798.2.

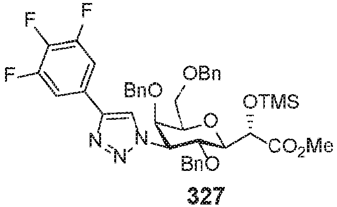

**Compound 328:** To a solution of compound **327** (2.65 g, 3.4 mmol) in anhydrous MeOH (40 mL) was added Pd(OH)₂ (0.27 g, 20% by wt). The mixture was cooled on an ice bath and stirred for 30 minutes. Triethylsilane (22 mL, 137 mmol) was added dropwise. The solution was allowed to slowly warm to ambient temperature and stirred for 16 hours. The reaction mixture was filtered through a bed of Celite and concentrated. The residue was purified by flash chromatography (hexanes to 100% EtOAc, gradient) to afford compound **328** (1.09 g, 73%).

¹H-NMR (400 MHz, CD₃OD): δ 8.57 (s, 1H), 7.77 - 7.53 (m, 2H), 4.91 - 4.82 (m, 1H), 4.66 - 4.59 (m, 1H), 4.55 (dd, J = 10.8, 9.4 Hz, 1H), 4.13 (d, J = 2.8 Hz, 1H), 3.86 (dd, J = 9.4, 2.1 Hz, 1H), 3.81 (s, 3H), 3.77 - 3.74 (m, 1H), 3.71 - 3.68 (m, 2H). LCMS (ESI): m/z (M+Na) calculated for C₁₇H₁₈F₃N₃O₇Na: 456.33, found 456.0.

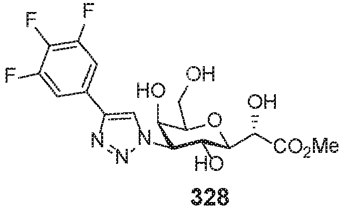

**Compound 329:** Compound **328** (1.09 g, 2.5 mmol) and CSA (0.115 g, 0.49 mmol) were suspended in anhydrous MeCN (80 mL) under an argon atmosphere. Benzaldehyde dimethyl acetal (0.45 mL, 2.99 mmol) was added dropwise. The reaction mixture was allowed to stir for 16 hours at ambient temperature, during which time it became a homogenous solution. The reaction mixture was then neutralized with a few drops of Et₃N, and concentrated. The residue was purified via flash chromatography (CH₂Cl₂ to 10% MeOH in CH₂Cl₂, gradient) to afford compound **329** (978 mg, 75%).

1H NMR (400 MHz, DMSO-d6): δ 8.84 (s, 1H), 7.95 - 7.73 (m, 2H), 7.33 (qdt, J = 8.4, 5.6, 2.7 Hz, 5H), 5.51 (t, J = 3.8 Hz, 2H), 5.47 (d, J = 6.8 Hz, 1H), 5.14 (dd, J = 10.8, 3.6 Hz, 1H), 4.54 (dd, J = 6.7, 2.2 Hz, 1H), 4.47 (ddd, J = 10.8, 9.3, 7.5 Hz, 1H), 4.40 (d, J = 4.0 Hz, 1H), 4.09 - 3.99 (m, 2H), 3.85 (dd, J = 9.3, 2.2 Hz, 1H), 3.81 - 3.76 (m, 1H), 3.71 (s, 3H). LCMS (ESI): m/z (M+Na) calculated for C₂₄H₂₂F₃N₃O₇Na: 544.43, found 544.1.

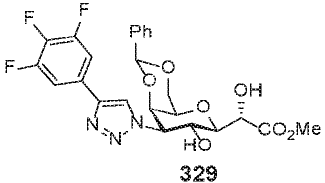

**Compound 330:** Compound **329** (25.2 mg, 0.048 mmol) was azeotroped with toluene 2 times under reduced pressure, dried under high vacuum for 2 hours, then dissolved in anhydrous DMF (2 mL) and cooled on an ice bath. Benzyl bromide (6 uL, 0.05 mmol) dissolved in 0.5 mL of anhydrous DMF was added and the reaction and was stirred under an atmosphere of argon for 30 minutes at 0 °C. Sodium hydride (2 mg, 0.05 mmol, 60%) was added and the reaction was allowed to gradually warm to ambient temperature while stirring for 16 hours. The reaction mixture was diluted with EtOAc (20 mL), transferred to a separatory funnel, and washed with H₂O (10 mL). The aqueous phase was separated and extracted with EtOAc (10 mL x 3). The combined organic phases were washed with H₂O (10 mL x 3), dried over Na₂SO₄, filtered, and concentrated. The residue was purified via preparative TLC (5% MeOH in CH₂Cl₂) to afford compound **330** (6.3 mg, 21%). LCMS (ESI): m/z (M+Na) calculated for C₃₁H₂₈F₃N₃O₇Na: 634.55, found 634.1.

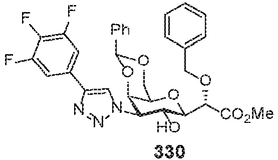

**Compound 331:** Compound **330** (6.3 mg, 0.01 mmol) was dissolved in anhydrous MeOH (1 mL) containing CSA (0.26 mg, 0.001 mmol). The reaction mixture was heated to 76 °C in a screw-cap scintillation vial while stirring. After 2 hours, an additional 0.13 mg of CSA in 0.5 mL of MeOH was added. The reaction mixture was stirred at 76 °C for 16 hours. The reaction mixture concentrated under reduced pressure. The residue was purified via preparative TLC (10% MeOH in CH₂Cl₂) to afford compound **331** (4.2 mg, 80%).

¹H NMR (400 MHz, DMSO-*d*₆) δ 8.80 (s, 1H), 7.94 - 7.86 (m, 2H), 7.48 - 7.42 (m, 2H), 7.38 (t, *J =* 7.4 Hz, 2H), 7.36 - 7.28 (m, 1H), 5.46 (d, *J* = 7.7 Hz, 1H), 5.28 (d, *J =* 6.0 Hz, 1H), 4.85 (dd, *J* = 10.7, 2.9 Hz, 1H), 4.67 (d, *J* = 11.0 Hz, 1H), 4.62 - 4.58 (m, 1H), 4.54 (d, *J* = 11.1 Hz, 1H), 4.44 (d, *J* = 2.5 Hz, 1H), 4.36 (q, *J* = 9.5 Hz, 1H), 3.95 - 3.90 (m, 1H), 3.78 (dd, *J* = 9.3, 2.5 Hz, 1H), 3.71 (s, 3H), 3.61 - 3.54 (m, 1H), 3.52 - 3.43 (m, 1H), 3.43 - 3.38 (m, 1H). LCMS (ESI): m/z (M+Na) calculated for C₂₄H₂₄F₃N₃O₇Na: 546.45, found 546.0.

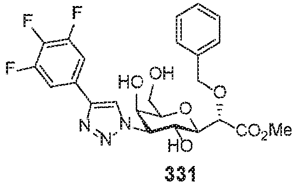

**Compound 332:** To a solution of compound **331** (3.5 mg, 0.007 mmoles) in methanol (0.5 mL) was added 1.0 M NaOH solution (0.1 mL). The reaction mixture was stirred overnight at room temperature then neutralized with acidic resin, filtered and concentrated. The residue was purified by reverse phase chromatography using a C-8 matrix to afford 3.0 mg compound **332** (90%).

¹H NMR (400 MHz, Deuterium Oxide) δ 8.39 (s, 1H), 8.37 (s, 2H), 7.54 - 7.45 (m, 1H), 7.43 (d, *J* = 7.4 Hz, 2H), 7.35 (dt, *J* = 14.3, 7.2 Hz, 3H), 4.86 (dd, *J* = 11.0, 2.9 Hz, 1H), 4.76 (d, *J* = 11.0 Hz, 1H), 4.40 - 4.30 (m, 2H), 4.16 (d, J= 1.9 Hz, 1H), 4.04 (d, *J* = 3.0 Hz, 1H), 3.81 (d, *J* = 9.6 Hz, 1H), 3.73 (d, *J* = 3.9 Hz, 0H), 3.67 (d, *J* = 7.6 Hz, 1H), 3.56 (dd, *J* = 11.7, 3.9 Hz, 1H). LCMS (ESI): m/z (M+Na) calculated for C₂₃H₂₂F₃N₃O₇: 509.1, found 508.2 (M-H).

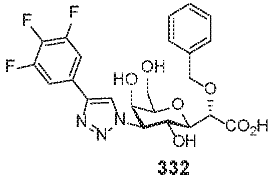

### EXAMPLE 239

### PROPHETIC SYNTHESIS OF BUILDING BLOCK 333

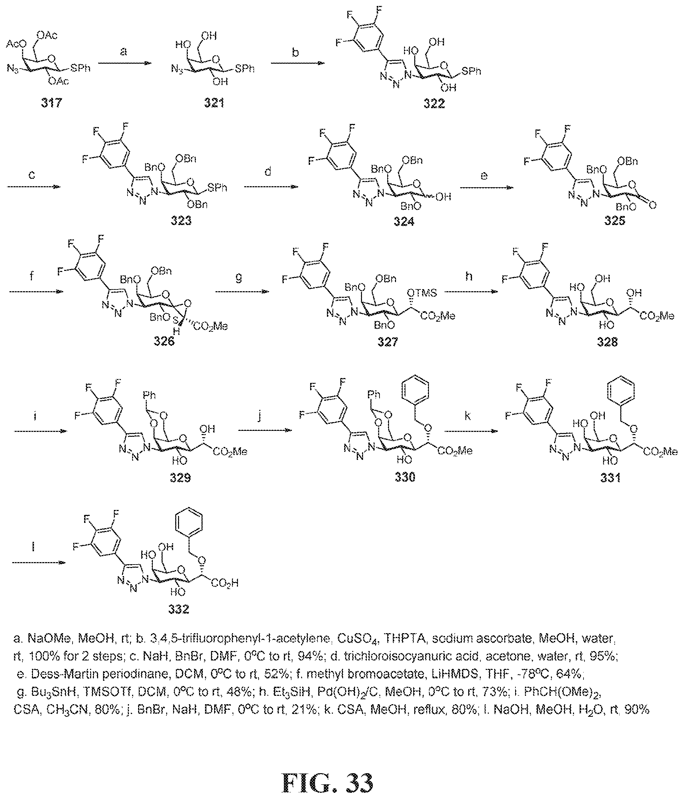

**Compound 333:** Compound 333 can be prepared in an analogous fashion to Figure 33 by replacing benzyl bromide with 4-chlorobenzyl bromide in step j.

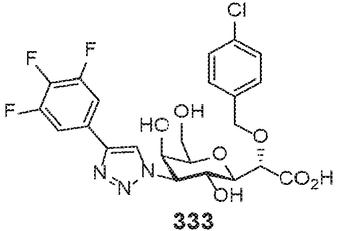

### EXAMPLE 240

### PROPHETIC SYNTHESIS OF BUILDING BLOCK 334

**Compound 334:** Compound 334 can be prepared in an analogous fashion to Figure 33 by replacing benzyl bromide with 4-methanesulfonylbenzyl bromide in step j.

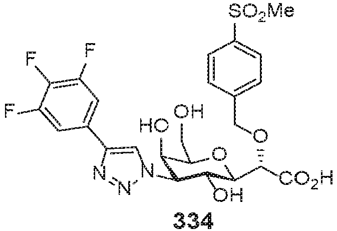

### EXAMPLE 241

### PROPHETIC SYNTHESIS OF BUILDING BLOCK 335

**Compound 335:** Compound **335** can be prepared in an analogous fashion to Figure 33 by replacing benzyl bromide with 3-picolyl bromide in step j.

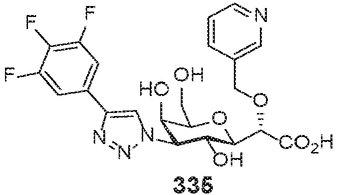

### EXAMPLE 242

### PROPHETIC SYNTHESIS OF MULTIMERIC COMPOUND 336

**Compound 336:** Compound **336** can be prepared in an analogous fashion to Figure 14 by replacing compound **145** with compound 332.

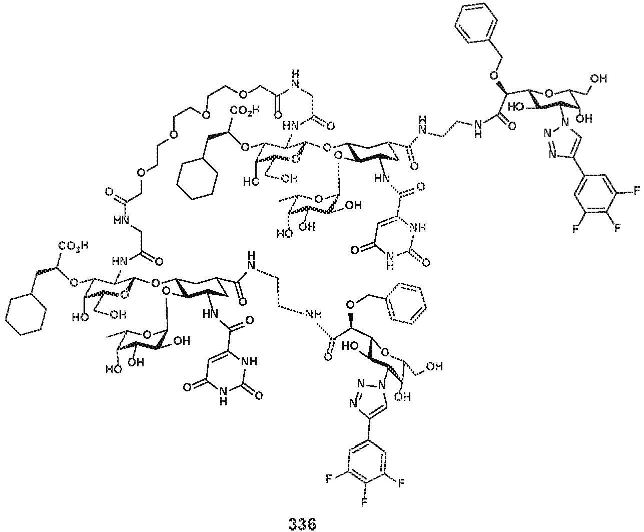

### EXAMPLE 243

### PROPHETIC SYNTHESIS OF MULTIMERIC COMPOUND 337

**Compound 337:** Compound **337** can be prepared in an analogous fashion to Figure 14 by replacing compound **145** with compound **333.**

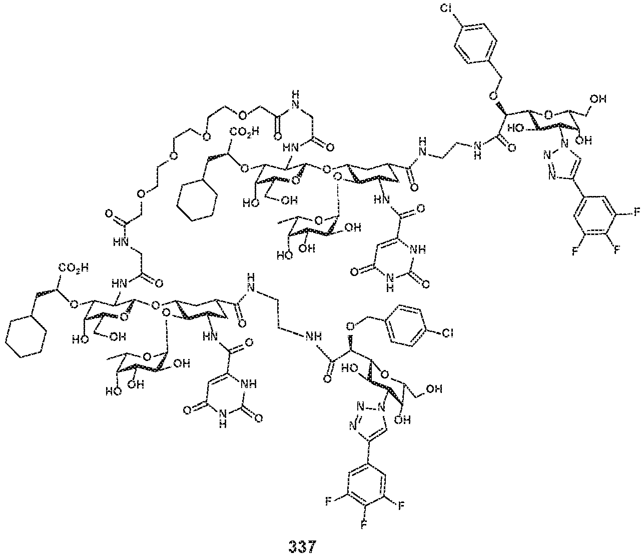

### EXAMPLE 244

### PROPHETIC SYNTHESIS OF MULTIMERIC COMPOUND 338

**Compound 338:** Compound **338** can be prepared in an analogous fashion to Figure 14 by replacing compound **145** with compound **334.**

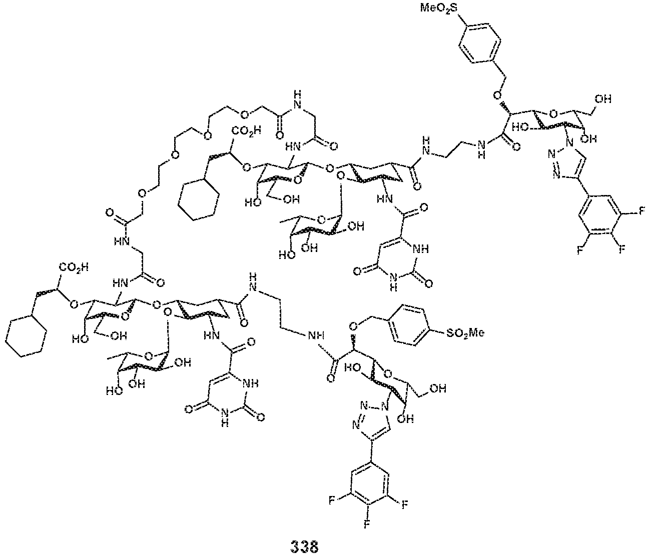

### EXAMPLE 245

### PROPHETIC SYNTHESIS OF MULTIMERIC COMPOUND 339

**Compound 339:** Compound **339** can be prepared in an analogous fashion to Figure 14 by replacing compound **145** with compound **335.**

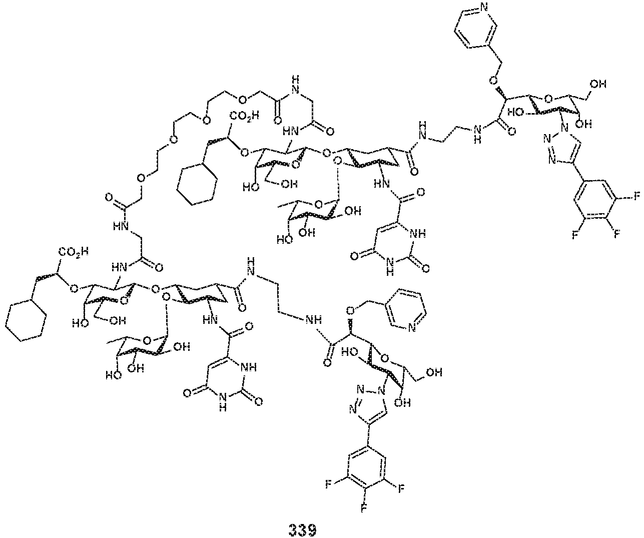

### EXAMPLE 246

### PROPHETIC SYNTHESIS OF MULTIMERIC COMPOUND 340

**Compound 340:** Compound **340** can be prepared in an analogous fashion to Figure 14 by replacing compound **22** with compound **40** and replacing compound **145** with compound **333.**

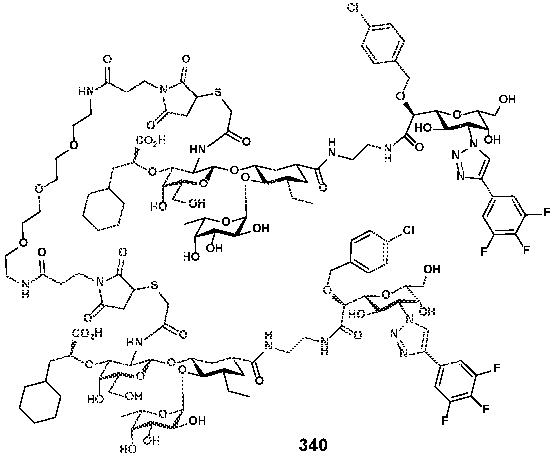

### EXAMPLE 247

### PROPHETIC SYNTHESIS OF MULTIMERIC COMPOUND 341

**Compound 341:** Compound **341** can be prepared in an analogous fashion to Figure 14 by replacing compound **22** with compound **78** and replacing compound **145** with compound **333.**

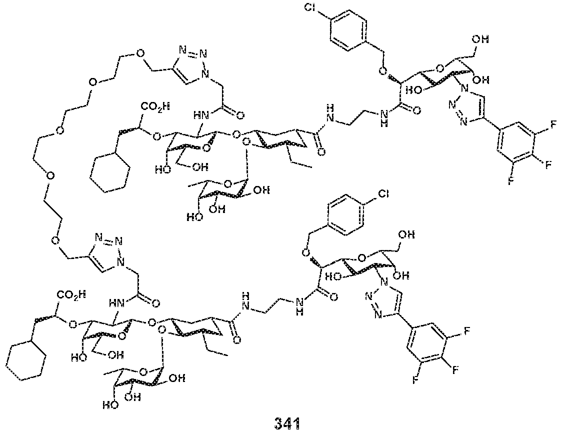

### EXAMPLE 248

### PROPHETIC SYNTHESIS OF MULTIMERIC COMPOUND 342

**Compound 342:** Compound **342** can be prepared in an analogous fashion to Figure 14 by replacing compound **22** with compound **87** and replacing compound **145** with compound **333.**

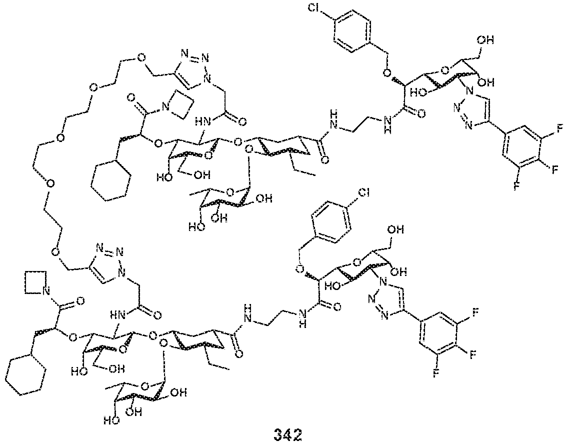

### EXAMPLE 249

### PROPHETIC SYNTHESIS OF MULTIMERIC COMPOUND 343

**Compound 343:** Compound **342** can be prepared in an analogous fashion to Figure 14 by replacing compound **22** with compound **88** and replacing compound **145** with compound **333.**

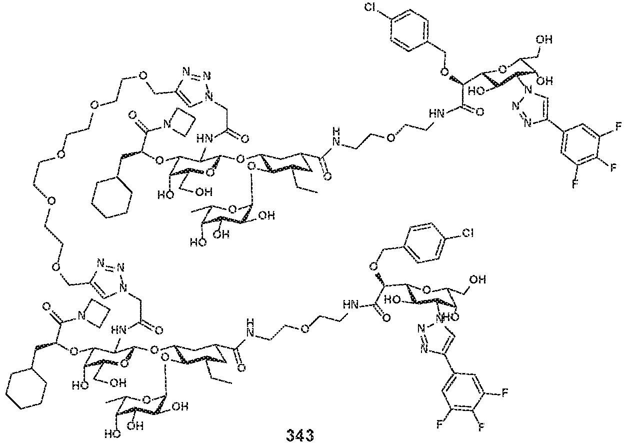

### EXAMPLE 250

### E-SELECTIN ACTIVITY - BINDING ASSAY

The inhibition assay to screen and characterize antagonists of E-selectin is a competitive binding assay, from which IC₅₀ values may be determined. E-selectin/Ig chimera are immobilized in 96 well microtiter plates by incubation at 37 °C for 2 hours. To reduce nonspecific binding, bovine serum albumin is added to each well and incubated at room temperature for 2 hours. The plate is washed and serial dilutions of the test compounds are added to the wells in the presence of conjugates of biotinylated, sLea polyacrylamide with streptavidin/horseradish peroxidase and incubated for 2 hours at room temperature.

To determine the amount of sLe^{a} bound to immobilized E-selectin after washing, the peroxidase substrate, 3,3',5,5' tetramethylbenzidine (TMB) is added. After 3 minutes, the enzyme reaction is stopped by the addition of H₃PO₄, and the absorbance of light at a wavelength of 450 nm is determined. The concentration of test compound required to inhibit binding by 50% is determined.

### E-Selectin Antagonist Activity

| Compound | IC50 (nM) |
|---|---|
| Compound 206 | 1.6 |

### EXAMPLE 251

### GALECTIN-3 ACTIVITY - ELISA ASSAY

Galectin-3 antagonists can be evaluated for their ability to inhibit binding of galectin-3 to a Galβ1-3GlcNAc carbohydrate structure. The detailed protocol is as follows. A 1 ug/mL suspension of a Galβ1-3GlcNAcβ1-3Galβ1-4GlcNAcβ-PAA-biotin polymer (Glycotech, catalog number 01-096) is prepared. A 100 uL aliquot of the polymer is added to the wells of a 96-well streptavidin-coated plate (R&D Systems, catalog number CP004). A 100 uL aliquot of 1X Tris Buffered Saline (TBS, Sigma, catalog number T5912 - 10X) is added to control wells. The polymer is allowed to bind to the streptavidin-coated wells for 1.5 hours at room temperature. The contents of the wells are discarded and 200 uL of 1X TBS containing 1% bovine serum albumin (BSA) is added to each well as a blocking reagent and the plate is kept at room temperature for 30 minutes. The wells are washed three times with 1X TBS containing 0.1% BSA. A serial dilution of test compounds is prepared in a separate V-bottom plate (Corning, catalog number 3897). A 75 uL aliquot of the highest concentration of the compound to be tested is added to the first well in a column of the V-bottom plate then 15 ul is serially transferred into 60 uL 1X TBS through the remaining wells in the column to generate a 1 to 5 serial dilution. A 60 uL aliquot of 2 ug/mL galectin-3 (IBL, catalog number IBATGP0414) is added to each well in the V-bottom plate. A 100 uL aliquot of the galectin-3/test compound mixture is transferred from the V-bottom plate into the assay plate containing the Galβ1-3GlcNAc polymer. Four sets of control wells in the assay plate are prepared in duplicate containing 1) both Galβ1-3GlcNAc polymer and galectin-3, 2) neither the polymer nor galectin-3, 3) galectin-3 only, no polymer, or 4) polymer only, no galectin-3. The plate is gently rocked for 1.5 hours at room temperature. The wells are washed four times with TBS/0.1%BSA. A 100 uL aliquot of anti-galectin-3 antibody conjugated to horse radish peroxidase (R&D Systems, from DGAL30 kit) is added to each well and the plate is kept at room temperature for 1 hour. The wells are washed four times with TBS/0.1%BSA. A 100 uL aliquot of TMB substrate solution is added to each well. The TMB substrate solution is prepared by making a 1:1 mixture of TMB Peroxidase Substrate (KPL, catalog number 5120-0048) and Peroxidase Substrate Solution B (KPL, catalog number 5120-0037). The plate is kept at room temperature for 10 to 20 minutes. The color development is stopped by adding 100 uL 10% phosphoric acid (RICCA Chemical Co., catalog number 5850-16). The absorbance at 450 nm (A₄₅₀) is measured using a FlexStation 3 plate reader (Molecular Devices). Plots of A₄₅₀ versus test compound concentration and IC₅₀ determinations are made using GraphPad Prism 6.

### EXAMPLE 252

### CXCR4 ASSAY - INHIBITION OF CYCLIC AMP

The CXCR4-cAMP assay measures the ability of a glycomimetic CXCR4 antagonist to inhibit the binding of CXCL12 (SDF-1α) to CHO cells that have been genetically engineered to express CXCR4 on the cell surface. Assay kits may be purchased from DiscoveRx (95-0081E2CP2M; cAMP Hunter eXpress CXCR4 CHO-K1). The Gᵢ-coupled receptor antagonist response protocol described in the kit instruction manual can be followed. GPCRs, such as CXCR4, are typically coupled to one of the 3 CT-proteins: Gs, Gi, or Gq. In the CHO cells supplied with the kit, CXCR4 is coupled to Gi. After activation of CXCR4 by ligand binding (CXCL12), Gi dissociates from the CXCR4 complex, becomes activated, and binds to adenylyl cyclase, thus inactivating it, resulting in decreased levels of intracellular cAMP. Intracellular cAMP is usually low, so the decrease of the low level of cAMP by a Gi-coupled receptor will be difficult to detect. Forskolin is added to the CHO cells to directly activate adenylyl cyclase (bypassing all GPCRs), thus raising the level of cAMP in the cell, so that a Gi response can be more easily observed. CXCL12 interaction with CXCR4 decreases the intracellular level of cAMP and inhibition of CXCL12 interaction with CXCR4 by a CXCR4 antagonist increases the intracellular cAMP level, which is measured by luminescence.

### EXAMPLE 253 (relevant for understanding the invention)

Compound A, a specific antagonist of E-selectin, enhanced HSC quiescence by preventing differentiation. Studies further showed that therapeutic blockade of E-selectin *in vivo* with Compound A specifically augmented the mobilization of HSC with highest self-renewal potential following G-CSF administration, and markedly improved subsequent engraftment and reconstitution in mice. Noteworthy is the fact that the studies focused on the role of E-selectin and the use of Compound A during HSC mobilization in current harvesting procedures of donors to accelerate recovery in transplant recipients (not part of the claimed invention).

Antagonism of E-selectin in the recipient could lead to a beneficial effect on survival of HSC-reconstituted recipients. For example, hepatic veno-occlusive disease (VOD), also known as sinusoidal obstruction syndrome (SOS), is a major complication of HSC transplantation and it carries a high mortality. In a murine model of VOD, hepatic inflammation was characteristic of SOS, and mice deficient in P- and E-selectins on the surface of vascular endothelial cells showed markedly reduced SOS, demonstrating a major role for leukocytes recruited from blood. Inhibition of SOS with an E-selectin antagonist such as Compound A could have a positive impact on survival.

### EXAMPLE 254 (according to the invention)

### Enhancing the Survival of Reconstituted, Bone Marrow Depleted Hosts

The survival outcome of lethally-irradiated, bone marrow depleted mice when reconstituted with HSC in combination with Compound A was investigated. C57BL/6 mice with bone marrow depleted by lethal-irradiation were reconstituted with bone-marrow derived from the congenic strain, B6.SJL-PtprcaPepcb/BoyJ (B6.SJL) mice. The use of a congenic strain in these studies allowed for the enumeration and differentiation between the donor strain (CD45.1+) and the recipient strain (CD45.2+).

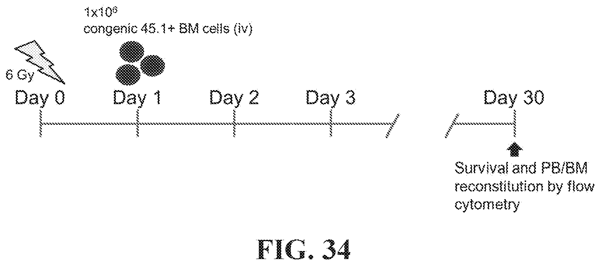

Twenty-four hours post irradiation (6Gy x2), cohorts of C57BL/6 mice (n=10/group) were injected i.v. with 1x10⁶ cells (study day 0) from B6.SJL donor mice with three IP dosing regimens with 40 mg/kg Compound A. These regimens were: (a) q12h on study days 0 and 1; (b) q12h on study days 1 and 2; and (c) q12h on study day 1 only. Control groups in this study included irradiated mice alone (expected survival = 0%), non-irradiated mice alone (expected survival = 100%), and irradiated, reconstituted mice (no Compound A). The survival of mice was determined over the course of the study (study days 0 to 30) (see Figure 34). Table 1, below, shows the study protocol to assess the effects of compound A on hematopoietic reconstitution of lethally irradiated C57BL/6 mice.

**Table 1**

| Group | N | Radiation | Transplant | Compound A (40 mg/kg/IP injection) | Parameters Assessed |
|---|---|---|---|---|---|
| 1 | 10 | + | - | - | Survival weight, and reconstitution of PB and BM |
| 2 | 10 | + | + | - | |
| 3 | 10 | + | + | Day 0 and 1; q12h | |
| 4 | 10 | + | + | Day 1 and 2; q12h | |
| 5 | 10 | + | + | Day 1; q12h | |
| 6 | 3 | - | - | - | |

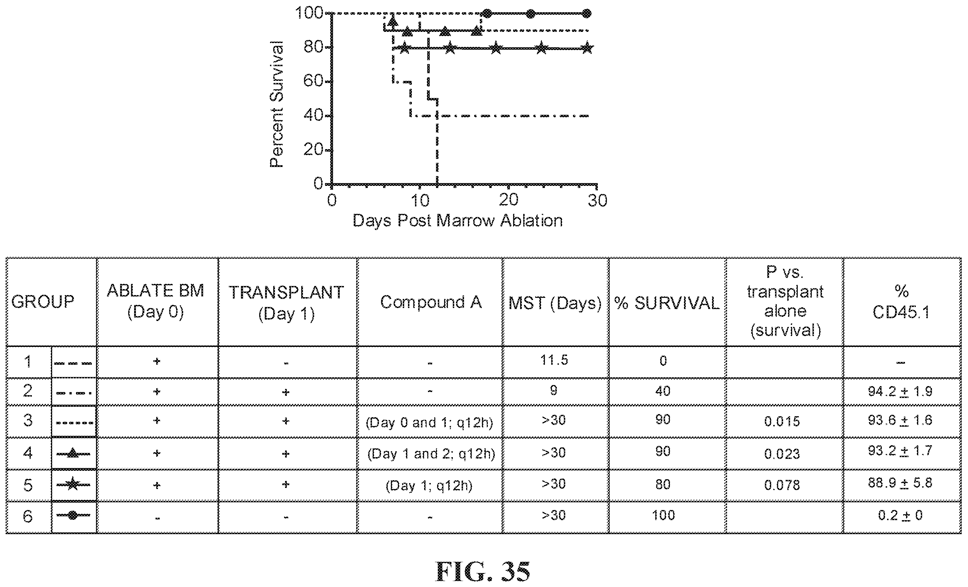

Treatment with Compound A as part of the transplant regimen significantly increased the median survival time (MST) of mice compared with the control group - the MST of mice treated with Compound A and HSC was >30 days with 80-90% of mice alive at study completion. In contrast, the MST of irradiated mice (no transplant) was 11.5 days with no survivors at study completion. The MST of mice irradiated and transplanted with congenic HSC was 9 days with 40% survival at study completion. The impact of Compound A on survival represented a >233.3% increase in life span (*See* Figure 35).

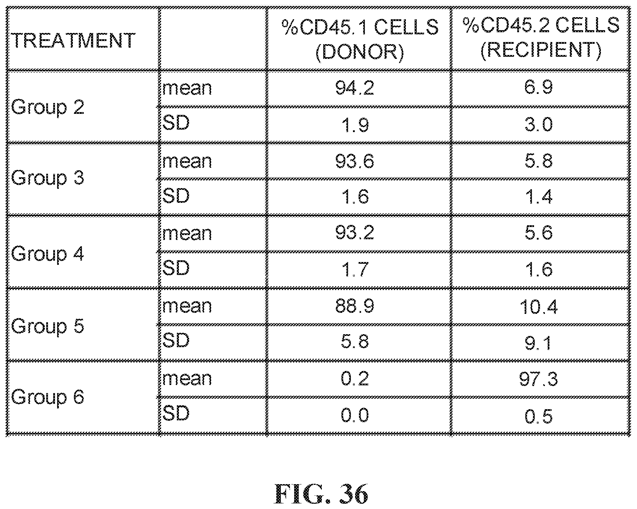

Flow cytometric analysis in all surviving mice on day 30 using PE-CD45.1 and APC-CD45.2 markers showed that the mean percentage of CD45.1+ cells from donor congenic mice was approximately 90% (blood and bone marrow), indicating that all surviving mice were successfully reconstituted (*See* Figure 36). These data suggest that administration of Compound A did not inhibit engraftment or expansion of donor HSCs in the recipient mice. Although not specifically evaluated, incorporation of Compound A into the reconstitution regimen may be speculated to have attenuated a sinusoidal obstructive syndrome such as hepatic veno-occlusive disease known to be E-selectin dependent and a major complication of HSC transplantation.

Accordingly, this novel therapeutic use of inhibitors of E-selectin, such as Compound A, results in the increased survival of mice when combined with HSC transplantation for reconstitution of depleted and compromised bone marrow. The impact on increased host survival could extend to the use of peripheral blood and stem cell transplantations as a therapeutic option in various malignancies where curative intent is intended.

### References

I.G. Winkler, V. Barbier, B. Nowlan, R.N. Jacobsen, C. E. Forristal, J. T. Patton, J. L. Magnani, J. Lévesque. Vascular Niche E-selectin Regulates Hematopoietic Stem Cell Dormancy, Self-Renewal and Chemoresistance. Nature Medicine 18: 1651, 2012.
I.G. Winkler, V. Barbier, A. C. Perkins, J. L. Magnani, J. Levesque. Mobilisation of Reconstituting HSC Is Boosted by Synergy Between G-CSF and E-Selectin Antagonist GMI 1271. Blood 124: 317, 2014.
P.S. Frenette, S. Subbarao, I.B. Mazo, U.H. von Andrian, D.D. Wagner. Endothelial selectins and vascular cell adhesion molecule-1 promote hematopoietic progenitor homing to bone marrow. Proc Natl Acad Sci USA 95: 14423, 1998.
I. Oancea, C.W. Png, I. Das, R Lourie, I.G. Winkler, R Eri, N Subramaniam, H A. Jinnah, B.C. McWhinney, J.-P. Levesque, M.A. McGuckin, J.A. Duley, T.H.J. Florin. A novel mouse model of veno-occlusive disease provides strategies to prevent thioguanine-induced hepatic toxicity. Gut 62: 594, 2013.
I.G. Winkler et al., Vascular E-Selectin Acts As a Gatekeeper Inducing Commitment and Loss of Self-Renewal in HSC Transmigrating through the Marrow Vasculature. Blood 132 (Supplement 1): 4552, 2018.

## Claims

1. At least one E-selectin inhibitor for use in a method of increasing survival of a subject that receives a hematopoietic stem cell (HSC) transplantation, wherein the subject has a hematological disease chosen from malignant and non-malignant diseases, the method comprising administering to the subject receiving HSC transplantation an effective amount of the at least one E-selectin inhibitor in combination with HSC transplantation, wherein the at least one E-selectin inhibitor is chosen from: 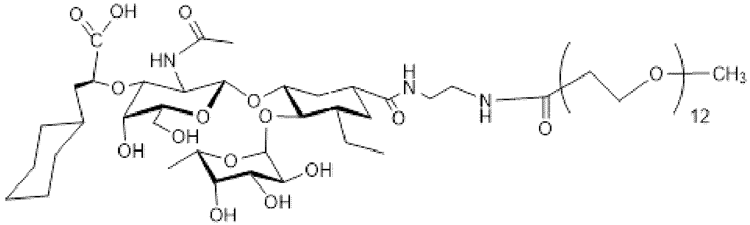 and pharmaceutically acceptable salts thereof.

2. At least one E-selectin inhibitor for use in a method of increasing engraftment and reconstitution of a hematopoietic stem cell (HSC) transplant in a subject receiving HSC transplantation, wherein the subject has a hematological disease chosen from malignant and non-malignant diseases, the method comprising administering to the subject receiving HSC transplantation an effective amount of the at least one E-selectin inhibitor in combination with HSC transplantation, wherein the at least one E-selectin inhibitor is chosen from: 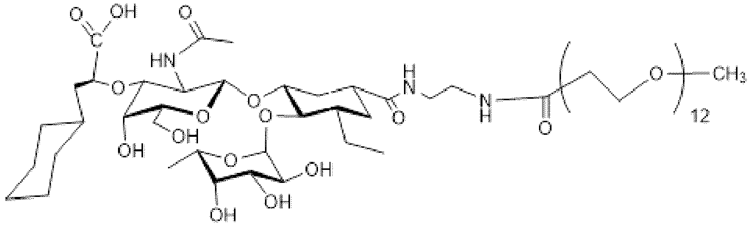 and pharmaceutically acceptable salts thereof.

3. The at least one E-selectin inhibitor for use according to claim 1 or 2, wherein the method further comprises inhibiting sinusoidal obstruction syndrome (SOS) in the subject.

4. The at least one E-selectin inhibitor for use according to claim 3, wherein the SOS is a hepatic veno-occlusive disease.

5. The at least one E-selectin inhibitor for use according to any one of claims 1-4, wherein the subject has depleted and/or compromised bone marrow.

6. The at least one E-selectin inhibitor for use according to any one of claims 1-5, wherein the HSC transplantation is from peripheral blood.

7. The at least one E-selectin inhibitor for use according to any one of claims 1-6, wherein the HSC transplantation is from bone marrow.

8. The at least one E-selectin inhibitor for use according to any one of claims 1-7, wherein the malignant diseases are chosen from multiple myeloma, Hodgkin and non-Hodgkin lymphoma, acute myeloid leukemia (AML), acute lymphoblastic leukemia (ALL), myelodysplastic syndrome, chronic myeloid leukemia (CML), chronic lymphocytic leukemia, myelofibrosis, essential thrombocytosis, and polycythemia vera.

9. The at least one E-selectin inhibitor for use according to any one of claims 1-7, wherein the non-malignant diseases are chosen from immunodeficiency, autoimmune disorders, and genetic disorders.

10. The at least one E-selectin inhibitor for use according to any one of claims 1-7 or 9, wherein the non-malignant diseases are chosen from aplastic amemia, severe combined immune deficiency syndrome (SCID), thalassemia, sickle cell anemia, chronic granulomatous disease, leukocyte adhesion deficiency, Chediak-Higashi syndrome, Kostman syndrome, Fanconi anemia, Blackfan-Diamond anemia, systemic sclerosis, systemic lupus erythematosus, mucopolysaccharidosis, pyruvate kinase deficiency, and multiple sclerosis.

## Patentansprüche

1. Mindestens ein E-Selektin-Inhibitor zur Verwendung in einem Verfahren zur Erhöhung des Überlebens eines Individuums, das eine Transplantation hämatopoetischer Stammzellen (HSC) empfängt, wobei das Individuum eine hämatologische Krankheit, ausgewählt aus malignen und nicht-malignen Krankheiten, aufweist, wobei das Verfahren die Verabreichung einer wirksamen Menge des mindestens einen E-Selektin-Inhibitors in Kombination mit HSC-Transplantation an das Individuum, das die HSC-Transplantation empfängt, umfasst, wobei der mindestens eine E-Selektin-Inhibitor ausgewählt wird aus: 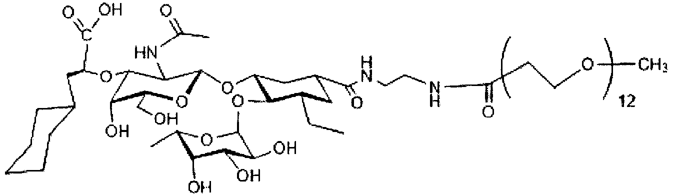 und pharmazeutisch akzeptablen Salzen davon.

2. Mindestens ein E-Selektin-Inhibitor zur Verwendung in einem Verfahren zur Erhöhung der Einnistung und Rekonstitution eines hämatopoetischen Stammzell (HSC)-Transplants in einem Individuum, das eine HSC-Transplantation empfängt, wobei das Individuum eine hämatologische Krankheit, ausgewählt aus malignen und nicht-malignen Krankheiten, aufweist, wobei das Verfahren die Verabreichung einer wirksamen Menge des mindestens einen E-Selektin-Inhibitors in Kombination mit HSC-Transplantation an das Individuum, das die HSC-Transplantation empfängt, umfasst, wobei der mindestens eine E-Selektin-Inhibitor ausgewählt wird aus: 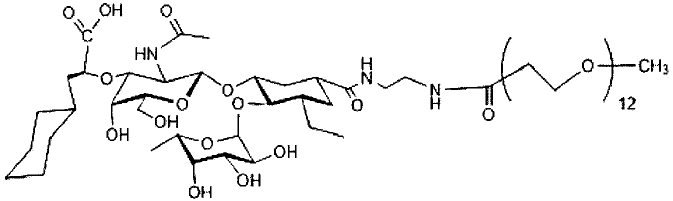 und pharmazeutisch akzeptablen Salzen davon.

3. Der mindestens eine E-Selektin-Inhibitor zur Verwendung nach Anspruch 1 oder 2, wobei das Verfahren ferner das Hemmen von sinusoidalem Obstruktionssyndrom (SOS) in dem Individuum umfasst.

4. Der mindestens eine E-Selektin-Inhibitor zur Verwendung nach Anspruch 3, wobei es sich bei dem SOS um eine venöse okklusive Leberkrankheit handelt.

5. Der mindestens eine E-Selektin-Inhibitor zur Verwendung nach einem der Ansprüche 1-4, wobei das Individuum abgereichertes und/oder beeinträchtigtes Knochenmark aufweist.

6. Der mindestens eine E-Selektin-Inhibitor zur Verwendung nach einem der Ansprüche 1-5, wobei die HSC-Transplantation von peripherem Blut ausgeht.

7. Der mindestens eine E-Selektin-Inhibitor zur Verwendung nach einem der Ansprüche 1-6, wobei die HSC-Transplantation von Knochenmark ausgeht.

8. Der mindestens eine E-Selektin-Inhibitor zur Verwendung nach einem der Ansprüche 1-7, wobei die malignen Krankheiten aus multiplem Myelom, Hodgkin- und Non-Hodgkin-Lymphom, akuter myeloischer Leukämie (AML), akuter lymphoblastischer Leukämie (ALL), myelodysplastischem Syndrom, chronischer myeloischer Leukämie (CML), chronischer lymphozytärer Leukämie, Myelofibrose, essentieller Thrombozytose und Polycythemia vera ausgewählt werden.

9. Der mindestens eine E-Selektin-Inhibitor zur Verwendung nach einem der Ansprüche 1-7, wobei die nicht-malignen Krankheiten aus Immunschwäche, Autoimmunstörungen und genetischen Störungen ausgewählt werden.

10. Der mindestens eine E-Selektin-Inhibitor zur Verwendung nach einem der Ansprüche 1-7 oder 9, wobei die nicht-malignen Krankheiten aus aplastischer Anämie, schwerem kombinierten Immundefizienz-Syndrom (SCID), Thalassämie, Sichelzellanämie, chronischer granulomatöser Erkrankung, Leukozytenadhäsionsdefizienz, Chediak-Higashi-Syndrom, Kostman-Syndrom, Fanconi-Anämie, Blackfan-Diamond-Anämie, systemischer Sklerose, systemischem Lupus erythematodes, Mucopolysaccharidose, Pyruvatkinase-Defizienz und multipler Sklerose ausgewählt werden.

## Revendications

1. Au moins un inhibiteur de la E-sélectine pour une utilisation dans une méthode d'augmentation de la survie d'un sujet recevant une greffe de cellules souches hématopoïétiques (CSH), le sujet étant atteint d'une maladie hématologique choisie parmi des maladies malignes ou non malignes, la méthode comprenant l'administration au sujet recevant une greffe de CSH d'une quantité efficace du ou des inhibiteurs de la E-sélectine en association avec la greffe de CSH, le ou les inhibiteurs de la E-sélectine étant choisis parmi : 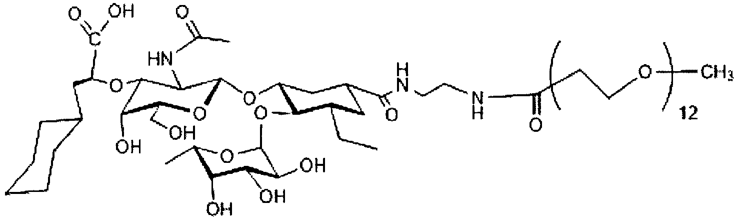 et des sels pharmaceutiquement acceptables de celui-ci.

2. Au moins un inhibiteur de la E-sélectine pour une utilisation dans une méthode d'augmentation de la prise de greffe et de la reconstitution d'une greffe de cellules souches hématopoïétiques (CSH) chez un sujet recevant une greffe de CSH, le sujet étant atteint d'une maladie hématologique choisie parmi des maladies malignes ou non malignes, la méthode comprenant l'administration au sujet recevant une greffe de CSH d'une quantité efficace du ou des inhibiteurs de la E-sélectine en association avec la greffe de CSH, le ou les inhibiteurs de la E-sélectine étant choisis parmi : 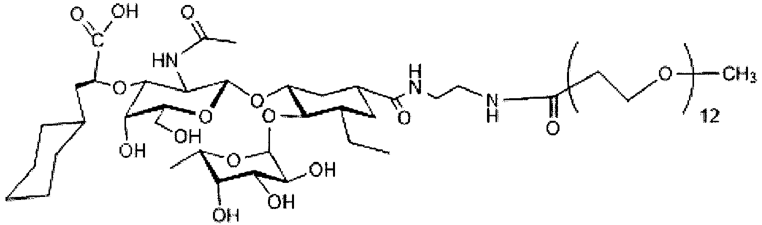 et des sels pharmaceutiquement acceptables de celui-ci.

3. Au moins un inhibiteur de la E-sélectine pour une utilisation selon la revendication 1 ou 2, le procédé comprenant en outre l'inhibition du syndrome d'obstruction sinusoïdale (SOS) chez le sujet.

4. Au moins un inhibiteur de la E-sélectine selon la revendication 3, le SOS étant une maladie veino-occlusive hépatique.

5. Au moins un inhibiteur de la E-sélectine selon l'une quelconque des revendications 1 à 4, le sujet ayant une moelle osseuse appauvrie et/ou altérée.

6. Au moins un inhibiteur de la E-sélectine selon l'une quelconque des revendications 1 à 5, la greffe de CSH provenant de sang périphérique.

7. Au moins un inhibiteur de la E-sélectine selon l'une quelconque des revendications 1 à 6, la greffe de CSH provenant de moelle osseuse.

8. Au moins un inhibiteur de la E-sélectine selon l'une quelconque des revendications 1 à 7, les maladies malignes étant choisies parmi le myélome multiple, le lymphome de Hodgkin et non hodgkinien, la leucémie myéloïde aiguë (LMA), la leucémie lymphoblastique aiguë (LLA), le syndrome myélodysplasique, la leucémie myéloïde chronique (LMC), la leucémie lymphoïde chronique, la myélofibrose, la thrombocytose essentielle, et la polyglobulie de Vaquez.

9. Au moins un inhibiteur de la E-sélectine selon l'une quelconque des revendications 1 à 7, les maladies non malignes étant choisies parmi l'immunodéficience, les troubles auto-immuns et les troubles génétiques.

10. Au moins un inhibiteur de la E-sélectine selon l'une quelconque des revendications 1 à 7 ou 9, les maladies non malignes étant choisies parmi l'anémie aplasique, le déficit immunitaire combiné sévère (SCID), la thalassémie, l'anémie falciforme, la granulomatose chronique, le déficit d'adhésion leucocytaire, le syndrome de Chediak-Higashi, le syndrome de Kostman, l'anémie de Fanconi, l'anémie de Blackfan-Diamond, la sclérose systémique, le lupus érythémateux disséminé, la mucopolysaccharidose, le déficit en pyruvate kinase et la sclérose en plaques.
